# EUROPEAN PATENT APPLICATION

(11) **EP 4 803 513 A1**
(43) Date of publication of application: **09.09.2026**
(21) Application number: 24884986.1
(22) Date of filing: 01.11.2024
(51) Int. Cl.: C07D 401/14, C07D 401/12, A61K 31/517, A61P 35/00

(54) **SUBSTITUTED QUINAZOLINE COMPOUND, PREPARATION METHOD THEREFOR, PHARMACEUTICAL COMBINATION THEREOF, AND USE THEREOF**

(30) Priority: 03.11.2023 CN 202311459950; 22.03.2024 CN 202410343136
(71) Applicant: Hongyun Biotech Co., Ltd., Taizhou, Jiangsu 225316 (CN)
(72) Inventor: CHEN, Yantao, Taizhou, Jiangsu 225316 (CN); HUANG, Wei, Taizhou, Jiangsu 225316 (CN); LV, Kun, Taizhou, Jiangsu 225316 (CN); YAN, Xiaoe, Taizhou, Jiangsu 225316 (CN); ZHANG, Baoding, Taizhou, Jiangsu 225316 (CN); CHEN, Jiejie, Taizhou, Jiangsu 225316 (CN)
(74) Representative: Zanoli, Enrico
(86) International application number: PCT/CN2024/129421
(87) International publication number: WO 2025/092989

(57) **Abstract**

The present invention relates to a substituted quinazoline compound, a preparation method therefor, a pharmaceutical composition thereof, and a use thereof. Specifically, provided are a compound represented by formula O or a stereoisomer of the compound, a prodrug thereof, a pharmaceutically acceptable salt thereof, or a pharmaceutically acceptable solvate thereof, a preparation method therefor, a pharmaceutical composition thereof, and a use thereof.

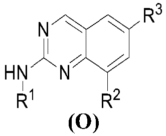

## Description

### TECHNICAL FIELD

The present invention relates to the field of biomedicine, and specifically to substituted quinazoline compounds, preparation methods therefor, pharmaceutical compositions and uses thereof. More specifically, the present invention provides a class of compounds having BRK kinase inhibitory activity, or stereoisomers thereof, prodrugs thereof, pharmaceutically acceptable salts thereof, or pharmaceutically acceptable hydrates or solvates thereof, pharmaceutical compositions comprising such compounds, and uses of these compounds in the preparation of medicaments for the prevention or treatment of diseases mediated by BRK kinase in organisms, particularly in the preparation of medicaments for the prevention or treatment of tumor growth and metastasis.

### BACKGROUND

BRK kinase (Breast tumor kinase) is a non-receptor tyrosine kinase associated with various tumors and plays an important role in tumorigenesis (Mitchell et al, Oncogene 1994, 9, 2383-2390). Studies have shown that BRK is highly expressed in breast cancer and many other types of tumors, including skin cancer, head and neck cancer, thyroid cancer, pancreatic cancer, cervical cancer, bladder cancer, etc., and can induce tumor cell proliferation, invasion, and metastasis (Barker et al, Oncogene, 1997, 15, 799-805; Gilic et al, Biochim Biophys Acta Rev Cancer 2020, 1874, 188432; Zhao et al, Onco Targets Ther 2013, 6, 183-188). BRK has been shown to suppress tumorigenesis by antagonizing EMT in colorectal cancer cell line models (Mathur et al, Mol. Cancer Res. 2016, 14, 563-573). BRK has also been demonstrated to have tumor-suppressive functions in esophageal cancer (Liu et al, J Transl Med 2013, 11, 59; Wu et al, Biochem Biophys Res Commun 2018, 503, 1378-1384). In HB4a, T47D, and Hela cells, overexpression of BRK promotes EGF- or serum-induced cell proliferation (Kamalati et al, J Biol Chem 1996, 271, 30956-30963); knockdown or downregulation of BRK expression can promote apoptosis, suppress tumor growth, and reduce metastasis (Park et al, Breast Cancer Res 2015, 17, 86; Ito et al, Cancer Res 2016, 76, 4406-4417). Moreover, small-molecule inhibitors targeting BRK can effectively inhibit the growth of BRK-overexpressing tumor cells both in vitro and in vivo (Jiang et al, Cancer Res 2017, 77, 175-186). Currently, no BRK inhibitor has been approved for clinical use or is undergoing clinical investigation. Therefore, there is an urgent need in the field to develop highly potent and selective BRK inhibitors to provide new treatment options for various tumors including breast cancer.

### SUMMARY OF THE INVENTION

Through extensive and in-depth research, the inventors of the present invention have designed and synthesized a series of substituted quinazoline compounds that are novel in structure, highly safe, and possess high inhibitory activity against BRK kinase.

The present invention provides compounds of the following general formula: 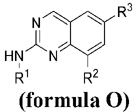 or a stereoisomer, a prodrug, a pharmaceutically acceptable salt, or a pharmaceutically acceptable solvate of the above compound.

More specifically, the present invention provides compounds of the following general formulae (I, II, III): 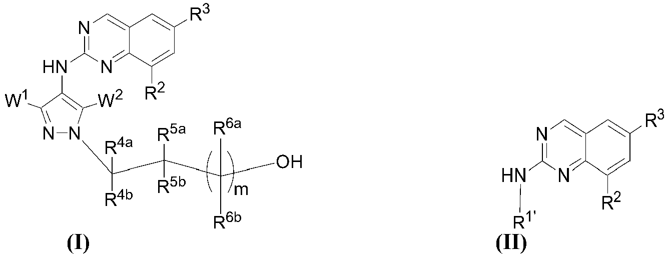 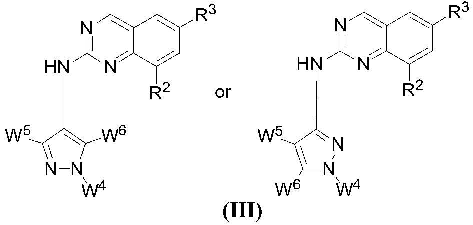 wherein the definitions of substituents and symbols are described in detail below.

One object of the present invention is to provide a series of compounds having BRK kinase inhibitory activity, and stereoisomers thereof, prodrugs thereof, pharmaceutically acceptable salts thereof, or pharmaceutically acceptable solvates thereof.

Another object of the present invention is to provide a method for preparing the above compounds.

Another object of the present invention is to provide a pharmaceutical composition comprising the above compounds.

Another object of the present invention is to provide use of the above compounds and pharmaceutical compositions comprising the above compounds in the preparation of medicaments for preventing and/or treating cancers or other diseases mediated by BRK kinase.

Another object of the present invention is to provide a method for treating cancer, comprising administering to a subject an effective amount of a compound or composition of the present invention.

### DETAILED DESCRIPTION OF THE INVENTION

Various specific embodiments, modes, and examples are described herein, including exemplary embodiments and definitions employed to understand the claimed invention. Although the following detailed description provides specific preferred embodiments, those skilled in the art will understand that these embodiments are exemplary only and that the invention may be practiced in other ways. For purposes of determining infringement, the scope of the invention will encompass any one or more of the appended claims, including equivalents thereof and elements or limitations equivalent to those described.

The present invention is implemented through the following technical solutions.

In the first aspect of the present invention, the present invention provides a compound of formula O, or a stereoisomer thereof, a prodrug thereof, a pharmaceutically acceptable salt thereof, or a pharmaceutically acceptable solvate thereof, 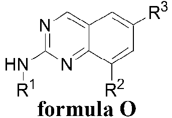 wherein:
R¹ is selected from the group consisting of 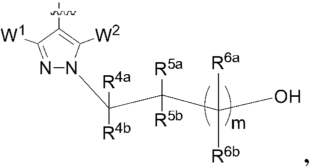 R^{1'}, 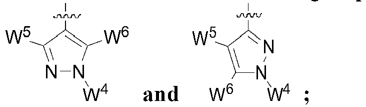
preferably, R¹ is selected from the group consisting of 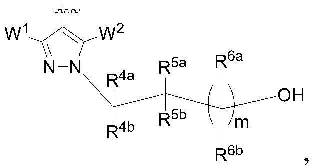 R^{1'} and 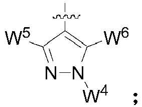
R^{1'} is selected from the group consisting of 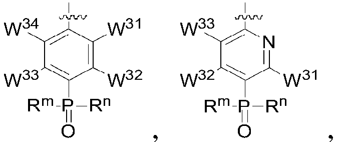 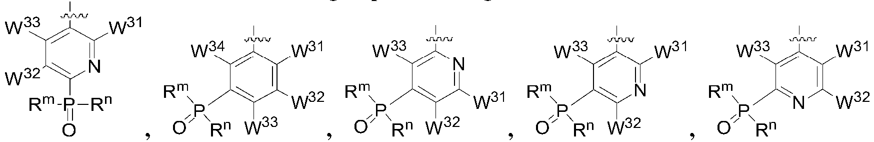 and 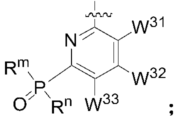
preferably, R^{1'} is selected from the group consisting of 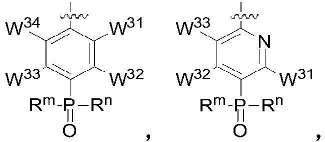 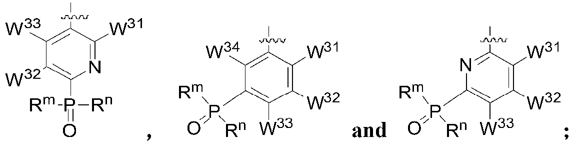
R^{m} and Rⁿ are each independently selected from the group consisting of C1-C6 alkyl;
preferably, R^{m} and Rⁿ are each independently selected from the group consisting of methyl and ethyl;
preferably, R^{1'} is selected from the group consisting of 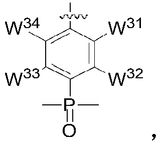 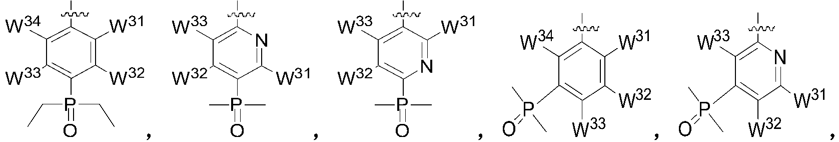 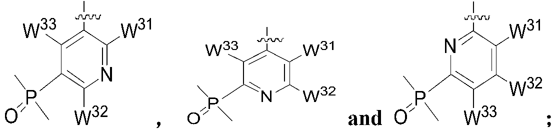
preferably, R^{1'} is selected from the group consisting of 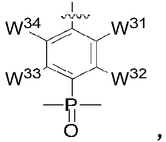 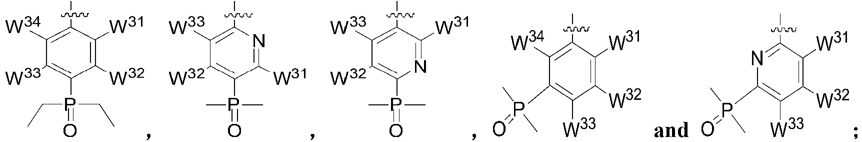
more preferably, R^{1'} is selected from the group consisting of 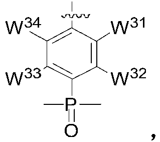 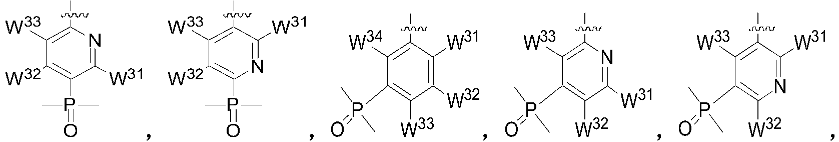 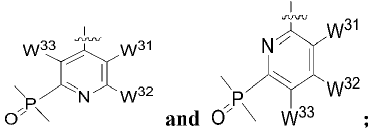
most preferably, R^{1'} is selected from the group consisting of 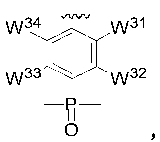 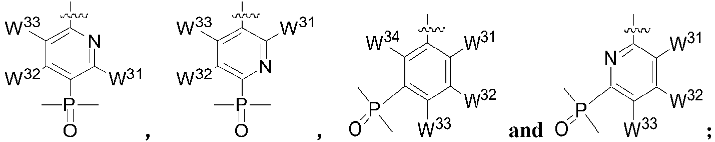
R^{4a} and R^{4b} are each independently selected from the group consisting of hydrogen and C1-C6 alkyl;
preferably, R^{4a} and R^{4b} are each independently selected from the group consisting of hydrogen and methyl;
R^{5a} and R^{5b} are each independently selected from the group consisting of hydrogen and C1-C6 alkyl;
preferably, R^{5a} and R^{5b} are each independently selected from the group consisting of hydrogen and methyl;
R^{6a} and R^{6b} are each independently selected from the group consisting of hydrogen and C1-C6 alkyl;
preferably, R^{6a} and R^{6b} are each independently selected from the group consisting of hydrogen and methyl;
m is selected from the group consisting of 0 and 1;
W¹ and W² are each independently selected from the group consisting of hydrogen, halogen, C1-C6 alkyl, and C1-C6 haloalkyl;
preferably, any one of W¹ and W² is selected from the group consisting of hydrogen, halogen, C1-C6 alkyl, and C1-C6 haloalkyl, and the other is hydrogen;
more preferably, any one of W¹ and W² is selected from the group consisting of hydrogen, chloro, methyl and difluoromethyl, and the other is hydrogen;
most preferably, 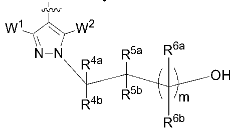 as a whole, is selected from the group consisting of: 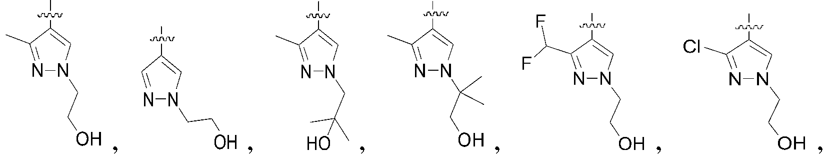 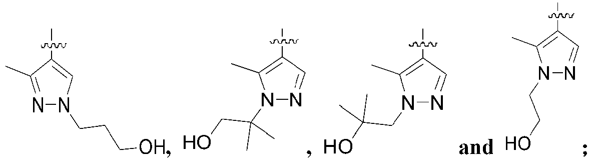
when W³¹, W³², W³³, and W³⁴ are simultaneously present, W³¹, W³², W³³, and W³⁴ are each independently selected from the group consisting of hydrogen, halogen, hydroxy, C1-C6 alkyl, hydroxy-substituted C1-C6 alkyl, C1-C6 alkoxy, and -NR^{x}R^{y};
preferably, when W³¹, W³², W³³, and W³⁴ are simultaneously present, W³¹, W³², W³³, and W³⁴ are each independently selected from the group consisting of hydrogen, halogen, C1-C6 alkyl, hydroxy-substituted C1-C6 alkyl, and C1-C6 alkoxy;
preferably, when W³¹, W³², W³³, and W³⁴ are simultaneously present, any one of W³¹, W³², W³³, and W³⁴ is selected from the group consisting of hydrogen, halogen, hydroxy, C1-C6 alkyl, C1-C6 alkoxy, hydroxy-substituted C1-C6 alkyl, and -NR^{x}R^{y}, and the remainder are hydrogen; or, any two of W³¹, W³², W³³, and W³⁴ are independently selected from the group consisting of hydrogen, halogen, hydroxy, C1-C6 alkyl, and C1-C6 alkoxy, and the remainder are hydrogen;
R^{x} and R^{y} are each independently selected from the group consisting of hydrogen and C1-C6 alkyl; preferably, any one of R^{x} and Ry is selected from the group consisting of C1-C6 alkyl, and the other is hydrogen; more preferably, any one of R^{x} and R^{y} is methyl, and the other is hydrogen;
preferably, wheₙ W³¹, W³², W³³, and W³⁴ are simultaneously present, any one of W³¹, W³², W³³, and W³⁴ is selected from the group consisting of hydrogen, halogen, C1-C6 alkyl, C1-C6 alkoxy, and hydroxy-substituted C1-C6 alkyl, and the remainder are hydrogen; or, any two of W³¹, W³², W³³, and W³⁴ are independently selected from the group consisting of hydrogen, halogen, C1-C6 alkyl, and C1-C6 alkoxy, and the remainder are hydrogen;
more preferably, when W³¹, W³², W³³, and W³⁴ are simultaneously present, any one of W³¹, W³², W³³, and W³⁴ is selected from the group consisting of hydrogen, fluoro, hydroxy, methyl, ethyl, methoxy, ethoxy, 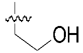 and 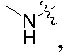 and the remainder are hydrogen; or, any two of W³¹, W³², W³³, and W³⁴ are independently selected from the group consisting of hydrogen, fluoro, chloro, hydroxy, methyl, and methoxy, and the remainder are hydrogen;
more preferably, when W³¹, W³², W³³, and W³⁴ are simultaneously present, any one of W³¹, W³², W³³, and W³⁴ is selected from the group consisting of hydrogen, fluoro, methyl, ethyl, methoxy and 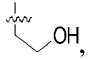 and the remainder are hydrogen; or, any two of W³¹, W³², W³³, and W³⁴ are independently selected from the group consisting of hydrogen, fluoro, chloro, methyl, and methoxy, and the remainder are hydrogen;
when W³¹, W³², and W³³ are simultaneously present but W³⁴ is absent, W³¹, W³², and W³³ are each independently selected from the group consisting of hydrogen, C1-C6 alkyl, and C1-C6 alkoxy;
preferably, when W³¹, W³², and W³³ are simultaneously present but W³⁴ is absent, any one of W³¹, W³², and W³³ is selected from the group consisting of hydrogen, C1-C6 alkyl, and C1-C6 alkoxy, and the remainder are hydrogen;
more preferably, wheₙ W³¹, W³², and W³³ are simultaneously present but W³⁴ is absent, any one of W³¹, W³², and W³³ is selected from the group consisting of hydrogen, methyl, and methoxy, and the remainder are hydrogen;
preferably, R^{1'} is selected from the group consisting of 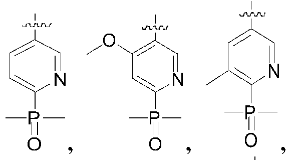 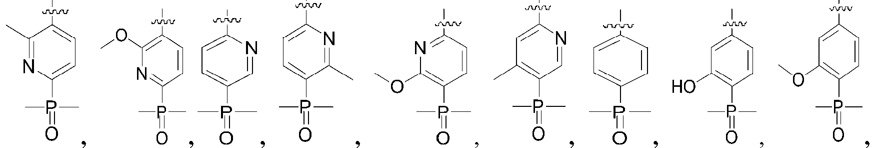 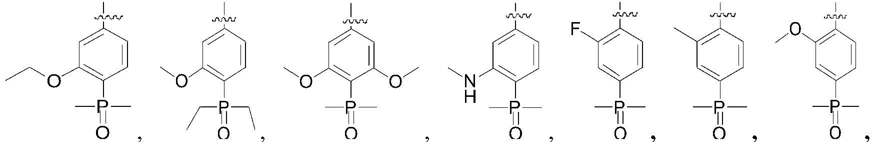 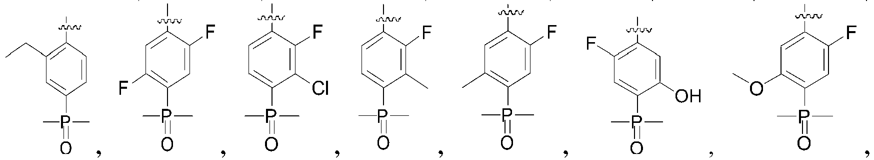 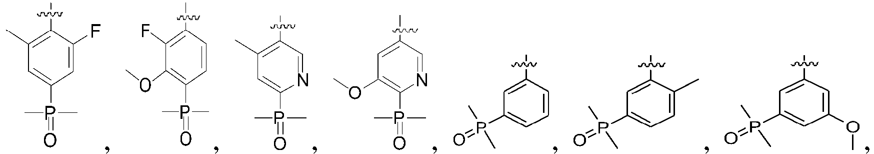 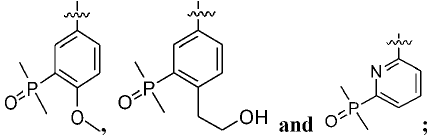
most preferably, R^{1'} is selected from the group consisting of 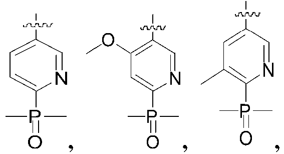 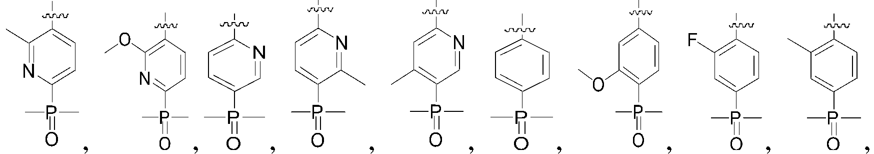 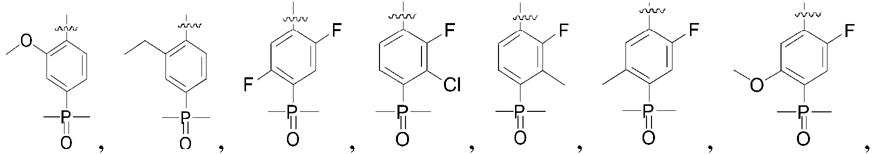 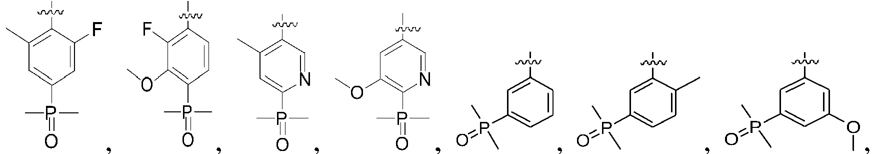 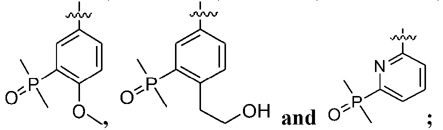
W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, O=S(R^{e}R^{f})=N-C1-C6 alkylene, R^{c}R^{d}N-C(=O)-C1-C6 alkylene, C3-C6 cycloalkyl-SO:-, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene and 8- to 10-membered saturated bridged heterocyclyl, and the 3-to 7-membered saturated heterocyclyl, the 5- to 6-membered heteroaryl and the 8- to 10-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)- and 3-to 6-membered saturated heterocyclyl, and the C1-C6 alkylene is optionally substituted by hydroxy;
preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{c}R^{d}N-C(=O)-C1-C6 alkylene, C3-C6 cycloalkyl-SO;-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, and the 3- to 7-membered saturated heterocyclyl and the 8-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)- and 4-membered saturated heterocyclyl, and the C1-C6 alkylene is optionally substituted by hydroxy;
more preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{c}R^{d}N-C(=O)-C1-C6 alkylene, C3-C6 cycloalkyl-SO;-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, and the 3- to 7-membered saturated heterocyclyl and the 8-membered saturated bridged heterocyclyl are each independently optionally substituted by 1 or 2 (preferably 1) substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl (preferably 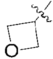 ), and the C1-C6 alkylene is optionally substituted by hydroxy, the 3- to 7-membered saturated heterocyclyl is selected from the group consisting of 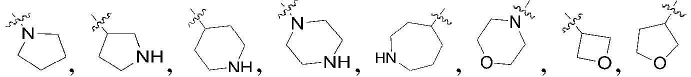 and 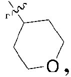 the 5- to 6-membered heteroaryl is selected from the group consisting of 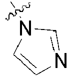 and 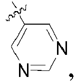 the 8-membered bridged heterocyclyl is 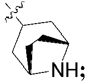
R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 3- to 6-membered saturated heterocyclyl; or, R^{c}, R^{d}, and the N atom to which they are both attached together form a 3- to 6-membered saturated heterocyclic ring, and the 3- to 6-membered saturated heterocyclic ring is optionally substituted by 1 or 2 substituent(s) selected from the group consisting of C1-C6 alkyl and halogen;
preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 5-membered saturated heterocyclyl; or, R^{c}, R^{d}, and the N atom to which they are both attached together form a 4- to 5-membered saturated heterocyclic ring, and the 4- to 5-membered saturated heterocyclic ring is optionally substituted by 1 or 2 substituent(s) selected from the group consisting of C1-C6 alkyl and halogen;
more preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 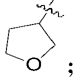 or, R^{c}, R^{d}, and the N atom to which they are both attached together form 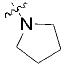 or 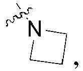 and the 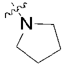 or 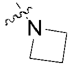 is independently optionally substituted by 1 or 2 substituent(s) selected from the group consisting of C1-C6 alkyl and halogen;
most preferably, R^{c} and R^{d} are each independently selected from the group consisting of hydrogen, methyl, ethyl, isopropyl, 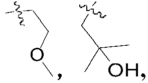 acetyl, 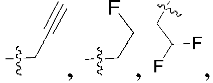 cyclopropyl, cyclobutyl, and 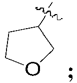 or, R^{c} and R^{d}, and the N atom to which they are both attached together form 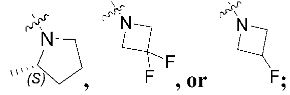
R^{e} and R^{f} are each independently selected from the group consisting of hydrogen and C1-C6 alkyl;
preferably, R^{e} and R^{f} are each independently selected from the group consisting of C1-C6 alkyl;
more preferably, R^{e} and R^{f} are methyl;
further preferably, W⁴ is selected from the group consisting of hydrogen, methyl, ethyl, difluoromethyl, trideuteromethyl, 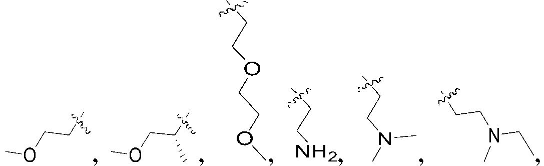 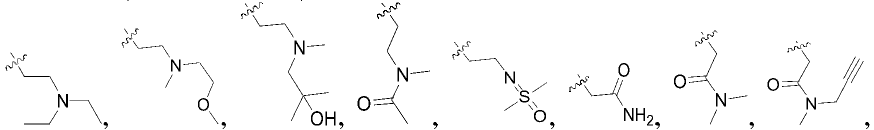 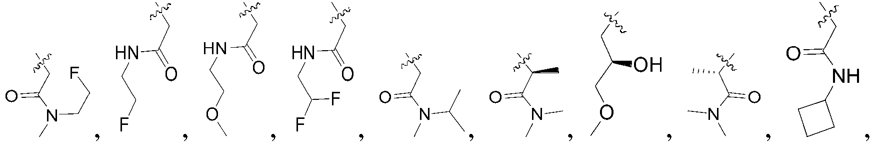 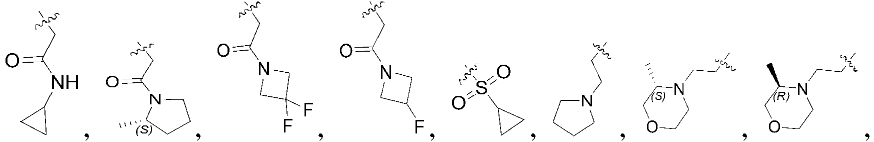 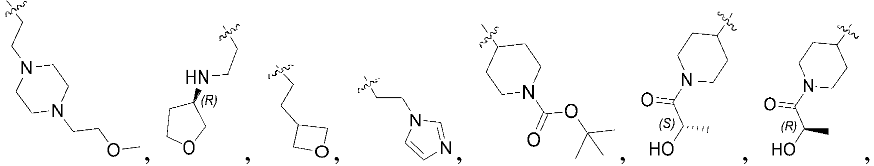 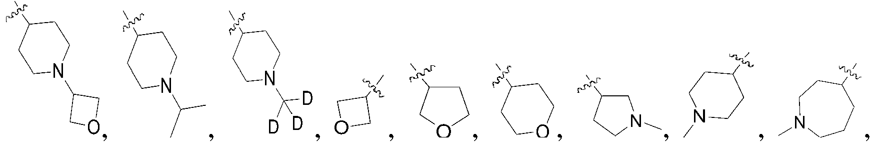 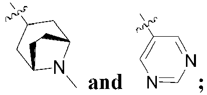
most preferably, W⁴ is selected from the group consisting of hydrogen, methyl, ethyl, difluoromethyl, trideuteromethyl, 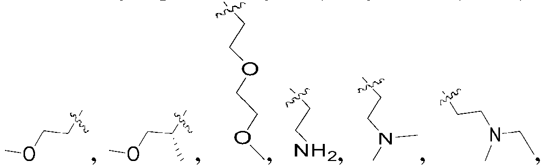 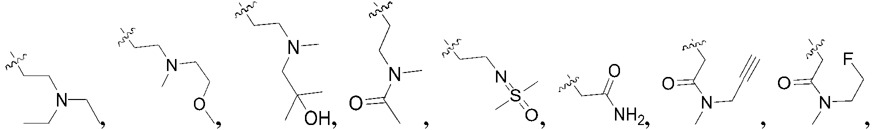 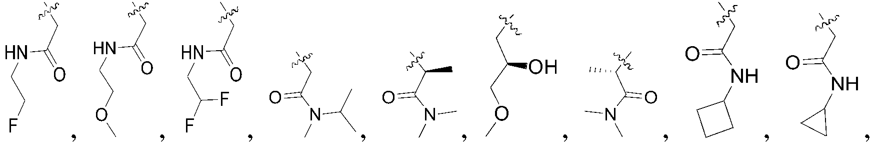 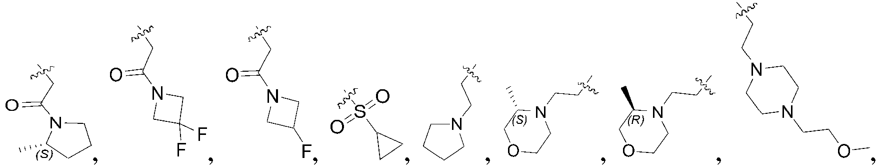 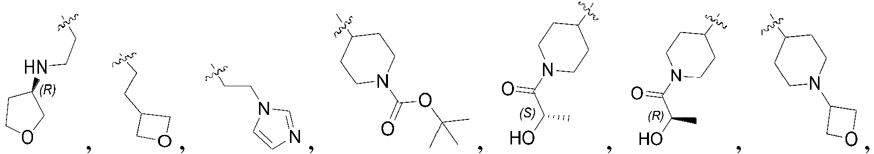 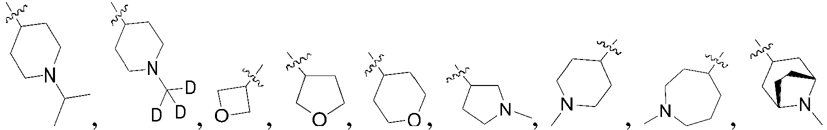 and 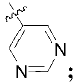
W⁵ and W⁶ are each independently selected from the group consisting of hydrogen, halogen, C1-C6 alkyl, C1-C6 alkoxy, C1-C6 haloalkyl and 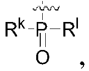 wherein W⁵ and W⁶ are not hydrogen at the same time;
preferably, W⁵ and W⁶ are each independently selected from the group consisting of hydrogen, halogen, C1-C6 alkyl, C1-C6 alkoxy and C1-C6 haloalkyl, wherein W⁵ and W⁶ are not hydrogen at the same time;
preferably, any one of W⁵ and W⁶ is selected from the group consisting of hydrogen, halogen, C1-C6 alkyl, C1-C6 alkoxy, C1-C6 haloalkyl and 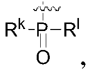 and the other is hydrogen;
R^{k} and R^{l} are each independently selected from the group consisting of C1-C6 alkyl;
preferably, R^{k} and R^{l} are methyl;
preferably, any one of W⁵ and W⁶ is selected from the group consisting of halogen, C1-C6 alkyl, C1-C6 alkoxy, and C1-C6 haloalkyl, and the other is hydrogen;
more preferably, any one of W⁵ and W⁶ is selected from the group consisting of hydrogen, chloro, methyl, ethyl, methoxy, difluoromethyl, trifluoromethyl and 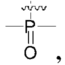 and the other is hydrogen;
more preferably, any one of W⁵ and W⁶ is selected from the group consisting of chloro, methyl, ethyl, methoxy, difluoromethyl and trifluoromethyl, and the other is hydrogen;
further preferably, 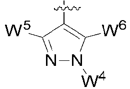 as a whole, is selected from the group consisting of 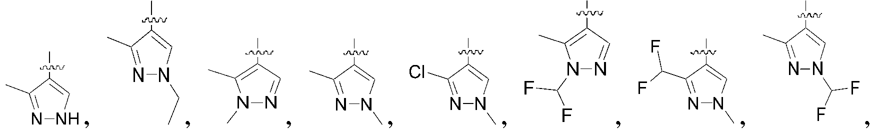 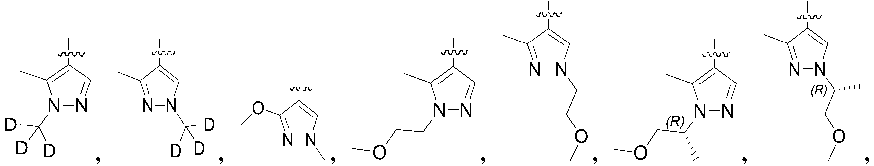 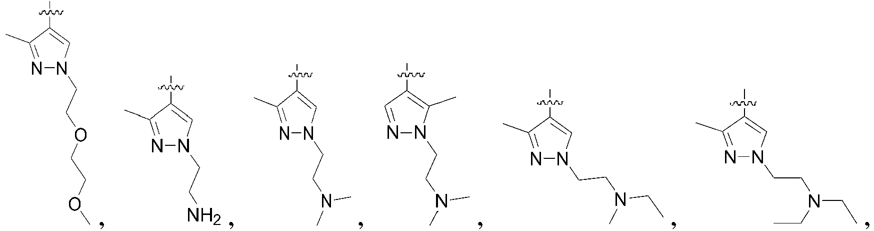 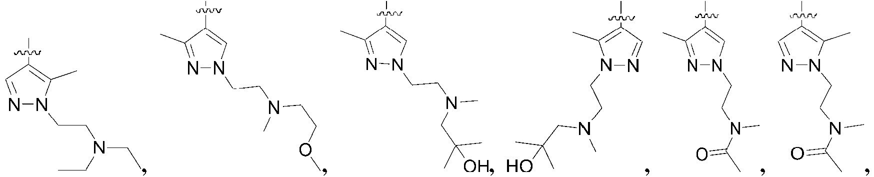 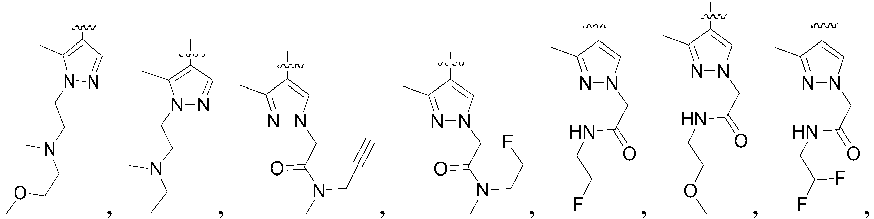 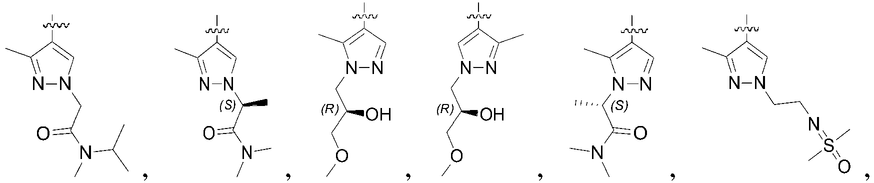 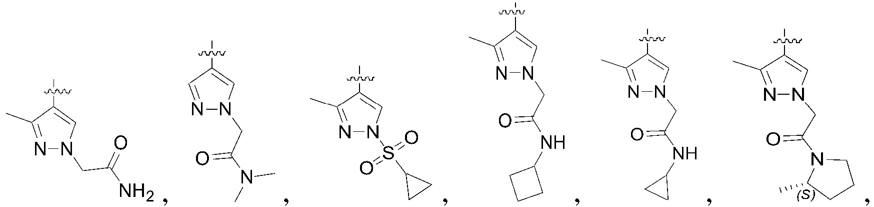 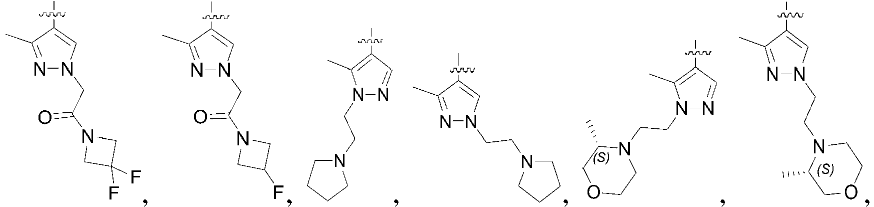 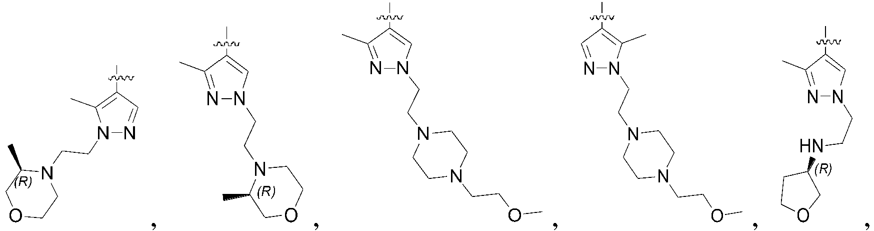 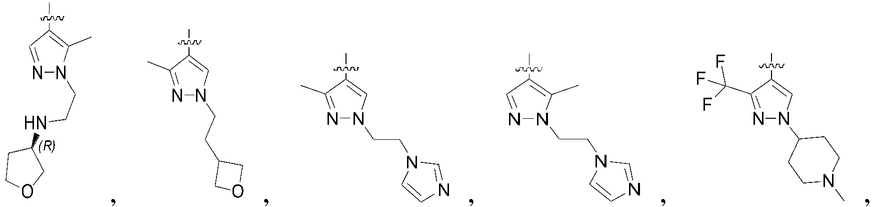 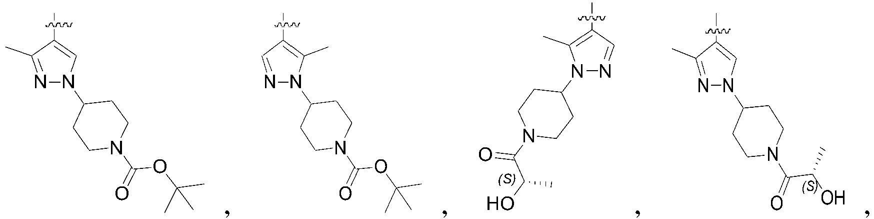 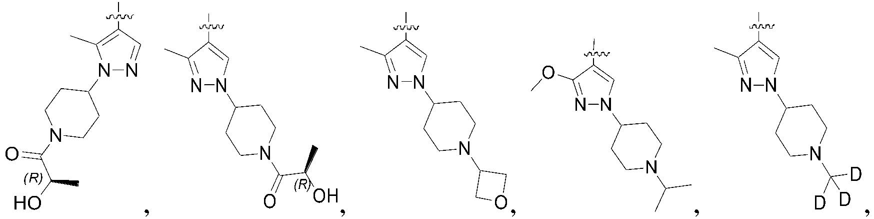 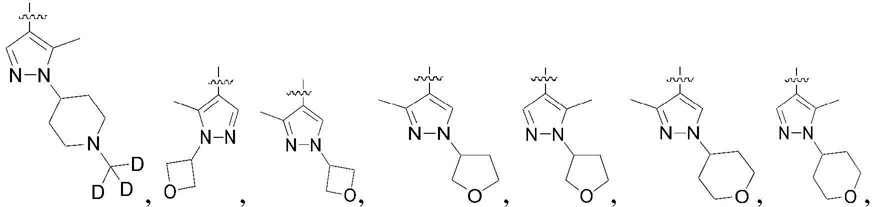 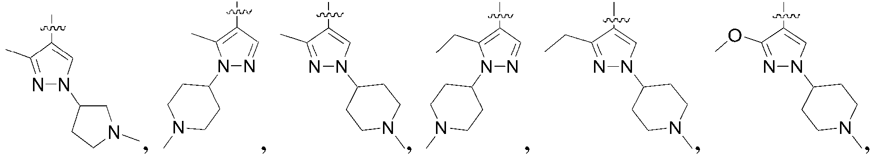 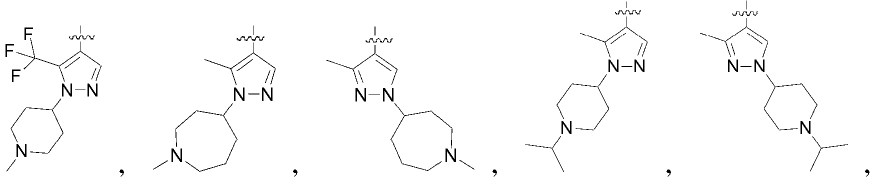 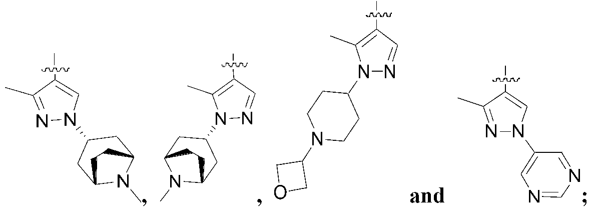
most preferably, 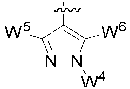 as a whole, is selected from the group consisting of 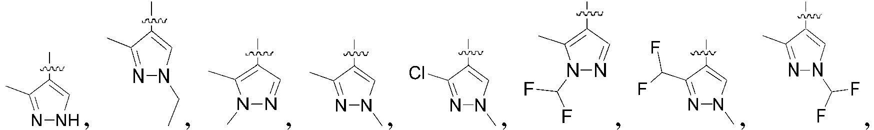 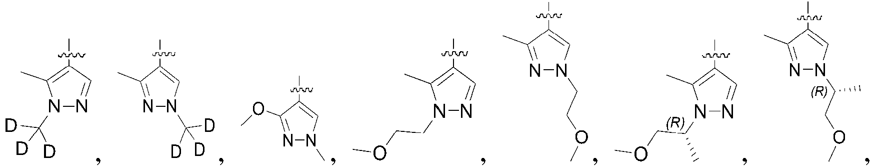 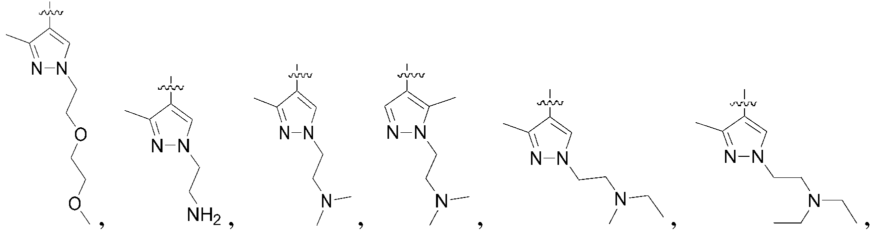 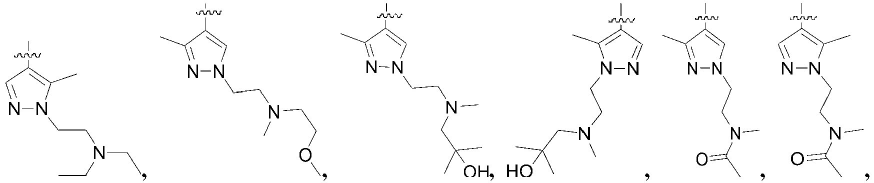 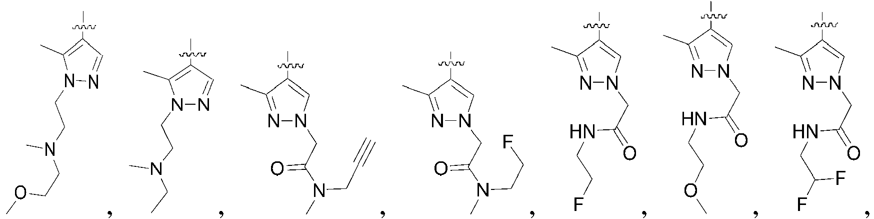 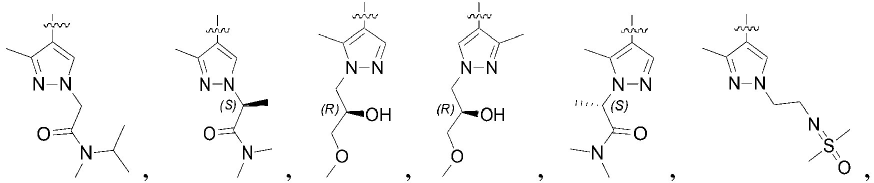 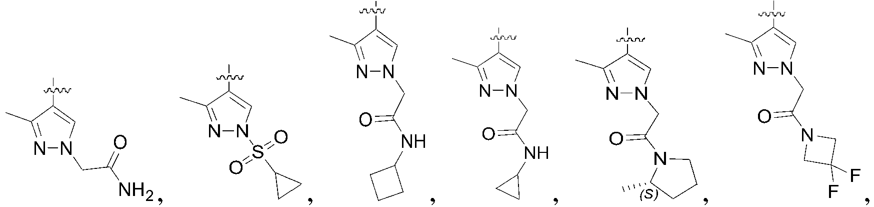 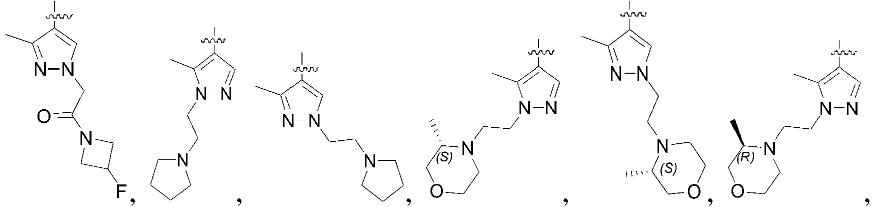 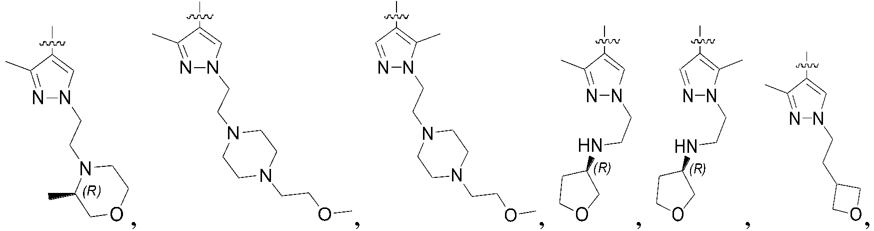 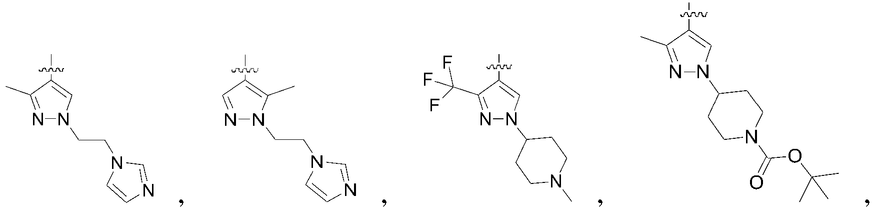 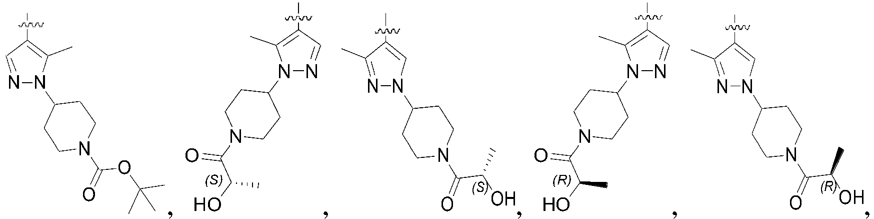 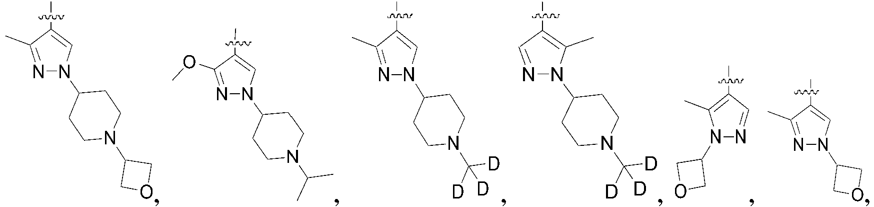 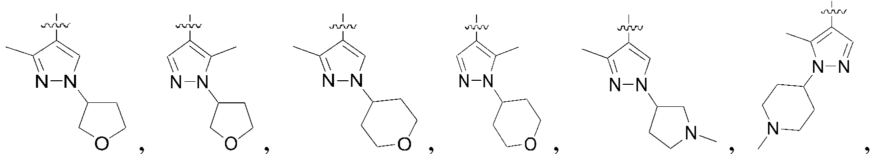 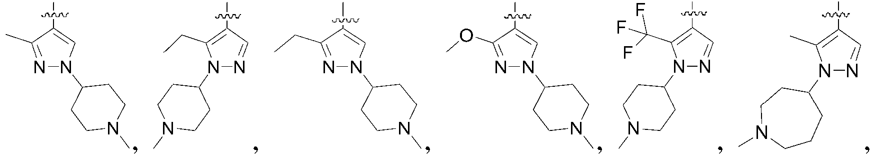 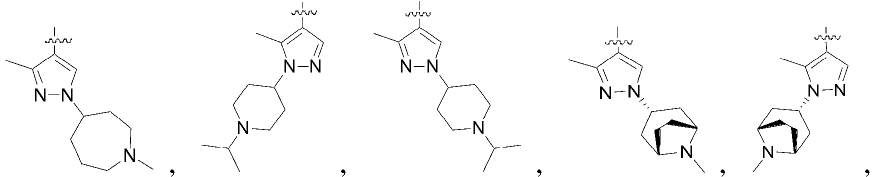 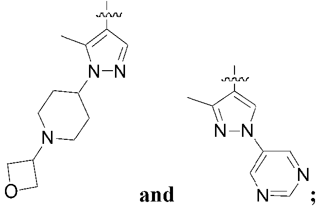
most preferably, 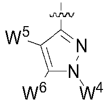 as a whole, is 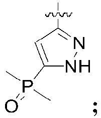
R² is selected from the group consisting of hydrogen, C1-C6 alkyl, C3-C6 cycloalkyl and C1-C6 haloalkyl;
preferably, R² is selected from the group consisting of C1-C6 alkyl, C3-C6 cycloalkyl and C1-C6 haloalkyl;
more preferably, R² is selected from the group consisting of methyl, ethyl, n-propyl, 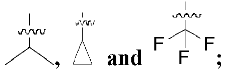
R³ is selected from the group consisting of:
   1) 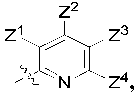 wherein:
      Z¹, Z², Z³, and Z⁴ are each independently selected from the group consisting of hydrogen, halogen, nitro, cyano, C1-C6 alkyl, C1-C6 alkoxy, C1-C6 haloalkyl, C3-C6 cycloalkyl, and - NR^{a}R^{b}, wherein R^{a} and R^{b} are each independently selected from the group consisting of hydrogen and C1-C6 alkyl;
      preferably, Z¹, Z², Z³, and Z⁴ are each independently selected from the group consisting of hydrogen, halogen, cyano, C1-C6 alkyl, C1-C6 haloalkyl, C1-C6 alkoxy, and -NR^{a}R^{b}, wherein R^{a} and R^{b} are each independently selected from the group consisting of hydrogen and C1-C6 alkyl;
      more preferably, among Z¹, Z², Z³, and Z⁴, Z¹ is selected from the group consisting of C1-C6 alkyl, halogen, and C1-C6 alkoxy, and the remainder are hydrogen; or, among Z¹, Z², Z³, and Z⁴, Z¹ and Z⁴ are each independently selected from the group consisting of halogen, cyano, C1-C6 alkyl, C1-C6 haloalkyl, and amino, and the remainder are hydrogen; or, Z¹, Z², Z³, and Z⁴ are all hydrogen;
      more preferably, Z¹, Z², Z³, and Z⁴ are each independently selected from the group consisting of hydrogen, fluoro, cyano, methyl, trifluoromethyl, amino, and methoxy;
      further preferably, among Z¹, Z², Z³, and Z⁴, Z¹ is selected from the group consisting of methyl, fluoro, and methoxy, and the remainder are hydrogen; or, among Z¹, Z², Z³, and Z⁴, Z¹ and Z⁴ are each independently selected from the group consisting of fluoro, cyano, methyl, trifluoromethyl, and amino, and the remainder are hydrogen; or, Z¹, Z², Z³, and Z⁴ are all hydrogen;
      most preferably, 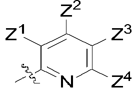 as a whole, is selected from the group consisting of 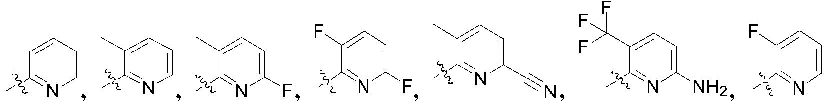 and 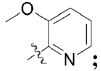
   2) 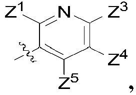 wherein: Z¹, Z³, Z⁴, and Z⁵ are each independently selected from the group consisting of hydrogen, nitro, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 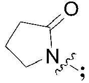 wherein R^{a} and R^{b} are each independently selected from the group consisting of hydrogen, C1-C6 alkyl, and C3-C6 cycloalkyl;
      preferably, Z¹, Z³, Z⁴, and Z⁵ are each independently selected from the group consisting of hydrogen, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 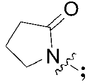 wherein R^{a} and R^{b} are each independently selected from the group consisting of hydrogen, C1-C6 alkyl, and C3-C6 cycloalkyl;
      more preferably, among Z¹, Z³, Z⁴, and Z⁵, any one is selected from the group consisting of cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 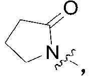 wherein R^{a} and R^{b} are each independently selected from the group consisting of hydrogen, C1-C6 alkyl, and C3-C6 cycloalkyl, and the remainder are hydrogen; or, among Z¹, Z³, Z⁴, and Z⁵, any two are selected from the group consisting of cyano, halogen, C1-C6 alkyl, amino and 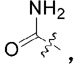 and the remainder are hydrogen; or, Z¹, Z³, Z⁴, and Z⁵ are all hydrogen;
      more preferably, Z¹, Z³, Z⁴, and Z⁵ are each independently selected from the group consisting of hydrogen, fluoro, chloro, bromo, cyano, methyl, ethyl, methoxy, isopropoxy, difluoromethyl, acetyl, cyclopropyl, 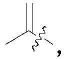 amino, 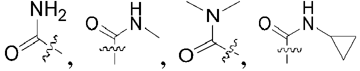 and 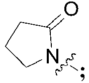
      further preferably, among Z¹, Z³, Z⁴, and Z⁵, any one is selected from the group consisting of cyano, fluoro, chloro, bromo, methyl, ethyl, methoxy, isopropoxy, difluoromethyl, acetyl, cyclopropyl, 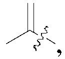 amino, 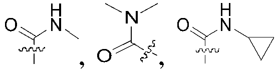 and 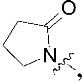 and the remainder are hydrogen; or, among Z¹, Z³, Z⁴, and Z⁵ any two are selected from the group consisting of fluoro, chloro, bromo, methyl, cyano, amino and 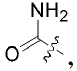 and the remainder are hydrogen; or, Z¹, Z³, Z⁴, and Z⁵ are all hydrogen;
      most preferably, 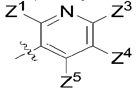 as a whole, is selected from the group consisting of 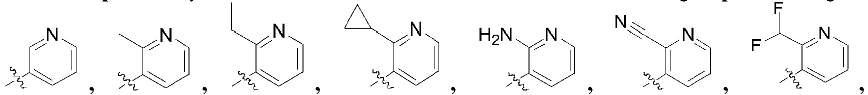 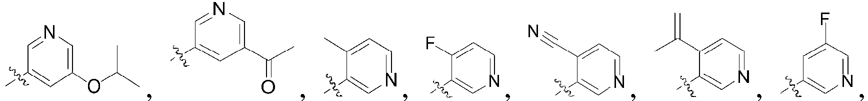 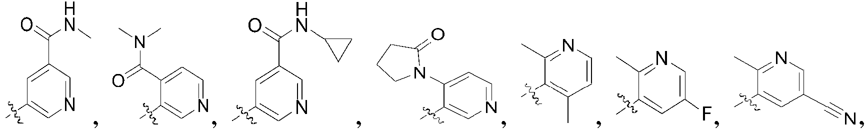 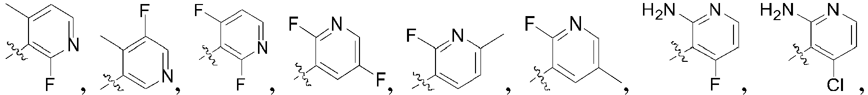 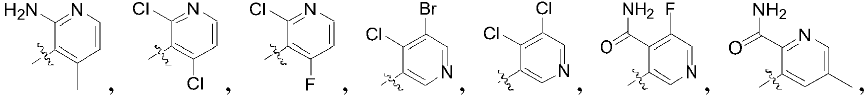 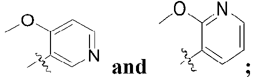
   3) 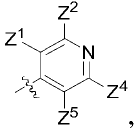 wherein:
      Z¹, Z², Z⁴, and Z⁵ are each independently selected from the group consisting of hydrogen, halogen, cyano, nitro, C1-C6 alkyl, C1-C6 alkoxy, C1-C6 alkyl-C(=O)-, and -NR^{a}R^{b}, wherein R^{a} and R^{b} are each independently selected from the group consisting of hydrogen and C1-C6 alkyl;
      preferably, Z¹, Z², Z⁴, and Z⁵ are each independently selected from the group consisting of hydrogen, halogen, C1-C6 alkyl, C1-C6 alkoxy, C1-C6 alkyl-C(=O)-, and -NR^{a}R^{b}, wherein R^{a} and R^{b} are each independently selected from the group consisting of hydrogen and C1-C6 alkyl;
      more preferably, among Z¹, Z², Z⁴, and Z⁵, any one is selected from the group consisting of C1-C6 alkyl, halogen, C1-C6 alkoxy, amino, and C1-C6 alkyl-C(=O)-, and the remainder are hydrogen; or, among Z¹, Z², Z⁴, and Z⁵, any two are selected from the group consisting of halogen and C1-C6 alkyl, and the remainder are hydrogen; or, Z¹, Z², Z⁴, and Z⁵ are all hydrogen;
      more preferably, Z¹, Z², Z⁴, and Z⁵ are each independently selected from the group consisting of hydrogen, fluoro, methyl, methoxy, acetyl, and amino;
      further preferably, among Z¹, Z², Z⁴, and Z⁵, any one is selected from the group consisting of methyl, fluoro, methoxy, amino, and acetyl, and the remainder are hydrogen; or, among Z¹, Z², Z⁴, and Z⁵, any two are selected from the group consisting of fluoro and methyl, and the remainder are hydrogen; or, Z¹, Z², Z⁴, and Z⁵ are all hydrogen;
      most preferably, 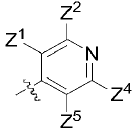 as a whole, is selected from the group consisting of 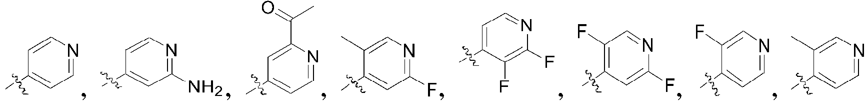 and 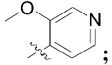
   4) 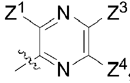 , wherein:
      Z¹, Z³, and Z⁴ are each independently selected from the group consisting of hydrogen, halogen, cyano, nitro, C1-C6 alkyl, C1-C6 alkoxy, hydroxy-substituted C1-C6 alkyl, -NR^{a}R^{b}, and R^{a}R^{b}N-C(=O)-; wherein R^{a} and R^{b} are each independently selected from the group consisting of hydrogen and C1-C6 alkyl;
      preferably, Z¹, Z³, and Z⁴ are each independently selected from the group consisting of hydrogen, halogen, cyano, C1-C6 alkyl, C1-C6 alkoxy, hydroxy-substituted C1-C6 alkyl, - NR^{a}R^{b}, and R^{a}R^{b}N-C(=O)-; wherein R^{a} and R^{b} are each independently selected from the group consisting of hydrogen and C1-C6 alkyl;
      more preferably, among Z¹, Z³, and Z⁴, any one is selected from the group consisting of halogen, cyano, C1-C6 alkyl, C1-C6 alkoxy, hydroxy-substituted C1-C6 alkyl, -NR^{a}R^{b}, and R^{a}R^{b}N-C(=O)-, wherein R^{a} and R^{b} are each independently selected from the group consisting of hydrogen and C1-C6 alkyl, and the remainder are hydrogen; or, among Z¹, Z³, and Z⁴, any two are selected from the group consisting of C1-C6 alkyl, and the remainder is hydrogen; or, Z¹, Z³, and Z⁴ are all hydrogen;
      more preferably, Z¹, Z³, and Z⁴ are each independently selected from the group consisting of hydrogen, fluoro, chloro, cyano, methyl, methoxy, hydroxymethyl, amino and 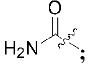
      further preferably, among Z¹, Z³, and Z⁴, any one is selected from the group consisting of fluoro, chloro, cyano, methyl, methoxy, hydroxymethyl, amino and 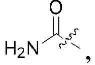 and the remainder are hydrogen; or, among Z¹, Z³, and Z⁴, any two are methyl, and the remainder is hydrogen; or, Z¹, Z³, and Z⁴ are all hydrogen;
      most preferably, 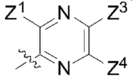 as a whole, is selected from the group consisting of 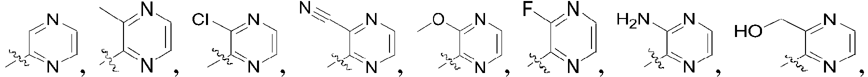 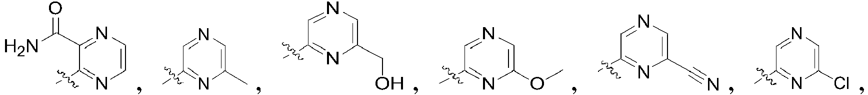 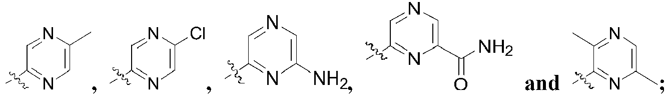
   5) 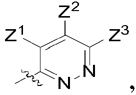 wherein:
      Z¹, Z², and Z³ are each independently selected from the group consisting of hydrogen, cyano, halogen, nitro, and C1-C6 alkyl;
      preferably, Z¹, Z², and Z³ are each independently selected from the group consisting of hydrogen, cyano, and C1-C6 alkyl;
      more preferably, among Z¹, Z², and Z³, any one is selected from the group consisting of hydrogen, cyano, and C1-C6 alkyl, and the remainder are hydrogen;
      more preferably, Z¹, Z², and Z³ are each independently selected from the group consisting of hydrogen, cyano, and methyl;
      further preferably, among Z¹, Z², and Z³, any one is selected from the group consisting of hydrogen, cyano, and methyl, and the remainder are hydrogen;
      most preferably, 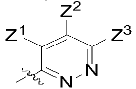 as a whole, is selected from the group consisting of 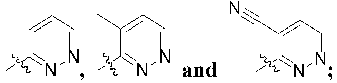
   6) 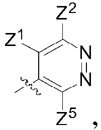 wherein:
      Z¹, Z², and Z⁵ are each independently selected from the group consisting of hydrogen, cyano, halogen, nitro, and C1-C6 alkyl;
      preferably, Z¹, Z², and Z⁵ are all hydrogen;
   7) 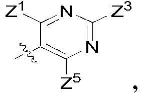 wherein:
      Z¹, Z³, and Z⁵ are each independently selected from the group consisting of hydrogen, hydroxy, halogen, cyano, nitro, C1-C6 alkyl, C1-C6 alkoxy, and -NR^{a}R^{b}, wherein R^{a} and R^{b} are each independently selected from the group consisting of hydrogen and C1-C6 alkyl;
      preferably, Z¹, Z³, and Z⁵ are each independently selected from the group consisting of hydrogen, hydroxy, halogen, C1-C6 alkyl, C1-C6 alkoxy, and -NR^{a}R^{b}, wherein R^{a} and R^{b} are each independently selected from the group consisting of hydrogen and C1-C6 alkyl;
      more preferably, among Z¹, Z³, and Z⁵, any one is selected from the group consisting of C1-C6 alkyl, C1-C6 alkoxy, and amino, and the remainder are hydrogen; or, among Z¹, Z³, and Z⁵, any two are selected from the group consisting of hydroxy, halogen, C1-C6 alkyl, C1-C6 alkoxy, and amino, and the remainder is hydrogen; or, Z¹, Z³, and Z⁵ are all hydrogen;
      more preferably, Z¹, Z³, and Z⁵ are each independently selected from the group consisting of hydrogen, hydroxy, fluoro, chloro, methyl, methoxy, and amino;
      further preferably, among Z¹, Z³, and Z⁵, any one is selected from the group consisting of methyl, methoxy, and amino, and the remainder are hydrogen; or, among Z¹, Z³, and Z⁵, any two are selected from the group consisting of hydroxy, fluoro, chloro, methyl, methoxy, and amino, and the remainder is hydrogen; or, Z¹, Z³, and Z⁵ are all hydrogen;
      most preferably, 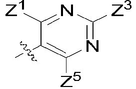 as a whole, is selected from the group consisting of 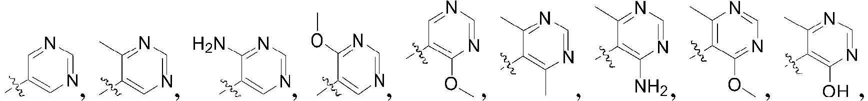 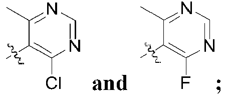
   8) 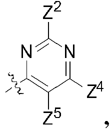 wherein:
      Z², Z⁴, and Z⁵ are each independently selected from the group consisting of hydrogen, halogen, cyano, nitro, and C1-C6 alkyl;
      preferably, Z², Z⁴, and Z⁵ are each independently selected from the group consisting of hydrogen, halogen, and C1-C6 alkyl;
      more preferably, among Z², Z⁴, and Z⁵, any one is selected from the group consisting of halogen and C1-C6 alkyl, and the remainder are hydrogen; or, among Z², Z⁴, and Z⁵, any two are selected from the group consisting of halogen and C1-C6 alkyl, and the remainder is hydrogen; or, Z², Z⁴, and Z⁵ are all hydrogen;
      more preferably, Z², Z⁴, Z⁵ are each independently selected from the group consisting of hydrogen, fluoro, chloro, bromo and methyl;
      further preferably, among Z², Z⁴, and Z⁵, any one is selected from the group consisting of fluoro, chloro, bromo, and methyl, and the remainder are hydrogen; or, among Z², Z⁴, and Z⁵, any two are selected from the group consisting of fluoro, bromo, and methyl, and the remainder is hydrogen; or, Z², Z⁴, and Z⁵ are all hydrogen;
      most preferably, 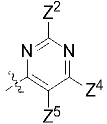 as a whole, is selected from the group consisting of 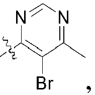 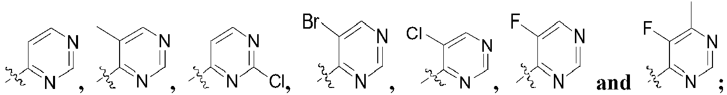
   9) 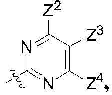 wherein:
      Z², Z³, and Z⁴ are each independently selected from the group consisting of hydrogen, cyano, halogen, nitro, and C1-C6 alkyl;
      preferably, Z², Z³, and Z⁴ are all hydrogen;
   10) 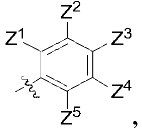 wherein:
      Z¹, Z², Z³, Z⁴, and Z⁵ are each independently selected from the group consisting of hydrogen, hydroxy, cyano, nitro, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, and the 5-membered heterocyclic ring or the 5-membered heteroaromatic ring is independently optionally substituted by 1 to 2 halogen atoms, and the remainder are each independently selected from the group consisting of hydrogen and C1-C6 alkyl;
      preferably, Z¹, Z², Z³, Z⁴, and Z⁵ are each independently selected from the group consisting of hydrogen, hydroxy, cyano, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, and the 5-membered heterocyclic ring or the 5-membered heteroaromatic ring is independently optionally substituted by 1 to 2 halogen atoms, and the remainder are each independently selected from the group consisting of hydrogen and C1-C6 alkyl;
      more preferably, among Z¹, Z², Z³, Z⁴, and Z⁵, any two are selected from the group consisting of hydroxy, cyano, halogen, and C1-C6 alkyl, and the remainder are hydrogen; or, among Z¹, Z², Z³, Z⁴, and Z⁵, Z¹ and Z² or Z² and Z³, and the carbon atoms to which they are respectively attached together form 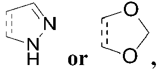 and the 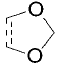 is optionally substituted by 1 or 2 halogen atoms, and the remainder are each independently selected from the group consisting of hydrogen and C1-C6 alkyl;
      further preferably, among Z¹, Z², Z³, Z⁴, and Z⁵, any two are selected from the group consisting of hydroxy, cyano, fluoro, and methyl, and the remainder are hydrogen; or, among Z¹, Z², Z³, Z⁴, and Z⁵, Z¹ and Z² or Z² and Z³, and the carbon atoms to which they are respectively attached together form 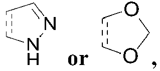 and the 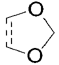 is optionally substituted by 1 or 2 fluoro atoms, and the remainder are each independently selected from the group consisting of hydrogen and methyl;
      most preferably, 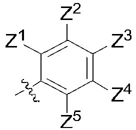 as a whole, is selected from the group consisting of 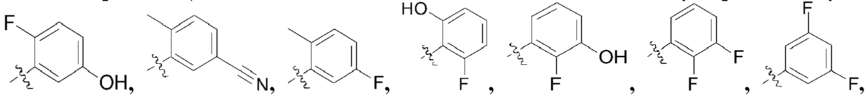 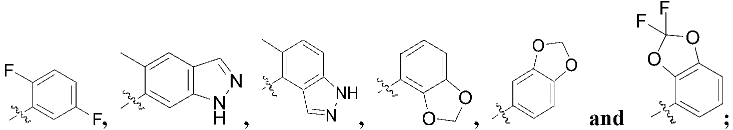
   11) 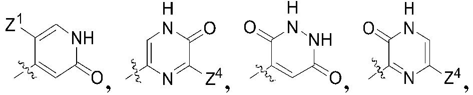 wherein Z¹ is selected from the group consisting of hydrogen and C1-C6 alkyl, preferably selected from the group consisting of hydrogen and methyl; each Z⁴ is independently selected from the group consisting of halogen and C1-C6 alkyl, preferably selected from the group consisting of chloro and methyl;
      and 12) 5- to 9-membered heteroaryl, and the 5- to 9-membered heteroaryl is optionally substituted by 1 or 2 substituent(s) selected from the group consisting of C1-C6 alkyl and cyano; preferably, the 5- to 9-membered heteroaryl is selected from the group consisting of 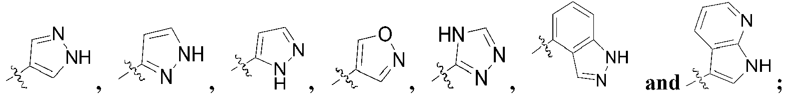 and the 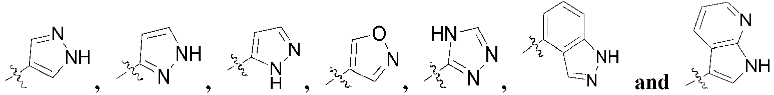 are each independently optionally substituted by 1 or 2 substituents selected from the group consisting of C1-C6 alkyl and cyano; more preferably, the 5- to 9-membered heteroaryl is selected from the group consisting of 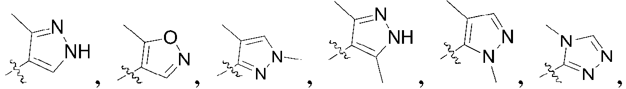 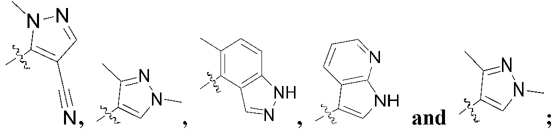
      preferably, R³ is selected from the group consisting of

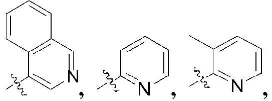

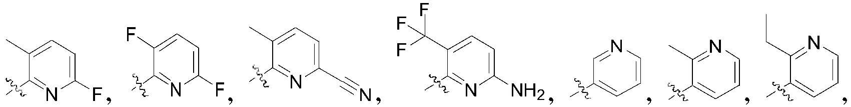

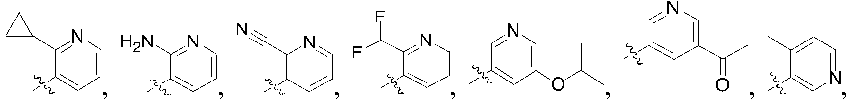

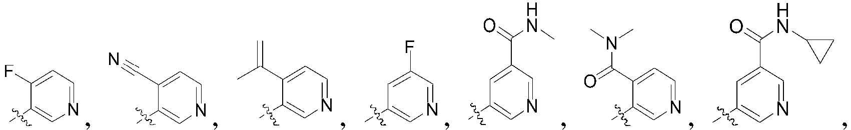

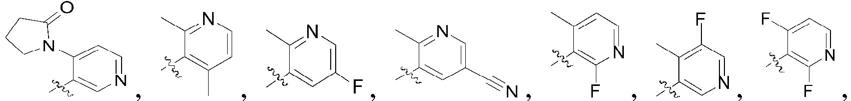

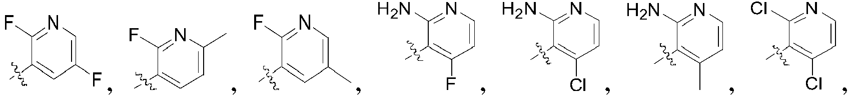

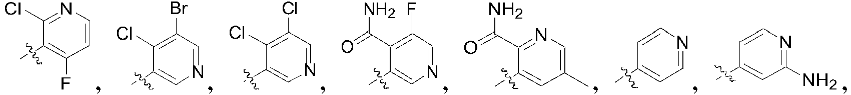

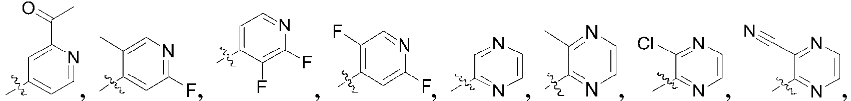

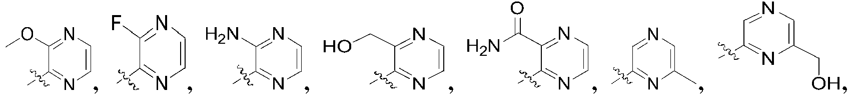

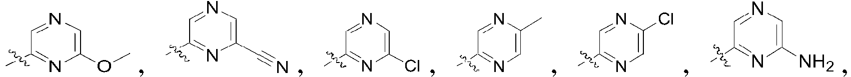

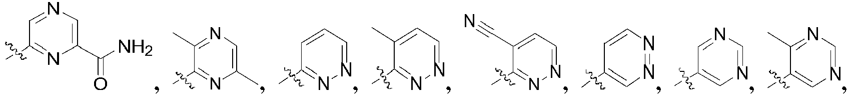

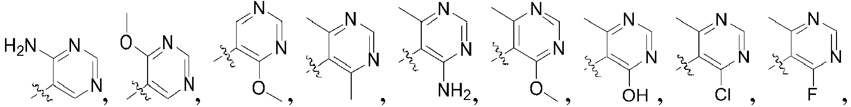

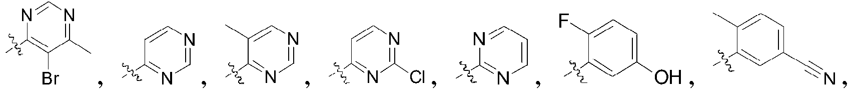

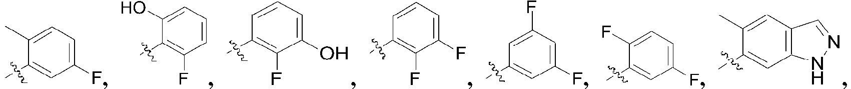

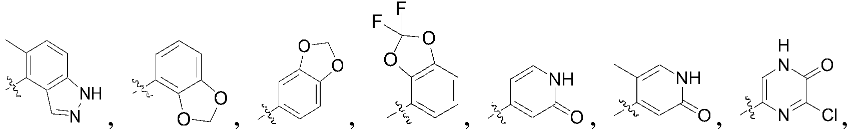

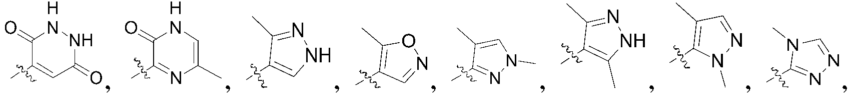

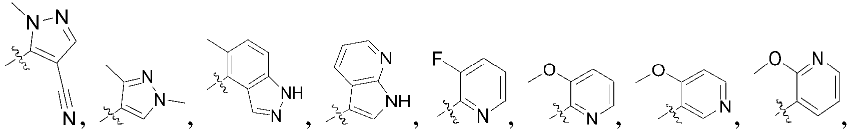

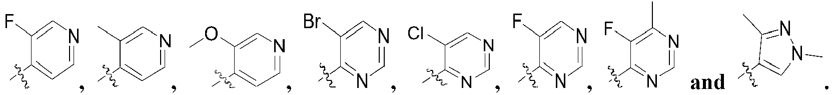

In some embodiments, the compound has the structure of formula I, 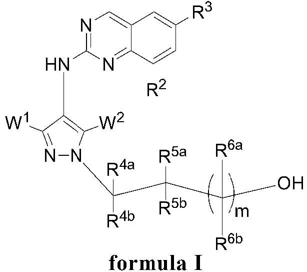 wherein:
R² is selected from the group consisting of hydrogen, C1-C6 alkyl, C3-C6 cycloalkyl and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 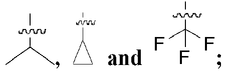
R³ is selected from the group consisting of:
   1) 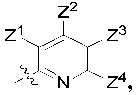 wherein:
      Z¹, Z², Z³, and Z⁴ are each independently selected from the group consisting of hydrogen, halogen, nitro, cyano, C1-C6 alkyl, C1-C6 alkoxy, C1-C6 haloalkyl, C3-C6 cycloalkyl, and - NR^{a}R^{b}, wherein R^{a} and R^{b} are each independently selected from the group consisting of hydrogen and C1-C6 alkyl;
      preferably, Z¹, Z², Z³, and Z⁴ are each independently selected from the group consisting of hydrogen, halogen, cyano, C1-C6 alkyl, C1-C6 haloalkyl, C1-C6 alkoxy, and -NR^{a}R^{b}, wherein R^{a} and R^{b} are each independently selected from the group consisting of hydrogen and C1-C6 alkyl;
      more preferably, among Z¹, Z², Z³, and Z⁴, Z¹ is selected from the group consisting of C1-C6 alkyl, halogen, and C1-C6 alkoxy, and the remainder are hydrogen; or, among Z¹, Z², Z³, and Z⁴, Z¹ and Z⁴ are each independently selected from the group consisting of halogen, cyano, C1-C6 alkyl, C1-C6 haloalkyl, and amino, and the remainder are hydrogen; or, Z¹, Z², Z³, and Z⁴ are all hydrogen;
      more preferably, Z¹, Z², Z³, and Z⁴ are each independently selected from the group consisting of hydrogen, fluoro, cyano, methyl, trifluoromethyl, amino, and methoxy;
      further preferably, among Z¹, Z², Z³, and Z⁴, Z¹ is selected from the group consisting of methyl, fluoro, and methoxy, and the remainder are hydrogen; or, among Z¹, Z², Z³, and Z⁴, Z¹ and Z⁴ are each independently selected from the group consisting of fluoro, cyano, methyl, trifluoromethyl, and amino, and the remainder are hydrogen; or, Z¹, Z², Z³, and Z⁴ are all hydrogen;
      most preferably, 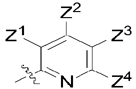 as a whole, is selected from the group consisting of 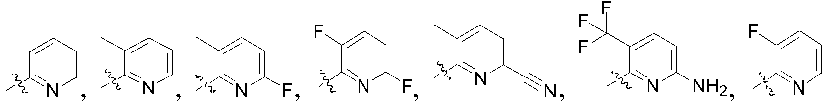 and 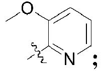
   2) 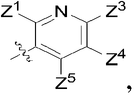 wherein:
      Z¹, Z³, Z⁴, and Z⁵ are each independently selected from the group consisting of hydrogen, nitro, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 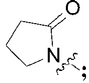 wherein R^{a} and R^{b} are each independently selected from the group consisting of hydrogen, C1-C6 alkyl, and C3-C6 cycloalkyl;
      preferably, Z¹, Z³, Z⁴, and Z⁵ are each independently selected from the group consisting of hydrogen, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 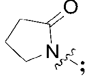 wherein R^{a} and R^{b} are each independently selected from the group consisting of hydrogen, C1-C6 alkyl, and C3-C6 cycloalkyl;
      more preferably, among Z¹, Z³, Z⁴, and Z⁵, any one is selected from the group consisting of cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 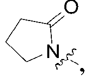 wherein R^{a} and R^{b} are each independently selected from the group consisting of hydrogen, C1-C6 alkyl, and C3-C6 cycloalkyl, and the remainder are hydrogen; or, among Z¹, Z³, Z⁴, and Z⁵, any two are selected from the group consisting of cyano, halogen, C1-C6 alkyl, and amino, and the remainder are hydrogen; or, Z¹, Z³, Z⁴, and Z⁵ are all hydrogen;
      more preferably, Z¹, Z³, Z⁴, and Z⁵ are each independently selected from the group consisting of hydrogen, fluoro, chloro, bromo, cyano, methyl, ethyl, methoxy, isopropoxy, difluoromethyl, acetyl, cyclopropyl, 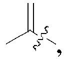 amino, 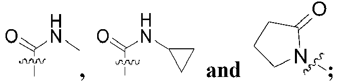
      further preferably, among Z¹, Z³, Z⁴, and Z⁵, any one is selected from the group consisting of cyano, fluoro, chloro, bromo, methyl, ethyl, methoxy, isopropoxy, difluoromethyl, acetyl, cyclopropyl, 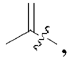 amino, 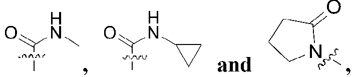 and the remainder are hydrogen; or, among Z¹, Z³, Z⁴, and Z⁵, any two are selected from the group consisting of fluoro, chloro, bromo, methyl, cyano, and amino, and the remainder are hydrogen; or, Z¹, Z³, Z⁴, and Z⁵ are all hydrogen;
      most preferably, 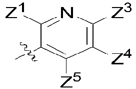 as a whole, is selected from the group consisting of 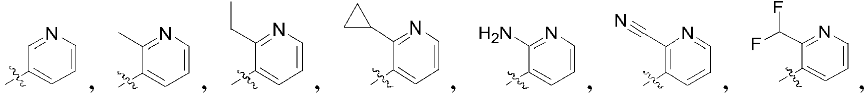 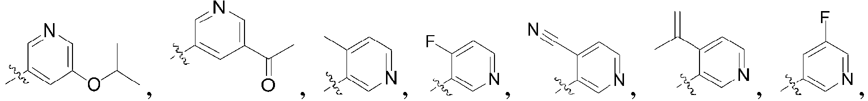 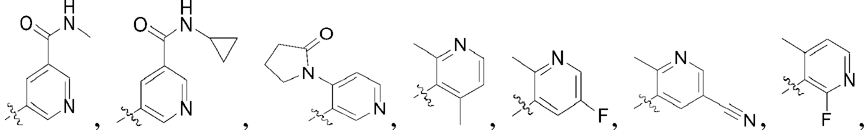 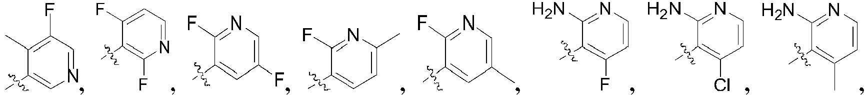 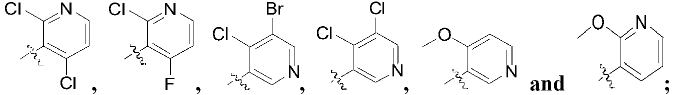
   3) 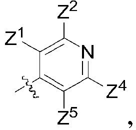 wherein:
      Z¹, Z², Z⁴, and Z⁵ are each independently selected from the group consisting of hydrogen, halogen, cyano, nitro, C1-C6 alkyl, C1-C6 alkoxy, C1-C6 alkyl-C(=O)- and -NR^{a}R^{b}, wherein R^{a} and R^{b} are each independently selected from the group consisting of hydrogen and C1-C6 alkyl;
      preferably, Z¹, Z², Z⁴, and Z⁵ are each independently selected from the group consisting of hydrogen, halogen, C1-C6 alkyl, C1-C6 alkoxy, C1-C6 alkyl-C(=O)- and -NR^{a}R^{b}, wherein R^{a} and R^{b} are each independently selected from the group consisting of hydrogen and C1-C6 alkyl;
      more preferably, among Z¹, Z², Z⁴, and Z⁵, any one is selected from the group consisting of C1-C6 alkyl, halogen, C1-C6 alkoxy, amino and C1-C6 alkyl-C(=O)-, and the remainder are hydrogen; or, among Z¹, Z², Z⁴, and Z⁵, any two are selected from the group consisting of halogen and C1-C6 alkyl, and the remainder are hydrogen; or, Z¹, Z², Z⁴, and Z⁵ are all hydrogen;
      more preferably, Z¹, Z², Z⁴, Z⁵ are each independently selected from the group consisting of hydrogen, fluoro, methyl, methoxy, acetyl and amino;
      further preferably, among Z¹, Z², Z⁴, and Z⁵, any one is selected from the group consisting of methyl, fluoro, methoxy, amino and acetyl, and the remainder are hydrogen; or, among Z¹, Z², Z⁴, and Z⁵, any two are selected from the group consisting of fluoro and methyl, and the remainder are hydrogen; or, Z¹, Z², Z⁴, and Z⁵ are all hydrogen;
      most preferably, 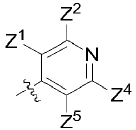 as a whole, is selected from the group consisting of 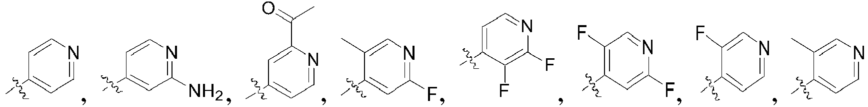 and 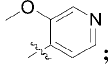
   4) 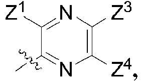 wherein:
      Z¹, Z³, and Z⁴ are each independently selected from the group consisting of hydrogen, halogen, cyano, nitro, C1-C6 alkyl, C1-C6 alkoxy, hydroxy-substituted C1-C6 alkyl, -NR^{a}R^{b}, and R^{a}R^{b}N-C(=O)-; wherein R^{a} and R^{b} are each independently selected from the group consisting of hydrogen and C1-C6 alkyl;
      preferably, Z¹, Z³, and Z⁴ are each independently selected from the group consisting of hydrogen, halogen, cyano, C1-C6 alkyl, C1-C6 alkoxy, hydroxy-substituted C1-C6 alkyl, - NR^{a}R^{b}, and R^{a}R^{b}N-C(=O)-; wherein R^{a} and R^{b} are each independently selected from the group consisting of hydrogen and C1-C6 alkyl;
      more preferably, among Z¹, Z³, and Z⁴, any one is selected from the group consisting of halogen, cyano, C1-C6 alkyl, C1-C6 alkoxy, hydroxy-substituted C1-C6 alkyl, -NR^{a}R^{b}, and R^{a}R^{b}N-C(=O)-, wherein R^{a} and R^{b} are each independently selected from the group consisting of hydrogen and C1-C6 alkyl, and the remainder are hydrogen; or among Z¹, Z³, and Z⁴, any two are independently selected from the group consisting of C1-C6 alkyl, and the remainder is hydrogen; or Z¹, Z³, and Z⁴ are all hydrogen;
      more preferably, Z¹, Z³, and Z⁴ are each independently selected from the group consisting of hydrogen, fluoro, chloro, cyano, methyl, methoxy, hydroxymethyl, amino and 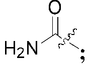
      further preferably, among Z¹, Z³, and Z⁴, any one is selected from the group consisting of fluoro, chloro, cyano, methyl, methoxy, hydroxymethyl, amino and 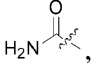 and the remainder are hydrogen; or, among Z¹, Z³, and Z⁴, any two are methyl, and the remainder is hydrogen; or, Z¹, Z³, and Z⁴ are all hydrogen;
      most preferably, 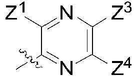 as a whole, is selected from the group consisting of 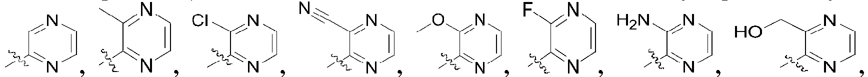 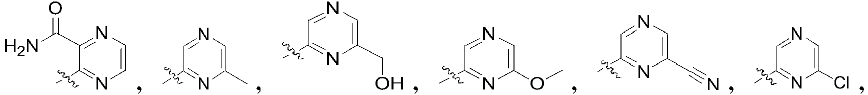 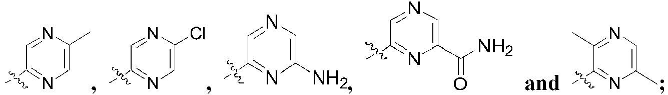
   5) 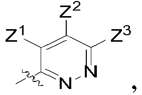 wherein:
      Z¹, Z², and Z³ are each independently selected from the group consisting of hydrogen, cyano, halogen, nitro, and C1-C6 alkyl;
      preferably, Z¹, Z², and Z³ are each independently selected from the group consisting of hydrogen, cyano, and C1-C6 alkyl;
      more preferably, among Z¹, Z², and Z³, any one is selected from the group consisting of hydrogen, cyano, and C1-C6 alkyl, and the remainder are hydrogen;
      more preferably, Z¹, Z², and Z³ are each independently selected from the group consisting of hydrogen, cyano, and methyl;
      further preferably, among Z¹, Z², and Z³, any one is selected from the group consisting of hydrogen, cyano, and methyl, and the remainder are hydrogen;
      most preferably, 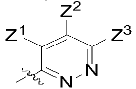 as a whole, is selected from the group consisting of 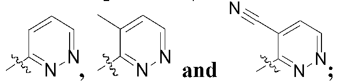
   6) 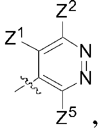 wherein:
      Z¹, Z², and Z⁵ are each independently selected from the group consisting of hydrogen, cyano, halogen, nitro, and C1-C6 alkyl;
      preferably, Z¹, Z², and Z⁵ are all hydrogen;
   7) 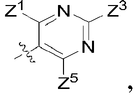 wherein:
      Z¹, Z³, and Z⁵ are each independently selected from the group consisting of hydrogen, hydroxy, halogen, cyano, nitro, C1-C6 alkyl, C1-C6 alkoxy, and -NR^{a}R^{b}, wherein R^{a} and R^{b} are each independently selected from the group consisting of hydrogen and C1-C6 alkyl;
      preferably, Z¹, Z³, and Z⁵ are each independently selected from the group consisting of hydrogen, hydroxy, halogen, C1-C6 alkyl, C1-C6 alkoxy, and -NR^{a}R^{b}, wherein R^{a} and R^{b} are each independently selected from the group consisting of hydrogen and C1-C6 alkyl;
      more preferably, among Z¹, Z³, and Z⁵, any one is selected from the group consisting of C1-C6 alkyl, C1-C6 alkoxy, and amino, and the remainder are hydrogen; or, among Z¹, Z³, and Z³, any two are selected from the group consisting of hydroxy, halogen, C1-C6 alkyl, C1-C6 alkoxy, and amino, and the remainder is hydrogen; or, Z¹, Z³, and Z⁵ are all hydrogen;
      more preferably, Z¹, Z³, and Z⁵ are each independently selected from the group consisting of hydrogen, hydroxy, fluoro, chloro, methyl, methoxy, and amino;
      further preferably, among Z¹, Z³, and Z⁵, any one is selected from the group consisting of methyl, methoxy, and amino, and the remainder are hydrogen; or, among Z¹, Z³, and Z⁵, any two are selected from the group consisting of hydroxy, fluoro, chloro, methyl, methoxy, and amino, and the remainder is hydrogen; or, Z¹, Z³, and Z⁵ are all hydrogen;
      most preferably, 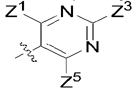 as a whole, is selected from the group consisting of 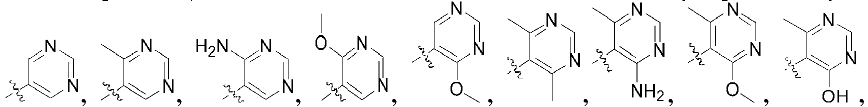 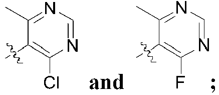
   8) 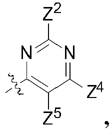 wherein:
      Z², Z⁴, and Z⁵ are each independently selected from the group consisting of hydrogen, halogen, cyano, nitro, and C1-C6 alkyl;
      preferably, Z², Z⁴, and Z⁵ are each independently selected from the group consisting of hydrogen, halogen, and C1-C6 alkyl;
      more preferably, among Z², Z⁴, and Z⁵, any one is selected from the group consisting of halogen and C1-C6 alkyl, and the remainder are hydrogen; or, among Z², Z⁴, and Z⁵, any two are selected from the group consisting of halogen and C1-C6 alkyl, and the remainder is hydrogen; or, Z², Z⁴, and Z⁵ are all hydrogen;
      more preferably, Z², Z⁴, and Z⁵ are each independently selected from the group consisting of hydrogen, fluoro, chloro, bromo, and methyl;
      further preferably, among Z², Z⁴, and Z⁵, any one is selected from the group consisting of fluoro, chloro, bromo, and methyl, and the remainder are hydrogen; or, among Z², Z⁴, and Z⁵, any two are selected from the group consisting of fluoro, bromo, and methyl, and the remainder is hydrogen; or, Z², Z⁴, and Z⁵ are all hydrogen;
      most preferably, 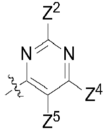 as a whole, is selected from the group consisting of 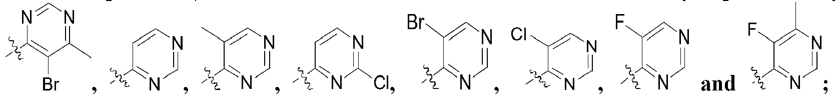
   9) 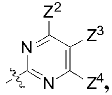 wherein:
      Z², Z³, and Z⁴ are each independently selected from the group consisting of hydrogen, cyano, halogen, nitro, and C1-C6 alkyl;
      preferably, Z², Z³, and Z⁴ are all hydrogen;
   10) 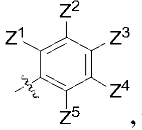 wherein:
      Z¹, Z², Z³, Z⁴, and Z⁵ are each independently selected from the group consisting of hydrogen, hydroxy, cyano, nitro, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, wherein the 5-membered heterocyclic ring or the 5-membered heteroaromatic ring is independently optionally substituted by 1 or 2 halogen atoms, and the remainder are each independently selected from the group consisting of hydrogen and C1-C6 alkyl;
      preferably, Z¹, Z², Z³, Z⁴, and Z⁵ are each independently selected from the group consisting of hydrogen, hydroxy, cyano, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, wherein the 5-membered heterocyclic ring or the 5-membered heteroaromatic ring is independently optionally substituted by 1 or 2 halogen atoms, and the remainder are each independently selected from the group consisting of hydrogen and C1-C6 alkyl;
      more preferably, among Z¹, Z², Z³, Z⁴, and Z⁵, any two are independently selected from the group consisting of hydroxy, cyano, halogen, and C1-C6 alkyl, and the remainder are hydrogen; or, among Z¹, Z², Z³, Z⁴, and Z⁵, Z¹ and Z² or Z² and Z³, and the carbon atoms to which they are respectively attached together form 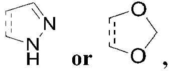 wherein 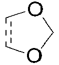 is optionally substituted by 1 or 2 halogen atoms, and the remainder are each independently selected from the group consisting of hydrogen and C1-C6 alkyl;
      further preferably, among Z¹, Z², Z³, Z⁴, and Z⁵, any two are independently selected from the group consisting of hydroxy, cyano, fluoro, and methyl, and the remainder are hydrogen; or, among Z¹, Z², Z³, Z⁴, and Z⁵, Z¹ and Z² or Z² and Z³, and the carbon atoms to which they are respectively attached together form 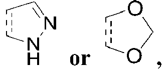 wherein the 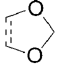 is optionally substituted by 1 or 2 fluoro atoms, and the remainder are each independently selected from the group consisting of hydrogen and methyl;
      most preferably, 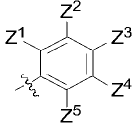 as a whole, is selected from the group consisting of 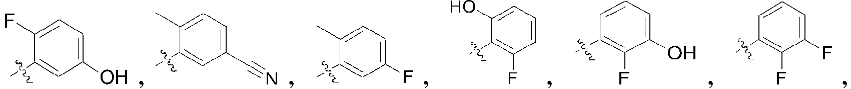 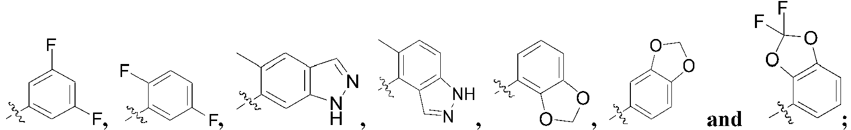
   11) 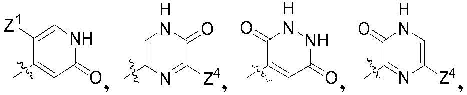 wherein, Z¹ is selected from the group consisting of C1-C6 alkyl, preferably is methyl; each Z⁴ is independently selected from the group consisting of halogen and C1-C6 alkyl, preferably is independently selected from the group consisting of chloro and methyl;
      and 12) 5- to 9-membered heteroaryl, and the 5- to 9-membered heteroaryl is optionally substituted by 1 or 2 substituents selected from the group consisting of C1-C6 alkyl; preferably, the 5- to 9-membered heteroaryl is selected from the group consisting of 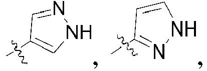 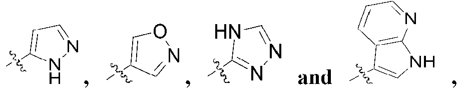 and the 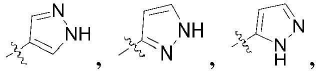 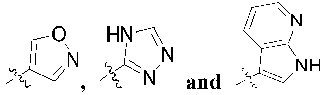 are each independently optionally substituted by 1 or 2 substituents selected from C1-C6 alkyl; more preferably, the 5- to 9-membered heteroaryl is selected from the group consisting of 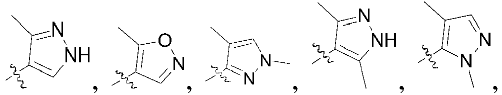 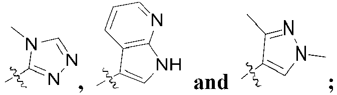
      preferably, R³ is selected from the group consisting of 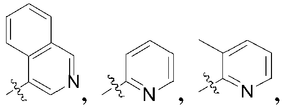 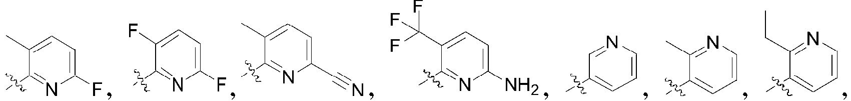 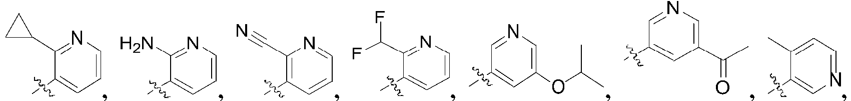 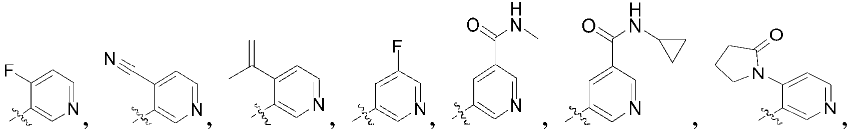 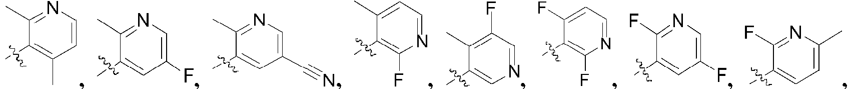 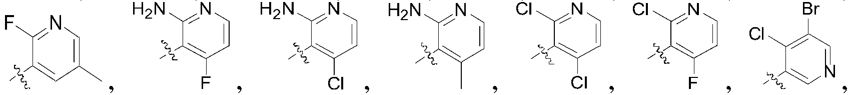 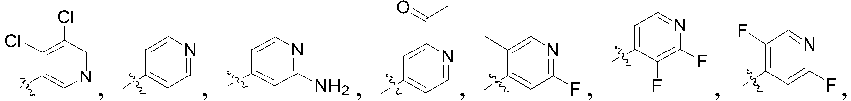 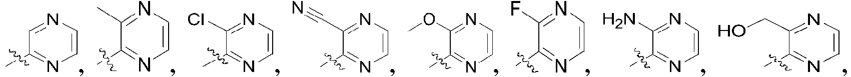 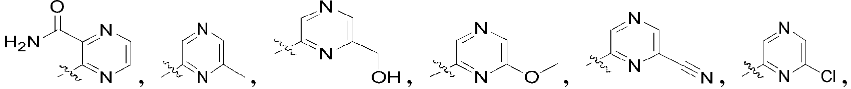 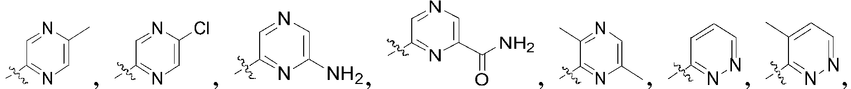 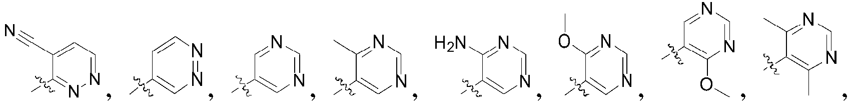 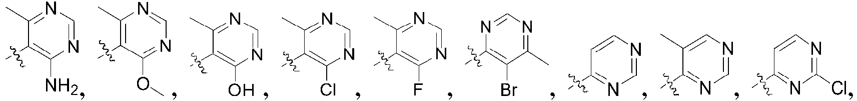 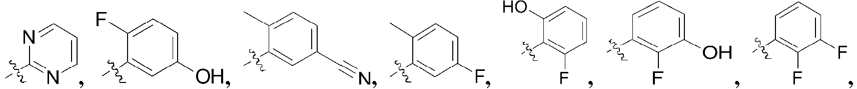 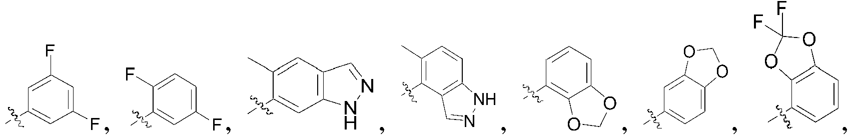 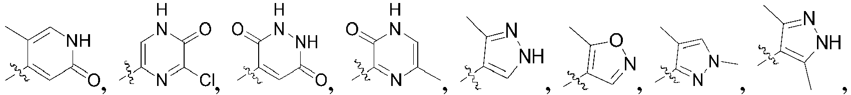 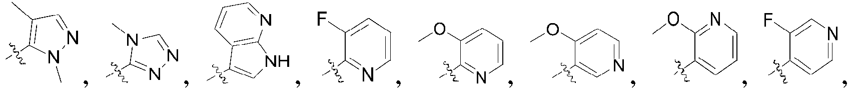 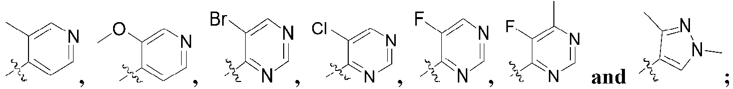
      R^{4a} and R^{4b} are each independently selected from the group consisting of hydrogen and C1-C6 alkyl;
      preferably, R^{4a} and R^{4b} are each independently selected from the group consisting of hydrogen and methyl;
      R^{5a} and R^{5b} are each independently selected from the group consisting of hydrogen and C1-C6 alkyl;
      preferably, R^{5a} and R^{5b} are each independently selected from the group consisting of hydrogen and methyl;
      R^{6a} and R^{6b} are each independently selected from the group consisting of hydrogen and C1-C6 alkyl;
      preferably, R^{6a} and R^{6b} are each independently selected from the group consisting of hydrogen and methyl;
      m is selected from the group consisting of 0 and 1;
      W¹ and W² are each independently selected from the group consisting of hydrogen, halogen, C1-C6 alkyl, and C1-C6 haloalkyl;
      preferably, any one of W¹ and W² is selected from the group consisting of hydrogen, halogen, C1-C6 alkyl, and C1-C6 haloalkyl, and the other is hydrogen;
      more preferably, any one of W¹ and W² is selected from the group consisting of hydrogen, chloro, methyl, and difluoromethyl, and the other is hydrogen;
      most preferably, 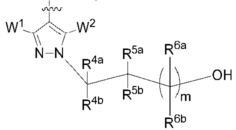 as a whole, is selected from the group consisting of:

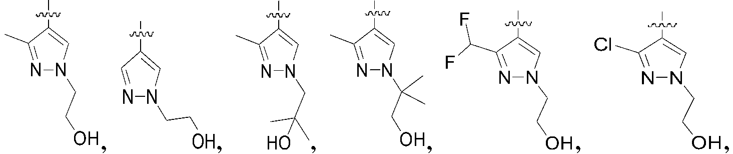

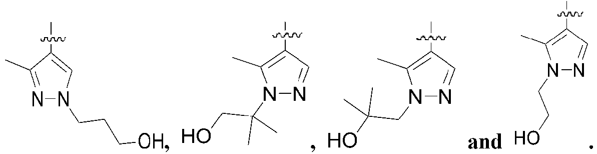

In some embodiments, the compound has the structure of formula II, 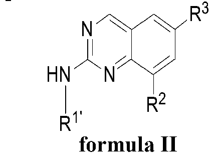 wherein:
R^{1'} is selected from the group consisting of 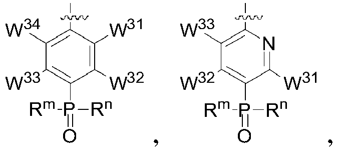 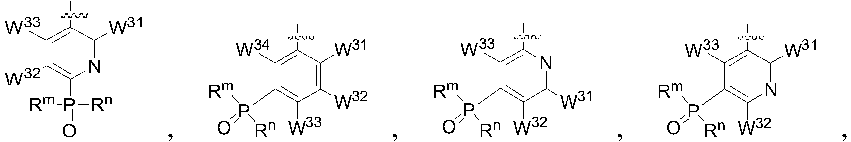 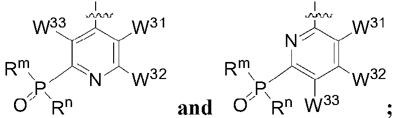
preferably, R^{1'} is selected from the group consisting of 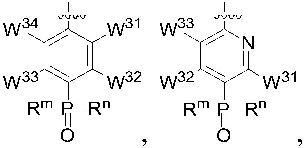 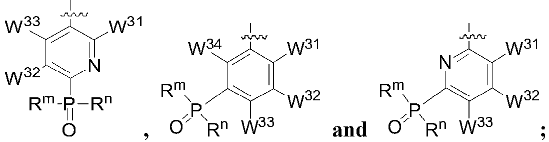
R^{m} and Rⁿ are each independently selected from the group consisting of C1-C6 alkyl;
preferably, R^{m} and Rⁿ are each independently selected from the group consisting of methyl and ethyl;
preferably, R^{1'} is selected from the group consisting of 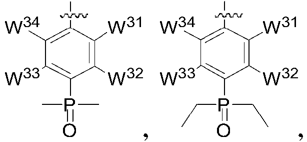 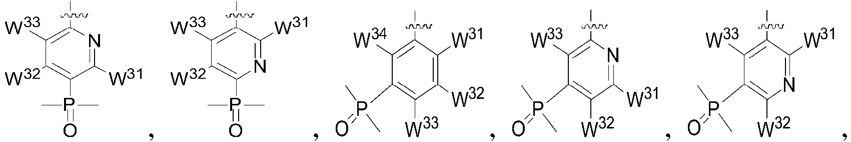 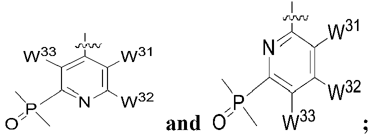
preferably, R^{1'} is selected from the group consisting of 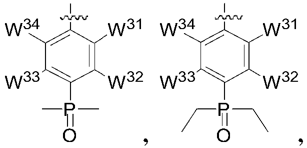 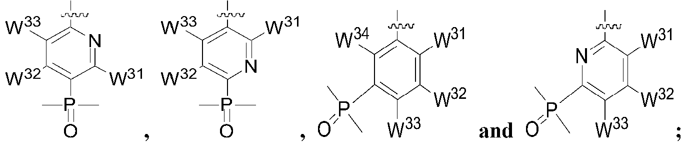
more preferably, R^{1'} is selected from the group consisting of 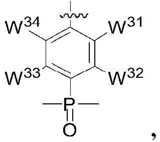 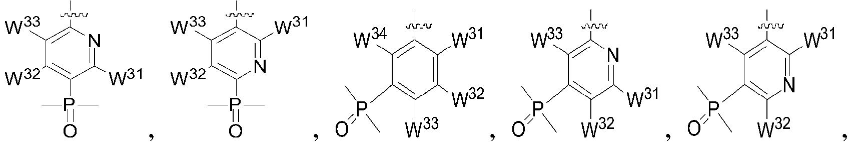 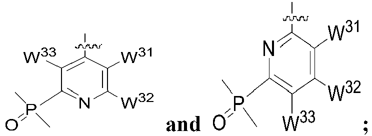
most preferably, R^{1'} is selected from the group consisting of 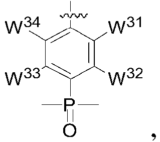 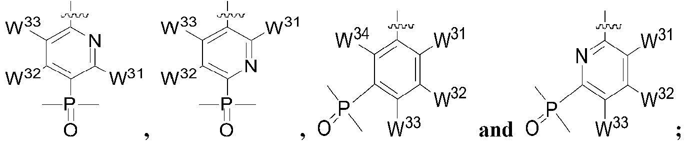
when W³¹, W³², W³³, and W³⁴ are simultaneously present, W³¹, W³², W³³, and W³⁴ are each independently selected from the group consisting of hydrogen, halogen, hydroxy, C1-C6 alkyl, hydroxy-substituted C1-C6 alkyl, C1-C6 alkoxy, and -NR^{x}R^{y};
preferably, when W³¹, W³², W³³, and W³⁴ are simultaneously present, W³¹, W³², W³³, and W³⁴ are each independently selected from the group consisting of hydrogen, halogen, C1-C6 alkyl, hydroxy-substituted C1-C6 alkyl, and C1-C6 alkoxy;
preferably, when W³¹, W³², W³³, and W³⁴ are simultaneously present, among W³¹, W³², W³³, and W³⁴, any one is selected from the group consisting of hydrogen, halogen, hydroxy, C1-C6 alkyl, C1-C6 alkoxy, hydroxy-substituted C1-C6 alkyl, and -NR^{x}R^{y}, and the remainder are hydrogen; or, among W³¹, W³², W³³, and W³⁴, any two are independently selected from the group consisting of hydrogen, halogen, hydroxy, C1-C6 alkyl, and C1-C6 alkoxy, and the remainder are hydrogen;
R^{x} and R^{y} are each independently selected from the group consisting of hydrogen and C1-C6 alkyl; preferably, any one of R^{x} and R^{y} is selected from the group consisting of C1-C6 alkyl, and the other is hydrogen; more preferably, any one of R^{x} and R^{y} is methyl, and the other is hydrogen;
preferably, when W³¹, W³², W³³, and W³⁴ are simultaneously present, among W³¹, W³², W³³, and W³⁴, any one is selected from the group consisting of hydrogen, halogen, C1-C6 alkyl, C1-C6 alkoxy, and hydroxy-substituted C1-C6 alkyl, and the remainder are hydrogen; or, among W³¹, W³², W³³, and W³⁴, any two are selected from the group consisting of hydrogen, halogen, C1-C6 alkyl, and C1-C6 alkoxy, and the remainder are hydrogen;
more preferably, when W³¹, W³², W³³, and W³⁴ are simultaneously present, among W³¹, W³², W³³, and W³⁴, any one is selected from the group consisting of hydrogen, fluoro, hydroxy, methyl, ethyl, methoxy, ethoxy, 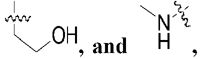 and the remainder are hydrogen; or, among W³¹, W³², W³³, and W³⁴, any two are selected from the group consisting of hydrogen, fluoro, chloro, hydroxy, methyl, and methoxy, and the remainder are hydrogen;
more preferably, when W³¹, W³², W³³, and W³⁴ are simultaneously present, among W³¹, W³², W³³, and W³⁴, any one is selected from the group consisting of hydrogen, fluoro, methyl, ethyl, methoxy, and 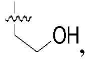 and the remainder are hydrogen; or, among W³¹, W³¹, W³³, and W³⁴, any two are selected from the group consisting of hydrogen, fluoro, chloro, methyl, and methoxy, and the remainder are hydrogen;
when W³¹, W³², and W³³ are simultaneously present but W³⁴ is absent, W³¹, W³², and W³³ are each independently selected from the group consisting of hydrogen, C1-C6 alkyl, and C1-C6 alkoxy;
preferably, wheₙ W³¹, W³², and W³³ are simultaneously present but W³⁴ is absent, among W³¹, W³², and W³³, any one is selected from the group consisting of hydrogen, C1-C6 alkyl, and C1-C6 alkoxy, and the remainder are hydrogen;
more preferably, wheₙ W³¹, W³², and W³³ are simultaneously present but W³⁴ is absent, among W³¹, W³², and W³³, any one is selected from the group consisting of hydrogen, methyl, and methoxy, and the remainder are hydrogen;
preferably, R^{1'} is selected from the group consisting of 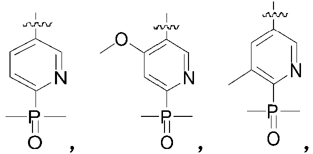 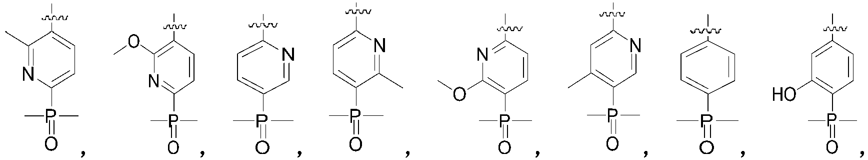 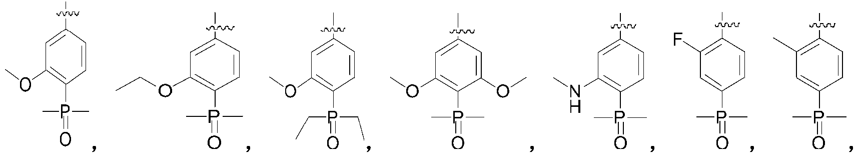 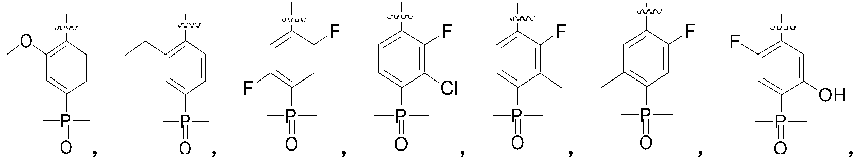 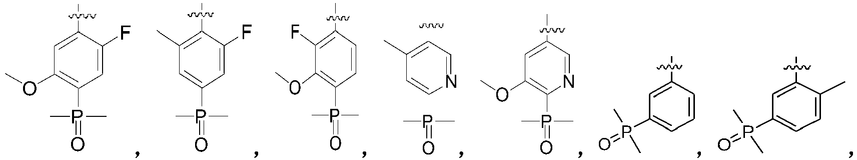 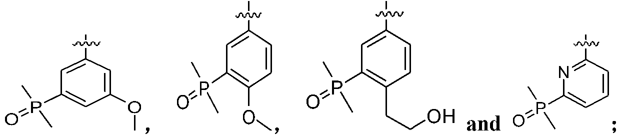
most preferably, R^{1'} is selected from the group consisting of 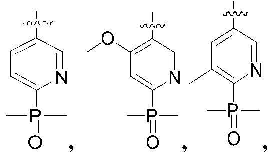 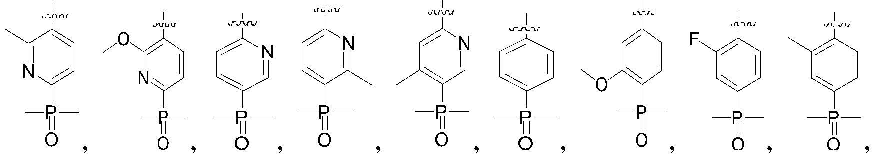 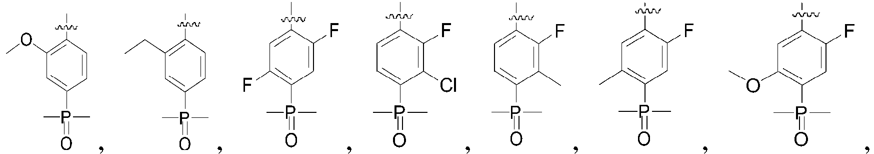 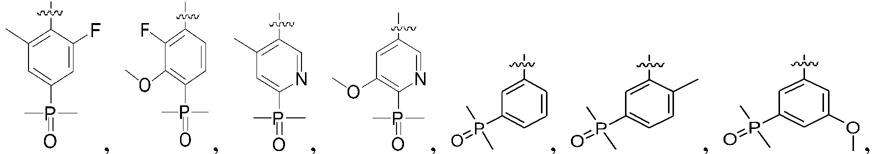 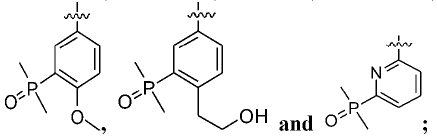
R² is selected from the group consisting of hydrogen, C1-C6 alkyl, C3-C6 cycloalkyl, and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 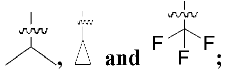
R³ is selected from the group consisting of:
   1) 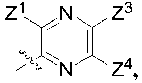 wherein:
      Z¹, Z³, and Z⁴ are each independently selected from the group consisting of hydrogen, cyano, halogen, nitro, and C1-C6 alkyl;
      preferably, Z¹, Z³, and Z⁴ are each independently selected from the group consisting of hydrogen and C1-C6 alkyl;
      more preferably, among Z¹, Z³, and Z⁴, any one is selected from the group consisting of C1-C6 alkyl, and the remainder are hydrogen;
      further preferably, among Z¹, Z³, and Z⁴, any one is methyl, and the remainder are hydrogen;
      most preferably, 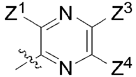 as a **whole,** is 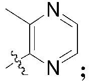
   2) 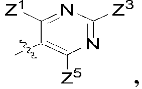 wherein:
      Z¹, Z³, and Z⁵ are each independently selected from the group consisting of hydrogen, cyano, halogen, nitro, and C1-C6 alkyl;
      preferably, Z¹, Z³, and Z⁵ are each independently selected from the group consisting of hydrogen and C1-C6 alkyl;
      more preferably, among Z¹, Z³, and Z⁵, any two are independently selected from the group consisting of C1-C6 alkyl, and the remainder is hydrogen;
      more preferably, Z¹, Z³, and Z⁵ are each independently selected from the group consisting of hydrogen and methyl;
      further preferably, among Z¹, Z³, and Z⁵, any two are methyl, and the remainder is hydrogen;
      most preferably, 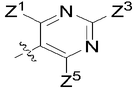 as a **whole,** is 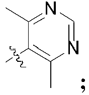
   3) 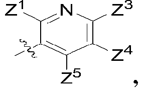 wherein:
      Z¹, Z³, Z⁴, and Z⁵ are each independently selected from the group consisting of hydrogen, nitro, cyano, halogen, and C1-C6 alkyl;
      preferably, Z¹, Z³, Z⁴, and Z⁵ are each independently selected from the group consisting of hydrogen and C1-C6 alkyl;
      more preferably, among Z¹, Z³, Z⁴, and Z⁵, any one is selected from the group consisting of hydrogen and C1-C6 alkyl, and the remainder are hydrogen;
      more preferably, Z¹, Z³, Z⁴, and Z⁵ are each independently selected from the group consisting of hydrogen and methyl;
      further preferably, among Z¹, Z³, Z⁴, and Z⁵, any one is selected from the group consisting of hydrogen and methyl, and the remainder are hydrogen;
      most preferably, 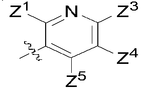 as a whole, is selected from the group consisting of 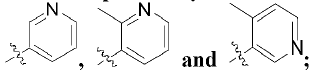
      and 4) 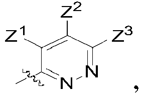 wherein:
         Z¹, Z², and Z³ are each independently selected from the group consisting of hydrogen, cyano, halogen, nitro, and C1-C6 alkyl;
         preferably, Z¹, Z², and Z³ are each independently selected from the group consisting of hydrogen and C1-C6 alkyl;
         more preferably, among Z¹, Z², and Z³, any one is selected from the group consisting of C1-C6 alkyl, and the remainder are hydrogen;
         further preferably, among Z¹, Z², and Z³, any one is methyl, and the remainder are hydrogen;
         most preferably, 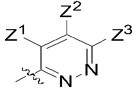 as a **whole,** is 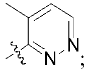
         preferably, R³ is selected from the group consisting of:
            1) 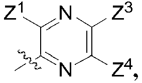 wherein:
               Z¹, Z³, and Z⁴ are each independently selected from the group consisting of hydrogen, cyano, halogen, nitro, and C1-C6 alkyl;
               preferably, Z¹, Z³, and Z⁴ are each independently selected from the group consisting of hydrogen and C1-C6 alkyl;
               more preferably, among Z¹, Z³, and Z⁴, any one is selected from the group consisting of C1-C6 alkyl, and the remainder are hydrogen;
               further preferably, among Z¹, Z³, and Z⁴, any one is methyl, and the remainder are hydrogen;
               most preferably, 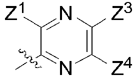 as a **whole,** is 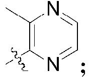
            and 2) 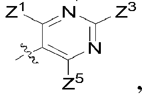 wherein:
               Z¹, Z³, and Z⁵ are each independently selected from the group consisting of hydrogen, cyano, halogen, nitro, and C1-C6 alkyl;
               preferably, Z¹, Z³, and Z⁵ are each independently selected from the group consisting of hydrogen and C1-C6 alkyl;
               more preferably, among Z¹, Z³, and Z⁵, any two are selected from the group consisting of C1-C6 alkyl, and the remainder is hydrogen;
               more preferably, Z¹, Z³, and Z⁵ are each independently selected from the group consisting of hydrogen and methyl;
               further preferably, among Z¹, Z³, and Z⁵, any two are methyl, and the remainder is hydrogen;
               most preferably, 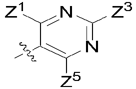 as a **whole,** is 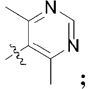
               preferably, R³ is selected from the group consisting of 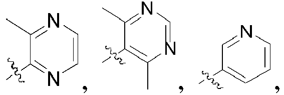 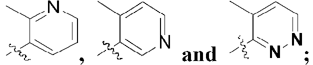
               most preferably, R³ is selected from the group consisting of

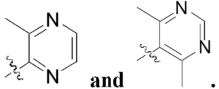

In some embodiments, the compound has the structure of formula III, 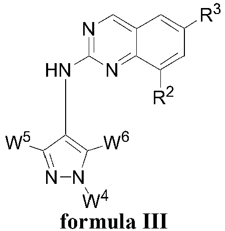 wherein:
W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, O=S(R^{e}R^{f})=N-C1-C6 alkylene, R^{e}R^{d}N-C(=O)-C1-C6 alkylene, C3-C6 cycloalkyl-SO₂-, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene and 8- to 10-membered saturated bridged heterocyclyl, and the 3-to 7-membered saturated heterocyclyl, the 5- to 6-membered heteroaryl, and the 8- to 10-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 3- to 6-membered saturated heterocyclyl, and the C1-C6 alkylene is optionally substituted by hydroxy;
preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{e}R^{d}N-C(=O)-C1-C6 alkylene, C3-C6 cycloalkyl-SO₂-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, wherein the 3- to 7-membered saturated heterocyclyl and the 8-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl, and the C1-C6 alkylene is optionally substituted by hydroxy;
more preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{e}R^{d}N-C(=O)-C1-C6 alkylene, C3-C6 cycloalkyl-SO₂-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, wherein the 3- to 7-membered saturated heterocyclyl and the 8-membered saturated bridged heterocyclyl are each independently optionally substituted by 1 or 2 (preferably 1) substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl (preferably 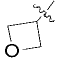 ), the C1-C6 alkylene is optionally substituted by hydroxy, the 3- to 7-membered saturated heterocyclyl is selected from the group consisting of 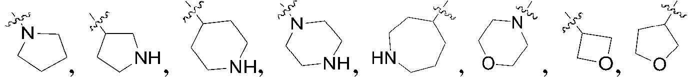 and 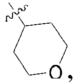 the 5- to 6-membered heteroaryl is selected from the group consisting of 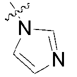 and 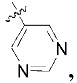 and the 8-membered bridged heterocyclyl is 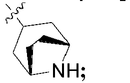 R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 3- to 6-membered saturated heterocyclyl; or, R^{c}, R^{d}, and the N atom to which they are both attached together form a 3- to 6-membered saturated heterocyclic ring, wherein the 3- to 6-membered saturated heterocyclic ring is optionally substituted by 1 or 2 substituent(s) selected from the group consisting of C1-C6 alkyl and halogen;
preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 5-membered saturated heterocyclyl; or, R^{c}, R^{d}, and the N atom to which they are both attached together form a 4- to 5-membered saturated heterocyclic ring, wherein the 4- to 5-membered saturated heterocyclic ring is optionally substituted by 1 or 2 substituent(s) selected from the group consisting of C1-C6 alkyl and halogen;
more preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl, and 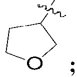 or, R^{c}, R^{d}, and the N atom to which they are both attached together form 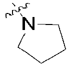 or 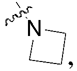 wherein the 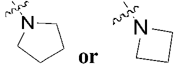 is independently optionally substituted by 1 or 2 substituents selected from the group consisting of C1-C6 alkyl and halogen;
most preferably, R^{c} and R^{d} are each independently selected from the group consisting of hydrogen, methyl, ethyl, isopropyl, 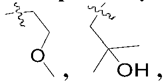 acetyl, 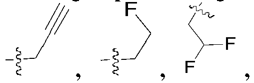 cyclopropyl, cyclobutyl and 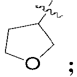 or, R^{c}, R^{d}, and the N atom to which they are both attached together form 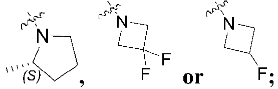
R^{e} and R^{f} are each independently selected from the group consisting of hydrogen and C1-C6 alkyl;
preferably, R^{e} and R^{f} are each independently selected from the group consisting of C1-C6 alkyl;
more preferably, R^{e} and R^{f} are methyl;
further preferably, W⁴ is selected from the group consisting of hydrogen, methyl, ethyl, difluoromethyl, trideuteromethyl, 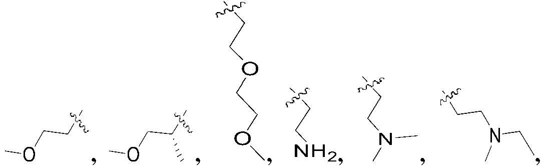 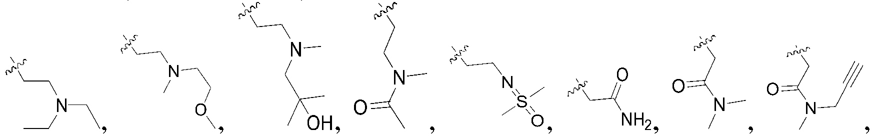 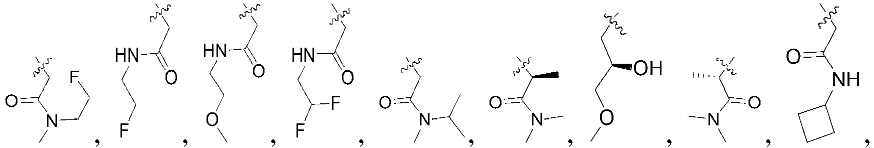 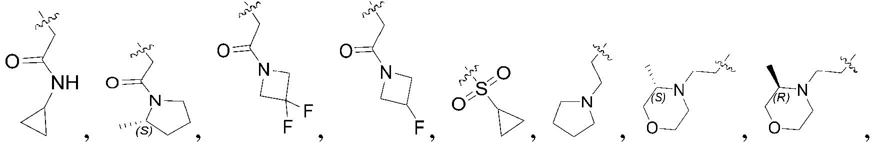 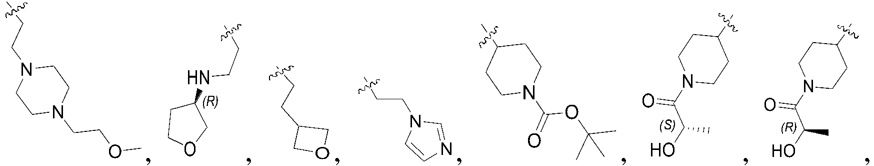 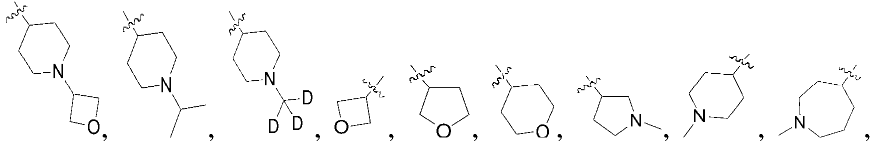 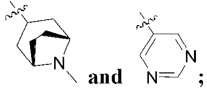
most preferably, W⁴ is selected from the group consisting of hydrogen, methyl, ethyl, difluoromethyl, trideuteromethyl, 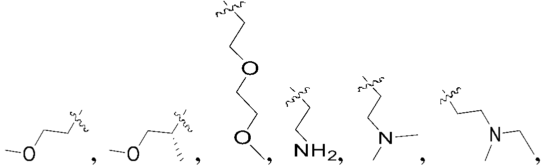 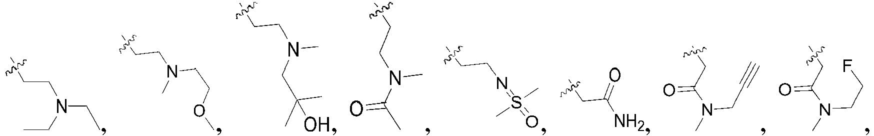 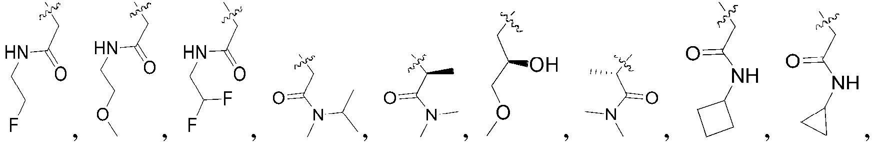 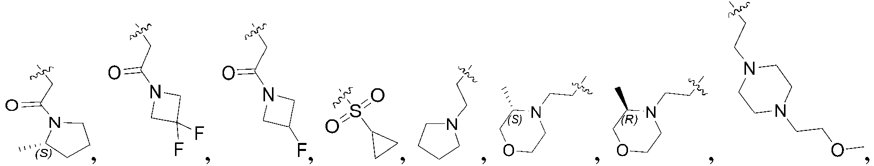 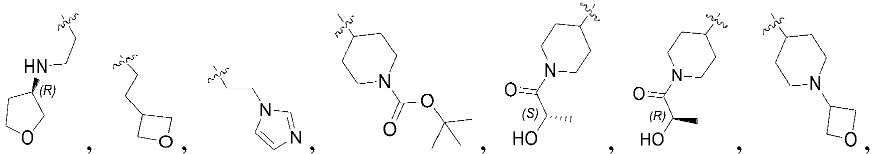 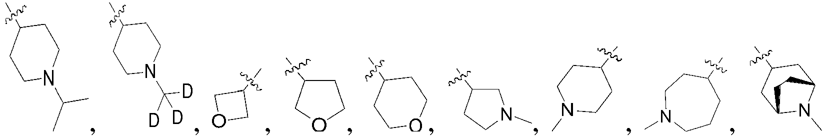 and 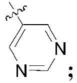
W⁵ and W⁶ are each independently selected from the group consisting of hydrogen, halogen, C1-C6 alkyl, C1-C6 alkoxy and C1-C6 haloalkyl, wherein W⁵ and W⁶ are not hydrogen at the same time;
preferably, any one of W⁵ and W⁶ is selected from the group consisting of hydrogen, halogen, C1-C6 alkyl, C1-C6 alkoxy, and C1-C6 haloalkyl, and the other is hydrogen;
preferably, any one of W⁵ and W⁶ is selected from the group consisting of halogen, C1-C6 alkyl, C1-C6 alkoxy, and C1-C6 haloalkyl, and the other is hydrogen;
more preferably, any one of W⁵ and W⁶ is selected from the group consisting of hydrogen, chloro, methyl, ethyl, methoxy, difluoromethyl, and trifluoromethyl, and the other is hydrogen;
more preferably, any one of W⁵ and W⁶ is selected from the group consisting of chloro, methyl, ethyl, methoxy, difluoromethyl, and trifluoromethyl, and the other is hydrogen;
further preferably, 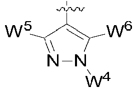 as a whole, is selected from the group consisting of 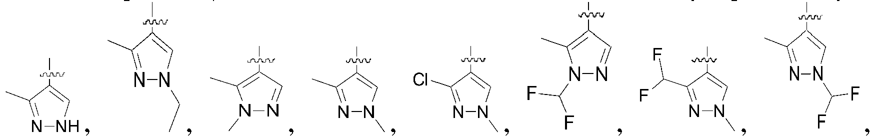 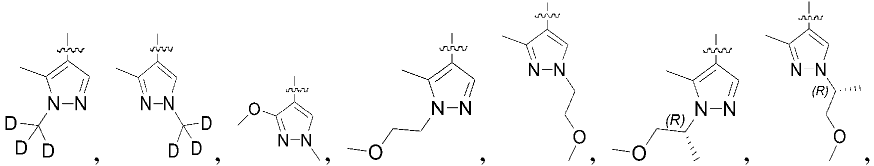 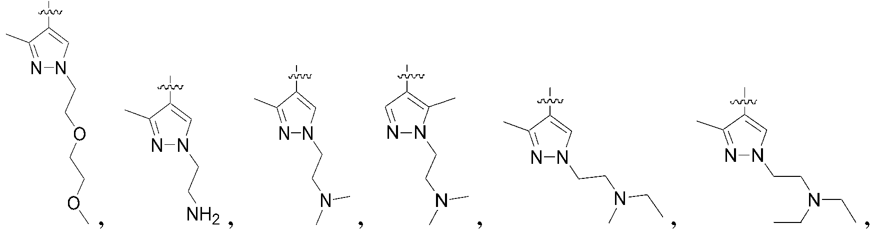 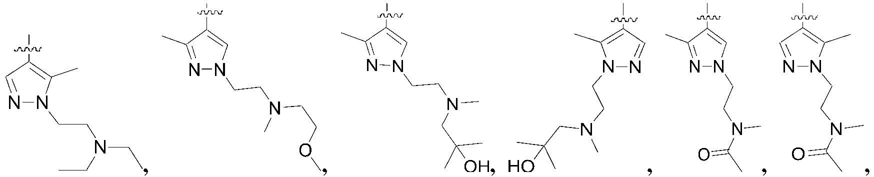 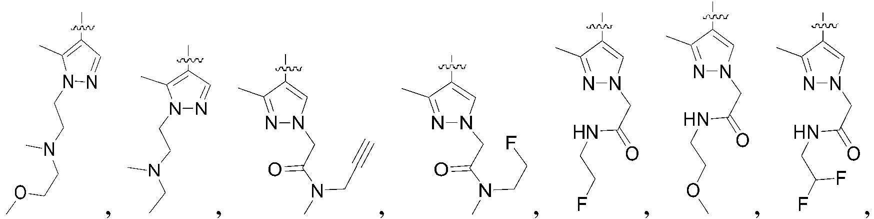 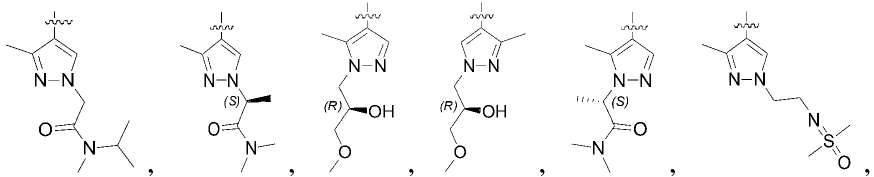 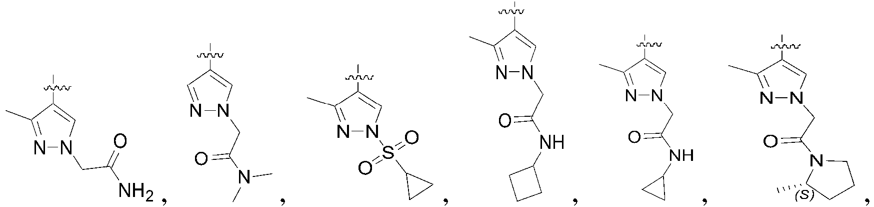 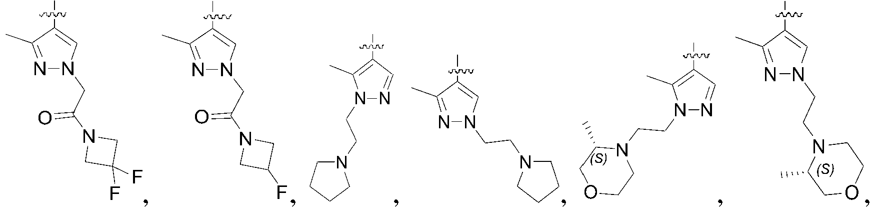 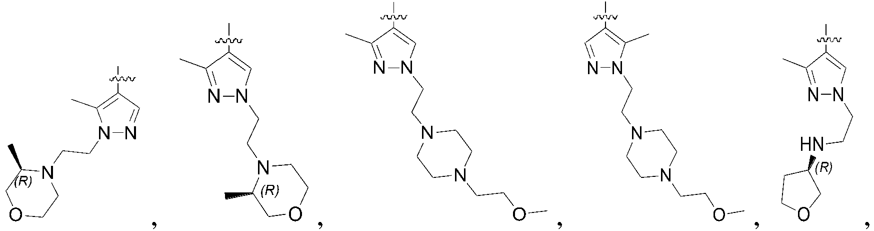 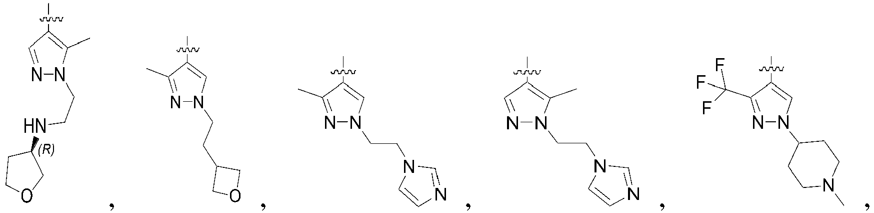 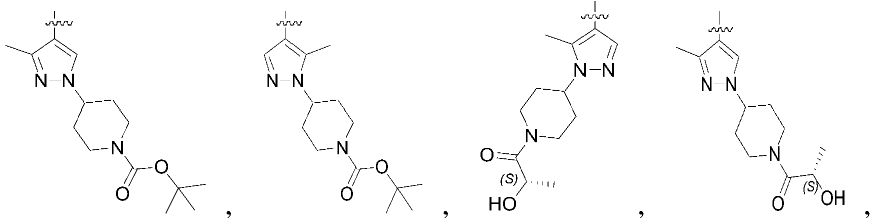 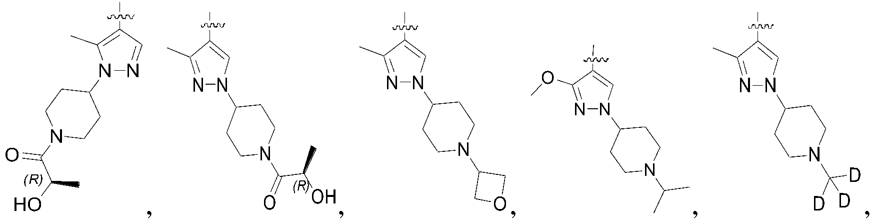 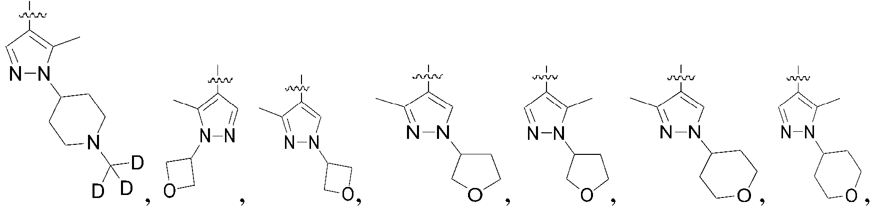 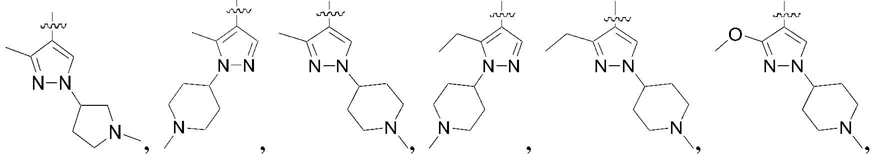 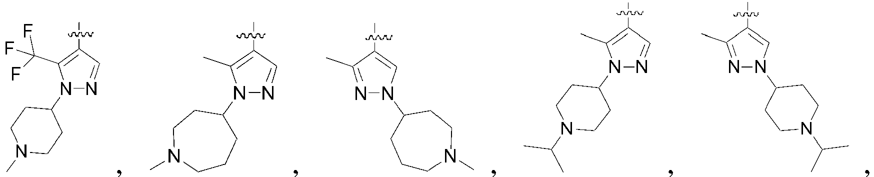 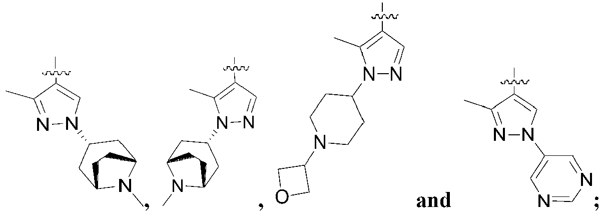
most preferably, 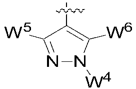 as a whole, is selected from the group consisting of 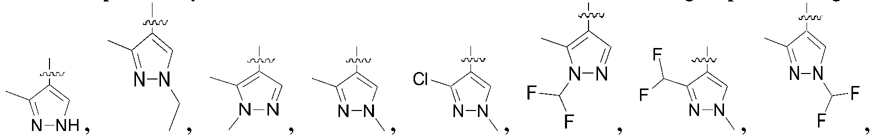 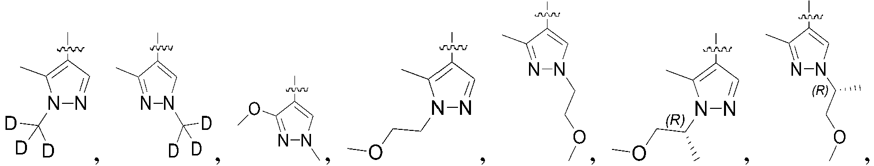 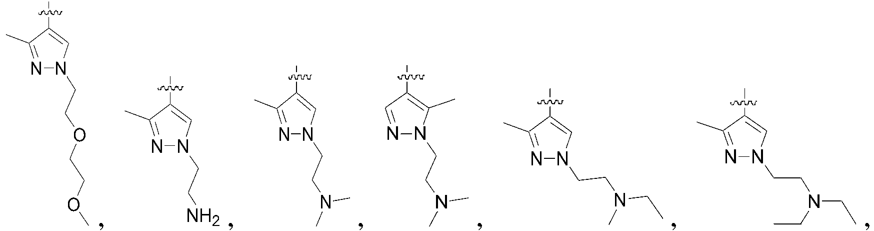 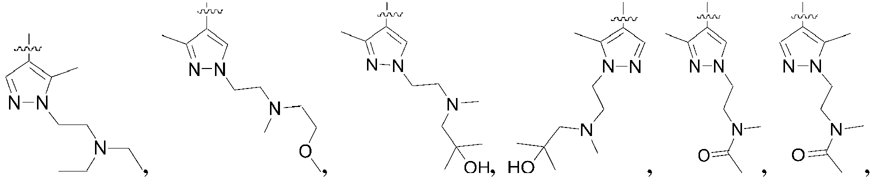 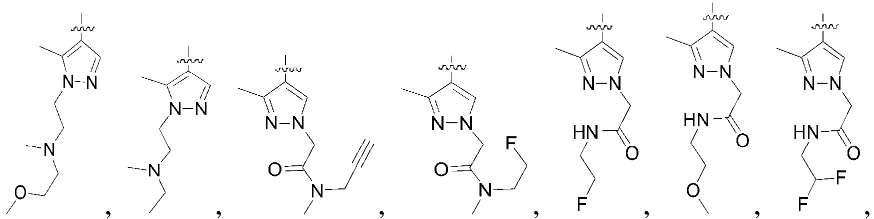 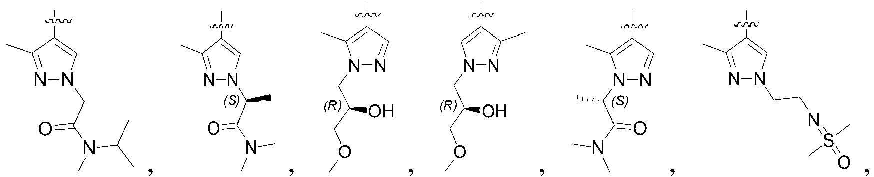 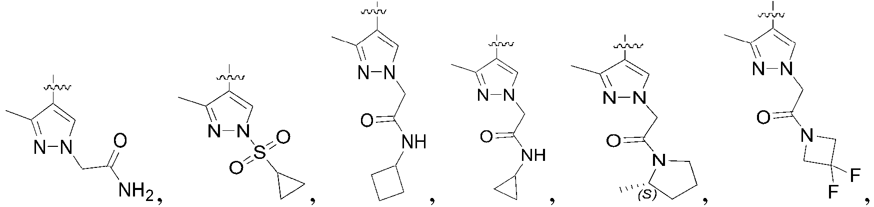 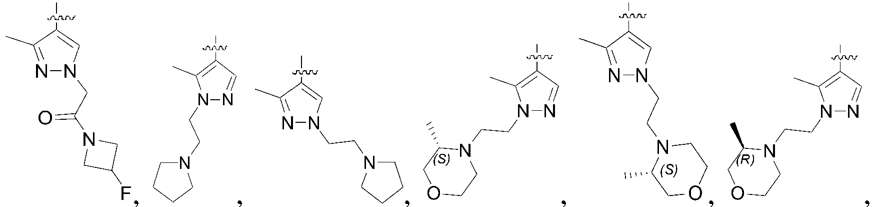 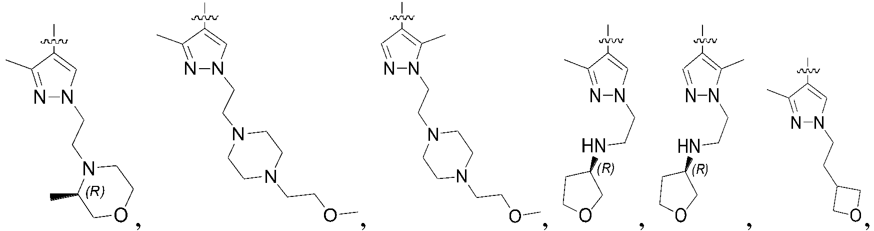 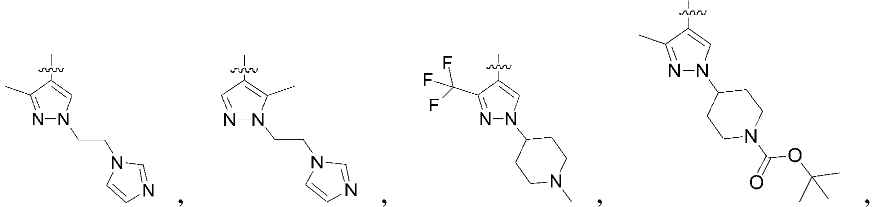 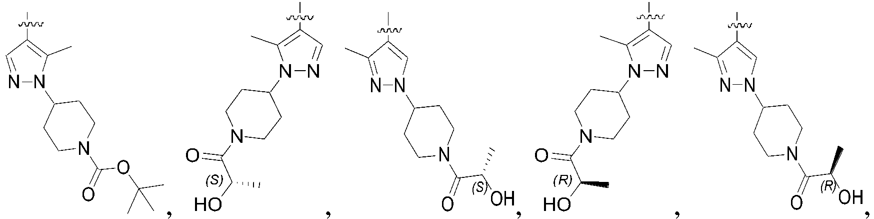 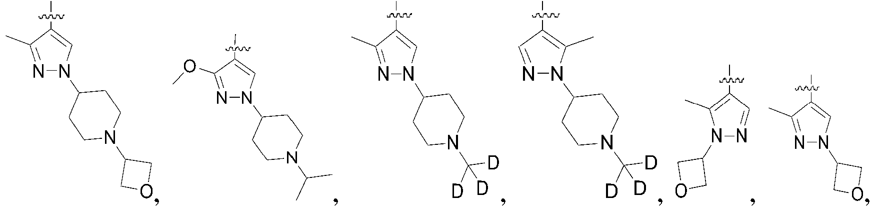 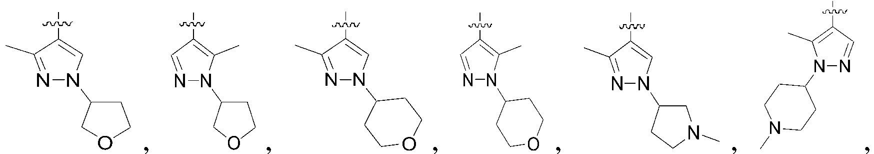 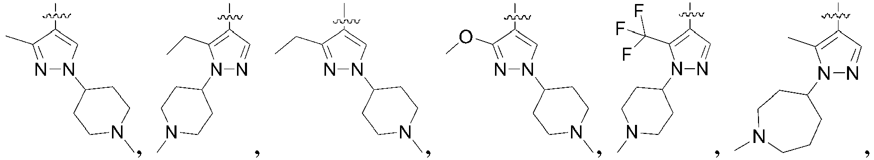 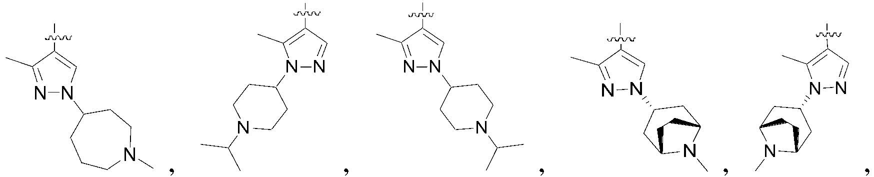 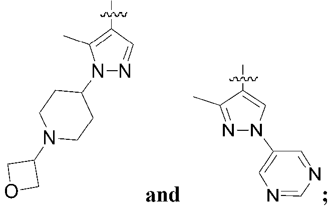 R² is selected from the group consisting of hydrogen, C1-C6 alkyl, C3-C6 cycloalkyl, and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 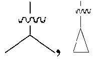 and trifluoromethyl;
R³ is selected from the group consisting of:
   1) 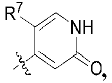 wherein:
      R⁷ is selected from the group consisting of hydrogen and C1-C6 alkyl;
      preferably, R⁷ is selected from the group consisting of hydrogen and methyl;
   2) 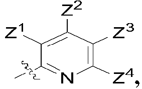 wherein:
      Z¹, Z², Z³, and Z⁴ are each independently selected from the group consisting of hydrogen, cyano, halogen, nitro, and C1-C6 alkyl;
      preferably, Z¹, Z², Z³, and Z⁴ are each independently selected from the group consisting of hydrogen and C1-C6 alkyl;
      more preferably, among Z¹, Z², Z³, and Z⁴, any one is selected from the group consisting of C1-C6 alkyl, and the remainder are hydrogen;
      further preferably, among Z¹, Z², Z³, and Z⁴, any one is methyl, and the remainder are hydrogen;
      most preferably, 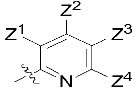 as a whole, is 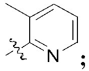
   3) 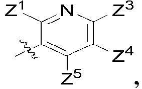 wherein:
      Z¹, Z³, Z⁴, and Z⁵ are each independently selected from the group consisting of hydrogen, halogen, nitro, cyano, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
      preferably, Z¹, Z³, Z⁴, and Z⁵ are each independently selected from the group consisting of hydrogen, halogen, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
      more preferably, among Z¹, Z³, Z⁴, and Z⁵, any one is selected from the group consisting of C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl, and the remainder are hydrogen; or, among Z¹, Z³, Z⁴, and Z⁵, any two are selected from the group consisting of halogen, C1-C6 alkyl, and R^{a}R^{b}N-C(=O)-, wherein R^{a} and R^{b} are each independently selected from the group consisting of hydrogen and C1-C6 alkyl, and the remainder are hydrogen;
      more preferably, Z¹, Z³, Z⁴, and Z⁵ are each independently selected from the group consisting of hydrogen, fluoro, methyl, difluoromethyl, cyclopropyl, 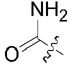 and 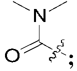
      further preferably, among Z¹, Z³, Z⁴, and Z⁵, any one is selected from the group consisting of methyl, 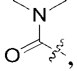 cyclopropyl, and difluoromethyl, and the remainder are hydrogen; or, among Z¹, Z³, Z⁴, and Z⁵, any two are independently selected from the group consisting of fluoro, methyl and 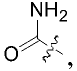 and the remainder are hydrogen;
      most preferably, 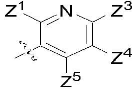 as a whole, is selected from the group consisting of 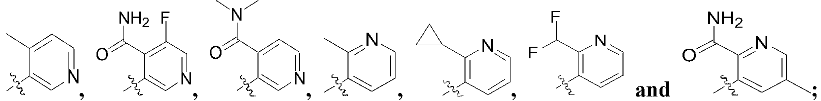
   4) 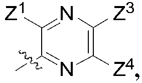 wherein:
      Z¹, Z³, and Z⁴ are each independently selected from the group consisting of hydrogen, cyano, halogen, nitro, and C1-C6 alkyl;
      preferably, Z¹, Z³, and Z⁴ are each independently selected from the group consisting of hydrogen and C1-C6 alkyl;
      more preferably, among Z¹, Z³, and Z⁴, any one is selected from the group consisting of C1-C6 alkyl, and the remainder are hydrogen;
      further preferably, among Z¹, Z³, and Z⁴, any one is methyl, and the remainder are hydrogen;
      most preferably, 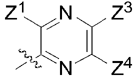 as a whole, is 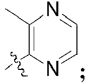
   5) 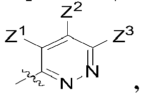 wherein:
      Z¹, Z², and Z³ are each independently selected from the group consisting of hydrogen, cyano, halogen, nitro, and C1-C6 alkyl;
      preferably, Z¹, Z², and Z³ are each independently selected from the group consisting of hydrogen and C1-C6 alkyl;
      more preferably, among Z¹, Z², and Z³, any one is selected from the group consisting of C1-C6 alkyl, and the remainder are hydrogen;
      further preferably, among Z¹, Z², and Z³, any one is methyl, and the remainder are hydrogen;
      most preferably, 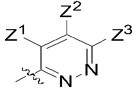 as a whole, is 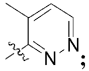
   6) 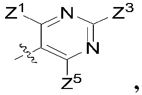 wherein:
      Z¹, Z³, and Z⁵ are each independently selected from the group consisting of hydrogen, cyano, halogen, nitro, and C1-C6 alkyl;
      preferably, Z¹, Z³, and Z⁵ are each independently selected from the group consisting of hydrogen and C1-C6 alkyl;
      more preferably, among Z¹, Z³, and Z⁵, any one is selected from the group consisting of C1-C6 alkyl, and the remainder are hydrogen; or, among Z¹, Z³, and Z⁵, any two are selected from the group consisting of C1-C6 alkyl, and the remainder is hydrogen;
      more preferably, Z¹, Z³, and Z⁵ are each independently selected from the group consisting of hydrogen and methyl;
      further preferably, among Z¹, Z³, and Z⁵, any one is methyl and the remainder are hydrogen; or, among Z¹, Z³, and Z⁵, any two are methyl and the remainder is hydrogen;
      most preferably, 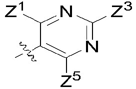 as a whole, is selected from the group consisting of 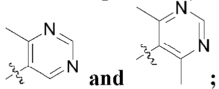
   7) 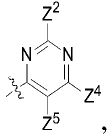 wherein:
      Z², Z⁴, and Z⁵ are each independently selected from the group consisting of hydrogen, cyano, halogen, nitro, and C1-C6 alkyl;
      preferably, Z², Z⁴, and Z⁵ are each independently selected from the group consisting of hydrogen and C1-C6 alkyl;
      more preferably, among Z², Z⁴, and Z⁵, any one is selected from the group consisting of C1-C6 alkyl, and the remainder are hydrogen;
      further preferably, among Z², Z⁴, and Z⁵, any one is methyl, and the remainder are hydrogen;
      most preferably, 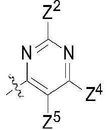 as a whole, is 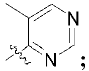
   8) 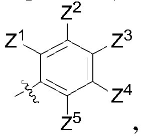 wherein:
      Z¹, Z², Z³, Z⁴, and Z⁵ are each independently selected from the group consisting of hydrogen, cyano, halogen, nitro, and C1-C6 alkyl;
      preferably, Z¹, Z², Z³, Z⁴, and Z⁵ are each independently selected from the group consisting of hydrogen, cyano, and C1-C6 alkyl;
      more preferably, among Z¹, Z², Z³, Z⁴, and Z⁵, any two are independently selected from the group consisting of cyano and C1-C6 alkyl, and the remainder are hydrogen;
      further preferably, among Z¹, Z², Z³, Z⁴, and Z⁵, any two are independently selected from the group consisting of cyano and methyl, and the remainder are hydrogen;
      most preferably, 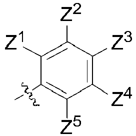 as a whole, is 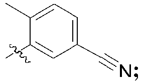
      and 9) 5- to 9-membered heteroaryl, wherein the 5- to 9-membered heteroaryl is optionally substituted by 1 or 2 substituent(s) selected from the group consisting of C1-C6 alkyl and cyano; preferably, the 5- to 9-membered heteroaryl is selected from the group consisting of 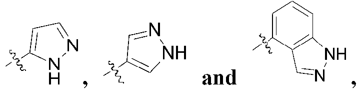 wherein the 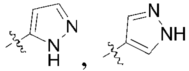 and 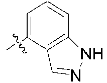 are each independently optionally substituted by 1 or 2 substituent(s) selected from the group consisting of C1-C6 alkyl and cyano; more preferably, the 5- to 9-membered heteroaryl selected from the group consisting of 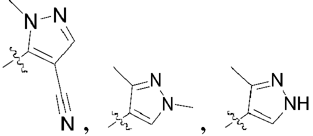 and 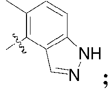
      preferably, R³ is selected from the group consisting of 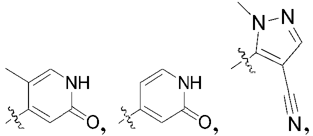 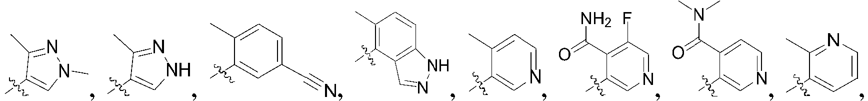 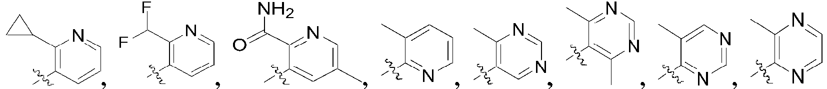 and

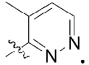

In some embodiments, the compound has the structure of formula III-1, 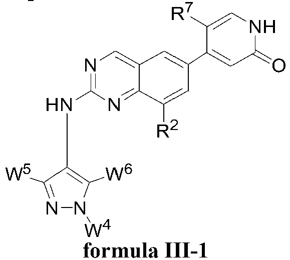 wherein:
W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, R^{e}R^{d}N-C(=O)-C1-C6 alkylene, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene, and 8- to 10-membered saturated bridged heterocyclyl, wherein the 3- to 7-membered saturated heterocyclyl, the 5-to 6-membered heteroaryl, and the 8- to 10-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 3- to 6-membered saturated heterocyclyl, and the C1-C6 alkylene is optionally substituted by hydroxy;
preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, R^{e}R^{d}N-C(=O)-C1-C6 alkylene, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene, and 8-membered saturated bridged heterocyclyl, wherein the 3- to 7-membered saturated heterocyclyl and the 8-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl, and the C1-C6 alkylene is optionally substituted by hydroxy;
more preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, R^{e}R^{d}N-C(=O)-C1-C6 alkylene, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, and the 3- to 7-membered saturated heterocyclyl, the 8-membered saturated bridged heterocyclyl are each independently optionally substituted by 1 or 2 (preferably 1) substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl (preferably 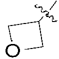 ), the C1-C6 alkylene is optionally substituted by hydroxy, the 3- to 7-membered saturated heterocyclyl is selected from the group consisting 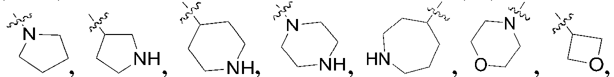 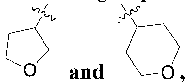 the 5- to 6-membered heteroaryl is selected from the group consisting of 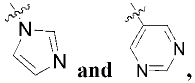 and the 8-membered bridged heterocyclyl is 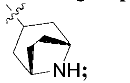
R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 3- to 6-membered saturated heterocyclyl; or, R^{c}, R^{d}, and the N atom to which they are both attached together form a 3- to 6-membered saturated heterocyclic ring, wherein the 3- to 6-membered saturated heterocyclic ring is optionally substituted by 1 or 2 substituent(s) selected from the group consisting of C1-C6 alkyl and halogen;
preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 5-membered saturated heterocyclyl; or, R^{c}, R^{d}, and the N atom to which they are both attached together form a 4- to 5-membered saturated heterocyclic ring, wherein the 4- to 5-membered saturated heterocyclic ring is optionally substituted by 1 or 2 substituent(s) selected from the group consisting of C1-C6 alkyl and halogen;
more preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O) -, C2-C6 alkynyl-C1-C6 alkylene, C1-C6 haloalkyl and 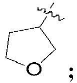 or, R^{c}, R^{d}, and the N atom to which they are both attached together form 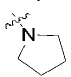 or 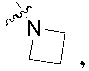 and the 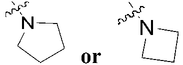 is independently optionally substituted by 1 or 2 substituent(s) selected from the group consisting of C1-C6 alkyl and halogen;
most preferably, R^{c}, R^{d} are each independently selected from the group consisting of hydrogen, methyl, ethyl, isopropyl, 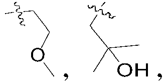 acetyl, 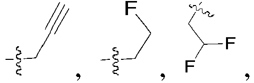 cyclopropyl, cyclobutyl and 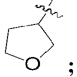 or, R^{c}, R^{d}, and the N atom to which they are both attached together form 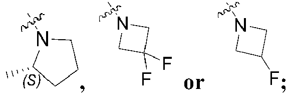
most preferably, W⁴ is selected from the group consisting of hydrogen, methyl, ethyl, difluoromethyl, trideuteromethyl, 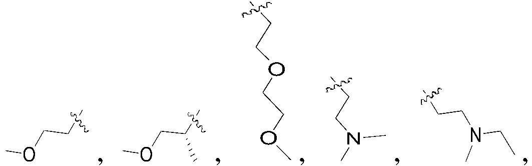 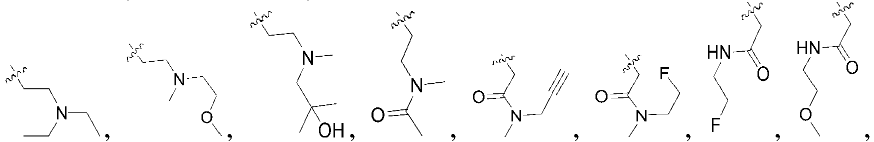 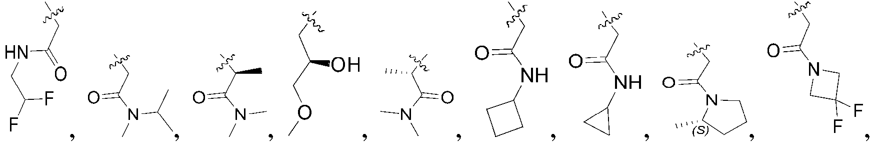 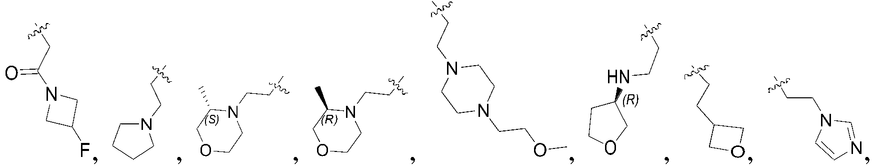 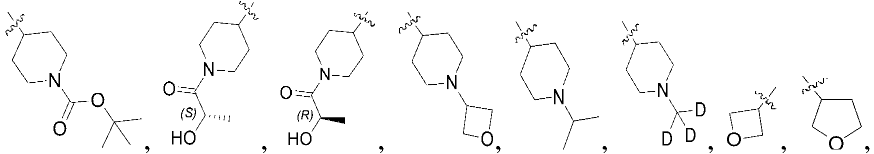 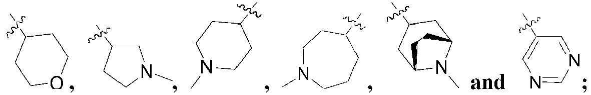
W⁵ and W⁶ are each independently selected from the group consisting of hydrogen, halogen, C1-C6 alkyl, C1-C6 alkoxy, and C1-C6 haloalkyl, wherein W⁵ and W⁶ are not hydrogen at the same time;
preferably, any one of W⁵ and W⁶ is selected from the group consisting of halogen, C1-C6 alkyl, C1-C6 alkoxy, and C1-C6 haloalkyl, and the other is hydrogen;
more preferably, any one of W⁵ and W⁶ is selected from the group consisting of chloro, methyl, ethyl, methoxy, difluoromethyl, and trifluoromethyl, and the other is hydrogen;
most preferably, 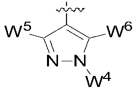 as a whole, is selected from the group consisting of 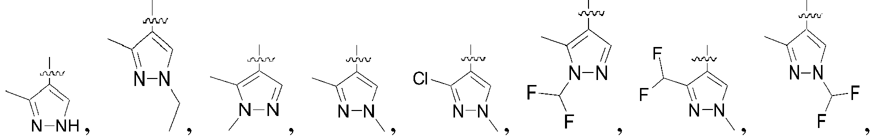 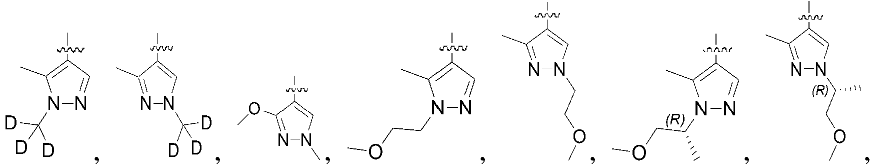 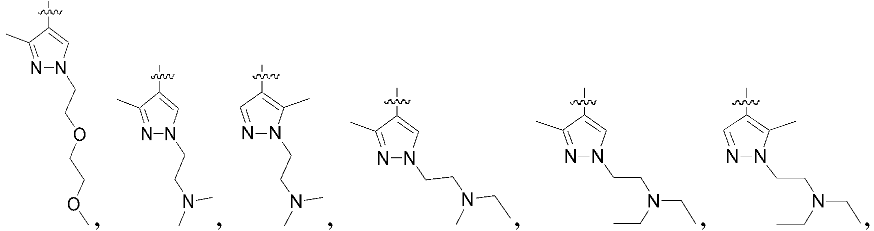 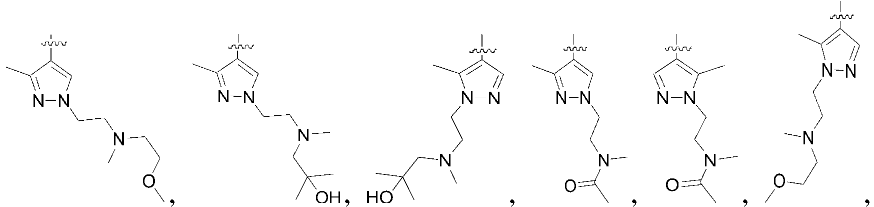 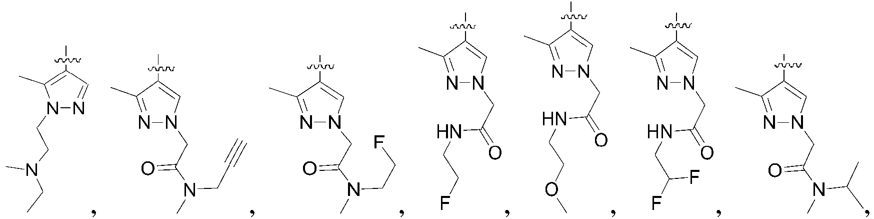 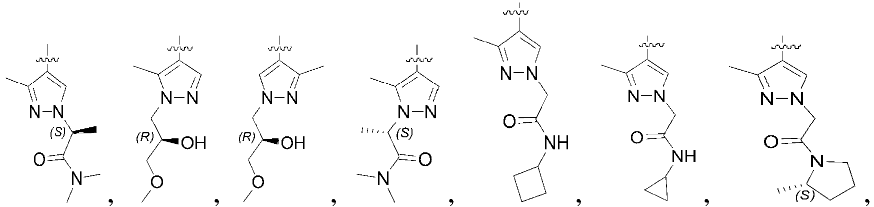 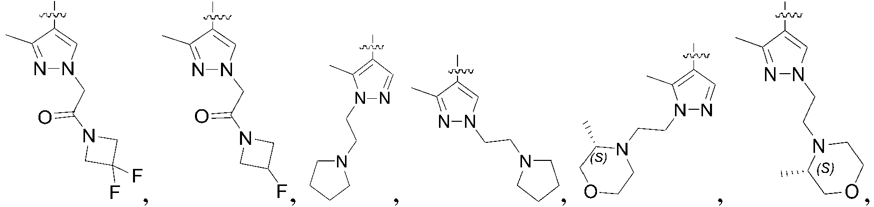 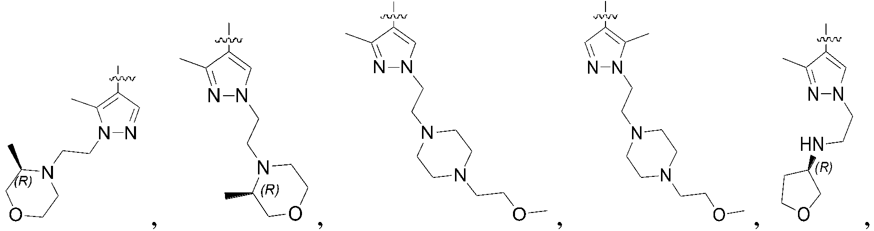 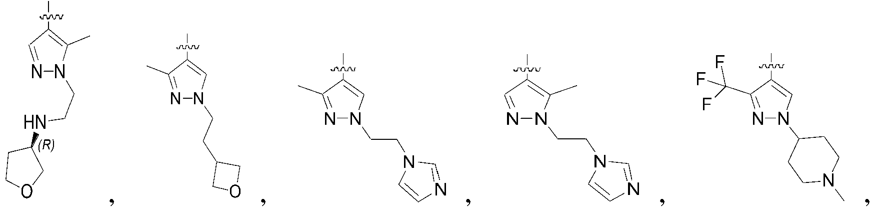 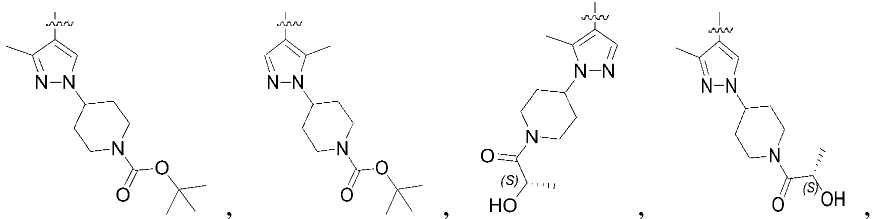 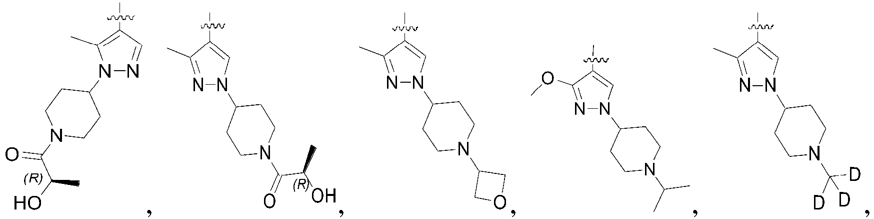 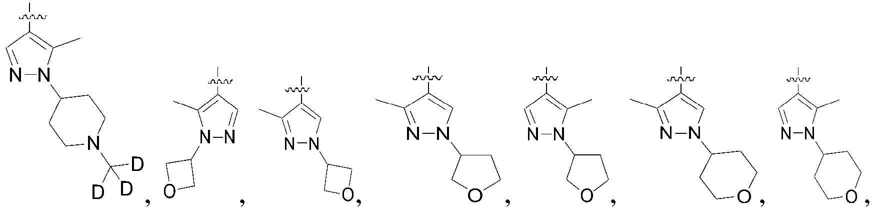 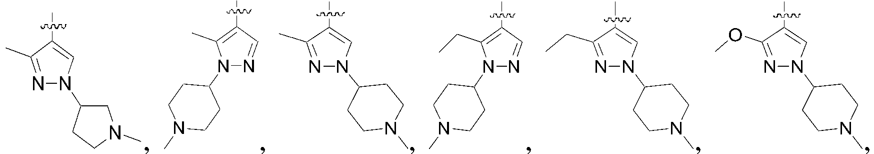 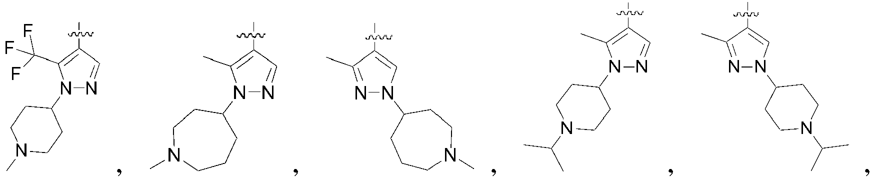 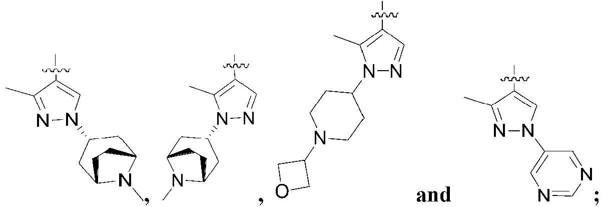
R² is selected from the group consisting of hydrogen, C1-C6 alkyl, C3-C6 cycloalkyl, and C1-C6 haloalkyl;
preferably, R² is selected from the group consisting of C1-C6 alkyl and C3-C6 cycloalkyl;
more preferably, R² is selected from the group consisting of methyl, ethyl, 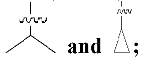
R⁷ is selected from the group consisting of hydrogen and C1-C6 alkyl;
preferably, R⁷ is selected from the group consisting of hydrogen and methyl.

In some embodiments, the compound has the structure of formula III-2, 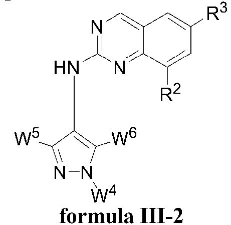 wherein:
W⁴ is selected from the group consisting of C1-C6 alkyl, deuterated C1-C6 alkyl, C1-C6 alkoxy-C1-C6 alkylene, 3- to 6-membered saturated heterocyclyl, O=S(R^{e}R^{f})=N-C1-C6 alkylene, R^{c}R^{d}N-C1-C6 alkylene, R^{e}R^{d}N-C(=O)-C1-C6 alkylene, and C3-C6 cycloalkyl-SO₂-, wherein the 3- to 6-membered saturated heterocyclyl is optionally substituted by C1-C6 alkyl;
preferably, W⁴ is selected from the group consisting of C1-C6 alkyl, deuterated C1-C6 alkyl, C1-C6 alkoxy-C1-C6 alkylene, 4- to 6-membered saturated heterocyclyl, O=S(R^{e}R^{f})=N-C1-C6 alkylene, R^{c}R^{d}N-C1-C6 alkylene, R^{e}R^{d}N-C(=O)-C1-C6 alkylene, and C3-C6 cycloalkyl-SO₂-, wherein the 4- to 6-membered saturated heterocyclyl is optionally substituted by C1-C6 alkyl;
more preferably, W⁴ is selected from the group consisting of C1-C6 alkyl, deuterated C1-C6 alkyl, C1-C6 alkoxy-C1-C6 alkylene, 4- to 6-membered saturated heterocyclyl, O=S(R^{e}R^{f})=N-C1-C6 alkylene, R^{c}R^{d}N-C1-C6 alkylene, R^{e}R^{d}N-C(=O)-C1-C6 alkylene, and C3-C6 cycloalkyl-SO₂-, wherein the 4- to 6-membered saturated heterocyclyl is optionally substituted by C1-C6 alkyl, and the 4- to 6-membered saturated heterocyclyl is selected from the group consisting of 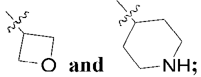
R^{c} and R^{d} are each independently selected from the group consisting of hydrogen, C1-C6 alkyl, and C1-C6 alkoxy-C1-C6 alkylene;
preferably, R^{c} and R^{d} are each independently selected from the group consisting of hydrogen, methyl, ethyl and 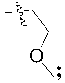
R^{e} and R^{f} are each independently selected from the group consisting of hydrogen and C1-C6 alkyl;
preferably, R^{e} and R^{f} are each independently selected from the group consisting of C1-C6 alkyl;
more preferably, R^{e} and R^{f} are methyl;
further preferably, W⁴ is selected from the group consisting of methyl, trideuteromethyl, 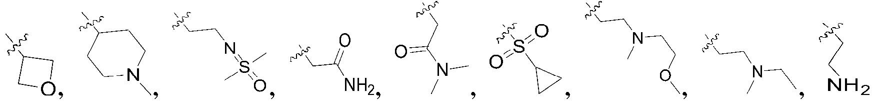 and 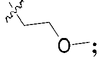
most preferably, W⁴ is selected from the group consisting of methyl, trideuteromethyl, 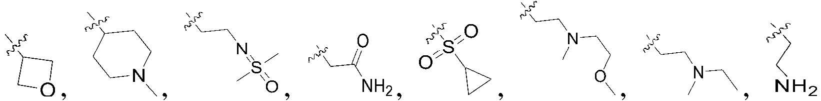 and 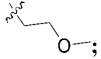
W⁵, W⁶ are each independently selected from the group consisting of hydrogen, halogen, C1-C6 alkyl and C1-C6 haloalkyl, and W⁵ and W⁶ are not hydrogen at the same time;
preferably, any one of W⁵ and W⁶ is selected from the group consisting of hydrogen, halogen, C1-C6 alkyl, and C1-C6 haloalkyl, and the other is hydrogen;
preferably, any one of W⁵ and W⁶ is selected from the group consisting of halogen, C1-C6 alkyl, and C1-C6 haloalkyl, and the other is hydrogen;
more preferably, any one of W⁵ and W⁶ is selected from the group consisting of hydrogen, chloro, methyl, and difluoromethyl, and the other is hydrogen;
more preferably, any one of W⁵ and W⁶ is selected from the group consisting of chloro, methyl, and difluoromethyl, and the other is hydrogen;
further preferably, 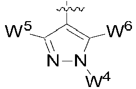 as a whole, is selected from the group consisting of 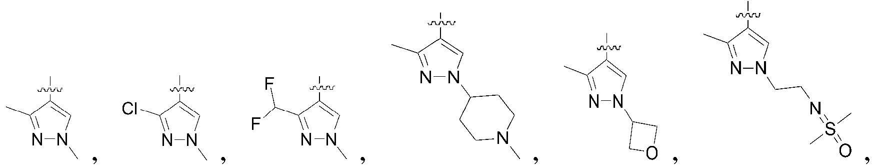 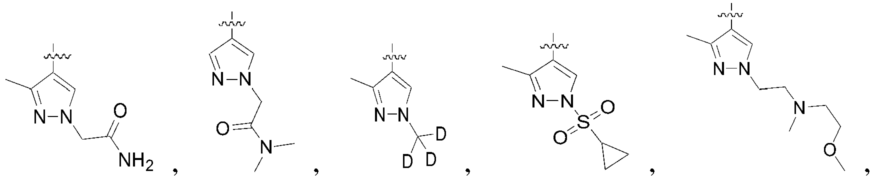 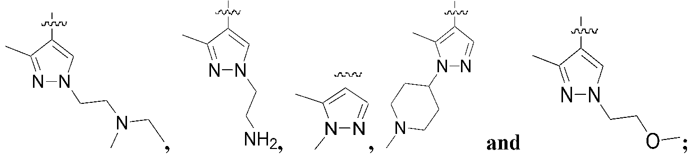
most preferably, 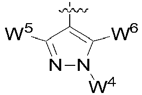 as a whole, is selected from the group consisting of 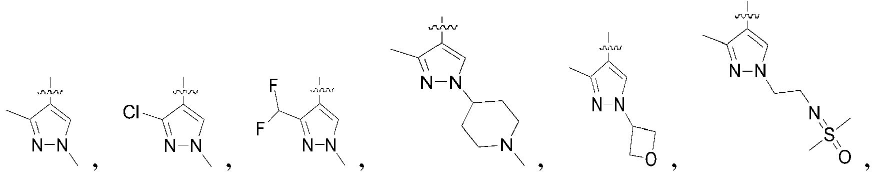 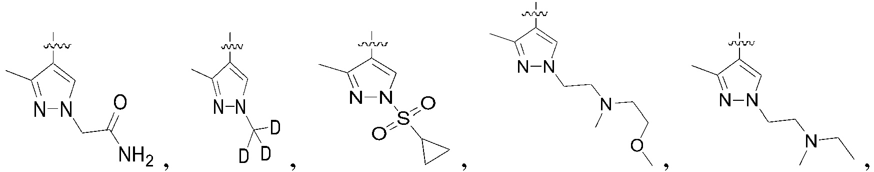 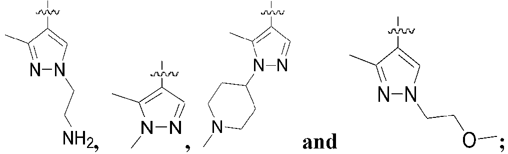
R² is selected from the group consisting of hydrogen, C1-C6 alkyl, C3-C6 cycloalkyl, and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 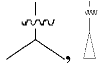 and trifluoromethyl;
R³ is selected from the group consisting of:
   1) 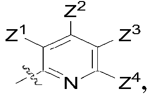 wherein:
      Z¹, Z², Z³, and Z⁴ are each independently selected from the group consisting of hydrogen, cyano, halogen, nitro, and C1-C6 alkyl;
      preferably, Z¹, Z², Z³, and Z⁴ are each independently selected from the group consisting of hydrogen and C1-C6 alkyl;
      more preferably, among Z¹, Z², Z³, and Z⁴, any one is selected from the group consisting of C1-C6 alkyl, and the remainder are hydrogen;
      further preferably, among Z¹, Z², Z³, and Z⁴, any one is methyl, and the remainder are hydrogen;
      most preferably, 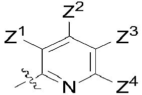 as a whole, is 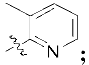
   2) 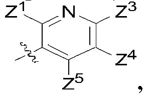 wherein:
      Z¹, Z³, Z⁴, and Z⁵ are each independently selected from the group consisting of hydrogen, halogen, nitro, cyano, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
      preferably, Z¹, Z³, Z⁴, and Z⁵ are each independently selected from the group consisting of hydrogen, halogen, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
      more preferably, among Z¹, Z³, Z⁴, and Z⁵, any one is selected from the group consisting of C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl, and the remainder are hydrogen; or, among Z¹, Z³, Z⁴, and Z⁵, any two are selected from the group consisting of halogen, C1-C6 alkyl, and R^{a}R^{b}N-C(=O)-, wherein R^{a} and R^{b} are each independently selected from the group consisting of hydrogen and C1-C6 alkyl, and the remainder are hydrogen;
      more preferably, Z¹, Z³, Z⁴, and Z⁵ are each independently selected from the group consisting of hydrogen, fluoro, methyl, difluoromethyl, cyclopropyl, 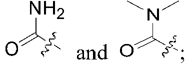
      further preferably, among Z¹, Z³, Z⁴, and Z⁵, any one is selected from the group consisting of methyl, 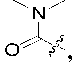 cyclopropyl, and difluoromethyl, and the remainder are hydrogen; or, among Z¹, Z³, Z⁴, and Z⁵, any two are selected from the group consisting of fluoro, methyl and 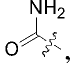 and the remainder are hydrogen;
      most preferably, 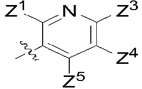 as a whole, is selected from the group consisting of 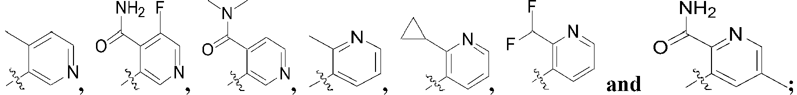
   3) 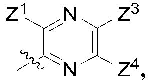 wherein:
      Z¹, Z³, and Z⁴ are each independently selected from the group consisting of hydrogen, cyano, halogen, nitro, and C1-C6 alkyl;
      preferably, Z¹, Z³, and Z⁴ are each independently selected from the group consisting of hydrogen and C1-C6 alkyl;
      more preferably, among Z¹, Z³, and Z⁴, any one is selected from the group consisting of C1-C6 alkyl, and the remainder are hydrogen;
      further preferably, among Z¹, Z³, and Z⁴, any one is methyl, and the remainder are hydrogen;
      most preferably, 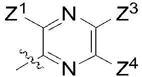 as a **whole,** is 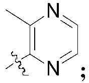
   4) 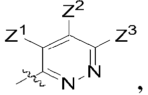 wherein:
      Z¹, Z², and Z³ are each independently selected from the group consisting of hydrogen, cyano, halogen, nitro, and C1-C6 alkyl;
      preferably, Z¹, Z², and Z³ are each independently selected from the group consisting of hydrogen and C1-C6 alkyl;
      more preferably, among Z¹, Z², and Z³, any one is selected from the group consisting of C1-C6 alkyl, and the remainder are hydrogen;
      further preferably, among Z¹, Z², and Z³, any one is methyl, and the remainder are hydrogen;
      most preferably, 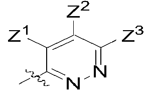 as a **whole,** is 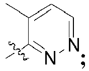
   5) 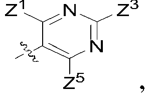 wherein:
      Z¹, Z³, and Z⁵ are each independently selected from the group consisting of hydrogen, cyano, halogen, nitro, and C1-C6 alkyl;
      preferably, Z¹, Z³, and Z⁵ are each independently selected from the group consisting of hydrogen and C1-C6 alkyl;
      more preferably, among Z¹, Z³, and Z⁵, any one is selected from the group consisting of C1-C6 alkyl, and the remainder are hydrogen; or, among Z¹, Z³, and Z⁵, any two are selected from the group consisting of C1-C6 alkyl, and the remainder is hydrogen;
      more preferably, Z¹, Z³, and Z⁵ are each independently selected from the group consisting of hydrogen and methyl.
      further preferably, among Z¹, Z³, and Z⁵, any one is methyl, and the remainder are hydrogen; or, among Z¹, Z³, and Z⁵, any two are methyl, and the remainder is hydrogen;
      most preferably, 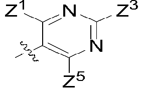 as a whole, is selected from the group consisting of 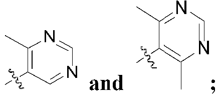
   6) 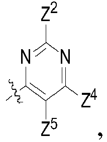 wherein:
      Z², Z⁴, and Z⁵ are each independently selected from the group consisting of hydrogen, cyano, halogen, nitro, and C1-C6 alkyl;
      preferably, Z², Z⁴, and Z⁵ are each independently selected from the group consisting of hydrogen and C1-C6 alkyl;
      more preferably, among Z², Z⁴, and Z⁷, any one is selected from the group consisting of C1-C6 alkyl, and the remainder are hydrogen;
      further preferably, among Z², Z⁴, and Z⁵, any one is methyl, and the remainder are hydrogen;
      most preferably, 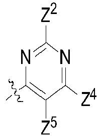 as a whole, is 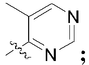
   7) 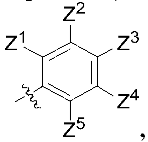 wherein:
      Z¹, Z², Z³, Z⁴, and Z⁵ are each independently selected from the group consisting of hydrogen, cyano, halogen, nitro, and C1-C6 alkyl;
      preferably, Z¹, Z², Z³, Z⁴, and Z⁵ are each independently selected from the group consisting of hydrogen, cyano, and C1-C6 alkyl;
      more preferably, among Z¹, Z², Z³, Z⁴, and Z⁵, any two are independently selected from the group consisting of cyano and C1-C6 alkyl, and the remainder are hydrogen;
      further preferably, among Z¹, Z², Z³, Z⁴, and Z⁵, any two are selected from the group consisting of cyano and methyl, and the remainder are hydrogen;
      most preferably, 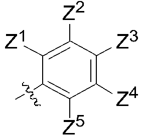 as a whole, is 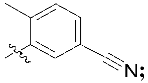
   and 8) 5- to 9-membered heteroaryl, and the 5- to 9-membered heteroaryl is optionally substituted by 1 or 2 substituent(s) selected from the group consisting of C1-C6 alkyl and cyano; preferably, the 5- to 9-membered heteroaryl is selected from the group consisting of 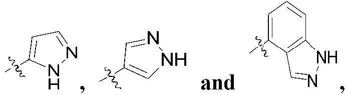 and the 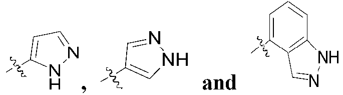 are each independently optionally substituted by 1 or 2 substituent(s) selected from the group consisting of C1-C6 alkyl and cyano; more preferably, the 5- to 9-membered heteroaryl is selected from the group consisting of 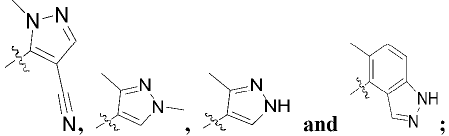
   preferably, R³ is selected from the group consisting of

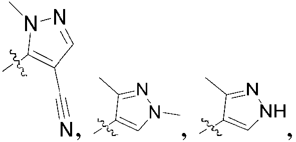

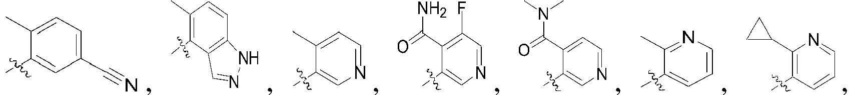

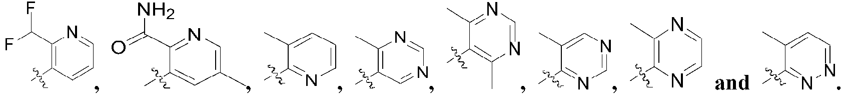

In the second aspect of the present invention, the present invention provides a method for preparing the compound according to any of the above technical solutions, comprising the following steps: 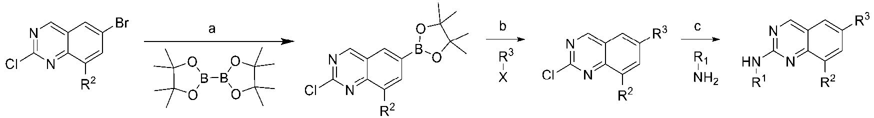 or 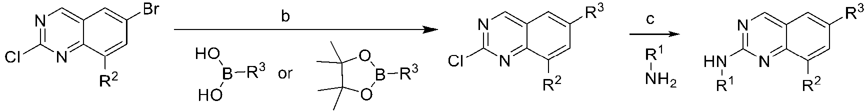 or 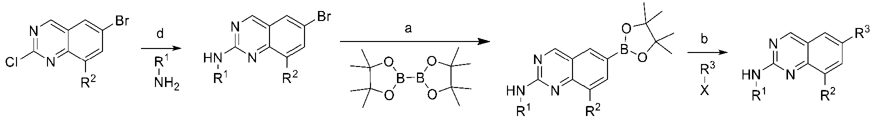 or 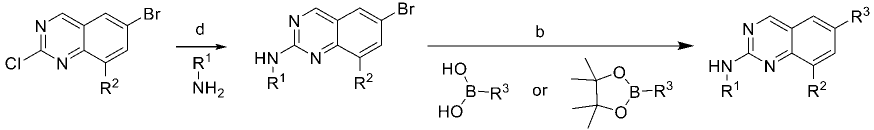 wherein X is halogen, and R¹, R², and R³ are as defined above,
reaction conditions are as follows:
   (a) a palladium-catalyzed coupling reaction;
   (b) a palladium-catalyzed coupling reaction;
   (c) a palladium-catalyzed coupling reaction, a nucleophilic substitution reaction under acidic conditions, or a nucleophilic substitution reaction under basic conditions;
   (d) a nucleophilic substitution reaction under acidic conditions, or a nucleophilic substitution reaction under basic conditions;
the palladium catalyst is selected from the group consisting of palladium acetate, tetrakis(triphenylphosphine)palladium, bis(triphenylphosphine)palladium(II) dichloride, [1,1'-bis(diphenylphosphino)ferrocene]palladium(II) dichloride, and tris(dibenzylideneacetone)dipalladium;
the basic conditions refer to conditions in the presence of any of the following: triethylamine, diisopropylethylamine, pyridine, sodium bicarbonate, sodium carbonate, potassium carbonate, cesium carbonate, lithium hydroxide, sodium hydroxide, potassium hydroxide, sodium hydride, or potassium hydride;
the acidic conditions refer to conditions in the presence of any of the following: acetic acid, trifluoroacetic acid, hydrochloric acid, methanesulfonic acid, p-toluenesulfonic acid, or camphorsulfonic acid.

In the third aspect of the present invention, the present invention provides a pharmaceutical composition comprising the aforementioned compound or a stereoisomer thereof, a prodrug thereof, a pharmaceutically acceptable salt thereof, or a pharmaceutically acceptable solvate thereof, and optionally a pharmaceutically acceptable excipient.

In the fourth aspect of the present invention, the present invention provides use of the aforementioned compound, or a stereoisomer thereof, a prodrug thereof, a pharmaceutically acceptable salt thereof, or a pharmaceutically acceptable solvate thereof, or the aforementioned pharmaceutical composition, in the manufacture of a medicament for preventing and/or treating a BRK kinase-mediated disease.

In the fifth aspect of the present invention, the present invention provides the aforementioned compound, or a stereoisomer thereof, a prodrug thereof, a pharmaceutically acceptable salt thereof, or a pharmaceutically acceptable solvate thereof, or the aforementioned pharmaceutical composition, for use in preventing and/or treating a BRK kinase-mediated disease.

In the sixth aspect of the present invention, the present invention provides a method for preventing and/or treating a BRK kinase-mediated disease, comprising administering to a subject in need thereof an effective amount of the aforementioned compound, or a stereoisomer thereof, a prodrug thereof, a pharmaceutically acceptable salt thereof, or a pharmaceutically acceptable solvate thereof, or the aforementioned pharmaceutical composition.

In some embodiments, the BRK kinase-mediated disease is selected from the group consisting of breast cancer, gastric cancer, leukemia, skin cancer, head and neck cancer, thyroid cancer, pancreatic cancer, cervical cancer, bladder cancer, ovarian cancer, nasopharyngeal carcinoma, non-small cell lung cancer, prostate cancer, colorectal cancer, and esophageal cancer.

In some embodiments, the BRK kinase-mediated disease is breast cancer or leukemia.

In some embodiments, the BRK kinase-mediated disease is breast cancer.

In some embodiments, the BRK kinase-mediated disease is gastric cancer.

### DEFINITIONS

It should be understood that the terminology employed herein is intended to describe specific embodiments and is not intended to be limiting. Furthermore, although any methods, devices, and materials similar or equivalent to those described herein can be used in the practice or testing of the present invention, the preferred methods, devices, and materials are described herein.

In the present invention, unless otherwise clearly indicated, the description "...are each independently selected from" used throughout this text may refer to that the specific options expressed between different groups, the same or different symbols do not affect each other, or may indicate that the specific options expressed between the same group, the same or different symbols do not affect each other.

The substituents of the compound of the present invention are disclosed according to the type or scope of the group. It is specifically pointed out that the present invention includes each independent sub-combination of each member of these group types and scopes. For example, the term "C1-C6 alkyl" specifically refers to independently disclosed methyl, ethyl, C3 alkyl, C4 alkyl, C5 alkyl, and C6 alkyl.

The term "C1-C6 alkyl" refers to an alkyl group having 1 to 6 carbon atoms, preferably "C1-C4 alkyl", more preferably "C1-C3 alkyl", and most preferably "C1-C2 alkyl". Examples of "C1-C6 alkyl" include, but are not limited to, methyl, ethyl, propyl (e.g., n-propyl, isopropyl), butyl (e.g., n-butyl, isobutyl, tert-butyl), pentyl (e.g., n-pentyl, isopentyl, neopentyl), and the like. Examples of "C1-C4 alkyl" include, but are not limited to, methyl, ethyl, propyl (e.g., n-propyl, isopropyl), butyl (e.g., n-butyl, isobutyl, tert-butyl), and the like. Examples of "C1-C3 alkyl" include methyl, ethyl, propyl (e.g., n-propyl, isopropyl), and the like. Examples of "C1-C2 alkyl" include methyl, ethyl.

The term "C1-C6 haloalkyl" refers to a group obtained by replacing one or more (e.g., 2, 3, or 4) hydrogen atoms in any of the aforementioned C1-C6 alkyl groups with a halogen atom (preferably fluoro). Examples include monofluoromethyl, difluoromethyl, difluoroethyl, trifluoromethyl, and the like, with trifluoromethyl being preferred.

The term "deuterated C1-C6 alkyl" refers to a group obtained by replacing one or more (e.g., 2, 3, or 4) hydrogen atoms in any of the aforementioned C1-C6 alkyl groups with deuterium. Examples include trideuteromethyl.

The term "C1-C6 alkoxy" refers to any of the aforementioned C1-C6 alkyl groups attached to the rest of the molecule through an oxygen atom (-O-). Examples include methoxy, ethoxy, isopropoxy, and the like.

The term "C1-C6 alkylene" refers to a divalent group obtained by removing one hydrogen atom from any of the aforementioned C1-C6 alkyl groups (e.g., C1-C4 alkyl, C1-C3 alkyl, etc.), e.g., 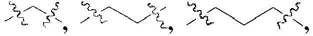 etc.

The term "C2-C6 alkenyl" refers to a straight-chain or branched hydrocarbon group containing 2 to 6 carbon atoms and comprising one or more (preferably one) carbon-carbon double bonds.

The term "C2-C6 alkynyl" refers to a straight-chain or branched hydrocarbon group containing 2 to 6 carbon atoms and comprising one or more (preferably one) carbon-carbon triple bonds.

The term "C3-C7 cycloalkyl" refers to a saturated monocyclic hydrocarbon comprising 3 to 7 carbon atoms, e.g., "C3-C6 cycloalkyl" refers to

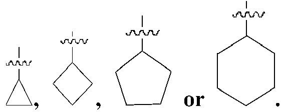

The term "halogen" refers to fluoro (F), chloro (Cl), bromo (Br) or iodo (I).

The term "heteroatom" refers to N, O or S.

The term "heterocyclyl" refers to a 3-, 4-, 5-, 6-, 7-, 8-, 9- or 10-membered (preferably 3- to 7-membered or 3- to 6-membered) saturated or partially unsaturated carbocyclic ring, wherein one or more carbon atoms are replaced by a heteroatom such as nitrogen, oxygen or sulfur. Non-limiting examples of heterocyclyl include, for example, pyran, pyrrolidine, pyrroline, imidazoline, imidazolidine, pyrazolidine, pyrazoline, thiazoline, thiazolidine, dihydrofuran, tetrahydrofuran, 1,3-dioxolane, piperidine, piperazine, morpholine, morpholinyl, pyrrolidinyl, thiomorpholinyl, and the like. For example, "6-membered heterocyclyl" refers to a 6-membered saturated or partially unsaturated carbocyclic ring, wherein one or more carbon atoms are replaced by a heteroatom such as nitrogen, oxygen or sulfur. Non-limiting examples of 6-membered heterocyclyl include, for example, pyran, piperidine, piperazine, morpholine, morpholinyl, thiomorpholinyl, and the like. Further, for example, "5-membered heterocyclyl" refers to a 5-membered saturated or partially unsaturated carbocyclic ring, wherein one or more carbon atoms are replaced by a heteroatom such as nitrogen, oxygen or sulfur. Non-limiting examples of 5-membered heterocyclyl include, for example, pyrrolidine, pyrroline, imidazoline, imidazolidine, pyrazolidine, pyrazoline, thiazoline, thiazolidine, 1,3-dioxolane, and the like. The term "heterocycle" can be understood correspondingly by reference to the aforementioned term "heterocyclyl".

The term "3- to 7-membered saturated heterocyclyl" refers to a group obtained by replacing at least one carbon atom in any of the aforementioned C3-C7 cycloalkyl with a heteroatom (N, O or S), e.g., pyrrolidinyl, morpholinyl, etc.

The term "8- to 10-membered saturated bridged heterocyclyl" refers to a group formed by replacing at least one (preferably one) carbon atom in an 8-, 9- or 10-membered bridged carbocyclic group with a heteroatom such as nitrogen, oxygen, or sulfur. Specific examples include, but are not limited to

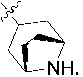

The term "heteroaryl" refers to a 5-, 6-, 7-, 8-, 9- or 10-membered aromatic monocyclic group, bicyclic group, tricyclic group or polycyclic group having at least one heteroatom (N, O or S) in at least one ring, wherein the heteroatom-containing ring optionally further has 1, 2 or 3 heteroatoms selected from N, O or S. For a heteroaryl that is bicyclic, tricyclic or polycyclic, each ring in the bicyclic, tricyclic or polycyclic system must constitute an aromatic system. Non-limiting examples of said heteroaryl are, e.g., pyridyl, pyrazinyl, pyrimidinyl, pyridazinyl, indolyl, imidazolyl, thiazolyl, isothiazolyl, thioxazolyl, pyrrolyl, phenyl-pyrrolyl, furyl, phenyl-furyl, oxazolyl, isoxazolyl, pyrazolyl, thienyl, benzofuryl, benzothienyl, benzimidazolyl, indazolyl, quinolyl, isoquinolyl, etc. The term "heteroaromatic ring" can be understood correspondingly by reference to the aforementioned term "heteroaryl".

From all the above descriptions, it is obvious to those skilled in the art that any group whose name is a composite name, e.g., "C1-C6 alkoxy-C1-C6 alkylene", should refer to the moiety conventionally derived from left to right, e.g., constructed from a "C1-C6 alkylene" substituted by "C1-C6 alkoxy", wherein "C1-C6 alkylene" is as defined above. Specifically, "C1-C6 alkoxy-C1-C6 alkylene" can be, for example, 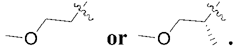 The remaining similar composite groups can be understood by reference to the foregoing.

The term "substituted" means that any one or more hydrogens on a designated atom or group are optionally replaced with a selection from designated groups, provided that the normal valence of the designated atom is not exceeded.

The term "hydroxy-substituted C1-C6 alkyl" refers to a group formed by replacing one or more (preferably one) hydrogen atom(s) in any of the aforementioned C1-C6 alkyl groups with a hydroxy group. Specific examples include, but are not limited to

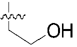

In the present invention, the phrase "the 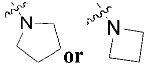 is independently optionally substituted by 1 or 2 groups selected from C1-C6 alkyl and halogen" means that 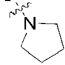 or 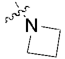 may be unsubstituted or substituted, and if substituted, the substituent(s) on each group may be the same or different. Furthermore, if substituted by 2 substituents, these 2 substituents may be the same or different. Other similar definitions should be understood by reference to the foregoing.

In the present invention, "treating" generally refers to obtaining a desired pharmacological and/or physiological effect. The effect may be prophylactic, in that it completely or partially prevents a disease or its symptoms; and/or therapeutic, in that it partially or completely stabilizes or cures a disease and/or alleviates side effects arising from the disease. As used herein, "treating" encompasses any treatment of a disease in a patient, including: (a) preventing the disease or symptom from occurring in a patient predisposed to the disease or symptom but not yet diagnosed as having it; (b) suppressing the symptoms of the disease, i.e., arresting its development; or (c) relieving the symptoms of the disease, i.e., causing regression of the disease or symptoms.

In the present invention, "subject" refers to a vertebrate. In certain embodiments, the vertebrate is a mammal. Mammals include, but are not limited to, farm animals (such as cattle), pets (such as cats, dogs, and horses), primates, mice and rats. In certain embodiments, the mammal is a human.

In the present invention, "effective amount" refers to an amount effective to achieve a desired therapeutic or prophylactic effect at the necessary dosage and time. A "therapeutically effective amount" of a substance/molecule of the invention may vary depending on factors such as the disease state, age, sex, and weight of the individual and the ability of the substance/molecule to elicit a desired response in the individual. The therapeutically effective amount also encompasses an amount where the therapeutic beneficial effect of the substance/molecule outweighs any toxic or deleterious effects. A "prophylactically effective amount" refers to an amount effective to achieve a desired prophylactic effect at the necessary dosage and time. Typically, but not necessarily, since prophylactic doses are used in subjects prior to the onset of a disease or in the early stages of a disease, the prophylactically effective amount will be lower than the therapeutically effective amount. In the case of cancer, a therapeutically effective amount of a drug may reduce the number of cancer cells; reduce tumor volume; inhibit (i.e., to some extent slow, preferably stop) cancer cell infiltration into surrounding organs; inhibit (i.e., to some extent slow, preferably stop) tumor metastasis; to some extent inhibit tumor growth; and/or to some extent alleviate one or more symptoms associated with cancer.

The pharmaceutical composition of the present invention may comprise a pharmaceutically acceptable excipient, including but not limited to: ion exchangers, alumina, aluminum stearate, lecithin, serum proteins such as human serum albumin, buffering substances such as phosphates, glycerol, sorbic acid, potassium sorbate, a mixture of partial glycerides of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinylpyrrolidone, cellulose-based materials, polyethylene glycol, sodium carboxymethylcellulose, polyacrylates, beeswax, lanolin, and the like.

The pharmaceutical composition of the present invention can be prepared in various forms according to different routes of administration. For example, the pharmaceutical composition may be administered by any of the following routes: oral, spray inhalation, rectal, nasal, buccal, vaginal, topical, parenteral such as subcutaneous, intravenous, intramuscular, intraperitoneal, intrathecal, intraventricular, intrasternal, and intracranial injection or infusion, or via an implanted reservoir. Among these, oral or intravenous administration is preferred.

The compound of the present invention may optionally be used in combination with one or more other active ingredients, wherein the respective dosages and ratios thereof can be adjusted by a person skilled in the art based on the specific disease condition, the specific circumstances of the patient, clinical needs, and the like.

As used herein, unless otherwise specified, the term "prodrug" refers to a derivative of a compound of the present invention that can be hydrolyzed, oxidized, or undergo other reactions under biological conditions (in vitro or in vivo). Prodrugs become active compounds only after undergoing such reactions under biological conditions, or they possess no or only low activity in their unreactive form. Prodrugs can generally be prepared using known methods, e.g., those described in Burger's Medicinal Chemistry and Drug Discovery (1995) 172-178, 949-982 (Edited by Manfred E. Wolff, 5th Edition).

The stereoisomers in the compounds described herein, when specifically designated as (R)- or (S)-isomers by chemical name, should be understood to have the predominant configuration as the (R)-isomer or (S)-isomer, respectively. Any asymmetric carbon atom may be present in the (R)-, (S)-, or (R,S)-configuration, preferably in the (R)- or (S)-configuration.

"Solvated compound" and "solvate" can be used interchangeably, referring to a compound that exists in combination with molecules of a solvent. This combination may include a stoichiometric amount of the solvent, e.g., a monohydrate or dihydrate, or may include any amount of water; for example, methanol or ethanol can form an "alcoholate", which may also be stoichiometric or non-stoichiometric. The term "solvate" as used herein refers to a solid form; that is, a compound in a solution of a solvent, although it may be solvated, is not a solvate as the term is used herein.

As used herein, the term "pharmaceutically acceptable salt" refers to (i) a salt formed from an acidic functional group (e.g., -COOH) present in the compound provided by the present invention and an appropriate inorganic or organic cation (base), and includes, but is not limited to, alkali metal salts such as sodium salt, potassium salt, lithium salt, etc.; alkaline earth metal salts such as calcium salt, magnesium salt, etc.; other metal salts such as aluminum salt, iron salt, zinc salt, copper salt, nickel salt, cobalt salt, etc.; inorganic base salts such as ammonium salt; organic base salts such as tert-octylamine salt, dibenzylamine salt, morpholine salt, glucamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt, tris(hydroxymethyl)aminomethane salt. And, (ii) a salt formed from a basic functional group (e.g., -NH2) present in the compound provided by the present invention and an appropriate inorganic or organic anion (acid), and includes, but is not limited to, hydrohalic acid salts such as hydrofluoric acid salt, hydrochloric acid salt, hydrobromic acid salt, hydroiodic acid salt, etc.; inorganic acid salts such as nitrate salt, perchlorate salt, sulfate salt, phosphate salt, etc.; lower alkylsulfonic acid salts such as methanesulfonic acid salt, trifluoromethanesulfonic acid salt, ethanesulfonic acid salt, etc.; arylsulfonic acid salts such as benzenesulfonic acid salt, p-toluenesulfonic acid salt, etc.; organic acid salts such as acetate salt, malate salt, fumarate salt, succinate salt, citrate salt, tartrate salt, oxalate salt, maleate salt, etc.; amino acid salts such as glycine salt, trimethylglycine salt, arginine salt, ornithine salt, glutamate salt, aspartate salt, etc.

### EXPERIMENTAL SECTION

For the examples discussed below, the compounds of the present invention were synthesized using the methods described herein or other methods well known in the art.

General Purification and Analytical Methods:
Thin-layer chromatography was performed on silica GF254 pre-coated plates (Qingdao Marine Chemical Plant). Column chromatographic separations were performed under medium pressure on silica gel (300-400 mesh, Yantai Zhifu District Huangwu Silica Gel Development Reagent Factory) or using an ISCO Combiflash Rf200 rapid purification system with pre-packed silica gel cartridges (ISCO or Welch). TLC was visualized by UV light (wavelength 254 nm) and by iodine vapor staining. When necessary, compounds were purified by preparative HPLC using a Waters automated purification system (SunFire^{™} C18 column, 5 µm, 19 × 50 mm), comprising an autosampler and fraction collector (2767), a quaternary solvent binary high-pressure gradient pump (2545), a system fluidics organizer (SFO), a 515 make-up pump, a diode array detector (2998), and a QDA quadrupole mass spectrometer, with mobile phase of methanol (containing 0.035% trifluoroacetic acid) and water (containing 0.035% trifluoroacetic acid); or an LC3000 I-type high-performance liquid chromatograph (Beijing Chuangxin Tongheng Chromatography Technology Co., Ltd.), with mobile phase of methanol (containing 0.4% acetic acid) and water (containing 0.4% acetic acid). Mass spectrometry was performed using a Waters LC-MS system with mobile phase of methanol (containing 0.035% trifluoroacetic acid) and water (containing 0.035% trifluoroacetic acid).

¹H-NMR spectra were recorded on a Bruker Avance 600 MHz or Agilent 400 MHz nuclear magnetic resonance spectrometer. Chemical shifts (δ) are reported in parts per million (ppm) and coupling constants (J) are in Hz. The tetramethylsilane signal was used as a reference (δ = 0 ppm). The following abbreviations are used for peak splitting: s = singlet; br. s. = broad signal; d = doublet; t = triplet; m = multiplet; dd = doublet of doublets.

Unless otherwise stated, all final compounds are homogeneous (purity not less than 95%), as determined by high-performance liquid chromatography (HPLC).

Reagent purification was performed with reference to Purification of Laboratory Chemicals (Perrin, D. D., Armarego, W. L. F. and Perrin, D. R.; Pergamon Press: Oxford, 1980). Petroleum ether is the 60-90°C fraction; ethyl acetate, methanol, and dichloromethane are of analytical grade.

### SPECIFIC EMBODIMENTS

The following specific examples are used to describe the embodiments of the present invention in detail, but they shall in no way be construed as limiting the present invention.

Compounds can be classified into the following major categories: 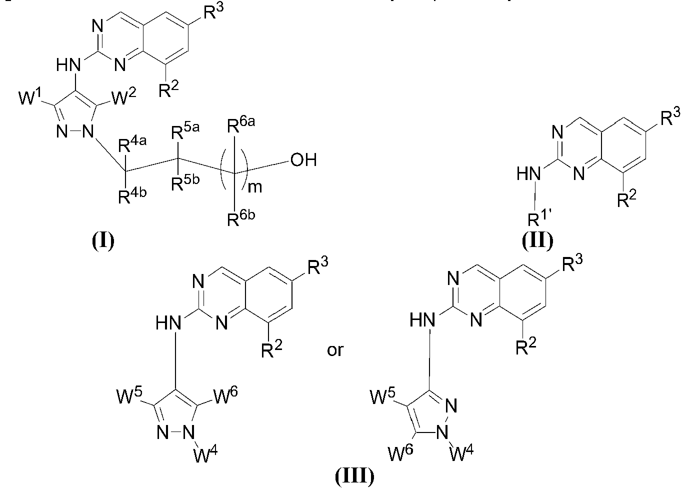 wherein W¹, W², R^{4a}, R^{4b}, R^{5a}, R^{5h}, R^{6a}, R^{6b}, m, R^{1'}, W⁴, W⁵, W⁶, R², and R³ are as described above.

Some of the starting materials and intermediates involved in the synthesis process are as follows:
1. 2-Chloro-6-bromo-8-methylquinazoline (CAS: 1388046-71-6) was obtained by cyclization of 5-bromo-2-fluoro-3-methylbenzaldehyde (CAS: 903875-64-9, Bidepharm, Shanghai) with guanidine carbonate (CAS: 593-85-1, Bidepharm, Shanghai) to give 2-amino-6-bromo-8-methylquinazoline, followed by reaction with antimony trichloride (CAS: 10025-91-9, Aladdin, Shanghai) and tert-butyl nitrite (CAS: 540-80-7, Aladdin, Shanghai).
2. 2-Chloro-6-bromo-8-ethylquinazoline (CAS: 2568323-82-8) was obtained by conversion of 4-bromo-2-ethyl-1-fluorobenzene (CAS: 627463-25-6, Bidepharm, Shanghai) with lithium diisopropylamide (CAS: 4111-54-0, Aladdin, Shanghai) and N,N-dimethylformamide (CAS: 68-12-2, Titan Technology, Shanghai) to 2-fluoro-3-ethyl-5-bromobenzaldehyde, followed by a method similar to that in (1); 2-chloro-6-bromo-8-trifluoromethylquinazoline was obtained from 5-bromo-2-fluorobenzotrifluoride (CAS: 393-37-3, Aladdin, Shanghai) by a similar method.
3. 2-Chloro-6-bromo-8-isopropylquinazoline was obtained by reacting 1-(2-fluoro-5-bromophenyl)ethanone (CAS: 198477-89-3, Bidepharm, Shanghai) and methylmagnesium bromide (CAS: 75-16-1, Aladdin, Shanghai) to give 2-(2-fluoro-5-bromophenyl)propan-2-ol, then the hydroxyl group was reduced to give 1-fluoro-2-isopropyl-4-bromobenzene (CAS: 112611-93-5), and was subsequently obtained by a method similar to that in (2).
4. 2-Chloro-6-bromo-8-cyclopropylquinazoline was obtained by reacting 2-iodo-4-bromofluorobenzene (CAS: 116272-41-4, Bidepharm, Shanghai) and cyclopropylboronic acid (CAS: 411235-57-9, Aladdin, Shanghai) to give 2-cyclopropyl-4-bromofluorobenzene, and was subsequently obtained by a method similar to that in (2).
5. 2-Chloro-6-bromo-8-cyclopropylquinazoline was obtained by reacting 2-fluoro-5-bromobenzaldehyde (CAS: 93777-26-5, Bidepharm, Shanghai) and ethyltriphenylphosphonium bromide (CAS: 1530-32-1, Bidepharm, Shanghai) to give 2-(1-propenyl)-4-bromofluorobenzene, then the double bond was reduced to give 2-n-propyl-4-bromofluorobenzene, and was subsequently obtained by a method similar to that in (2).
6. 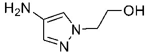 was obtained by reacting 4-nitropyrazole (CAS: 2075-46-9, Bidepharm, Shanghai) and 2-bromoethanol (CAS: 540-51-2, Bidepharm, Shanghai) to give 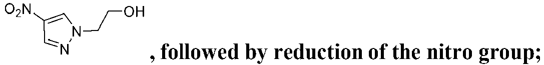
7. 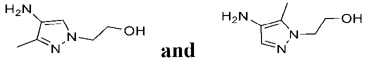 was obtained by reacting 3-methyl-4-nitropyrazole (CAS: 5334-39-4, Bidepharm, Shanghai) and 2-bromoethanol (CAS: 540-51-2, Bidepharm, Shanghai) to give a mixture of 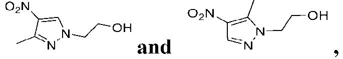 which was separated and purified to afford two individual isomers, followed by reduction of the nitro group;
   The following intermediates were obtained by similar methods: 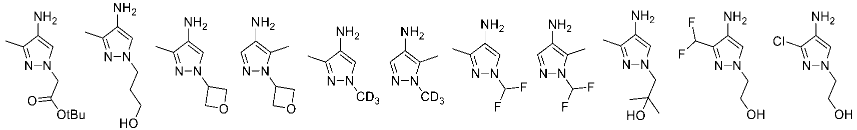 The raw materials involved were: tert-butyl bromoacetate (CAS: 5292-43-3, Energy Chemical, Shanghai), 3-bromo-1-propanol (CAS: 627-18-9, Bidepharm, Shanghai), 3-iodooxetane (CAS: 26272-85-5, Bidepharm, Shanghai), deuterated iodomethane (CAS: 865-50-9, Aladdin, Shanghai), sodium difluorochloroacetate (CAS: 1895-39-2, Bidepharm, Shanghai), 1,2-epoxy-2-methylpropane (CAS: 558-30-5, Energy Chemical, Shanghai), 3-(difluoromethyl)-4-nitro-1H-pyrazole (CAS: 1789048-54-9, Bidepharm, Shanghai), 3-chloro-4-nitro-1H-pyrazole (CAS: 35852-75-6, Bidepharm, Shanghai);
8. 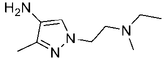 was obtained by reacting 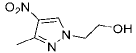 in (7) with methanesulfonyl chloride (CAS: 124-63-0, Xiya Reagent, Shandong) to give 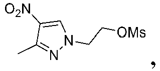 which was then reacted with methylethylamine (CAS: 624-78-2, Meryer, Shanghai) to give 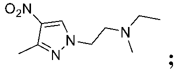 further reduction of the nitro group afforded the target compound. 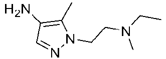 was obtained by similar methods starting from 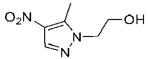 in (7).
   The following intermediates were obtained by similar methods: 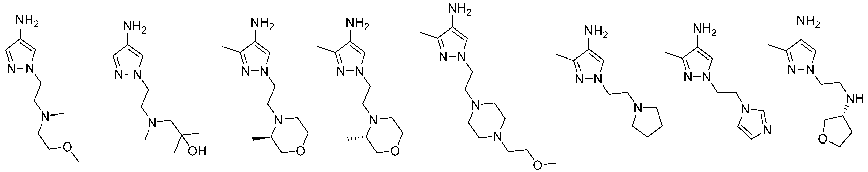 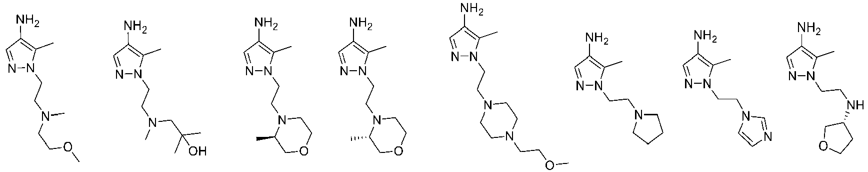 The raw materials involved are: N-(2-methoxyethyl)methylamine (CAS: 38256-93-8, Bidepharm, Shanghai), 2-methyl-1-(methylamino)propan-2-ol (CAS: 67622-86-0, Bidepharm, Shanghai), (R)-3-methylmorpholine (CAS: 74572-04-6, Bidepharm, Shanghai), (S)-3-methylmorpholine (CAS: 350595-57-2, Bidepharm, Shanghai), 1-(2-methoxyethyl)piperazine (CAS: 13484-40-7, Bidepharm, Shanghai), pyrrolidine (CAS: 123-75-1, Bidepharm, Shanghai), imidazole (CAS: 288-32-4, Macklin, Shanghai), (R)-3-aminotetrahydrofuran (CAS: 111769-26-7, Bidepharm, Shanghai).
9. 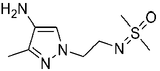 was obtained by reacting 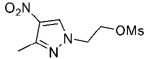 in (8) with dimethyl sulfoximide (CAS: 1520-31-6, Aladdin, Shanghai) to give 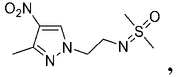 followed by reduction of the nitro group to afford the target compound;
10. 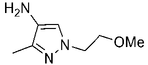 was obtained by reacting 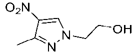 in (7) with iodomethane (CAS: 74-88-4, (Xiya Reagent, Shandong)) to give 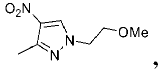 followed by reduction of the nitro group;
11. 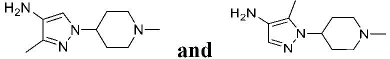 were obtained by reacting 1-methyl-4-piperidinol (CAS: 106-52-5, Bidepharm, Shanghai) and methanesulfonyl chloride (CAS: 124-63-0, (Xiya Reagent, Shandong)) to give 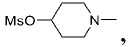 followed by reaction with 3-methyl-4-nitropyrazole (CAS: 5334-39-4, Bidepharm, Shanghai) to give a mixture of 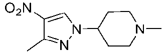 and 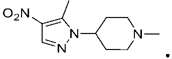 After the two individual isomers were obtained by separation and purification, reduction of the nitro group afforded the target compounds. The following intermediates were obtained by similar methods: 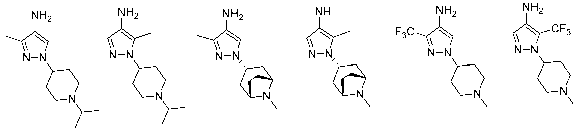 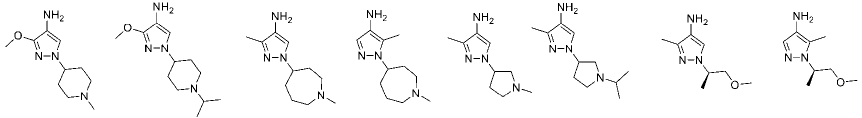 The raw materials involved were: 1-isopropyl-4-piperidinol (CAS: 5570-78-5, Energy Chemical, Shanghai), tropine (CAS: 120-29-6, Bidepharm, Shanghai), 4-nitro-5-trifluoromethylpyrazole (CAS: 1046462-99-0, Bidepharm, Shanghai), 3-methoxy-4-nitropyrazole (CAS: 400755-41-1, Bidepharm, Shanghai), 4-hydroxy-1-methylhexahydroazepine (CAS: 19065-49-7, Bidepharm, Shanghai), 3-hydroxy-1-methylpyrrolidine (CAS: 13220-33-2, Bidepharm, Shanghai), 1-isopropyl-3-hydroxypyrrolidine (CAS: 42729-56-6, Bidepharm, Shanghai), (S)-1-methoxy-2-propanol (CAS: 26550-55-0, Bidepharm, Shanghai);
12. A mixture of 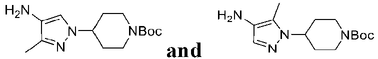 was obtained by reacting N-Boc-4-iodopiperidine (CAS: 301673-14-3, Bidepharm, Shanghai) and 3-methyl-4-nitropyrazole (CAS: 5334-39-4, Bidepharm, Shanghai) to give a mixture of 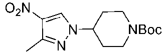 and 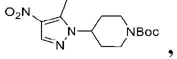 followed by reduction of the nitro group to afford the target regioisomeric mixture. The following regioisomeric mixtures were obtained by similar methods: 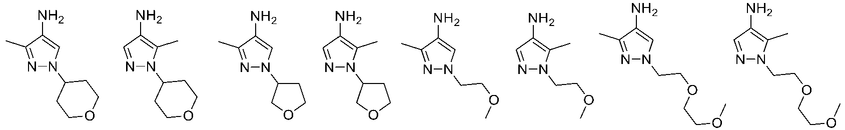 The raw materials involved were: 4-iodotetrahydropyran (CAS: 25637-18-7, Bidepharm, Shanghai), 3-iodotetrahydrofuran (CAS: 121138-01-0, Bidepharm, Shanghai), 2-bromoethyl methyl ether (CAS: 6482-24-2, Bidepharm, Shanghai), 1-bromo-2-(2-methoxyethoxy)ethane (CAS: 54149-17-6, Bidepharm, Shanghai);
13. A mixture of 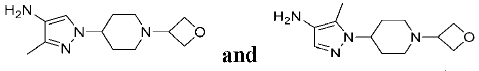 was **obtained** by deprotecting the Boc of a mixture of 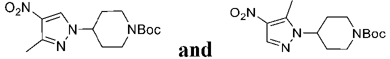 to give a mixture of 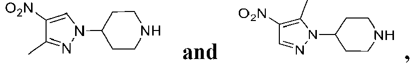 which was then reacted with 3-oxetanone (CAS: 6704-31-0, Bidepharm, Shanghai) to give a mixture of 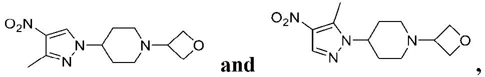 and further reduction of the nitro group afforded the final mixture;
14. A mixture of 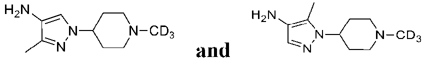 was obtained by deprotecting the Boc group of a mixture of 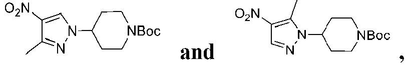 followed by reaction with deuterated iodomethane (CAS: 865-50-9, (Aladdin, Shanghai)) to give a mixture of 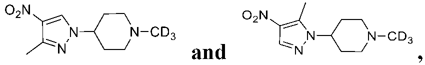 followed by reduction of the nitro group;
15. A mixture of 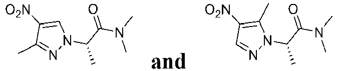 was **obtained** by Mitsunobu reaction of 3-methyl-4-nitropyrazole (CAS: 5334-39-4, Bidepharm, Shanghai) and methyl (R)-2-hydroxypropionate (CAS: 17392-83-5, Bidepharm, Shanghai) to give a mixture of 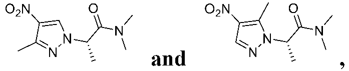 followed by reduction of the nitro group; replacing methyl (R)-2-hydroxypropionate with 3-(hydroxymethyl)oxetane (CAS: 251922-46-0, Bidepharm, Shanghai), a mixture of 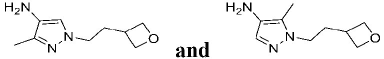 was **obtained** by a similar method;
16. A mixture of 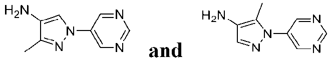 was **obtained** by reacting 3-methyl-4-nitropyrazole (CAS: 5334-39-4, Bidepharm, Shanghai) and 5-pyrimidineboronic acid (CAS: 109299-78-7, Bidepharm, Shanghai) to give a mixture of 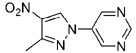 and 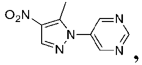 followed by reduction of the nitro group;
17. A mixture of 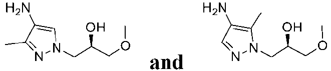 was **obtained** by reacting 3-methyl-4-nitropyrazole (CAS: 5334-39-4, Bidepharm, Shanghai) and (R)-glycidyl methyl ether (CAS: 64491-70-9, Aladdin, Shanghai) to give a mixture of 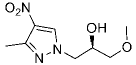 and 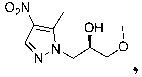 followed by reduction of the nitro group;
18. A mixture of 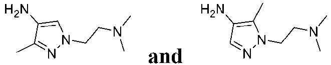 was **obtained** by reacting the mixture of 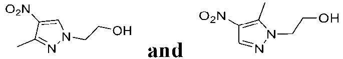 in (7) with methanesulfonyl chloride (CAS: 124-63-0, Xiya Reagent, Shandong) to give a mixture of 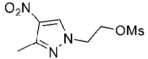 and 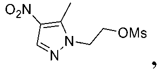 which was further reacted with dimethylamine hydrochloride (CAS: 506-59-2, Energy Chemical, Shanghai) to give a mixture of 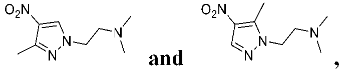 followed by reduction of the nitro group; replacing dimethylamine hydrochloride with diethylamine (CAS: 109-89-7, Energy Chemical, Shanghai), a mixture of 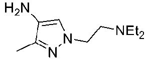 and 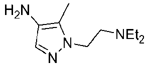 was obtained by a similar method;
19. A mixture of 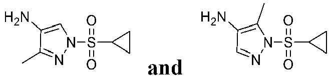 was obtained by reacting 3-methyl-4-nitropyrazole (CAS: 5334-39-4, Bidepharm, Shanghai) and cyclopropylsulfonyl chloride (CAS: 139631-62-2, Energy Chemical, Shanghai) to give a mixture of 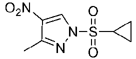 and 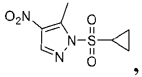 followed by reduction of the nitro group;
20. 2-methoxyethylamine (CAS: 109-85-3, Bidepharm, Shanghai);
21. 2-fluoroethylamine hydrochloride salt (CAS: 460-08-2, Bidepharm, Shanghai);
22. 2,2-difluoroethylamine (CAS: 430-67-1, Bidepharm, Shanghai);
23. cyclobutylamine (CAS: 2516-34-9, Energy Chemical, Shanghai);
24. cyclopropylamine (CAS: 765-30-0, Bidepharm, Shanghai);
25. N-isopropylmethylamine (CAS: 4747-21-1, Bidepharm, Shanghai);
26. 2,2-difluoro-N-methylethylamine hydrochloride (CAS: 139364-36-6, Bidepharm, Shanghai);
27. N-methylprop-2-yn-1-amine (CAS: 35161-71-8, Bidepharm, Shanghai);
28. 3-fluoroazetidine hydrochloride (CAS: 617718-46-4, Bidepharm, Shanghai);
29. 3,3-difluoroazetidine hydrochloride (CAS: 288315-03-7, Bidepharm, Shanghai);
30. 1,3-dimethyl-4-aminopyrazole (CAS: 64517-88-0, Bidepharm, Shanghai);
31. 1,5-dimethyl-4-aminopyrazole (CAS: 121983-36-6, Bidepharm, Shanghai);
32. 3-methyl-1H-pyrazol-4-amine (CAS: 113140-10-6, Bidepharm, Shanghai);
33. 1-ethyl-3-methyl-1H-pyrazol-4-amine (CAS: 947763-34-0, Bidepharm, Shanghai);
34. 1-methyl-3-(difluoromethyl)-1H-pyrazol-4-amine, obtained by reacting 1-methyl-3-(difluoromethyl)-1H-pyrazole-4-carboxylic acid (CAS: 176969-34-9, Bidepharm, Shanghai) and diphenylphosphoryl azide (CAS: 26386-88-9, Energy Chemical, Shanghai) to give 1-methyl-3-(difluoromethyl)-4-[(tert-butoxycarbonyl)amino]-1H-pyrazole, followed by removal of the tert-butoxycarbonyl group;
35. 1-methyl-3-chloro-1H-pyrazol-4-amine, obtained by reduction of the nitro group of 1-methyl-3-chloro-4-nitro-1H-pyrazole (CAS: 299930-70-4, Jizhi Biochemical, Shanghai);
36. 1-methyl-3-methoxy-4-aminopyrazole, obtained by reduction of the nitro group of 1-methyl-3-methoxy-4-nitropyrazole (CAS: 1201935-85-4, Jizhi Biochemical, Shanghai);
37. 2-bromopyridine (CAS: 109-04-6, Bidepharm, Shanghai);
38. 2-bromo-3-fluoropyridine (CAS: 40273-45-8, Bidepharm, Shanghai);
39. 2-bromo-3-methylpyridine (CAS: 3430-17-9, Bidepharm, Shanghai);
40. 2-bromo-3-methoxypyridine (CAS: 24100-18-3, Bidepharm, Shanghai);
41. 5-methyl-6-bromopyridine-2-carbonitrile (CAS: 450844-27-6, Bidepharm, Shanghai);
42. 2-bromo-3-methyl-6-fluoropyridine (CAS: 1211536-01-4, Bidepharm, Shanghai);
43. 2-bromo-3,6-difluoropyridine (CAS: 1382786-22-2, Bidepharm, Shanghai);
44. 6-chloro-5-(trifluoromethyl)pyridin-2-amine (CAS: 79456-28-3, Bidepharm, Shanghai);
45. pyridine-4-boronic acid (CAS: 1692-15-5, Bidepharm, Shanghai);
46. 3-fluoropyridine-4-boronic acid (CAS: 458532-97-3, Bidepharm, Shanghai);
47. 3-methylpyridine-4-boronic acid (CAS: 894808-72-1, Bidepharm, Shanghai);
48. 3-methoxypyridine-4-boronic acid (CAS: 1008506-24-8, Bidepharm, Shanghai);
49. 2-aminopyridine-4-boronic acid (CAS: 903513-62-2, Bidepharm, Shanghai);
50. 2,3-difluoropyridin-4-ylboronic acid (CAS: 1263374-42-0, Bidepharm, Shanghai);
51. 2,5-difluoropyridine-4-boronic acid (CAS: 1263375-23-0, Bidepharm, Shanghai);
52. 2-fluoro-5-methylpyridine-4-boronic acid (CAS: 929194-41-2, Bidepharm, Shanghai);
53. 1-(4-bromopyridin-2-yl)ethanone (CAS: 1060805-69-7, Bidepharm, Shanghai);
54. 2-fluoro-4-iodo-5-methylpyridine (CAS: 153034-94-7, Aikang, Jiangsu);
55. 3-pyridineboronic acid (CAS: 1692-25-7, Energy Chemical, Shanghai);
56. 2-methylpyridin-3-ylboronic acid pinacol ester (CAS: 1012084-56-8, Bidepharm, Shanghai);
57. 3-bromo-2-(difluoromethyl)pyridine (CAS: 1211520-77-2, Bidepharm, Shanghai);
58. 3-bromo-2-ethylpyridine (CAS: 38749-81-4, Bidepharm, Shanghai);
59. 2-methoxypyridin-3-ylboronic acid (CAS: 163105-90-6, Bidepharm, Shanghai);
60. 2-cyano-3-bromopyridine (CAS: 55758-02-6, Bidepharm, Shanghai);
61. 2-aminopyridine-3-boronic acid, pinacol ester (CAS: 1073354-97-8, Jizhi Biochemical, Shanghai)
62. 2-cyclopropyl-3-bromopyridine (CAS: 944718-27-8, Shaoyuan, Shanghai);
63. 3-bromo-5-acetylpyridine (CAS: 38940-62-4, Jizhi Biochemical, Shanghai)
64. 5-fluoropyridin-3-ylboronic acid (CAS: 872041-86-6, Bidepharm, Shanghai);
65. 4-fluoropyridin-3-ylboronic acid, pinacol ester (CAS: 1220219-91-9, Bidepharm, Shanghai);
66. 4-methylpyridin-3-ylboronic acid (CAS: 148546-82-1, Bidepharm, Shanghai);
67. 4-methoxypyridin-3-ylboronic acid, pinacol ester (CAS: 758699-74-0, Bidepharm, Shanghai);
68. 4-cyanopyridine-3-boronic acid pinacol ester (CAS: 878194-91-3, Bidepharm, Shanghai);
69. 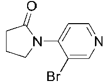 was obtained by reacting 3-bromo-4-aminopyridine (CAS: 13534-98-0, Pharmablock, Jiangsu) and 4-chlorobutyryl chloride (CAS: 4635-59-0, Bidepharm, Shanghai);
70. 3-bromo-4-methyl-5-fluoropyridine (CAS: 1211517-76-8, Jizhi Biochemical, Shanghai);
71. 3,5-dibromo-4-chloropyridine (CAS: 13626-17-0, Bidepharm, Shanghai);
72. 3-bromo-4,5-dichloropyridine (CAS: 1001056-83-2, Bidepharm, Shanghai);
73. 2-fluoro-3-bromo-6-methylpyridine (CAS: 375368-78-8, Bidepharm, Shanghai);
74. 3-bromo-2,5-difluoropyridine (CAS: 1211331-43-9, Aladdin, Shanghai)
75. 2-methyl-3-bromo-5-fluoropyridine (CAS: 1211542-29-8, Bidepharm, Shanghai);
76. 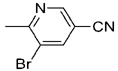 was obtained by reacting 5-bromo-6-methylnicotinic acid (CAS: 1190862-72-6, Bidepharm, Shanghai) and ammonium chloride (CAS: 12125-02-9, Aladdin, Shanghai) to give 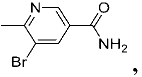 followed by dehydration;
77. 3-bromo-2,4-difluoropyridine (CAS: 1227502-60-4, Bidepharm, Shanghai);
78. 2,4-dichloro-3-iodopyridine (CAS: 343781-36-2, Bidepharm, Shanghai);
79. 2-chloro-3-iodo-4-fluoropyridine (CAS: 1271477-28-1, Leyan, Shanghai);
80. 2-fluoro-4-methylpyridine-3-boronic acid (CAS: 1029654-30-5, Leyan, Shanghai);
81. 3-bromo-2,4-dimethylpyridine (CAS: 27063-93-0, (Leyan, Shanghai))
82. 2-amino-3-bromo-4-fluoropyridine (CAS: 1417407-29-4, Bidepharm, Shanghai);
83. 2-amino-3-bromo-4-chloropyridine (CAS: 221297-82-1, Bidepharm, Shanghai);
84. 2-amino-3-bromo-4-methylpyridine (CAS: 40073-38-9, Bidepharm, Shanghai);
85. 5-bromo-N-methylnicotinamide (CAS: 153435-68-8, Bidepharm, Shanghai);
86. 5-bromo-N-cyclopropylnicotinamide (CAS: 385382-48-9, Bidepharm, Shanghai);
87. 4-chloropyridin-3-ylboronic acid pinacol ester (CAS: 452972-15-5, KeyWe, Shanghai)
88. 2-fluoro-3-iodo-5-methylpyridine (CAS: 153034-78-7, Bidepharm, Shanghai);
89. pyrazine-2-boronic acid (CAS: 762263-64-9, Bidepharm, Shanghai);
90. 2-bromo-3-fluoropyrazine (CAS: 206278-27-5, Bidepharm, Shanghai);
91. 2-bromo-3-chloropyrazine (CAS: 1206250-01-2, Bidepharm, Shanghai);
92. 2-bromo-3-methylpyrazine (CAS: 120984-76-1, Bidepharm, Shanghai);
93. 2-chloro-3-methoxypyrazine (CAS: 40155-28-0, Bidepharm, Shanghai);
94. 2-(hydroxymethyl)-3-chloropyrazine (CAS: 89283-32-9, Bidepharm, Shanghai);
95. 2-amino-3-chloropyrazine (CAS: 6863-73-6, Bidepharm, Shanghai);
96. 2-chloro-3-cyanopyrazine (CAS: 55557-52-3, Bidepharm, Shanghai);
97. 3-chloropyrazine-2-carboxamide (CAS: 21279-62-9, Bidepharm, Shanghai);
98. 2,5-dichloropyrazine (CAS: 19745-07-4, Bidepharm, Shanghai);
99. 2-chloro-5-methylpyrazine (CAS: 59303-10-5, Bidepharm, Shanghai);
100. 2-bromo-6-chloropyrazine (CAS: 916791-07-6, Bidepharm, Shanghai);
101. 2-chloro-6-methylpyrazine (CAS: 38557-71-0, Energy Chemical, Shanghai);
102. 2-chloro-6-methoxypyrazine (CAS: 33332-30-8, Bidepharm, Shanghai);
103. 2-(hydroxymethyl)-6-chloropyrazine (CAS: 1240602-95-2, Bidepharm, Shanghai);
104. 2-amino-6-chloropyrazine (CAS: 33332-28-4, Bidepharm, Shanghai);
105. 6-chloropyrazine-2-carbonitrile (CAS: 6863-74-7, Bidepharm, Shanghai);
106. 6-chloropyrazine-2-carboxamide (CAS: 36070-79-8, Bidepharm, Shanghai);
107. 3-chloro-2,5-dimethylpyrazine (CAS: 95-89-6, Bidepharm, Shanghai);
108. 4-chloropyridazine hydrochloride (CAS: 1193386-63-8, Bidepharm, Shanghai);
109. 3-bromopyridazine (CAS: 88491-61-6, Bidepharm, Shanghai);
110. 3-chloro-4-methylpyridazine (CAS: 68206-04-2, Bidepharm, Shanghai);
111. 3-chloropyridazine-4-carbonitrile (CAS: 1445-56-3, Bidepharm, Shanghai);
112. pyrimidine-5-boronic acid pinacol ester (CAS: 321724-19-0, Bidepharm, Shanghai);
113. 4-amino-5-bromopyrimidine (CAS: 1439-10-7, Bidepharm, Shanghai);
114. 4-methoxy-5-bromopyrimidine (CAS: 4319-85-1, Bidepharm, Shanghai);
115. 4-chloro-5-iodo-6-methylpyrimidine (CAS: 83410-15-5, Jizhi Biochemical, Shanghai);
116. 5-bromo-4,6-dimethylpyrimidine (CAS: 157335-97-2, Bidepharm, Shanghai);
117. 4-amino-5-bromo-6-methylpyrimidine (CAS: 7752-48-9, Kaiwei, Shanghai);
118. 4-methoxy-5-bromo-6-methylpyrimidine (CAS: 4319-87-3, Bidepharm, Shanghai);
119. 2-bromopyrimidine (CAS: 4595-60-2, Bidepharm, Shanghai);
120. 2-chloro-4-bromopyrimidine (CAS: 885702-34-1, Bidepharm, Shanghai);
121. 4-iodopyrimidine hydroiodide (CAS: 1965309-31-2, Bidepharm, Shanghai);
122. 4,5-dibromopyrimidine (CAS: 1381946-49-1, Bidepharm, Shanghai);
123. 4-bromo-5-chloropyrimidine (CAS: 1261628-82-3, Bidepharm, Shanghai);
124. 4-bromo-5-fluoropyrimidine (CAS: 1003706-87-3, Bidepharm, Shanghai);
125. 4-chloro-5-fluoro-6-methylpyrimidine (CAS: 898044-55-8, Bidepharm, Shanghai);
126. 4-methyl-5-bromopyrimidine (CAS: 1439-09-4, Bidepharm, Shanghai);
127. 4-methoxy-5-bromopyrimidine (CAS: 4319-85-1, Bidepharm, Shanghai);
128. 4-chloro-5-methylpyrimidine (CAS: 51957-32-5, Bidepharm, Shanghai);
129. 2-fluoro-6-hydroxybenzeneboronic acid (CAS: 1256345-60-4, Bidepharm, Shanghai);
130. 2,3-difluorobenzeneboronic acid (CAS: 121219-16-7, Bidepharm, Shanghai);
131. 2-fluoro-3-hydroxybenzeneboronic acid (CAS: 855230-60-3, Bidepharm, Shanghai);
132. 3,5-difluorobenzeneboronic acid (CAS: 156545-07-2, Bidepharm, Shanghai);
133. 2,5-difluorobenzeneboronic acid (CAS: 193353-34-3, Bidepharm, Shanghai);
134. 2-fluoro-5-hydroxybenzeneboronic acid (CAS: 1150114-52-5, Bidepharm, Shanghai);
135. 2-methyl-5-fluorobenzeneboronic acid (CAS: 163517-62-2, Bidepharm, Shanghai);
136. 2-methyl-5-cyanobenzeneboronic acid (CAS: 867333-43-5, Bidepharm, Shanghai);
137. 4-bromo-1,3-benzodioxole (CAS: 6698-13-1, Bidepharm, Shanghai);
138. 4-bromo-2,2-difluoro-1,3-benzodioxole (CAS: 144584-66-7, Bidepharm, Shanghai);
139. 1-bromo-3,4-(methylenedioxy)benzene (CAS: 2635-13-4, Bidepharm, Shanghai);
140. 3,5-dichloro-2(1H)-pyrazinone (CAS: 130879-62-8, Bidepharm, Shanghai);
141. 2-hydroxy-3-bromo-5-methylpyrazine (CAS: 1260810-61-4, Bidepharm, Shanghai);
142. 4-bromo-3-hydroxy-6-pyrazinone (CAS: 15456-86-7, Bidepharm, Shanghai);
143. 2-hydroxy-3-chloro-5-bromopyrazine (CAS: 21943-17-9, Bidepharm, Shanghai);
144. 4-iodo-5-methylpyridin-2(1H)-one (CAS: 1227570-92-4, Bidepharm, Shanghai);
145. 4-bromo-5-methyl-1H-indazole (CAS: 926922-40-9, Bidepharm, Shanghai);
146. 4-isoquinolineboronic acid (CAS: 192182-56-2, Leyan, Shanghai);
147. 5-methyl-6-bromo-1H-indazole (CAS: 1000343-69-0, Bidepharm, Shanghai);
148. 3-bromo-7-azaindole (CAS: 74420-15-8, Bidepharm, Shanghai);
149. 3-methylpyrazol-4-ylboronic acid pinacol ester (CAS: 936250-20-3, Bidepharm, Shanghai);
150. 1,3-dimethylpyrazol-4-ylboronic acid pinacol ester (CAS: 1046832-21-6, Bidepharm, Shanghai);
151. 3,5-dimethylpyrazol-4-ylboronic acid pinacol ester (CAS: 857530-80-4, Bidepharm, Shanghai);
152. 3-bromo-1,4-dimethyl-1H-pyrazole (CAS: 13745-59-0, Bidepharm, Shanghai);
153. 1,4-dimethylpyrazole-5-boronic acid pinacol ester (CAS: 1047644-76-7, Bidepharm, Shanghai);
154. 3-bromo-4-methyl-4H-1,2,4-triazole (CAS: 16681-73-5, Bidepharm, Shanghai);
155. 5-methylisoxazole-4-boronic acid pinacol ester (CAS: 1346808-41-0, Bidepharm, Shanghai);
156. 4-Iodo-2-pyridone (CAS: 858839-90-4, Bidepharm, Shanghai);
157. 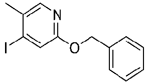 was obtained by reacting 2-fluoro-4-iodo-5-methylpyridine (CAS: 153034-94-7, Aikang, Jiangsu) and benzyl alcohol (CAS: 100-51-6, Bidepharm, Shanghai);
158. 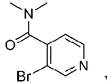 was obtained by reacting 3-bromoisonicotinic acid (CAS: 13959-02-9, Bidepharm, Shanghai) and dimethylamine hydrochloride (CAS: 506-59-2, Energy Chemical, Shanghai);
159. 3-bromo-5-fluoroisonicotinamide (CAS: 1353636-72-2, Bidepharm, Shanghai);
160. 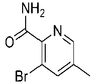 was obtained by reacting 3-bromo-5-methylpyridine-2-carboxylic acid (CAS: 1211515-68-2, Bidepharm, Shanghai) and ammonium chloride (CAS: 12125-02-9, (Aladdin, Shanghai));
161. 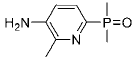 was obtained by reacting 2-bromo-5-amino-6-methylpyridine (CAS: 126325-47-1, Bidepharm, Shanghai) and dimethylphosphine oxide (CAS: 7211-39-4, Aikang, Jiangsu); the following intermediates were obtained by a similar method: 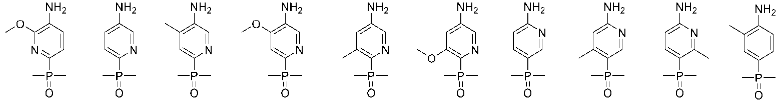 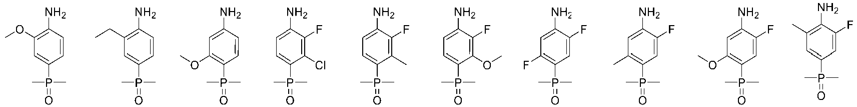 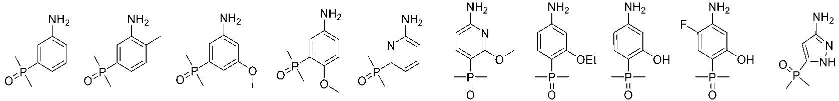 The raw materials involved are: 2-methoxy-3-amino-6-bromopyridine (CAS: 89466-18-2, Bidepharm, Shanghai), 3-amino-6-bromopyridine (CAS: 13534-97-9, Bidepharm, Shanghai), 2-bromo-4-methyl-5-aminopyridine (CAS: 156118-16-0, Bidepharm, Shanghai), 6-chloro-4-methoxy-3-pyridinamine (CAS: 1256805-54-5, Bidepharm, Shanghai), 5-amino-2-bromo-3-methylpyridine (CAS: 38186-83-3, Bidepharm, Shanghai), 3-amino-6-chloro-5-methoxypyridine (CAS: 75711-01-2, Bidepharm, Shanghai), 2-amino-5-bromopyridine (CAS: 1072-97-5, Bidepharm, Shanghai), 2-amino-5-bromo-4-methylpyridine (CAS: 98198-48-2, Bidepharm, Shanghai), 2-amino-5-bromo-6-methylpyridine (CAS: 42753-71-9, Bidepharm, Shanghai), 2-methyl-4-bromoaniline (CAS: 583-75-5, Bidepharm, Shanghai), 2-methoxy-4-bromoaniline (CAS: 59557-91-4, Bidepharm, Shanghai), 4-bromo-2-ethylaniline (CAS: 45762-41-2, Bidepharm, Shanghai), 3-methoxy-4-bromoaniline (CAS: 19056-40-7, Bidepharm, Shanghai), 4-bromo-3-chloro-2-fluoroaniline (CAS: 115843-99-7, Bidepharm, Shanghai), 4-bromo-3-chloro-2-fluoroaniline (CAS: 1540204-53-2, (Jizhi Biochemical, Shanghai)), 4-bromo-2-fluoro-3-methoxyaniline (CAS: 1272719-19-3, (Leyan, Shanghai)), 4-bromo-2,5-difluoroaniline (CAS: 112279-60-4, (Aladdin, Shanghai)), 4-bromo-2-fluoro-5-methylaniline (CAS: 418762-26-2, Bidepharm, Shanghai), 4-bromo-2-fluoro-5-methoxyaniline (CAS: 108310-38-9, Bidepharm, Shanghai), 4-bromo-2-fluoro-6-methylaniline (CAS: 429683-46-5, Bidepharm, Shanghai), m-bromoaniline (CAS: 591-19-5, Bidepharm, Shanghai), 5-bromo-2-methylaniline (CAS: 39478-78-9, Bidepharm, Shanghai), 3-bromo-5-methoxyaniline (CAS: 16618-68-1, Bidepharm, Shanghai), 3-bromo-4-methoxyaniline (CAS: 19056-41-8, (Aladdin, Shanghai)), 2-amino-6-bromopyridine (CAS: 19798-81-3, Bidepharm, Shanghai), 5-bromo-6-methoxypyridin-2-amine (CAS: 1211533-83-3, Bidepharm, Shanghai), 2-bromo-5-nitrobenzene ethanol (CAS: 423165-33-7, Bidepharm, Shanghai), 2-bromo-5-nitrophenol (CAS: 52427-05-1, Bidepharm, Shanghai), 2-bromo-4-fluoro-5-nitrophenol (CAS: 84478-87-5, Bidepharm, Shanghai), 5-bromo-1H-pyrazol-3-amine (CAS: 950739-21-6, Bidepharm, Shanghai);
162. (4-aminophenyl)dimethylphosphine oxide (CAS: 737751-54-1, Bidepharm, Shanghai);
163. (4-amino-3-fluorophenyl)dimethylphosphine oxide (CAS: 1197956-35-6, Bidepharm, Shanghai);
164. 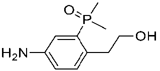 was obtained by reacting p-nitrobenzeneethanol (CAS: 100-27-6, Bidepharm, Shanghai) and 1,3-dibromo-5,5-dimethylhydantoin (CAS: 77-48-5, Bidepharm, Shanghai) to give 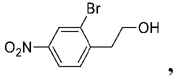 followed by reduction of the nitro group to give 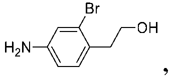 which was then reacted with dimethylphosphine oxide (CAS: 7211-39-4, Aikang, Jiangsu);
165. bis(pinacolato)diboron (CAS: 73183-34-3, Bidepharm, Shanghai);
166. tris(dibenzylideneacetone)dipalladium(0) (CAS: 51364-51-3, Energy Chemical, Shanghai);
167. [1,1'-bis(diphenylphosphino)ferrocene]palladium(II) dichloride dichloromethane complex (CAS: 95464-05-4, Bidepharm, Shanghai);
168. palladium acetate (CAS: 3375-31-3, Bidepharm, Shanghai);
169. tricyclohexylphosphine (CAS: 2622-14-2, Bidepharm, Shanghai);
170. (S)-2-hydroxypropanoic acid (CAS: 79-33-4, Bidepharm, Shanghai);
171. (R)-2-hydroxypropanoic acid (CAS: 10326-41-7, Bidepharm, Shanghai);
172. 5-bromo-1-methyl-1H-pyrazole-4-carbonitrile (CAS: 1269293-80-2, Bidepharm, Shanghai).
173. 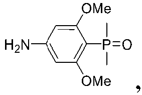 obtained by converting 4-bromo-3,5-dimethoxybenzoic acid (CAS: 56518-42-4, Bidepharm, Shanghai) to 4-bromo-3,5-dimethoxybenzoyl azide, followed by rearrangement to obtain 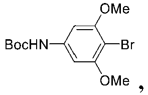 followed by deprotection to obtain 4-bromo-3,5-dimethoxybenzenamine, which was then reacted with dimethylphosphine oxide (CAS: 7211-39-4, Aikang, Jiangsu);
174. 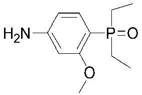 was obtained by reacting 3-methoxy-4-bromobenzenamine (CAS: 19056-40-7, Bidepharm, Shanghai) and diethylphosphine oxide (CAS: 7215-33-0, Bidepharm, Shanghai);
175. 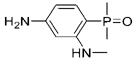 was obtained by reacting 2-bromo-5-nitroaniline (CAS: 10403-47-1, Bidepharm, Shanghai) and iodomethane (CAS: 74-88-4, Xiya Reagent, Shandong) to give 2-bromo-5-nitro-N-methylaniline, and was subsequently obtained by a method similar to that described in 161.
176. 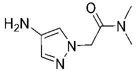 was obtained by reacting 4-nitropyrazole (CAS: 2075-46-9, Bidepharm, Shanghai) and N,N-dimethylbromoacetamide (CAS: 5468-77-9, Aladdin, Shanghai) to give 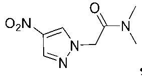 , followed by reduction of the nitro group.

### Synthesis of Compound I-1

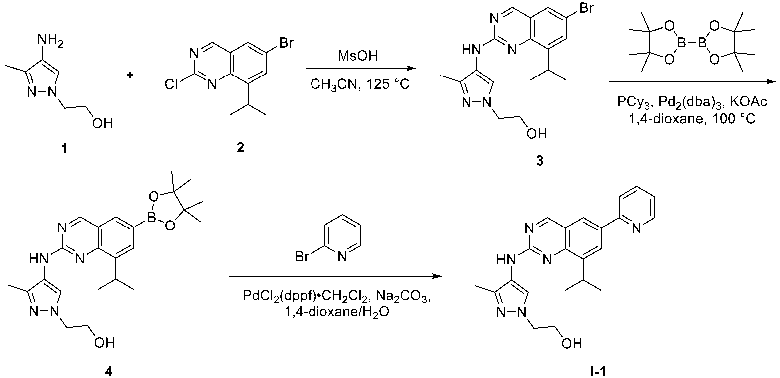

1) Preparation of compound 3:
   Compound 1 (373 mg, 2.65 mmol) and compound 2 (630 mg, 2.21 mmol) and methanesulfonic acid (318 mg, 3.32 mmol) were dissolved in 20 mL of acetonitrile, and the mixture was reacted at 125°C for 12 h. TLC and LC-MS analysis indicated the reaction was complete. **In** an ice bath, aqueous ammonia was added to the reaction mixture, and a precipitate formed. The solid was collected by filtration, washed with water, and dried to give compound 3 (575 mg), which was used directly in the next step.
2) Preparation of compound 4:
   Compound 3 (145 mg, 0.37 mmol), bis(pinacolato)diboron (123 mg, 0.48 mmol), tricyclohexylphosphine (21 mg, 0.074 mmol), tris(dibenzylideneacetone)dipalladium (34 mg, 0.037 mmol), and potassium acetate (110 mg, 1.11 mmol) were dispersed in 1,4-dioxane (5 mL). After purging with nitrogen, the mixture was reacted at 100°C for 2 h. TLC and LC-MS analysis indicated the reaction was complete. The reaction mixture was diluted with water and extracted with dichloromethane. The organic layer was concentrated and purified by column chromatography to give compound 4 (95 mg).
3) Preparation of compound I-1:
   Compound 4 (10 mg, 0.023 mmol), 2-bromopyridine (5.4 mg, 0.034 mmol), [1,1'-bis(diphenylphosphino)ferrocene]palladium(II) dichloride dichloromethane complex (2 mg, 0.0022 mmol), and sodium carbonate (10 mg, 0.091 mmol) were dispersed in a mixed solvent of 1,4-dioxane (0.6 mL) and water (0.3 mL). After purging with nitrogen, the mixture was stirred at 100°C overnight. TLC and LC-MS analysis indicated the reaction was complete. The reaction mixture was diluted with water and extracted with dichloromethane. The organic layer was concentrated and purified by silica gel column chromatography, and further purified by preparative HPLC to give compound I-1 (5.8 mg).

### Synthesis of Compound I-64

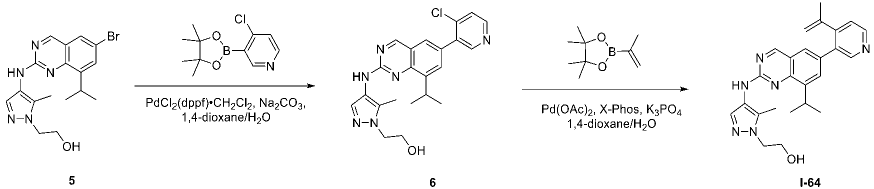

1) Preparation of compound 5:
   Compound 5 was obtained by a method analogous to that for the preparation of compound 4.
2) Preparation of compound 6:
   Compound 5 (30 mg, 0.077 mmol), 4-chloropyridine-3-boronic acid pinacol ester (27.6 mg, 0.11 mmol), [1,1'-bis(diphenylphosphino)ferrocene]palladium(II) dichloride dichloromethane complex (6.2 mg, 0.0077 mmol) and sodium carbonate (32 mg, 0.31 mmol) were dispersed in a mixed solvent of 1,4-dioxane (0.6 mL) and water (0.3 mL). After purging with nitrogen, the mixture was stirred at 100°C overnight. TLC and LC-MS analysis indicated the reaction was complete. The reaction mixture was diluted with water and extracted with DCM. The organic layer was concentrated and purified by silica gel column chromatography to give compound 6 (20 mg).
3) Preparation of compound I-64:
   Compound 6 (20 mg, 0.047 mmol), 4-chloropyridine-3-boronic acid pinacol ester (9.5 mg, 0.057 mmol), palladium acetate (1.1 mg, 0.0047 mmol), 2-dicyclohexylphosphino-2',4',6'-triisopropylbiphenyl (4.5 mg, 0.0094 mmol) and potassium phosphate (30 mg, 0.14 mmol) were dispersed in a mixed solvent of 1,4-dioxane (0.8 mL) and water (0.4 mL). After purging with nitrogen, the mixture was stirred at 100°C overnight. TLC and LC-MS analysis indicated the reaction was complete. The reaction mixture was diluted with water and extracted with DCM. The organic layer was concentrated and purified by silica gel column chromatography, and further purified by preparative HPLC to give compound I-64 (10.2 mg).

The remaining compounds were obtained by similar methods.

The specific compounds and structural identification data are listed in the table below.

| **No.** | **Structure** | **NMR and/or MS data** |
|---|---|---|
| **I-1** | 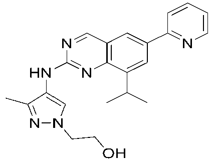 | **¹H NMR (600 MHz, DMSO-*d*₆) δ 9.41 - 9.27 (m, 2H), 8.74 (dd, *J =* 5.0, 1.7 Hz, 1H), 8.43 (d, *J =* 2.1 Hz, 1H), 8.38 (d, *J =* 2.1 Hz, 1H), 8.35 - 8.26 (m, 1H), 8.14 (d, *J* = 8.0 Hz, 1H), 8.02 (td, *J =* 7.8, 1.8 Hz, 1H), 7.46 (dd, *J* = 7.5, 4.9 Hz, 1H), 4.36 (s, 1H), 4.09 (t, *J* = 5.6 Hz, 2H), 4.04 - 3.93 (m, 1H), 3.75 (t, *J =* 5.7 Hz, 2H), 2.24 (s, 3H), 1.40 (d, *J* = 6.9 Hz, 6H). MS (ESI) m/z: 389 [M+H]⁺.** |
| **I-2** | 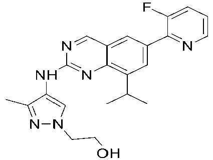 | **¹H NMR (600 MHz, DMSO-*d*₆) δ 9.37 - 9.29 (m, 2H), 8.62 - 8.55 (m, 1H), 8.36 - 8.28 (m, 2H), 8.26 (s, 1H), 7.93 - 7.83 (m, 1H), 7.50 (dt, *J =* 8.3, 4.1 Hz, 1H), 4.92 (t, *J =* 5.3 Hz, 1H), 4.09 (t, *J =* 5.6 Hz, 2H), 4.06 - 3.95 (m, 1H), 3.75 (q, *J =* 5.6 Hz, 2H), 2.24 (s, 3H), 1.38 (d, *J =* 6.9 Hz, 6H). MS (ESI) m/z: 407 [M+H]⁺.** |
| **I-3** | 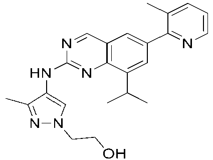 | **MS (ESI) *m*/*z:* 403 [M+H]⁺.** |
| **I-4** | 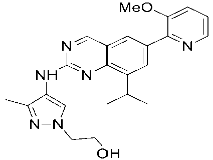 | **¹H NMR (600 MHz, DMSO-*d*₆) δ 9.28 (s, 2H), 8.35 (d, *J* = 4.7 Hz, 1H), 8.29 (d, *J =* 2.0 Hz, 2H), 8.21 (d, *J =* 1.9 Hz, 1H), 7.73 (d, *J =* 8.4 Hz, 1H), 7.49 (dd, *J =* 8.4, 4.7 Hz, 1H), 4.09 (t, *J =* 5.6 Hz, 2H), 4.03 - 3.96 (m, 1H), 3.94 (s, 3H), 3.75 (t, *J =* 5.6 Hz, 2H), 2.24 (s, 3H), 1.37 (d, *J =* 6.9 Hz, 6H). MS (ESI) *m*/*z*: 419 [M+H]⁺.** |
| **I-5** | 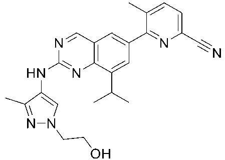 | **¹H NMR (600 MHz, DMSO-*d*₆) δ 9.34 - 9.28 (m, 2H), 8.30 (s, 1H), 8.03 (dd, *J =* 7.9, 0.8 Hz, 1H), 7.96 (dd, *J* = 4.9, 2.9 Hz, 2H), 7.83 (d, *J =* 2.0 Hz, 1H), 4.90 (t, *J =* 5.3 Hz, 1H), 4.08 (t, *J* = 5.6 Hz, 2H), 4.03 - 3.94 (m, 1H), 3.78 - 3.71 (m, 2H), 2.49 (s, 3H), 2.23 (s, 3H), 1.36 (d, *J =* 6.9 Hz, 6H). MS (ESI) *m*/*z*: 428 [M+H]⁺.** |
| **I-6** | 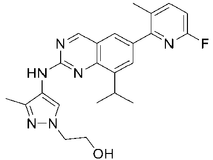 | **¹H NMR (600 MHz, DMSO-*d*₆) δ 9.29 (s, 1H), 8.30 (s, 1H), 7.99 - 7.92 (m, 2H), 7.84 (s, 1H), 7.14 - 7.09 (m, 1H), 4.91 (t, *J =* 5.3 Hz, 1H), 4.08 (t, *J =* 5.6 Hz, 2H), 4.03 - 3.93 (m, 1H), 3.75 (q, *J =* 5.6 Hz, 2H), 2.43 (s, 3H), 2.24 (s, 3H), 1.36 (d, *J =* 6.9 Hz, 6H). MS (ESI) *m*/*z*: 421 [M+H]⁺.** |
| **I-7** | 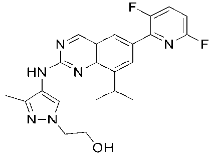 | **MS (ESI) m/z: 425 [M+H]⁺.** |
| **I-8** | 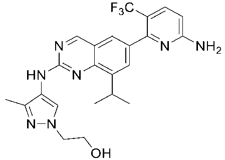 | **¹H NMR (600 MHz, DMSO-*d*₆) δ 9.35 - 9.22 (m, 2H), 8.30 (s, 1H), 7.83 (d, *J =* 8.9 Hz, 1H), 7.76 (d, *J* = 1.9 Hz, 1H), 7.65 (d, *J =* 1.9 Hz, 1H), 6.64 (d, *J =* 8.9 Hz, 1H), 4.08 (t, *J =* 5.7 Hz, 2H), 3.96 - 3.94 (m, 1H), 3.74 (t, *J =* 5.7 Hz, 2H), 2.23 (s, 3H), 1.32 (d, *J =* 6.9 Hz, 6H). MS (ESI) *m*/*z*: 472 [M+H]⁺.** |
| **I-9** | 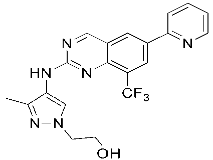 | **¹H NMR (600 MHz, DMSO-*d*₆) δ 9.76 (s, 1H), 9.43 (s, 1H), 8.92 - 8.84 (m, 2H), 8.73 (ddd, *J =* 4.8, 1.9, 0.9 Hz, 1H), 8.36 (s, 1H), 8.16 (dt, *J* = 8.1, 1.1 Hz, 1H), 7.97 (td, *J=* 7.7, 1.8 Hz, 1H), 7.43 (ddd, *J =* 7.5, 4.7, 1.0 Hz, 1H), 4.88 (t, *J =* 5.4 Hz, 1H), 4.05 (t, *J =* 5.9 Hz, 2H), 3.74 (q, *J* = 5.7 Hz, 2H), 2.26 (s, 3H). MS (ESI) *m*/*z*: 415 [M+H]⁺.** |
| **I-10** | 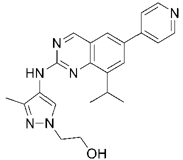 | **¹H NMR (600 MHz, DMSO-*d*₆) δ 9.40 - 9.30 (m, 1H), 9.28 (s, 1H), 8.69 - 8.65 (m, 2H), 8.29 (s, 1H), 8.19 (d, *J* = 2.1 Hz, 1H), 8.02 (d, *J =* 2.1 Hz, 1H), 7.86 - 7.77 (m, 2H), 4.90 (t, *J =* 5.3 Hz, 1H), 4.11 - 4.06 (m, 2H), 4.02 - 3.96 (m, 1H), 3.74 (q, *J =* 5.5 Hz, 2H), 2.23 (s, 3H), 1.39 (d, *J* = 6.9 Hz, 6H). MS (ESI) m/z: 389 [M+H]⁺.** |
| **I-11** | 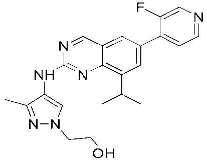 | **¹H NMR (600 MHz, DMSO-*d*₆) δ 9.44 - 9.33 (m, 1H), 9.30 (s, 1H), 8.69 (d, *J =* 2.5 Hz, 1H), 8.54 (d, *J =* 4.9 Hz, 1H), 8.33 - 8.26 (m, 1H), 8.07 (t, *J* = 1.6 Hz, 1H), 7.92 - 7.86 (m, 1H), 7.76 (dd, *J =* 7.1, 4.9 Hz, 1H), 4.91 (t, *J =* 5.3 Hz, 1H), 4.08 (t, *J =* 5.6 Hz, 2H), 4.02 - 3.93 (m, 1H), 3.74 (q, *J=* 5.5 Hz, 2H), 2.23 (s, 3H), 1.37 (d, *J =* 6.9 Hz, 6H). MS (ESI) m/z: 407 [M+H]⁺.** |
| **I-12** | 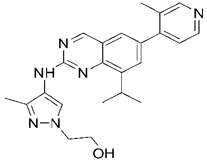 | **¹H NMR (600 MHz, DMSO-*d*₆) δ 9.48 - 9.35 (m, 1H), 9.30 (s, 1H), 8.82 (s, 1H), 8.75 (d, *J* = 5.7 Hz, 1H), 8.30 (s, 1H), 7.92 (d, *J =* 2.0 Hz, 1H), 7.86 (d, *J =* 5.6 Hz, 1H), 7.74 (d, *J =* 2.1 Hz, 1H), 4.09 (t, *J =* 5.6 Hz, 2H), 4.03 - 3.96 (m, 1H), 3.75 (t, *J =* 5.6 Hz, 2H), 2.47 (s, 3H), 2.24 (s, 3H), 1.37 (d, *J =* 6.9 Hz, 6H). MS (ESI) *m*/*z*: 403 [M+H]⁺.** |
| **I-13** | 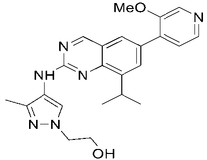 | **¹H NMR (600 MHz, DMSO-*d*₆) δ 9.34 - 9.15 (m, 2H), 8.50 (s, 1H), 8.36 - 8.20 (m, 2H), 7.95 (s, 1H), 7.84 (s, 1H), 7.48 (d, *J =* 4.8 Hz, 1H), 4.91 (s, 1H), 4.13 - 4.04 (m, 2H), 4.02 - 3.87 (m, 4H), 3.80 - 3.72 (m, 2H), 2.23 (s, 3H), 1.36 (d, *J* = 6.9 Hz, 6H). MS (ESI) m/z: 419 [M+H]⁺.** |
| **I-14** | 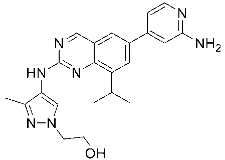 | **¹H NMR (600 MHz, DMSO-*d*₆) δ 9.35 - 9.19 (m, 2H), 8.28 (s, 1H), 8.04 - 7.97 (m, 2H), 7.87 (d, *J* = 2.1 Hz, 1H), 6.89 (dd, *J =* 5.3, 1.6 Hz, 1H), 6.81 (s, 1H), 6.00 (s, 2H), 4.90 (t, *J =* 5.3 Hz, 1H), 4.08 (t, *J =* 5.7 Hz, 2H), 3.98 (s, 1H), 3.74 (q, *J =* 5.6 Hz, 2H), 2.23 (s, 3H), 1.38 (d, *J =* 6.9 Hz, 6H). MS (ESI) *m*/*z*: 404 [M+H]⁺.** |
| **I-15** | 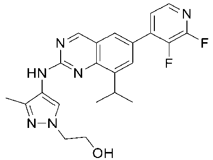 | **MS (ESI) m/z: 425 [M+H]⁺.** |
| **I-16** | 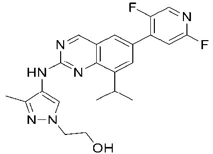 | **MS (ESI) m/z: 425 [M+H]⁺.** |
| **I-17** | 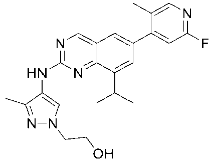 | **MS (ESI) m/z: 421 [M+H]⁺.** |
| **I-18** | 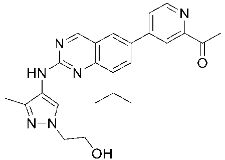 | **¹H NMR (600 MHz, DMSO-*d*₆) δ 9.51 - 9.41 (m, 1H), 9.39 (s, 1H), 8.88 (d, *J =* 5.1 Hz, 1H), 8.41 - 8.37 (m, 1H), 8.36 (d, *J =* 2.1 Hz, 1H), 8.18 (d, *J =* 5.2 Hz, 1H), 8.11 (d, *J =* 2.1 Hz, 1H), 4.98 (t, *J =* 5.3 Hz, 1H), 4.14 (t, *J =* 5.6 Hz, 2H), 4.08 - 4.00 (m, 1H), 3.80 (q, *J =* 5.6 Hz, 2H), 2.77 (s, 3H), 2.30 (s, 3H), 1.46 (d, *J =* 6.9 Hz, 6H). MS (ESI) *m*/*z:* [431 M+H]⁺.** |
| **I-19** | 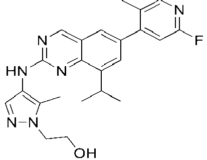 | **¹H NMR (600 MHz, DMSO-*d*₆) δ 9.22 (s, 1H), 9.19 (s, 1H), 8.18 (s, 1H), 8.00 (s, 1H), 7.80 (d, *J =* 2.0 Hz, 1H), 7.67 (d, *J =* 2.0 Hz, 1H), 7.17 (d, *J* = 1.7 Hz, 1H), 4.91 (t, *J =* 5.4 Hz, 1H), 4.07 (t, *J =* 5.9 Hz, 2H), 3.95 (s, 1H), 3.71 (q, *J =* 5.8 Hz, 2H), 2.29 (s, 3H), 2.28 (s, 3H), 1.31 (d, *J =* 6.9 Hz, 6H). MS (ESI) m/z: 421 [M+H]⁺.** |
| **I-20** | 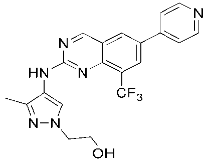 | **¹H NMR (600 MHz, DMSO-*d*₆) δ 9.93 (s, 1H), 9.42 (s, 1H), 8.86 (d, *J =* 5.6 Hz, 2H), 8.78 (d, *J =* 2.2 Hz, 1H), 8.58 (d, *J =* 2.1 Hz, 1H), 8.35 (s, 1H), 8.23 (d, *J =* 5.6 Hz, 2H), 4.05 (t, *J =* 5.9 Hz, 2H), 3.74 (t, *J =* 5.9 Hz, 2H), 2.26 (s, 3H). MS (ESI) m/z: 415 [M+H]⁺.** |
| **I-21** | 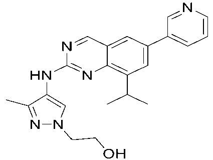 | **¹H NMR (600 MHz, DMSO-*d*₆) δ 9.29 - 9.24 (m, 2H), 9.01 (dd, *J=* 2.5, 0.9 Hz, 1H), 8.59 (dd, *J =* 4.7, 1.6 Hz, 1H), 8.32 - 8.24 (m, 1H), 8.18 (ddd, *J =* 8.0, 2.5, 1.6 Hz, 1H), 8.09 (d, *J =* 2.1 Hz, 1H), 7.97 (d, *J =* 2.1 Hz, 1H), 7.53 (ddd, *J =* 7.9, 4.7, 0.9 Hz, 1H), 4.90 (t, *J =* 5.3 Hz, 1H), 4.08 (t, *J =* 5.7 Hz, 2H), 4.02 - 3.97 (m, 1H), 3.76 - 3.72 (m, 2H), 2.23 (s, 3H), 1.39 (d, *J =* 6.9 Hz, 6H). MS (ESI) m/z: 389 [M+H]⁺.** |
| **I-22** | 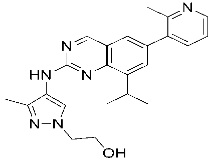 | **MS (ESI) *m*/*z:* 403 [M+H]⁺.** |
| **I-23** | 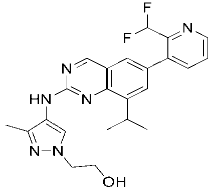 | **¹H NMR (600 MHz, DMSO-*d*₆) δ 9.34 - 9.26 (m, 2H), 8.77 (d, *J =* 4.7 Hz, 1H), 8.35 - 8.27 (m, 1H), 8.01 (d, *J* = 7.8 Hz, 1H), 7.75 (s, 1H), 7.71 (dd, *J =* 7.7, 5.0 Hz, 1H), 7.65 (s, 1H), 6.89 (t, *J =* 53.6 Hz, 1H), 4.92 (t, *J*= 5.3 Hz, 1H), 4.09 (t, *J =* 5.7 Hz, 2H), 4.03 - 3.95 (m, 1H), 3.75 (q, *J=* 5.6 Hz, 2H), 2.24 (s, 3H), 1.36 (d, *J=* 6.9 Hz, 6H). MS (ESI) m/z: 439 [M+H]⁺.** |
| **I-24** | 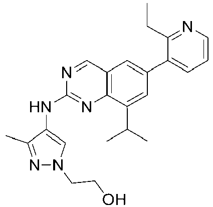 | **¹H NMR (600 MHz, DMSO-*d*₆) δ 9.37 - 9.31 (m, 1H), 9.28 (s, 1H), 8.80 (d, *J =* 5.5 Hz, 1H), 8.32 (d, *J =* 8.0 Hz, 1H), 7.84 (t, *J =* 6.8 Hz, 1H), 7.80 (s, 1H), 7.68 (s, 1H), 4.14 - 4.05 (m, 2H), 4.03 - 3.94 (m, 1H), 3.75 (t, *J* = 5.8 Hz, 2H), 2.93 (q, *J =* 7.6 Hz, 2H), 2.24 (s, 3H), 1.35 (d, *J =* 6.9 Hz, 6H), 1.20 (t, *J =* 7.5 Hz, 3H). MS (ESI) m/z: 417 [M+H]⁺.** |
| **I-25** | 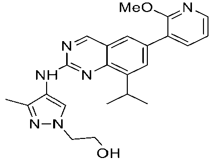 | **¹H NMR (600 MHz, DMSO-*d*₆) δ 9.24 (s, 1H), 9.23 - 9.17 (m, 1H), 8.35 - 8.24 (m, 1H), 8.21 (d, *J =* 5.0 Hz, 1H), 7.90 (d, *J =* 2.0 Hz, 1H), 7.87 (d, *J =* 7.4 Hz, 1H), 7.83 (d, *J =* 2.0 Hz, 1H), 7.14 (dd, *J =* 7.3, 4.9 Hz, 1H), 4.09 (t, *J =* 5.6 Hz, 2H), 4.02 - 3.95 (m, 1H), 3.93 (s, 3H), 3.75 (t, *J =* 5.6 Hz, 2H), 2.24 (s, 3H), 1.36 (d, *J =* 6.9 Hz, 6H). MS (ESI) m/z: 419 [M+H]⁺.** |
| **I-26** | 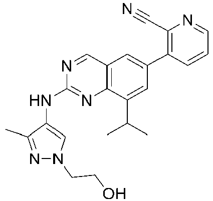 | **MS (ESI) m/z: 414 [M+H]⁺.** |
| **I-27** | 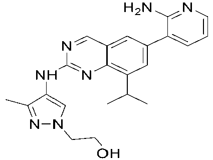 | **MS (ESI) *m*/*z*: 404 [M+H]⁺.** |
| **I-28** | 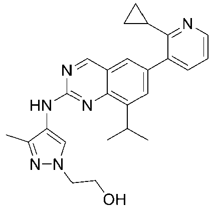 | **¹H NMR (600 MHz, DMSO-*d*₆) δ 9.26 (s, 1H), 9.25 - 9.18 (m, 1H), 8.45 (dd, *J* = 4.8,1.7 Hz, 1H), 8.36 - 8.25 (m, 1H), 7.80 (d, *J* = 2.0 Hz, 1H), 7.74 (d, *J =* 2.0 Hz, 1H), 7.69 (dd, *J* = 7.6, 1.7 Hz, 1H), 7.25 (dd, *J* = 7.6, 4.7 Hz, 1H), 4.94 - 4.87 (m, 1H), 4.13 - 4.05 (m, 2H), 4.05 - 3.95 (m, 1H), 3.80 - 3.72 (m, 2H), 2.23 (s, 3H), 2.06 - 2.00 (m, 1H), 1.36 (d, *J* = 6.9 Hz, 6H), 1.09 - 1.04 (m, 2H), 0.95 - 0.86 (m, 2H). MS (ESI) m/z: 429 [M+H]⁺.** |
| **I-29** | 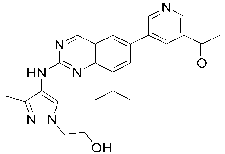 | **MS (ESI) *m*/*z:* 431 [M+H]⁺.** |
| **I-30** | 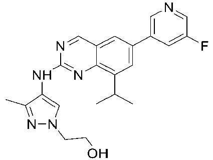 | **¹H NMR (600 MHz, DMSO-*d*₆) δ 9.38 - 9.28 (m, 1H), 9.25 (s, 1H), 8.92 (t, *J* = 1.9 Hz, 1H), 8.59 (d, *J* = 2.7 Hz, 1H), 8.34 - 8.24 (m, 1H), 8.19 (dt, *J =* 10.4, 2.3 Hz, 1H), 8.16 (d, *J =* 2.1 Hz, 1H), 8.01 (d, *J =* 2.1 Hz, 1H), 4.90 (t, *J =* 5.3 Hz, 1H), 4.08 (t, *J =* 5.6 Hz, 2H), 4.04 - 3.92 (m, 1H), 3.74 (q, *J =* 5.6 Hz, 2H), 2.23 (s, 3H), 1.40 (d, *J =* 6.9 Hz, 6H). MS (ESI) *m*/*z*: 407 [M+H]⁺.** |
| **I-31** | 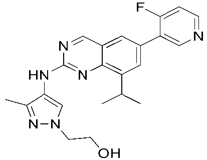 | **¹H NMR (600 MHz, DMSO-*d*₆) δ 9.44 - 9.34 (m, 1H), 9.30 (s, 1H), 8.69 (d, *J =* 2.6 Hz, 1H), 8.54 (d, *J* = 4.9 Hz, 1H), 8.36 - 8.24 (m, 1H), 8.07 (t, *J* = 1.6 Hz, 1H), 7.88 (t, *J =* 1.7 Hz, 1H), 7.76 (dd, *J =* 7.1, 4.9 Hz, 1H), 4.90 (t, *J =* 5.3 Hz, 1H), 4.08 (t, *J =* 5.6 Hz, 2H), 4.03 - 3.94 (m, 1H), 3.74 (q, *J =* 5.6 Hz, 2H), 2.23 (s, 3H), 1.37 (d, *J =* 6.9 Hz, 6H). MS (ESI) *m*/*z*: 407 [M+H]⁺.** |
| **I-32** | 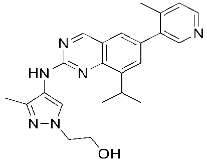 | **MS (ESI) *m*/*z:* 403 [M+H]⁺.** |
| **I-33** | 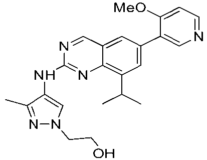 | **¹H NMR (600 MHz, DMSO-*d*₆) δ 9.30 - 9.11 (m, 2H), 8.53 - 8.45 (m, 2H), 8.35 - 8.22 (m, 1H), 7.85 (s, 1H), 7.78 (s, 1H), 7.21 (d, *J =* 5.7 Hz, 1H), 4.91 (t, *J =* 5.3 Hz, 1H), 4.08 (t, *J =* 5.6 Hz, 2H), 4.01 - 3.94 (m, 1H), 3.90 (s, 3H), 3.75 (q, *J =* 5.6 Hz, 2H), 2.23 (s, 3H), 1.36 (d, *J =* 6.9 Hz, 6H). MS (ESI) *m*/*z*: 419 [M+H]⁺.** |
| **I-34** | 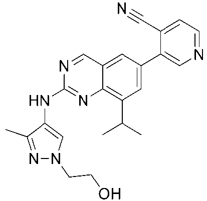 | **¹H NMR (600 MHz, DMSO-*d*₆) δ 9.47 - 9.35 (m, 1H), 9.30 (s, 1H), 9.04 (s, 1H), 8.84 (d, *J =* 5.0 Hz, 1H), 8.36 - 8.23 (m, 1H), 8.04 (d, *J =* 2.1 Hz, 1H), 8.01 (d, *J =* 5.0 Hz, 1H), 7.95 (d, *J* = 2.1 Hz, 1H), 4.90 (t, *J* = 5.3 Hz, 1H), 4.08 (t, *J =* 5.6 Hz, 2H), 4.06 - 3.96 (m, 1H), 3.75 (q, *J* = 5.5 Hz, 2H), 2.24 (s, 3H), 1.38 (d, *J* = 6.9 Hz, 6H). MS (ESI) *m*/*z*: 414 [M+H]⁺.** |
| **I-35** | 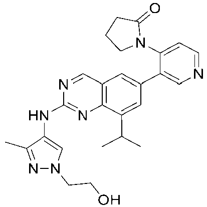 | **¹H NMR (600 MHz, DMSO-*d*₆) δ 9.39 - 9.29 (m, 1H), 9.27 (s, 1H), 8.82 (s, 1H), 8.73 (d, *J =* 5.7 Hz, 1H), 8.35 - 8.24 (m, 1H), 7.83 (s, 1H), 7.72 (d, *J =* 5.6 Hz, 1H), 7.60 (s, 1H), 4.08 (t, *J* = 5.7 Hz, 2H), 4.01 - 3.92 (m, 1H), 3.78 - 3.71 (m, 4H), 2.29 (t, *J =* 7.9 Hz, 2H), 2.23 (s, 3H), 1.97 (p, *J* = 7.5 Hz, 2H), 1.31 (d, *J* = 6.9 Hz, 6H). MS (ESI) *m*/*z*: 472 [M+H]⁺.** |
| **I-36** | 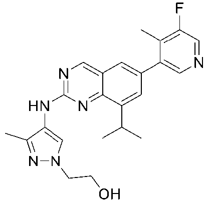 | **MS (ESI) m/z: 421 [M+H]⁺.** |
| **I-37** | 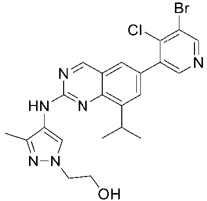 | **¹H NMR (600 MHz, DMSO-*d*₆) δ 9.43 - 9.35 (m, 1H), 9.32 (s, 1H), 8.96 (s, 1H), 8.71 (s, 1H), 8.40 - 8.29 (m, 1H), 7.93 (s, 1H), 7.82 (s, 1H), 4.96 (t, *J =* 5.3 Hz, 1H), 4.14 (t, *J =* 5.6 Hz, 2H), 4.08 - 4.00 (m, 1H), 3.81 (q, *J* = 5.5 Hz, 2H), 2.29 (s, 3H), 1.41 (d, *J =* 6.9 Hz, 6H). MS (ESI) m/z: 501 [M+H]⁺.** |
| **I-38** | 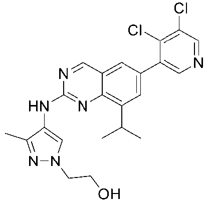 | **¹H NMR (600 MHz, DMSO-*d*₆) δ 9.40 - 9.29 (m, 1H), 9.26 (s, 1H), 8.82 (s, 1H), 8.65 (s, 1H), 8.35 - 8.20 (m, 1H), 7.88 (d, *J =* 2.0 Hz, 1H), 7.77 (d, *J =* 2.0 Hz, 1H), 4.90 (t, *J =* 5.3 Hz, 1H), 4.08 (t, *J =* 5.7 Hz, 2H), 3.98 (s, 1H), 3.75 (q, *J =* 5.5 Hz, 2H), 2.23 (s, 3H), 1.35 (d, *J* = 6.9 Hz, 6H). MS (ESI) m/z: 457 [M+H]⁺.** |
| **I-39** | 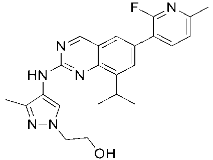 | **¹H NMR (600 MHz, DMSO-*d*₆) δ 9.33 - 9.22 (m, 2H), 8.36 - 8.22 (m, 1H), 8.11 (dd, *J* = 10.5, 7.6 Hz, 1H), 7.94 (s, 1H), 7.82 (s, 1H), 7.36 (d, *J =* 7.6 Hz, 1H), 4.89 (t, *J* = 5.3 Hz, 1H), 4.08 (t, *J =* 5.7 Hz, 2H), 4.03 - 3.92 (m, 1H), 3.74 (q, *J =* 5.4 Hz, 2H), 2.50 (s, 3H), 2.23 (s, 3H), 1.36 (d, *J* = 6.9 Hz, 6H). MS (ESI) m/z: 421 [M+H]⁺.** |
| **I-40** | 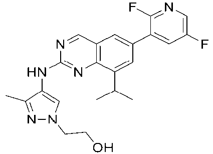 | **MS (ESI) m/z: 425 [M+H]⁺.** |
| **I-41** | 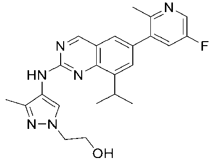 | **¹H NMR (600 MHz, DMSO-*d*₆) δ 9.31 - 9.23 (m, 2H), 8.52 (d, *J =* 2.9 Hz, 1H), 8.33 - 8.26 (m, 1H), 7.79 (d, *J* = 2.1 Hz, 1H), 7.76 (dd, *J =* 9.4, 2.9 Hz, 1H), 7.70 (d, *J* = 2.1 Hz, 1H), 4.08 (t, *J =* 5.6 Hz, 2H), 4.02 - 3.96 (m, 1H), 3.75 (t, *J =* 5.6 Hz, 2H), 2.49 (s, 3H), 2.23 (s, 3H), 1.36 (d, *J =* 6.9 Hz, 6H). MS (ESI) *m*/*z*: 421 [M+H]⁺.** |
| **I-42** | 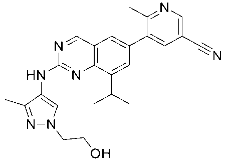 | **¹H NMR (600 MHz, DMSO-*d*₆) δ 9.43 - 9.36 (m, 1H), 9.29 (s, 1H), 8.63 (dd, *J =* 1.9, 0.7 Hz, 1H), 8.35 - 8.24 (m, 1H), 8.08 (dd, *J* = 2.0, 0.9 Hz, 1H), 8.00 (d, *J =* 2.1 Hz, 1H), 7.91 (d, *J =* 2.1 Hz, 1H), 4.90 (t, *J* = 5.3 Hz, 1H), 4.11 - 4.05 (m, 2H), 4.06 - 3.97 (m, 1H), 3.79 - 3.72 (m, 2H), 2.49 (s, 3H), 2.24 (s, 3H), 1.37 (d, *J =* 6.9 Hz, 6H). MS (ESI) m/z: 428 [M+H]⁺.** |
| **I-43** | 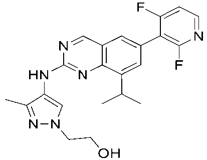 | **¹H NMR (600 MHz, DMSO-*d*₆) δ 9.37 (s, 1H), 9.27 (s, 1H), 8.39 - 8.25 (m, 2H), 7.90 (s, 1H), 7.74 (s, 1H), 7.53 (dd, *J =* 8.4, 5.7 Hz, 1H), 4.94 (t, *J =* 5.3 Hz, 1H), 4.08 (t, *J =* 5.6 Hz, 2H), 4.02 - 3.93 (m, 1H), 3.74 (q, *J =* 5.5 Hz, 2H), 2.24 (s, 3H), 1.34 (d, *J* = 6.9 Hz, 6H). MS (ESI) *m*/*z*: 425 [M+H]⁺.** |
| **I-44** | 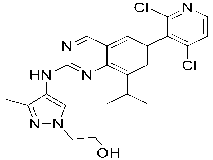 | **¹H NMR (600 MHz, DMSO-*d*₆) δ 9.38 - 9.27 (m, 1H), 9.24 (s, 1H), 8.45 (d, *J =* 5.3 Hz, 1H), 8.33 - 8.23 (m, 1H), 7.79 (d, *J =* 5.4 Hz, 1H), 7.74 (d, *J =* 2.0 Hz, 1H), 7.58 (d, *J =* 2.0 Hz, 1H), 4.90 (t, *J =* 5.1 Hz, 1H), 4.08 (t, *J* = 5.5 Hz, 2H), 4.03 - 3.90 (m, 1H), 3.74 (q, *J =* 5.3 Hz, 2H), 2.23 (s, 3H), 1.38 - 1.30 (m, 6H). MS (ESI) *m*/*z*: 457 [M+H]⁺.** |
| **I-45** | 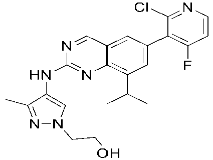 | **MS (ESI) m/z: 441 [M+H]⁺.** |
| **I-46** | 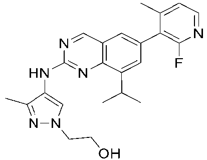 | **¹H NMR (600 MHz, DMSO-*d*₆) δ 9.36 - 9.26 (m, 1H), 9.23 (s, 1H), 8.37 - 8.25 (m, 1H), 8.14 (d, *J* = 5.1 Hz, 1H), 7.73 (d, *J =* 1.9 Hz, 1H), 7.59 (d, *J =* 1.9 Hz, 1H), 7.38 (d, *J =* 5.1 Hz, 1H), 4.09 (t, *J =* 5.6 Hz, 2H), 4.04 - 3.94 (m, 1H), 3.75 (t, *J* = 5.6 Hz, 2H), 2.26 (s, 3H), 2.24 (s, 3H), 1.34 (d, *J* = 6.9 Hz, 6H). MS (ESI) m/z: 421 [M+H]⁺.** |
| **I-47** | 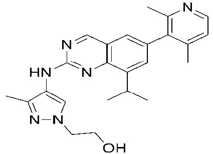 | **MS (ESI) m/z: 417 [M+H]⁺.** |
| **I-48** | 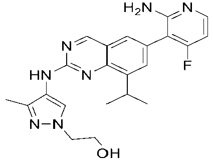 | **¹H NMR (600 MHz, DMSO-*d*₆) δ 9.28 - 9.10 (m, 2H), 8.36 - 8.20 (m, 1H), 7.98 (dd, *J =* 8.8, 5.7 Hz, 1H), 7.72 (d, *J* = 1.9 Hz, 1H), 7.53 (s, 1H), 6.58 (dd, *J =* 8.9, 5.7 Hz, 1H), 5.90 (s, 2H), 4.90 (t, *J =* 5.4 Hz, 1H), 4.07 (t, *J* = 5.8 Hz, 2H), 3.99 - 3.90 (m, 1H), 3.74 (q, *J =* 5.6 Hz, 2H), 2.23 (s, 3H), 1.34 (d, *J* = 6.9 Hz, 6H). MS (ESI) *m*/*z:* 422 [M+H]⁺.** |
| **I-49** | 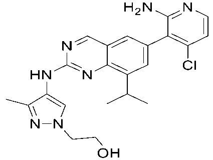 | **¹H NMR (600 MHz, DMSO-*d*₆) δ 9.37 - 9.27 (m, 1H), 9.25 (s, 1H), 8.36 - 8.22 (m, 1H), 8.02 (d, *J* = 6.6 Hz, 1H), 7.72 (s, 1H), 7.52 (s, 1H), 7.49 - 7.25 (m, 2H), 7.12 (d, *J =* 6.6 Hz, 1H), 4.08 (t, *J =* 5.7 Hz, 2H), 4.02 - 3.94 (m, 1H), 3.75 (t, *J =* 5.7 Hz, 2H), 2.23 (s, 3H), 1.34 (t, *J* = 7.8 Hz, 6H). MS (ESI) m/z: 438 [M+H]⁺.** |
| **I-50** | 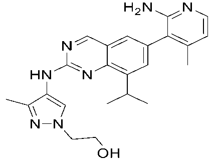 | **¹H NMR (600 MHz, DMSO-*d*₆) δ 9.33 - 9.26 (m, 1H), 9.23 (s, 1H), 8.37 - 8.22 (m, 1H), 7.94 (d, *J =* 6.6 Hz, 1H), 7.66 (d, *J =* 1.9 Hz, 1H), 7.47 (d, *J =* 1.9 Hz, 1H), 7.40 (s, 2H), 6.96 (d, *J =* 6.6 Hz, 1H), 4.08 (t, *J =* 5.6 Hz, 2H), 4.02 - 3.92 (m, 1H), 3.74 (t, *J =* 5.7 Hz, 2H), 2.23 (s, 3H), 2.10 (s, 3H), 1.35 (d, *J =* 6.9 Hz, 3H), 1.33 (d, *J =* 6.9 Hz, 3H). MS (ESI) *m*/*z*: 418 [M+H]⁺.** |
| **I-51** | 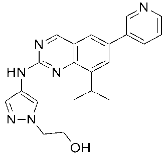 | **MS (ESI) m/z: 375 [M+H]⁺.** |
| **I-52** | 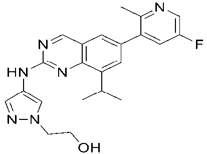 | **MS (ESI) m/z: 407 [M+H]⁺.** |
| **I-53** | 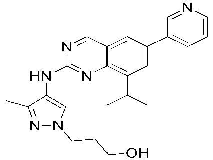 | **¹H NMR (600 MHz, DMSO-*d*₆) δ 9.40 - 9.30 (m, 1H), 9.28 (s, 1H), 9.18 (d, *J =* 2.2 Hz, 1H), 8.74 (d, *J =* 5.1 Hz, 1H), 8.57 (d, *J =* 8.1 Hz, 1H), 8.25 - 8.19 (m, 1H), 8.17 (d, *J =* 2.1 Hz, 1H), 8.02 (d, *J =* 2.1 Hz, 1H), 7.83 (dd, *J =* 8.0, 5.2 Hz, 1H), 4.11 (t, *J =* 6.9 Hz, 2H), 4.01 - 3.93 (m, 1H), 3.44 (t, *J =* 6.2 Hz, 2H), 2.23 (s, 3H), 1.93 (p, *J =* 6.5 Hz, 2H), 1.40 (d, *J =* 6.9 Hz, 6H). MS (ESI) *m*/*z*: 403 [M+H]⁺.** |
| **I-54** | 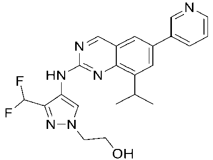 | **¹H NMR (600 MHz, DMSO-*d*₆) δ 9.59 - 9.43 (m, 1H), 9.34 (s, 1H), 9.16 (s, 1H), 8.73 (d, *J =* 5.1 Hz, 1H), 8.56 - 8.41 (m, 2H), 8.20 (s, 1H), 8.06 (s, 1H), 7.79 (dd, *J =* 8.0, 5.1 Hz, 1H), 7.31 (t, *J =* 53.8 Hz, 1H), 4.24 (t, *J =* 5.4 Hz, 2H), 4.06 - 3.98 (m, 1H), 3.80 (t, *J* = 5.4 Hz, 2H), 1.41 (d, *J* = 6.9 Hz, 6H). MS (ESI) m/z: 425 [M+H]⁺.** |
| **I-55** | 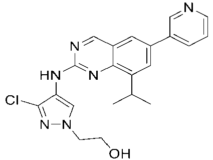 | **¹H NMR (600 MHz, DMSO-*d*₆) δ 9.32 (s, 1H), 9.27 - 9.20 (m, 1H), 9.16 (s, 1H), 8.77 - 8.68 (m, 1H), 8.50 (d, *J* = 8.0 Hz, 1H), 8.38 - 8.22 (m, 1H), 8.19 (d, *J =* 2.1 Hz, 1H), 8.04 (d, *J =* 2.1 Hz, 1H), 7.78 (dd, *J =* 8.1, 5.0 Hz, 1H), 4.15 (t, *J =* 5.4 Hz, 2H), 4.02 - 3.93 (m, 1H), 3.77 (t, *J =* 5.4 Hz, 2H), 1.38 (d, *J =* 6.9 Hz, 6H). MS (ESI) *m*/*z:* 409 [M+H]⁺.** |
| **I-56** | 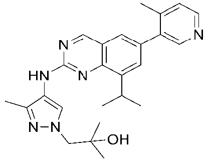 | **MS (ESI) *m*/*z:* 431 [M+H]⁺.** |
| **I-57** | 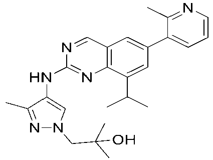 | **MS (ESI) *m*/*z:* 431 [M+H]⁺.** |
| **I-58** | 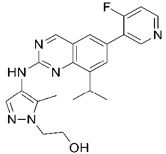 | **MS (ESI) m/z: 407 [M+H]⁺.** |
| **I-59** | 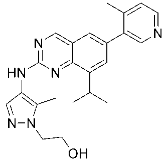 | **¹H NMR (600 MHz, DMSO-*d*₆) δ 9.21 (s, 1H), 9.15 (s, 1H), 8.47 (s, 1H), 8.44 (d, *J* = 5.0 Hz, 1H), 8.03 (s, 1H), 7.72 (d, *J* = 2.0 Hz, 1H), 7.63 (d, *J =* 2.0 Hz, 1H), 7.37 (d, *J* = 5.0 Hz, 1H), 4.93 (t, *J* = 5.4 Hz, 1H), 4.07 (t, *J =* 5.9 Hz, 2H), 3.96 (s, 1H), 3.71 (q, *J* = 5.8 Hz, 2H), 2.32 (s, 3H), 2.30 (s, 3H), 1.31 (d, *J* = 6.9 Hz, 6H). MS (ESI) *m*/*z*: 403 [M+H]⁺.** |
| **I-60** | 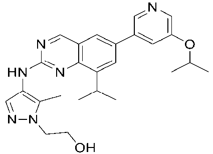 | **MS (ESI) *m*/*z*: 447 [M+H]⁺.** |
| **I-61** | 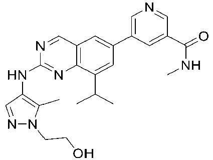 | **¹H NMR (600 MHz, DMSO-*d*₆) δ 9.25 (s, 1H), 9.23 - 9.16 (m, 1H), 9.12 (d, *J* = 2.2 Hz, 1H), 8.97 (d, *J* = 2.0 Hz, 1H), 8.80 - 8.73 (m, 1H), 8.50 (t, *J* = 2.2 Hz, 1H), 8.13 (d, *J* = 2.1 Hz, 1H), 8.02 (d, *J* = 2.1 Hz, 1H), 4.87 (t, *J* = 5.4 Hz, 1H), 4.08 (t, *J* = 5.9 Hz, 2H), 4.03 - 3.93 (m, 1H), 3.72 (q, *J* = 5.8 Hz, 2H), 2.86 (d, *J* = 4.5 Hz, 3H), 2.31 (s, 3H), 1.37 (d, *J* = 6.9 Hz, 6H). MS (ESI) *m*/*z*: 446 [M+H]⁺.** |
| **I-62** | 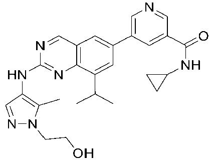 | **¹H NMR (600 MHz, DMSO-*d*₆) δ 9.25 (s, 1H), 9.23 - 9.15 (m, 1H), 9.11 (d, *J* = 2.2 Hz, 1H), 8.95 (d, *J* = 2.0 Hz, 1H), 8.78 - 8.73 (m, 1H), 8.47 (d, *J* = 2.3 Hz, 1H), 8.12 (d, *J* = 2.1 Hz, 1H), 8.04 - 7.98 (m, 1H), 4.08 (t, *J* = 5.9 Hz, 2H), 4.02 - 3.91 (m, 1H), 3.72 (t, *J* = 5.9 Hz, 2H), 2.92 - 2.88 (m, 1H), 2.31 (s, 3H), 1.37 (d, *J* = 6.9 Hz, 7H), 0.81 - 0.70 (m, 2H), 0.68 - 0.55 (m, 2H). MS (ESI) *m*/*z*: 472 [M+H]⁺.** |
| **I-63** | 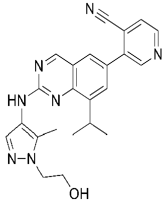 | **MS (ESI) *m*/*z*: 414 [M+H]⁺.** |
| **I-64** | 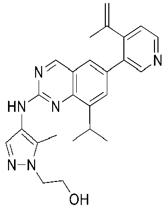 | **¹H NMR (600 MHz, DMSO-*d*₆) δ 9.31 - 9.20 (m, 2H), 8.82 (s, 1H), 8.73 (d, *J* = 5.5 Hz, 1H), 8.10 - 7.99 (m, 1H), 7.84 (d, *J* = 1.9 Hz, 1H), 7.69 (d, *J* = 1.9 Hz, 1H), 7.67 (d, *J* = 5.5 Hz, 1H), 5.33 (s, 1H), 5.14 (s, 1H), 4.08 (t, *J* = 5.9 Hz, 2H), 3.96 - 3.93 (m, 1H), 3.72 (t, *J* = 5.9 Hz, 2H), 2.31 (s, 3H), 1.76 (s, 3H), 1.28 (d, *J* = 6.9 Hz, 6H). MS (ESI) *m*/*z*: 429 [M+H]⁺.** |
| **I-65** | 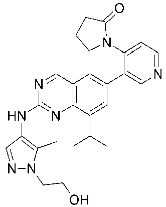 | **¹H NMR (600 MHz, DMSO-*d*₆) δ 9.23 (s, 1H), 9.21 - 9.09 (m, 1H), 8.69 (d, *J* = 0.6 Hz, 1H), 8.62 (d, *J* = 5.3 Hz, 1H), 8.10 - 7.93 (m, 1H), 7.77 (d, *J* = 2.0 Hz, 1H), 7.55 (d, *J* = 2.0 Hz, 1H), 7.48 (d, *J* = 5.1 Hz, 1H), 4.87 (t, *J* = 5.4 Hz, 1H), 4.07 (t, *J* = 5.9 Hz, 2H), 3.99 - 3.89 (m, 1H), 3.72 (q, *J* = 5.8 Hz, 2H), 3.51 (t, *J* = 6.9 Hz, 2H), 2.30 (s, 3H), 2.27 (t, *J* = 8.0 Hz, 2H), 1.94 (p, *J* = 7.5 Hz, 2H), 1.28 (d, *J* = 6.9 Hz, 6H). MS (ESI) *m*/*z*: 472 [M+H]⁺.** |
| **I-66** | 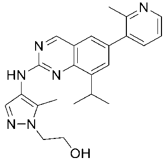 | **MS (ESI) *m*/*z:* 403 [M+H]⁺.** |
| **I-67** | 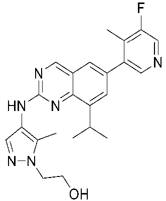 | **¹H NMR (600 MHz, DMSO-*d*₆) δ 9.22 (s, 1H), 9.16 (s, 1H), 8.51 (s, 1H), 8.39 (s, 1H), 8.02 (s, 1H), 7.76 (d, *J* = 2.0 Hz, 1H), 7.65 (d, *J* = 2.0 Hz, 1H), 4.91 (t, *J* = 5.4 Hz, 1H), 4.07 (t, *J* = 5.9 Hz, 2H), 3.96 (s, 1H), 3.71 (q, *J* = 5.7 Hz, 2H), 2.29 (s, 3H), 2.26 (d, *J* = 2.0 Hz, 3H), 1.31 (d, *J* = 6.9 Hz, 6H). MS (ESI) *m*/*z*: 421 [M+H]⁺.** |
| **I-68** | 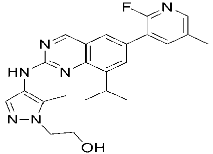 | **MS (ESI) *m*/*z*: 421 [M+H]⁺.** |
| **I-69** | 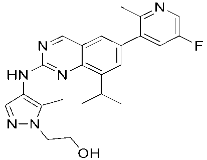 | **¹H NMR (600 MHz, DMSO-*d*₆) δ 9.28 - 9.13 (m, 2H), 8.53 (d, *J* = 2.7 Hz, 1H), 8.14 - 7.95 (m, 1H), 7.83 - 7.75 (m, 2H), 7.69 (s, 1H), 4.09 (t, *J =* 5.9 Hz, 2H), 4.01 - 3.93 (m, 1H), 3.73 (t, *J* = 5.9 Hz, 2H), 2.49 (s, 3H), 2.32 (s, 3H), 1.33 (d, *J* = 6.9 Hz, 6H). MS (ESI) *m*/*z*: 421 [M+H]⁺.** |
| **I-70** | 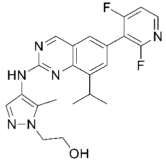 | **MS (ESI) *m*/*z*: 425 [M+H]⁺.** |
| **I-71** | 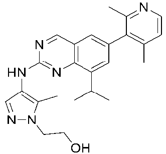 | **MS (ESI) *m*/*z*: 417 [M+H]⁺.** |
| **I-72** | 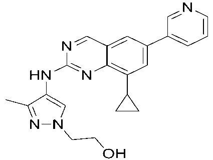 | **MS (ESI) *m*/*z*: 387 [M+H]⁺.** |
| **I-73** | 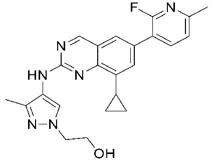 | **MS (ESI) *m*/*z*: 419 [M+H]⁺.** |
| **I-74** | 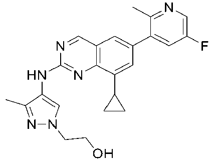 | **MS (ESI) *m*/*z*: 419 [M+H]⁺.** |
| **I-75** | 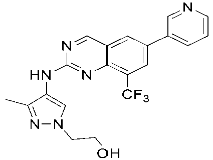 | **¹H NMR (600 MHz, DMSO-*d*₆) δ 9.75 (s, 1H), 9.39 (s, 1H), 9.05 (d, *J* = 2.4 Hz, 1H), 8.63 (dd, *J* = 4.8, 1.5 Hz, 1H), 8.55 (d, *J* = 2.1 Hz, 1H), 8.44 (d, *J* = 2.1 Hz, 1H), 8.36 (s, 1H), 8.25 (dt, *J* = 8.1, 1.9 Hz, 1H), 7.55 (dd, *J* = 7.9, 4.7 Hz, 1H), 4.88 (t, *J* = 5.4 Hz, 1H), 4.05 (t, *J* = 5.9 Hz, 2H), 3.74 (q, *J* = 5.7 Hz, 2H), 2.25 (s, 3H). MS (ESI) *m*/*z*: 415 [M+H]⁺.** |
| **I-76** | 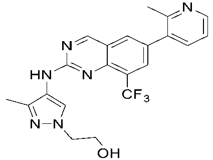 | **¹H NMR (600 MHz, DMSO-*d*₆) δ 9.79 (s, 1H), 9.39 (s, 1H), 8.74 (d, *J* = 5.4 Hz, 1H), 8.36 (s, 1H), 8.31 - 8.24 (m, 2H), 8.22 (d, *J* = 2.0 Hz, 1H), 7.77 (t, *J* = 6.6 Hz, 1H), 4.05 (t, *J* = 5.9 Hz, 2H), 3.74 (t, *J* = 5.9 Hz, 2H), 2.63 (s, 3H), 2.26 (s, 3H). MS (ESI) *m*/*z*: 429 [M+H]⁺.** |
| **I-77** | 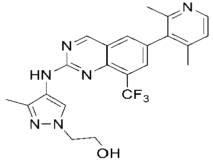 | **¹H NMR (600 MHz, DMSO-*d*₆) δ 9.70 (s, 1H), 9.34 (s, 1H), 8.39 - 8.35 (m, 2H), 8.04 (s, 1H), 7.99 (s, 1H), 7.24 (d, *J* = 5.0 Hz, 1H), 4.94 - 4.83 (m, 1H), 4.05 (t, *J* = 5.8 Hz, 2H), 3.73 (q, *J* = 5.7 Hz, 2H), 2.26 (s, 3H), 2.23 (s, 3H), 2.06 (s, 3H). MS (ESI) *m*/*z*: 443 [M+H]⁺.** |
| **I-78** | 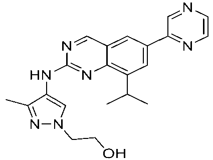 | **¹H NMR (600 MHz, DMSO-*d*₆) δ 9.44 - 9.34 (m, 2H), 9.30 (s, 1H), 8.75 (dd, *J* = 2.5, 1.5 Hz, 1H), 8.62 (d, *J* = 2.5 Hz, 1H), 8.53 (d, *J =* 2.1 Hz, 1H), 8.40 (d, *J* = 2.0 Hz, 1H), 8.34 - 8.26 (m, 1H), 4.90 (t, *J =* 5.3 Hz, 1H), 4.08 (t, *J =* 5.6 Hz, 2H), 4.04 - 3.95 (m, 1H), 3.74 (q, *J* = 5.5 Hz, 2H), 2.23 (s, 3H), 1.39 (d, *J* = 6.9 Hz, 6H). MS (ESI) *m*/*z*: 390 [M+H]⁺.** |
| **I-79** | 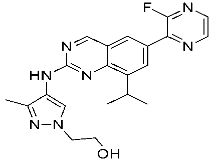 | **¹H NMR (600 MHz, DMSO-*d*₆) δ 9.49 - 9.40 (m, 1H), 9.37 (s, 1H), 8.78 (dd, *J =* 4.9, 2.5 Hz, 1H), 8.40 (t, *J =* 1.8 Hz, 1H), 8.35 (t, *J* = 2.1 Hz, 1H), 8.32 (d, *J* = 2.0 Hz, 2H), 4.93 (t, *J* = 5.3 Hz, 1H), 4.09 (t, *J* = 5.7 Hz, 2H), 4.03 - 3.96 (m, 1H), 3.75 (q, *J* = 5.6 Hz, 2H), 2.24 (s, 3H), 1.38 (d, *J* = 6.9 Hz, 6H). MS (ESI) *m*/*z*: 408 [M+H]⁺.** |
| **I-80** | 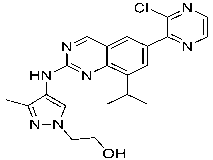 | **¹H NMR (600 MHz, DMSO-*d*₆) δ 9.42 - 9.36 (m, 1H), 9.32 (s, 1H), 8.81 (d, *J =* 2.4 Hz, 1H), 8.54 (d, *J* = 2.4 Hz, 1H), 8.35 - 8.27 (m, 1H), 8.21 (d, *J =* 2.1 Hz, 1H), 8.04 (d, *J =* 2.1 Hz, 1H), 4.90 (t, *J =* 5.3 Hz, 1H), 4.08 (t, *J* = 5.6 Hz, 2H), 4.03 - 3.92 (m, 1H), 3.75 (q, *J* = 5.6 Hz, 2H), 2.24 (s, 3H), 1.36 (d, *J* = 6.9 Hz, 6H). MS (ESI) *m*/*z*: 424 [M+H]⁺.** |
| **I-81** | 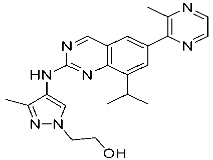 | **¹H NMR (400 MHz, DMSO-*d*₆) δ 9.28 (s, 2H), 8.60 (d, *J =* 2.4 Hz, 1H), 8.54 (d, *J* = 2.4 Hz, 1H), 8.29 (s, 1H), 8.03 (d, *J =* 2.0 Hz, 1H), 7.93 (d, *J =* 2.0 Hz, 1H), 4.89 (t, *J* = 5.2 Hz, 1H), 4.09 - 4.05 (m, 2H), 4.04 - 3.98 (m, 1H), 3.77 - 3.72 (m, 2H), 2.67 (s, 3H), 2.23 (s, 3H), 1.36 (d, *J* = 6.8 Hz, 6H). MS (ESI) *m*/*z*: 404 [M+H]⁺.** |
| **I-82** | 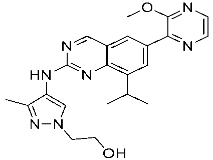 | **¹H NMR (600 MHz, DMSO-*d*₆) δ 9.31 (s, 1H), 8.47 (d, *J* = 2.0 Hz, 1H), 8.37 (d, *J* = 2.6 Hz, 1H), 8.35 (d, *J =* 2.0 Hz, 1H), 8.33 - 8.27 (m, 1H), 8.23 (d, *J* = 2.6 Hz, 1H), 4.90 (t, *J =* 5.3 Hz, 1H), 4.08 (t, *J =* 5.7 Hz, 2H), 4.05 (s, 3H), 4.02 - 3.95 (m, 1H), 3.74 (q, *J* = 5.5 Hz, 2H), 2.23 (s, 3H), 1.37 (d, *J* = 6.9 Hz, 6H). MS (ESI) *m*/*z*: 420 [M+H]⁺.** |
| **I-83** | 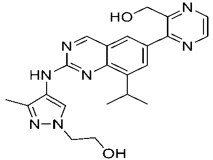 | **¹H NMR (600 MHz, DMSO-*d*₆) δ 9.39 - 9.32 (m, 1H), 9.29 (d, *J =* 3.3 Hz, 1H), 8.72 (d, *J =* 3.1 Hz, 1H), 8.67 (d, *J* = 3.0 Hz, 1H), 8.38 - 8.29 (m, 1H), 8.19 - 8.13 (m, 1H), 8.11 (d, *J* = 2.9 Hz, 1H), 4.69 - 4.63 (m, 2H), 4.12 - 4.07 (m, 2H), 4.04 - 3.94 (m, 1H), 3.80 - 3.71 (m, 2H), 2.57 - 2.52 (m, 1H), 2.25 (s, 3H), 1.42 - 1.23 (m, 6H). MS (ESI) *m*/*z*: 420 [M+H]⁺.** |
| **I-84** | 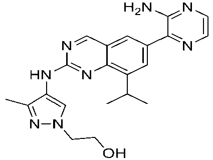 | **¹H NMR (600 MHz, DMSO-*d*₆) δ 9.31 - 9.26 (m, 1H), 9.25 (s, 1H), 8.37 - 8.24 (m, 1H), 8.10 (d, *J* = 2.0 Hz, 1H), 7.97 - 7.93 (m, 3H), 4.08 (t, *J =* 5.6 Hz, 2H), 3.99 - 3.94 (m, 1H), 3.76 - 3.73 (m, 2H), 2.24 (s, 3H), 1.36 (d, *J* = 6.9 Hz, 6H). MS (ESI) *m*/*z*: 405 [M+H]⁺.** |
| **I-85** | 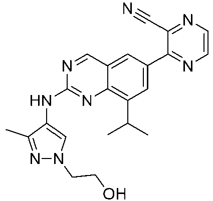 | **¹H NMR (600 MHz, DMSO-*d*₆) δ 9.54 - 9.46 (m, 1H), 9.37 (s, 1H), 9.07 (d, *J* = 2.3 Hz, 1H), 8.85 (d, *J* = 2.4 Hz, 1H), 8.36 (d, *J* = 2.1 Hz, 1H), 8.34 - 8.28 (m, 1H), 8.23 (d, *J* = 2.1 Hz, 1H), 4.91 (t, *J* = 5.3 Hz, 1H), 4.09 (t, *J* = 5.6 Hz, 2H), 4.04 - 3.94 (m, 1H), 3.75 (q, *J* = 5.5 Hz, 2H), 2.24 (s, 3H), 1.38 (d, *J* = 6.9 Hz, 6H). MS (ESI) *m*/*z*: 415 [M+H]⁺.** |
| **I-86** | 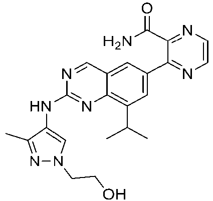 | **¹H NMR (600 MHz, DMSO-*d*₆) δ 9.37 - 9.30 (m, 1H), 9.27 (s, 1H), 8.83 (d, *J* = 2.4 Hz, 1H), 8.63 (d, *J* = 2.5 Hz, 1H), 8.34 - 8.27 (m, 1H), 8.22 (s, 1H), 8.11 (d, *J* = 2.1 Hz, 1H), 8.03 (d, *J* = 2.1 Hz, 1H), 7.84 (s, 1H), 4.90 (t, *J* = 5.3 Hz, 1H), 4.08 (t, *J* = 5.7 Hz, 2H), 4.01 - 3.92 (m, 1H), 3.74 (q, *J =* 5.6 Hz, 2H), 2.23 (s, 3H), 1.35 (d, *J* = 6.9 Hz, 6H). MS (ESI) *m*/*z:* 433 [M+H]⁺.** |
| **I-87** | 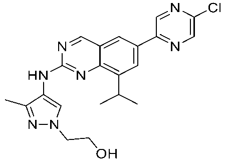 | **¹H NMR (600 MHz, DMSO-*d*₆) δ 9.45 - 9.37 (m, 1H), 9.30 (s, 1H), 9.23 (d, *J =* 1.4 Hz, 1H), 8.88 (d, *J* = 1.4 Hz, 1H), 8.53 (d, *J =* 2.1 Hz, 1H), 8.36 (d, *J =* 2.0 Hz, 1H), 8.32 - 8.27 (m, 1H), 4.90 (t, *J =* 5.3 Hz, 1H), 4.08 (t, *J* = 5.7 Hz, 2H), 4.04 - 3.94 (m, 1H), 3.74 (q, *J =* 5.5 Hz, 2H), 2.23 (s, 3H), 1.39 (d, *J* = 6.9 Hz, 6H). MS (ESI) *m*/*z*: 424 [M+H]⁺.** |
| **I-88** | 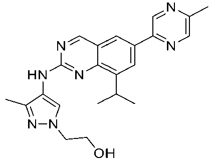 | **¹H NMR (600 MHz, DMSO-*d*₆) δ 9.38 - 9.31 (m, 1H), 9.29 (s, 1H), 9.22 (d, *J* = 1.5 Hz, 1H), 8.64 (dd, *J* = 1.5, 0.7 Hz, 1H), 8.48 (d, *J =* 2.1 Hz, 1H), 8.37 (d, *J* = 2.0 Hz, 1H), 8.33 - 8.24 (m, 1H), 4.90 (t, *J =* 5.3 Hz, 1H), 4.08 (t, *J =* 5.6 Hz, 2H), 4.04 - 3.93 (m, 1H), 3.75 (q, *J* = 5.6 Hz, 2H), 2.56 (s, 3H), 2.23 (s, 3H), 1.39 (d, *J* = 6.9 Hz, 6H). MS (ESI) *m*/*z*: 404 [M+H]⁺.** |
| **I-89** | 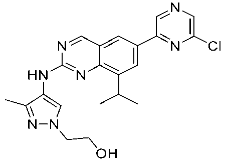 | **¹H NMR (600 MHz, DMSO-*d*₆) δ 9.46 - 9.41 (m, 1H), 9.39 (s, 1H), 9.33 (s, 1H), 8.74 (s, 1H), 8.54 (d, *J* = 2.1 Hz, 1H), 8.33 (d, *J* = 2.0 Hz, 1H), 8.32 - 8.29 (m, 1H), 4.90 (t, *J* = 5.3 Hz, 1H), 4.08 (t, *J* = 5.6 Hz, 2H), 4.03 - 3.93 (m, 1H), 3.75 (q, *J* = 5.5 Hz, 2H), 2.24 (s, 3H), 1.39 (d, *J* = 6.9 Hz, 6H). MS (ESI) *m*/*z*: 424 [M+H]⁺.** |
| **I-90** | 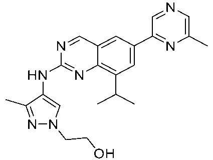 | **¹H NMR (600 MHz, DMSO-*d*₆) δ 9.40 - 9.32 (m, 1H), 9.31 (s, 1H), 9.16 (s, 1H), 8.51 (s, 1H), 8.50 (d, *J* = 2.1 Hz, 1H), 8.38 (d, *J* = 2.0 Hz, 1H), 8.31 (s, 1H), 4.09 (t, *J* = 5.6 Hz, 2H), 4.03 - 3.94 (m, 1H), 3.75 (t, *J* = 5.7 Hz, 2H), 2.60 (s, 3H), 2.24 (s, 3H), 1.39 (d, *J* = 6.9 Hz, 6H). MS (ESI) *m*/*z*: 404 [M+H]⁺.** |
| **I-91** | 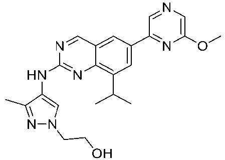 | **¹H NMR (600 MHz, DMSO-*d*₆) δ 9.39 - 9.32 (m, 1H), 9.30 (s, 1H), 8.95 - 8.93 (m, 1H), 8.52 (d, *J* = 2.1 Hz, 1H), 8.39 (d, *J* = 2.0 Hz, 1H), 8.35 - 8.29 (m, 1H), 8.27 (d, *J* = 0.6 Hz, 1H), 4.90 (t, *J* = 5.3 Hz, 1H), 4.09 - 4.08 (m, 2H), 4.08 (s, 3H), 4.02 - 3.95 (m, 1H), 3.75 (q, *J* = 5.6 Hz, 2H), 2.23 (s, 3H), 1.40 (d, *J* = 6.9 Hz, 6H). MS (ESI) *m*/*z*: 420 [M+H]⁺.** |
| **I-92** | 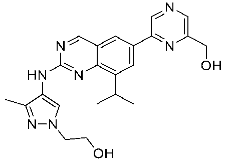 | **¹H NMR (600 MHz, DMSO-*d*₆) δ 9.37 - 9.33 (m, 1H), 9.31 (s, 1H), 9.24 (s, 1H), 8.66 (s, 1H), 8.51 (d, *J =* 2.1 Hz, 1H), 8.38 (d, *J =* 2.0 Hz, 1H), 8.34 - 8.26 (m, 1H), 5.66 (t, *J =* 5.9 Hz, 1H), 4.90 (t, *J =* 5.3 Hz, 1H), 4.75 (d, *J* = 5.8 Hz, 2H), 4.08 (t, *J =* 5.7 Hz, 2H), 4.04 - 3.96 (m, 1H), 3.74 (q, *J* = 5.5 Hz, 2H), 2.23 (s, 3H), 1.39 (d, *J* = 6.9 Hz, 6H). MS (ESI) *m*/*z*: 420 [M+H]⁺.** |
| **I-93** | 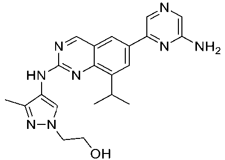 | **¹H NMR (600 MHz, DMSO-*d*₆) δ 9.26 (s, 1H), 8.39 (s, 1H), 8.33 (d, *J =* 2.0 Hz, 1H), 8.30 (s, 1H), 8.27 (d, *J =* 2.0 Hz, 1H), 7.85 (s, 1H), 6.54 (s, 2H), 4.95 - 4.85 (m, 1H), 4.08 (t, *J* = 5.7 Hz, 2H), 4.02 - 3.95 (m, 1H), 3.76 - 3.73 (m, 2H), 2.23 (s, 3H), 1.38 (d, *J* = 6.9 Hz, 6H). MS (ESI) *m*/*z*: 405 [M+H]⁺.** |
| **I-94** | 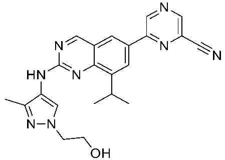 | **¹H NMR (600 MHz, DMSO-*d*₆) δ 9.68 (d, *J* = 3.0 Hz, 1H), 9.51 - 9.45 (m, 1H), 9.33 (d, *J* = 3.0 Hz, 1H), 9.16 (d, *J* = 3.1 Hz, 1H), 8.60 (d, *J* = 2.8 Hz, 1H), 8.40 - 8.35 (m, 1H), 8.30 (s, 1H), 4.95 - 4.86 (m, 1H), 4.16 - 4.05 (m, 2H), 4.04 - 3.93 (m, 1H), 3.82 - 3.72 (m, 2H), 2.24 (s, 3H), 1.40 (d, *J* = 6.7 Hz, 6H). MS (ESI) *m*/*z*: 415 [M+H]⁺.** |
| **I-95** | 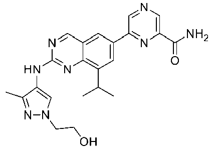 | **¹H NMR (600 MHz, DMSO-*d*₆) δ 9.57 (s, 1H), 9.45 - 9.35 (m, 1H), 9.27 (s, 1H), 9.11 (s, 1H), 8.79 (d, *J* = 2.0 Hz, 1H), 8.53 (d, *J* = 2.0 Hz, 1H), 8.51 (s, 1H), 8.37 - 8.23 (m, 1H), 7.99 (s, 1H), 4.09 (t, *J* = 5.7 Hz, 2H), 4.05 - 3.94 (m, 1H), 3.75 (t, *J* = 5.6 Hz, 2H), 2.24 (s, 3H), 1.43 (d, *J* = 6.9 Hz, 6H). MS (ESI) *m*/*z:* 433 [M+H]⁺.** |
| **I-96** | 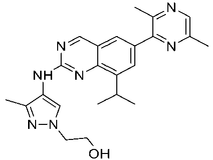 | **MS (ESI) *m*/*z*: 418 [M+H]⁺.** |
| **I-97** | 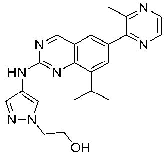 | **¹H NMR (400 MHz, DMSO-*d*₆) δ 9.91 (s, 1H), 9.27 (s, 1H), 8.60 (d, *J* = 2.4 Hz, 1H), 8.54 (d, *J =* 2.4 Hz, 1H), 8.16 (s, 1H), 8.03 (d, *J =* 2.0 Hz, 1H), 7.94 (d, *J* = 2.0 Hz, 1H), 7.76 (s, 1H), 4.92 (t, *J =* 5.4 Hz, 1H), 4.15 (t, *J* = 5.4 Hz, 2H), 4.07 - 4.00 (m, 1H), 3.78 - 3.74 (m, 2H), 2.67 (s, 3H), 1.40 (d, *J* = 6.8 Hz, 6H). MS (ESI) *m*/*z*: 390 [M+H]⁺.** |
| **I-98** | 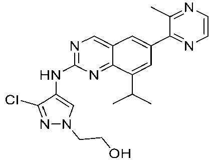 | **MS (ESI) *m*/*z*: 424 [M+H]⁺.** |
| **I-99** | 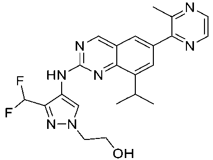 | **MS (ESI) *m*/*z*: 440 [M+H]⁺.** |
| **I-100** | 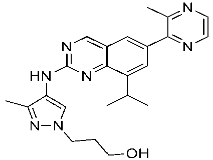 | **MS (ESI) *m*/*z*: 418 [M+H]⁺.** |
| **I-101** | 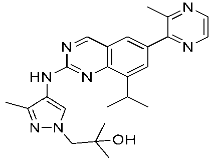 | **MS (ESI) *m*/*z*: 432 [M+H]⁺.** |
| **I-102** | 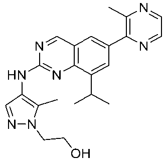 | **MS (ESI) *m*/*z*: 404 [M+H]⁺.** |
| **I-103** | 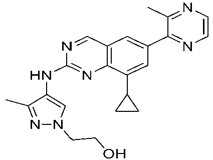 | **¹H NMR (400 MHz, DMSO-*d*₆) δ 9.31 (s, 1H), 9.28 (s, 1H), 8.58 (d, *J* = 2.4 Hz, 1H), 8.53 (d, *J* = 2.4 Hz, 1H), 8.38 (s, 1H), 7.97 (d, *J* = 2.0 Hz, 1H), 7.54 (d, *J* = 2.0 Hz, 1H), 4.90 - 4.83 (m, 1H), 4.07 (t, *J* = 5.4 Hz, 2H), 3.74 - 3.71 (m, 2H), 2.89 - 2.77 (m, 1H), 2.63 (s, 3H), 2.24 (s, 3H), 1.15 - 1.10 (m, 2H), 0.85 - 0.78 (m, 2H). MS (ESI) *m*/*z*: 402 [M+H]⁺.** |
| **I-104** | 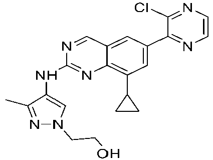 | **MS (ESI) *m*/*z*: 422 [M+H]⁺.** |
| **I-105** | 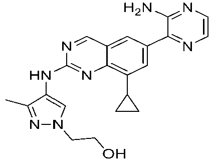 | **MS (ESI) *m*/*z:* 403 [M+H]⁺.** |
| **I-106** | 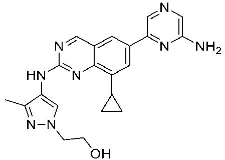 | **MS (ESI) *m*/*z:* 403 [M+H]⁺.** |
| **I-107** | 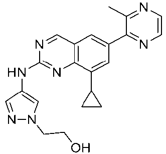 | **MS (ESI) *m*/*z*: 388 [M+H]⁺.** |
| **I-108** | 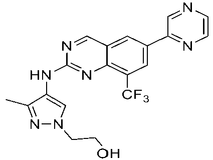 | **¹H NMR (600 MHz, DMSO-*d*₆) δ 9.85 (s, 1H), 9.44 (s, 1H), 9.43 (s, 1H), 8.97 (s, 1H), 8.85 (s, 1H), 8.78 (s, 1H), 8.68 (s, 1H), 8.35 (s, 1H), 4.96 - 4.85 (m, 1H), 4.05 (t, *J* = 6.0 Hz, 2H), 3.77 - 3.71 (m, 2H), 2.26 (s, 3H). MS (ESI) *m*/*z*: 416 [M+H]⁺.** |
| **I-109** | 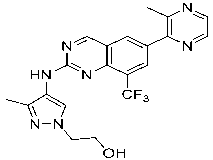 | **MS (ESI) *m*/*z:* 430 [M+H]⁺.** |
| **I-110** | 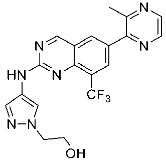 | **¹H NMR (600 MHz, DMSO-*d*₆) δ 10.33 (s, 1H), 9.40 (s, 1H), 8.63 (d, *J* = 2.1 Hz, 1H), 8.59 (d, *J* = 2.2 Hz, 1H), 8.50 (s, 1H), 8.43 (s, 1H), 8.18 (s, 1H), 7.82 (s, 1H), 4.91 (t, *J* = 5.1 Hz, 1H), 4.13 (t, *J* = 5.4 Hz, 2H), 3.75 (q, *J* = 5.1 Hz, 2H), 2.70 (s, 3H). MS (ESI) *m*/*z*: 416 [M+H]⁺.** |
| **I-111** | 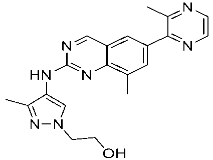 | **¹H NMR (600 MHz, DMSO-*d*₆) δ 9.47 - 9.36 (m, 1H), 9.34 (s, 1H), 8.68 - 8.62 (m, 1H), 8.60 (d, *J* = 2.5 Hz, 1H), 8.47 - 8.34 (m, 1H), 8.09 (d, *J* = 2.0 Hz, 1H), 8.00 (s, 1H), 4.96 (t, *J* = 5.3 Hz, 1H), 4.15 (t, *J* = 5.6 Hz, 2H), 3.80 (q, *J* = 5.6 Hz, 2H), 2.73 (s, 3H), 2.69 (s, 3H), 2.30 (s, 3H). MS (ESI) *m*/*z*: 376 [M+H]⁺.** |
| **I-112** | 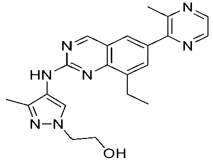 | **MS (ESI) *m*/*z*: 390 [M+H]⁺.** |
| **I-113** | 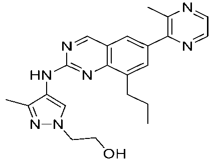 | **MS (ESI) *m*/*z*: 404 [M+H]⁺.** |
| **I-114** | 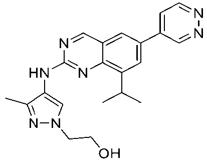 | **¹H NMR (600 MHz, DMSO-*d*₆) δ 9.76 (dd, *J* = 2.6, 1.2 Hz, 1H), 9.42 (s, 1H), 9.29 (dd, *J* = 5.4, 1.2 Hz, 1H), 9.28 (s, 1H), 8.35 (d, *J =* 2.1 Hz, 1H), 8.32 - 8.25 (m, 1H), 8.15 - 8.09 (m, 2H), 4.90 (t, *J =* 5.3 Hz, 1H), 4.08 (t, *J* = 5.6 Hz, 2H), 4.04 - 3.94 (m, 1H), 3.74 (q, *J* = 5.5 Hz, 2H), 2.23 (s, 3H), 1.40 (d, *J* = 6.9 Hz, 6H). MS (ESI) *m*/*z*: 390 [M+H]⁺.** |
| **I-115** | 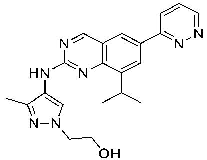 | **¹H NMR (600 MHz, DMSO-*d*₆) δ 9.41 - 9.34 (m, 1H), 9.32 (s, 1H), 9.22 (dd, *J* = 4.8,1.5 Hz, 1H), 8.52 (d, *J* = 2.0 Hz, 1H), 8.47 (d, *J* = 2.0 Hz, 1H), 8.34 (dd, *J* = 8.7, 1.5 Hz, 1H), 8.32 - 8.29 (m, 1H), 7.82 (dd, *J* = 8.6, 4.9 Hz, 1H), 4.91 (t, *J* = 5.2 Hz, 1H), 4.09 (t, *J* = 5.7 Hz, 2H), 4.05 - 3.96 (m, 1H), 3.75 (q, *J* = 5.5 Hz, 2H), 2.24 (s, 3H), 1.41 (d, *J* = 6.9 Hz, 6H). MS (ESI) *m*/*z*: 390 [M+H]⁺.** |
| **I-116** | 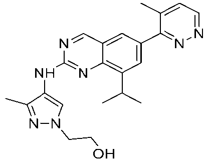 | **¹H NMR (600 MHz, DMSO-*d*₆) δ 9.40 - 9.24 (m, 2H), 9.10 (d, *J* = 5.2 Hz, 1H), 8.39 - 8.25 (m, 1H), 8.05 - 7.98 (m, 1H), 7.91 (s, 1H), 7.68 (d, *J* = 5.2 Hz, 1H), 5.01 - 4.76 (m, 1H), 4.08 (t, *J* = 5.7 Hz, 2H), 4.06 - 3.96 (m, 1H), 3.75 (t, *J* = 5.7 Hz, 2H), 2.43 (s, 3H), 2.24 (s, 3H), 1.37 (d, *J* = 6.9 Hz, 6H). MS (ESI) *m*/*z*: 404 [M+H]⁺.** |
| **I-117** | 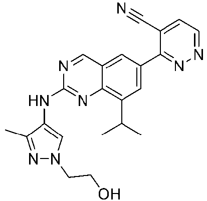 | **¹H NMR (600 MHz, DMSO-*d*₆) δ 9.55 (d, *J* = 5.2 Hz, 1H), 9.50 (s, 1H), 9.38 (s, 1H), 8.40 (d, *J* = 5.2 Hz, 1H), 8.36 (d, *J* = 2.1 Hz, 1H), 8.34 - 8.30 (m, 1H), 8.22 (d, *J* = 2.1 Hz, 1H), 4.91 (t, *J* = 5.3 Hz, 1H), 4.09 (t, *J* = 5.6 Hz, 2H), 4.06 - 3.97 (m, 1H), 3.75 (q, *J* = 5.5 Hz, 2H), 2.25 (s, 3H), 1.39 (d, *J* = 6.9 Hz, 6H). MS (ESI) *m*/*z*: 415 [M+H]⁺.** |
| **I-118** | 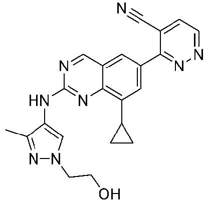 | **MS (ESI) *m*/*z*: 413 [M+H]⁺.** |
| **I-119** | 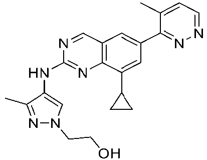 | **MS (ESI) *m*/*z*: 402 [M+H]⁺.** |
| **I-120** | 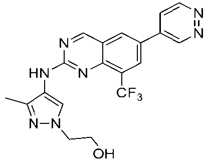 | **¹H NMR (600 MHz, DMSO-*d*₆) δ 9.89 (s, 1H), 9.80 (s, 1H), 9.40 (d, *J* = 2.0 Hz, 1H), 9.34 (d, *J* = 5.4 Hz, 1H), 8.78 (s, 1H), 8.61 (s, 1H), 8.36 (s, 1H), 8.21 - 8.15 (m, 1H), 4.90 (t, *J* = 5.6 Hz, 1H), 4.05 (t, *J* = 5.8 Hz, 2H), 3.78 - 3.69 (m, 2H), 2.26 (s, 2H). MS (ESI) *m*/*z*: 416 [M+H]⁺.** |
| **I-121** | 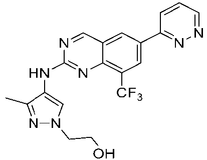 | **¹H NMR (600 MHz, DMSO-*d*₆) δ 9.91 - 9.82 (m, 1H), 9.45 (s, 1H), 9.27 (d, *J* = 4.8 Hz, 1H), 8.98 (s, 1H), 8.92 (s, 1H), 8.43 (d, *J* = 8.7 Hz, 1H), 8.36 (s, 1H), 7.87 (dd, *J* = 8.6, 5.0 Hz, 1H), 4.89 (t, *J* = 5.1 Hz, 1H), 4.09 - 4.03 (m, 2H), 3.74 (q, *J* = 5.0 Hz, 2H), 2.26 (s, 3H). MS (ESI) *m*/*z*: 416 [M+H]⁺.** |
| **I-122** | 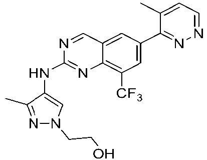 | **¹H NMR (600 MHz, DMSO-*d*₆) δ 9.80 (s, 1H), 9.43 (s, 1H), 9.13 (d, *J* = 5.2 Hz, 1H), 8.49 (d, *J* = 1.9 Hz, 1H), 8.41 (s, 1H), 8.37 (s, 1H), 7.72 (d, *J* = 5.2 Hz, 1H), 4.94 - 4.85 (m, 1H), 4.05 (t, *J* = 5.9 Hz, 2H), 3.74 (q, *J* = 5.7 Hz, 2H), 2.46 (s, 3H), 2.26 (s, 3H). MS (ESI) *m*/*z:* 430 [M+H]⁺.** |
| **I-123** | 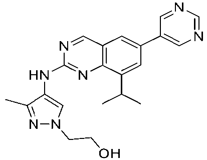 | **¹H NMR (600 MHz, DMSO-*d*₆) δ 9.33 (s, 1H), 9.28 - 9.24 (m, 3H), 9.20 (s, 1H), 8.34 - 8.24 (m, 1H), 8.18 (d, *J* = 2.1 Hz, 1H), 8.04 (d, *J* = 2.1 Hz, 1H), 4.90 (t, *J* = 5.3 Hz, 1H), 4.08 (t, *J* = 5.6 Hz, 2H), 4.04 - 3.96 (m, 1H), 3.74 (q, *J* = 5.6 Hz, 2H), 2.23 (s, 3H), 1.40 (d, *J* = 6.9 Hz, 6H). MS (ESI) *m*/*z*: 390 [M+H]⁺.** |
| **I-124** | 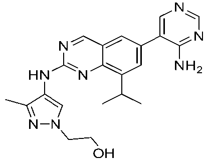 | **¹H NMR (600 MHz, DMSO-*d*₆) δ 9.22 (s, 1H), 8.39 (s, 1H), 8.34 - 8.23 (m, 1H), 8.11 (s, 1H), 7.78 (d, *J* = 2.0 Hz, 1H), 7.63 (d, *J* = 2.0 Hz, 1H), 6.85 - 6.62 (m, 2H), 4.89 (t, *J* = 5.3 Hz, 1H), 4.07 (t, *J* = 5.7 Hz, 2H), 4.00 - 3.92 (m, 1H), 3.74 (q, *J* = 5.6 Hz, 2H), 2.23 (s, 3H), 1.36 (d, *J* = 6.9 Hz, 6H). MS (ESI) *m*/*z*: 405 [M+H]⁺.** |
| **I-125** | 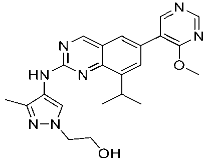 | **¹H NMR (600 MHz, DMSO-*d*₆) δ 9.34 - 9.26 (m, 1H), 9.24 (s, 1H), 8.81 (s, 1H), 8.71 (s, 1H), 8.37 - 8.09 (m, 1H), 7.96 (d, *J* = 2.0 Hz, 1H), 7.86 (d, *J* = 2.0 Hz, 1H), 4.89 (t, *J =* 5.3 Hz, 1H), 4.08 (t, *J =* 5.6 Hz, 2H), 4.02 (s, 3H), 4.00 - 3.94 (m, 1H), 3.74 (q, *J =* 5.6 Hz, 2H), 2.23 (s, 3H), 1.36 (d, *J* = 6.9 Hz, 6H). MS (ESI) *m*/*z*: 420 [M+H]⁺.** |
| **I-126** | 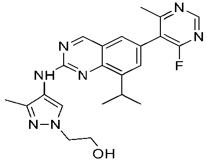 | **MS (ESI) *m*/*z*: 422 [M+H]⁺.** |
| **I-127** | 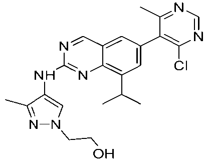 | **MS (ESI) *m*/*z*: 438 [M+H]⁺.** |
| **I-128** | 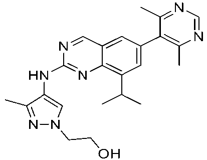 | **¹H NMR (600 MHz, DMSO-*d*₆) δ 9.39 - 9.31 (m, 1H), 9.29 - 9.24 (m, 1H), 9.00 - 8.96 (m, 1H), 8.46 - 8.27 (m, 1H), 7.75 - 7.69 (m, 1H), 7.64 - 7.59 (m, 1H), 5.03 - 4.94 (m, 1H), 4.18 - 4.11 (m, 2H), 4.09 - 3.99 (m, 1H), 3.85 - 3.75 (m, 2H), 2.40 - 2.23 (m, 9H), 1.45 - 1.36 (m, 6H). MS (ESI) *m*/*z*: 418 [M+H]⁺.** |
| **I-129** | 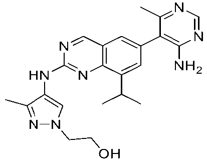 | **¹H NMR (600 MHz, Methanol-*d*₄) δ 9.12 (s, 1H), 8.32 - 8.29 (m, 1H), 8.23 (s, 1H), 7.60 (d, *J* = 1.9 Hz, 1H), 7.50 (d, *J* = 1.9 Hz, 1H), 4.19 (t, *J* = 5.4 Hz, 2H), 4.07 (p, *J =* 6.9 Hz, 1H), 3.91 (t, *J =* 5.5 Hz, 2H), 2.30 (s, 3H), 2.28 (s, 3H), 1.40 (d, *J =* 6.9 Hz, 6H). MS (ESI) *m*/*z*: 419 [M+H]⁺.** |
| **I-130** | 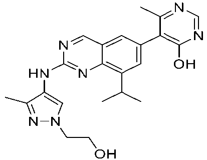 | **¹H NMR (600 MHz, DMSO-*d*₆) δ 9.25 - 9.12 (m, 2H), 8.33 (s, 1H), 8.30 - 8.20 (m, 1H), 7.64 (d, *J* = 1.9 Hz, 1H), 7.53 (s, 1H), 4.08 (t, *J* = 5.6 Hz, 2H), 3.98 - 3.94 (m, 1H), 3.76 - 3.73 (m, 2H), 2.23 (s, 3H), 2.18 (s, 3H), 1.33 (d, *J* = 6.9 Hz, 6H). MS (ESI) *m*/*z*: 420 [M+H]⁺.** |
| **I-131** | 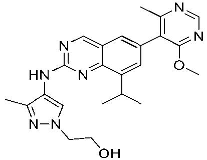 | **¹H NMR (600 MHz, DMSO-*d*₆) δ 9.20 (s, 2H), 8.69 (s, 1H), 8.35 - 8.18 (m, 1H), 7.66 (d, *J* = 1.9 Hz, 1H), 7.55 (d, *J* = 1.9 Hz, 1H), 4.89 (t, *J* = 5.3 Hz, 1H), 4.07 (t, *J* = 5.7 Hz, 2H), 4.02 - 3.92 (m, 1H), 3.87 (s, 3H), 3.74 (q, *J* = 5.6 Hz, 2H), 2.28 (s, 3H), 2.23 (s, 3H), 1.33 (d, *J* = 6.9 Hz, 6H). MS (ESI) *m*/*z*: 434 [M+H]⁺.** |
| **I-132** | 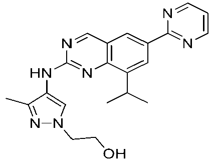 | **¹H NMR (600 MHz, DMSO-*d*₆) δ 9.44 - 9.34 (m, 2H), 8.94 (d, *J* = 4.8 Hz, 2H), 8.80 - 8.76 (m, 1H), 8.67 (s, 1H), 8.38 - 8.26 (m, 1H), 7.45 (t, *J* = 4.9 Hz, 1H), 4.90 (t, *J* = 5.3 Hz, 1H), 4.08 (t, *J* = 5.6 Hz, 2H), 4.03 - 3.95 (m, 1H), 3.75 (q, *J* = 5.5 Hz, 2H), 2.24 (s, 3H), 1.39 (d, *J* = 6.9 Hz, 6H). MS (ESI) *m*/*z*: 390 [M+H]⁺.** |
| **I-133** | 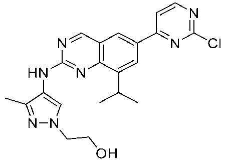 | **¹H NMR (600 MHz, DMSO-*d*₆) δ 9.51 (s, 1H), 9.35 (s, 1H), 8.83 (d, *J* = 5.3 Hz, 1H), 8.64 (d, *J* = 2.1 Hz, 1H), 8.35 (d, *J =* 2.1 Hz, 1H), 8.33 - 8.26 (m, 1H), 8.25 (d, *J* = 5.3 Hz, 1H), 4.95 - 4.83 (m, 1H), 4.11 - 4.07 (m, 2H), 4.02 - 3.92 (m, 1H), 3.75 (d, *J* = 5.2 Hz, 2H), 2.24 (s, 3H), 1.39 (d, *J* = 6.9 Hz, 6H). MS (ESI) *m*/*z*: 424 [M+H]⁺.** |
| **I-134** | 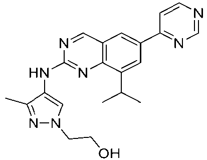 | **¹H NMR (600 MHz, DMSO-*d*₆) δ 9.51 - 9.39 (m, 1H), 9.33 (s, 1H), 9.27 (d, *J* = 1.3 Hz, 1H), 8.88 (d, *J* = 5.4 Hz, 1H), 8.62 (d, *J* = 2.0 Hz, 1H), 8.45 (d, *J* = 2.0 Hz, 1H), 8.35 - 8.24 (m, 1H), 8.20 (dd, *J* = 5.4, 1.4 Hz, 1H), 4.91 (t, *J* = 5.3 Hz, 1H), 4.08 (t, *J* = 5.6 Hz, 2H), 4.02 - 3.95 (m, 1H), 3.75 (q, *J* = 5.4 Hz, 2H), 2.24 (s, 3H), 1.39 (d, *J* = 6.9 Hz, 6H). MS (ESI) *m*/*z*: 390 [M+H]⁺.** |
| **I-135** | 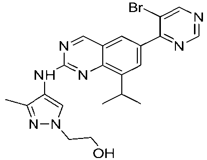 | **MS (ESI) *m*/*z*: 468 [M+H]⁺.** |
| **I-136** | 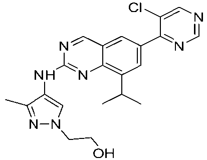 | **MS (ESI) *m*/*z*: 424 [M+H]⁺.** |
| **I-137** | 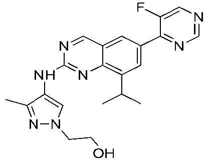 | **¹H NMR (600 MHz, DMSO-*d*₆) δ 9.51 (s, 1H), 9.39 (s, 1H), 9.16 (d, *J* = 3.1 Hz, 1H), 8.98 (d, *J* = 3.8 Hz, 1H), 8.50 (s, 1H), 8.38 (s, 1H), 8.31 (s, 1H), 5.00 - 4.86 (m, 1H), 4.09 (t, *J* = 5.6 Hz, 2H), 4.01 - 3.94 (m, 1H), 3.80 - 3.70 (m, 2H), 2.24 (s, 3H), 1.38 (d, *J* = 6.9 Hz, 6H). MS (ESI) *m*/*z*: 408 [M+H]⁺.** |
| **I-138** | 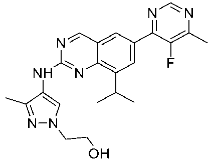 | **MS (ESI) *m*/*z*: 422 [M+H]⁺.** |
| **I-139** | 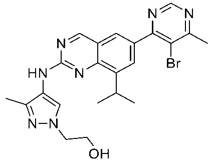 | **¹H NMR (600 MHz, Methanol-*d*₄) δ 9.17 (s, 1H), 8.97 (s, 1H), 8.46 - 8.25 (m, 1H), 8.09 (d, *J* = 2.0 Hz, 1H), 8.00 (d, *J* = 2.0 Hz, 1H), 4.19 (t, *J* = 5.4 Hz, 2H), 4.07 (p, *J* = 7.0 Hz, 1H), 3.91 (t, *J* = 5.4 Hz, 2H), 2.78 (s, 3H), 2.31 (s, 3H), 1.41 (d, *J* = 6.9 Hz, 6H). MS (ESI) *m*/*z*: 482 [M+H]⁺.** |
| **I-140** | 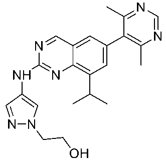 | **¹H NMR (400 MHz, DMSO-*d*₆) δ 9.85 (s, 1H), 9.19 (s, 1H), 8.91 (s, 1H), 8.15 (s, 1H), 7.75 (s, 1H), 7.65 (d, *J* = 2.0 Hz, 1H), 7.56 (d, *J* = 2.0 Hz, 1H), 4.95 - 4.88 (m, 1H), 4.15 (t, *J* = 5.6 Hz, 2H), 4.07 - 4.00 (m, 1H), 3.78 - 3.75 (m, 2H), 2.25 (s, 6H), 1.38 (d, *J* = 6.8 Hz, 6H). MS (ESI) *m*/*z*: 404 [M+H]⁺.** |
| **I-141** | 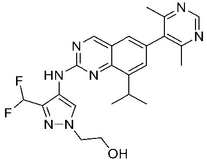 | **MS (ESI) *m*/*z*: 454 [M+H]⁺.** |
| **I-142** | 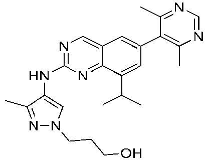 | **MS (ESI) *m*/*z*: 432 [M+H]⁺.** |
| **I-143** | 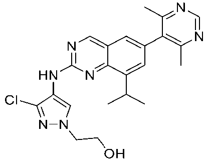 | **MS (ESI) *m*/*z*: 438 [M+H]⁺.** |
| **I-144** | 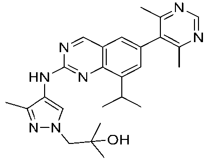 | **MS (ESI) *m*/*z*: 446 [M+H]⁺.** |
| **I-145** | 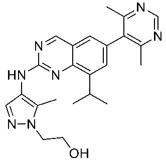 | **¹H NMR (600 MHz, DMSO-*d*₆) δ 9.26 - 9.10 (m, 2H), 8.95 (s, 1H), 8.15 - 7.94 (m, 1H), 7.65 (t, *J* = 1.5 Hz, 1H), 7.55 (s, 1H), 4.10 (t, *J* = 5.9 Hz, 2H), 4.03 - 3.92 (m, 1H), 3.73 (t, *J* = 5.9 Hz, 2H), 2.32 (s, 3H), 2.27 (s, 6H), 1.31 (d, *J* = 7.0 Hz, 6H). MS (ESI) *m*/*z*: 418 [M+H]⁺.** |
| **I-146** | 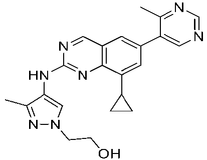 | **MS (ESI) *m*/*z*: 402[M+H]⁺.** |
| **I-147** | 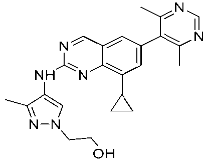 | **¹H NMR (400 MHz, DMSO-*d*₆) δ 9.23 (s, 1H), 9.19 (s, 1H), 8.89 (s, 1H), 8.34 (s, 1H), 7.59 (d, *J* = 2.0 Hz, 1H), 7.19 (d, *J* = 2.0 Hz, 1H), 4.93 - 4.78 (m, 1H), 4.06 (t, *J* = 5.6 Hz, 2H), 3.72 (t, *J* = 5.6 Hz, 2H), 2.90 - 2.78 (m, 1H), 2.24 - 2.21 (m, 9H), 1.14 - 1.05 (m, 2H), 0.85 - 0.77 (m, 2H). MS (ESI) *m*/*z*: 416 [M+H]⁺.** |
| **I-148** | 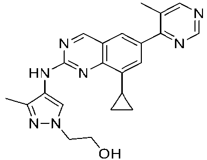 | **MS (ESI) *m*/*z*: 402 [M+H]⁺.** |
| **I-149** | 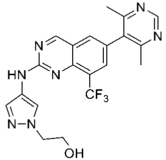 | **¹H NMR (600 MHz, DMSO-*d*₆) δ 10.28 (s, 1H), 9.31 (s, 1H), 8.95 (s, 1H), 8.17 (s, 1H), 8.13 (s, 1H), 8.12 (s, 1H), 7.82 (s, 1H), 4.13 (t, *J* = 5.7 Hz, 2H), 3.75 (t, *J* = 5.7 Hz, 2H), 2.26 (s, 6H). MS (ESI) *m*/*z:* 430 [M+H]⁺.** |
| **I-150** | 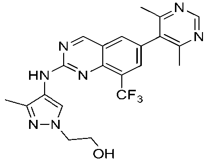 | **¹H NMR (600 MHz, DMSO-*d*₆) δ 9.74 (s, 1H), 9.33 (s, 1H), 8.94 (s, 1H), 8.36 (s, 1H), 8.12 (s, 2H), 4.88 (t, *J* = 5.4 Hz, 1H), 4.05 (t, *J* = 5.9 Hz, 2H), 3.74 (t, *J* = 5.7 Hz, 2H), 2.27 - 2.23 (m, 9H). MS (ESI) *m*/*z*: 444 [M+H]⁺.** |
| **I-151** | 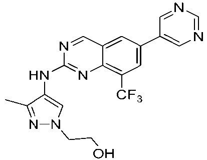 | **¹H NMR (600 MHz, DMSO-*d*₆) δ 9.81 (s, 1H), 9.38 (s, 1H), 9.32 - 9.27 (m, 2H), 9.24 (s, 1H), 8.62 (s, 1H), 8.54 (s, 1H), 8.36 (s, 1H), 4.89 (t, *J* = 4.6 Hz, 1H), 4.05 (t, *J* = 6.2 Hz, 2H), 3.73 (q, *J* = 5.6 Hz, 2H), 2.26 (s, 3H). MS (ESI) *m*/*z*: 416 [M+H]⁺.** |
| **I-152** | 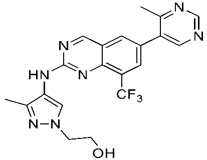 | **¹H NMR (600 MHz, DMSO-*d*₆) δ 9.76 (s, 1H), 9.37 (s, 1H), 9.10 (d, *J* = 1.1 Hz, 1H), 8.76 (d, *J* = 1.1 Hz, 1H), 8.40 - 8.33 (m, 1H), 8.28 (s, 1H), 8.25 (s, 1H), 4.92 - 4.82 (m, 1H), 4.05 (t, *J* = 5.9 Hz, 2H), 3.74 (q, *J* = 5.6 Hz, 2H), 2.52 (s, 3H), 2.26 (s, 3H). MS (ESI) *m*/*z*: *430* [M+H]⁺.** |
| **I-153** | 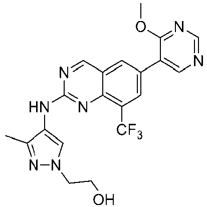 | **¹H NMR (600 MHz, DMSO-*d*₆) δ 9.75 (s, 1H), 9.38 (s, 1H), 8.85 (s, 1H), 8.76 (s, 1H), 8.40 (s, 1H), 8.38 - 8.32 (m, 2H), 5.04 - 4.71 (m, 1H), 4.07 - 4.04 (m, 2H), 4.03 (s, 3H), 3.73 (t, *J* = 5.9 Hz, 2H), 2.25 (s, 3H). MS (ESI) *m*/*z*: 446 [M+H]⁺.** |
| **I-154** | 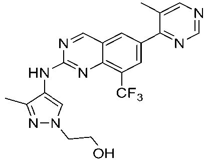 | **¹H NMR (600 MHz, DMSO-*d*₆) δ 9.85 (s, 1H), 9.45 (s, 1H), 9.15 (s, 1H), 8.81 (s, 1H), 8.57 (s, 1H), 8.48 (s, 1H), 8.35 (s, 1H), 4.91 - 4.85 (m, 1H), 4.04 (s, 2H), 3.78 - 3.68 (m, 2H), 2.39 (s, 3H), 2.26 (s, 3H). MS (ESI) *m*/*z*: 430 [M+H]⁺.** |
| **I-155** | 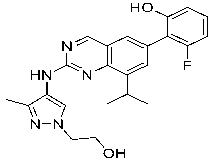 | **¹H NMR (600 MHz, Methanol-*d*₄) δ 9.08 (s, 1H), 8.33 (s, 1H), 7.71 (s, 1H), 7.70 (s, 1H), 7.17 (td, *J =* 8.2, 6.4 Hz, 1H), 6.76 (d, *J* = 8.2 Hz, 1H), 6.70 (t, *J* = 9.0 Hz, 1H), 4.18 (t, *J* = 5.4 Hz, 2H), 4.09 - 4.03 (m, 1H), 3.91 (t, *J =* 5.4 Hz, 2H), 2.31 (s, 3H), 1.40 (d, *J =* 6.9 Hz, 6H). MS (ESI) *m*/*z*: 422 [M+H]⁺.** |
| **I-156** | 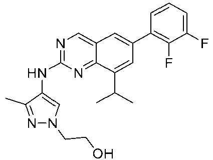 | **MS (ESI) *m*/*z*: 424 [M+H]⁺.** |
| **I-157** | 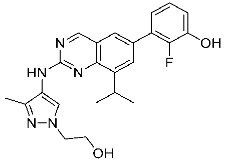 | **¹H NMR (600 MHz, Methanol-*d*₄) δ 9.12 (s, 1H), 8.32 (s, 1H), 7.82 (s, 1H), 7.79 (s, 1H), 7.07 (t, *J* = 7.9 Hz, 1H), 6.99 - 6.96 (m, 1H), 6.94 (td, *J* = 8.1, 1.6 Hz, 1H), 4.18 (t, *J* = 5.4 Hz, 2H), 4.06 (p, *J* = 7.0 Hz, 1H), 3.91 (t, *J =* 5.4 Hz, 2H), 2.31 (s, 3H), 1.41 (d, *J =* 7.0 Hz, 6H). MS (ESI) *m*/*z*: 422 [M+H]⁺.** |
| **I-158** | 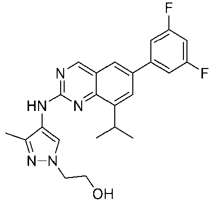 | **MS (ESI) *m*/*z*: 424[M+H]⁺.** |
| **I-159** | 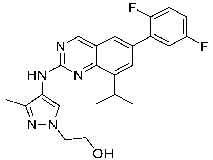 | **MS (ESI) *m*/*z*: 424 [M+H]⁺.** |
| **I-160** | 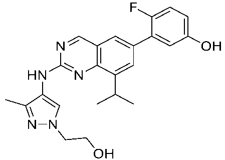 | **¹H NMR (600 MHz, Methanol-*d*₄) δ 9.12 (s, 1H), 8.32 (s, 1H), 7.80 (s, 1H), 7.79 (s, 1H), 7.04 (dd, *J* = 10.4, 8.8 Hz, 1H), 6.94 (dd, *J* = 6.5, 3.0 Hz, 1H), 6.77 (dt, *J =* 8.8, 3.4 Hz, 1H), 4.18 (t, *J* = 5.4 Hz, 2H), 4.06 (p, *J =* 7.1 Hz, 1H), 3.91 (t, *J* = 5.4 Hz, 2H), 2.31 (s, 3H), 1.41 (d, *J* = 6.9 Hz, 6H). MS (ESI) *m*/*z*: 422 [M+H]⁺.** |
| **I-161** | 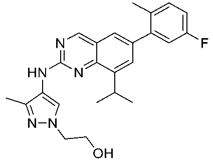 | **MS (ESI) *m*/*z*: 420 [M+H]⁺.** |
| **I-162** | 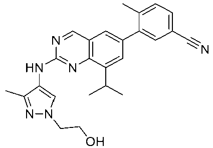 | **MS (ESI) *m*/*z*: 427 [M+H]⁺.** |
| **I-163** | 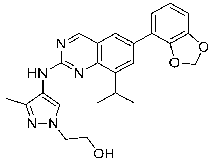 | **¹H NMR (600 MHz, DMSO-*d*₆) δ 9.25 (s, 1H), 8.29 (s, 1H), 8.06 (s, 1H), 8.01 (s, 1H), 7.24 (d, *J* = 7.9 Hz, 1H), 7.00 (t, *J =* 7.8 Hz, 1H), 6.95 (d, *J =* 7.7 Hz, 1H), 6.13 (s, 2H), 4.08 (t, *J* = 5.7 Hz, 2H), 3.98 (p, *J* = 7.0 Hz, 1H), 3.75 (t, *J* = 5.7 Hz, 2H), 2.23 (s, 3H), 1.37 (d, *J* = 6.9 Hz, 6H). MS (ESI) *m*/*z*: 432 [M+H]⁺.** |
| **I-164** | 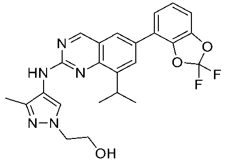 | **¹H NMR (600 MHz, DMSO-*d*₆) δ 9.32 (s, 1H), 8.30 (s, 1H), 8.09 (s, 1H), 7.95 (s, 1H), 7.61 (d, *J* = 8.0 Hz, 1H), 7.43 (d, *J =* 8.0 Hz, 1H), 7.37 (t, *J =* 8.0 Hz, 1H), 4.92 (t, *J* = 5.4 Hz, 1H), 4.09 (t, *J* = 5.7 Hz, 2H), 4.03 - 3.95 (m, 1H), 3.75 (q, *J* = 5.6 Hz, 2H), 2.24 (s, 3H), 1.38 (d, *J* = 6.9 Hz, 6H). MS (ESI) *m*/*z*: 468 [M+H]⁺.** |
| **I-165** | 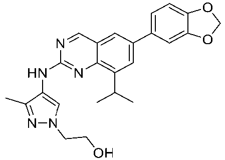 | **¹H NMR (600 MHz, DMSO-*d*₆) δ 9.21 (s, 1H), 9.20 - 9.07 (m, 1H), 8.35 - 8.17 (m, 1H), 7.92 (d, *J* = 2.2 Hz, 1H), 7.86 (s, 1H), 7.35 (s, 1H), 7.24 (d, *J* = 8.0 Hz, 1H), 7.04 (d, *J* = 8.1 Hz, 1H), 6.08 (s, 2H), 4.89 (t, *J* = 5.4 Hz, 1H), 4.07 (t, *J =* 5.8 Hz, 2H), 4.01 - 3.92 (m, 1H), 3.74 (q, *J =* 5.6 Hz, 2H), 2.23 (s, 3H), 1.38 (d, *J* = 6.9 Hz, 6H). MS (ESI) *m*/*z*: 432 [M+H]⁺.** |
| **I-166** | 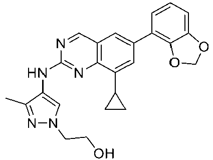 | **MS (ESI) *m*/*z:* 430 [M+H]⁺.** |
| **I-167** | 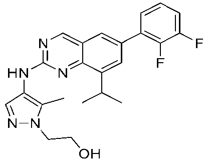 | **¹H NMR (600 MHz, DMSO-*d*₆) δ 9.24 (s, 1H), 9.22 (s, 1H), 8.03 (s, 1H), 7.91 (d, *J* = 1.7 Hz, 1H), 7.81 - 7.76 (m, 1H), 7.60 - 7.42 (m, 2H), 7.33 (q, *J =* 7.9, 7.4 Hz, 1H), 4.93 (t, *J =* 5.4 Hz, 1H), 4.07 (t, *J =* 5.9 Hz, 2H), 3.94 (s, 1H), 3.71 (q, *J* = 5.8 Hz, 2H), 2.29 (s, 3H), 1.31 (d, *J* = 7.0 Hz, 6H). MS (ESI) *m*/*z*: 424 [M+H]⁺.** |
| **I-168** | 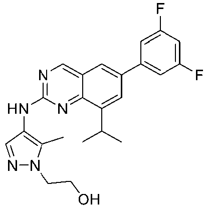 | **¹H NMR (600 MHz, DMSO-*d*₆) δ 9.24 (s, 1H), 9.21 (s, 1H), 8.09 (d, *J* = 2.2 Hz, 1H), 8.02 (s, 1H), 7.95 (d, *J* = 2.2 Hz, 1H), 7.57 - 7.54 (m, 2H), 7.22 (tt, *J* = 9.2, 2.3 Hz, 1H), 4.93 (t, *J* = 5.4 Hz, 1H), 4.07 (t, *J* = 5.9 Hz, 2H), 3.94 (s, 1H), 3.71 (q, *J* = 5.8 Hz, 2H), 2.29 (s, 3H), 1.34 (d, *J* = 6.9 Hz, 6H). MS (ESI) *m*/*z*: 424 [M+H]⁺.** |
| **I-169** | 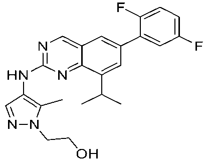 | **¹H NMR (600 MHz, DMSO-*d*₆) δ 9.23 (s, 1H), 9.21 (s, 1H), 8.03 (s, 1H), 7.91 (s, 1H), 7.78 (s, 1H), 7.52 (ddd, *J = 9.3,* 6.2, 3.2 Hz, 1H), 7.40 (ddd, *J* = 10.2, 9.0, 4.6 Hz, 1H), 7.30 - 7.24 (m, 1H), 4.93 (t, *J =* 5.4 Hz, 1H), 4.07 (t, *J* = 5.9 Hz, 2H), 3.94 (s, 1H), 3.71 (q, *J* = 5.8 Hz, 2H), 2.29 (s, 3H), 1.31 (d, *J* = 7.0 Hz, 6H). MS (ESI) *m*/*z*: 424 [M+H]⁺.** |
| **I-170** | 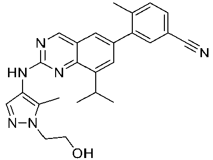 | **¹H NMR (600 MHz, DMSO-*d*₆) δ 9.20 (s, 1H), 9.15 (s, 1H), 8.02 (s, 1H), 7.78 (d, *J* = 1.8 Hz, 1H), 7.75 (dd, *J* = 7.9, 1.8 Hz, 1H), 7.71 (d, *J =* 2.1 Hz, 1H), 7.62 (d, *J* = 2.0 Hz, 1H), 7.55 (d, 1H), 4.07 (t, *J* = 5.9 Hz, 2H), 3.95 (s, 1H), 3.71 (q, *J* = 5.8 Hz, 2H), 2.35 (s, 3H), 2.29 (s, 3H), 1.30 (d, *J* = 6.9 Hz, 6H). MS (ESI) *m*/*z*: 427 [M+H]⁺.** |
| **I-171** | 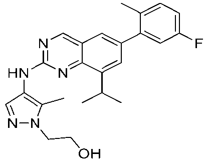 | **¹H NMR (600 MHz, DMSO-*d*₆) δ 9.20 (s, 1H), 9.13 (s, 1H), 8.03 (s, 1H), 7.68 (d, *J* = 2.0 Hz, 1H), 7.60 (d, *J* = 2.0 Hz, 1H), 7.36 (dd, *J* = 8.3, 6.0 Hz, 1H), 7.17 - 7.10 (m, 2H), 4.07 (t, *J* = 5.9 Hz, 2H), 3.95 (s, 1H), 3.71 (t, *J* = 5.9 Hz, 2H), 2.29 (s, 3H), 2.24 (s, 3H), 1.30 (d, *J* = 7.0 Hz, 6H). MS (ESI) *m*/*z*: 420 [M+H]⁺.** |
| **I-172** | 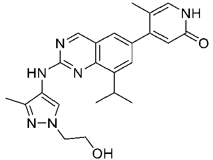 | **¹H NMR (400 MHz, DMSO-*d*₆) δ 11.51 (s, 1H), 9.30 (s, 1H), 9.24 (s, 1H), 8.29 (s, 1H), 7.74 (d, *J* = 2.0 Hz, 1H), 7.59 (d, *J* = 2.0 Hz, 1H), 7.32 (s, 1H), 6.29 (s, 1H), 4.93 (s, 1H), 4.06 (t, *J* = 5.6 Hz, 2H), 3.98 - 3.88 (m, 1H), 3.75 (t, *J* = 5.6 Hz, 2H), 2.23 (s, 3H), 1.97 (s, 3H), 1.35 (d, *J* = 7.2 Hz, 6H). MS (ESI) *m*/*z*: 419 [M+H]⁺.** |
| **I-173** | 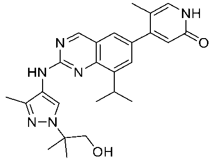 | **MS (ESI) *m*/*z*: 447 [M+H]⁺.** |
| **I-174** | 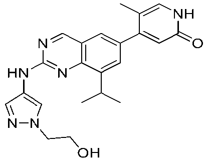 | **MS (ESI) *m*/*z*: 405 [M+H]⁺.** |
| **I-175** | 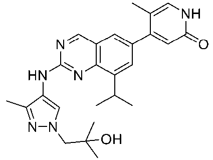 | **MS (ESI) *m*/*z*: 447[M+H]⁺.** |
| **I-176** | 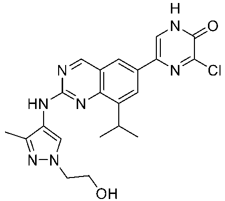 | **MS (ESI) *m*/*z*: 440 [M+H]⁺.** |
| **I-177** | 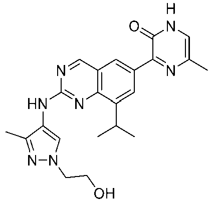 | **MS (ESI) *m*/*z*: 420 [M+H]⁺.** |
| **I-178** | 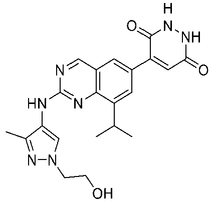 | **MS (ESI) *m*/*z*: 422[M+H]⁺.** |
| **I-179** | 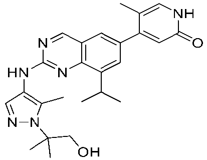 | **MS (ESI) *m*/*z*: 447 [M+H]⁺.** |
| **I-180** | 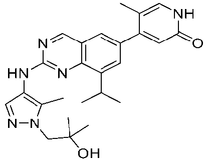 | **MS (ESI) *m*/*z*: 447 [M+H]⁺.** |
| **I-181** | 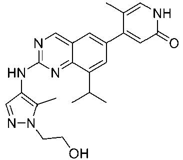 | **¹H NMR (400 MHz, DMSO-*d*₆) δ 11.53 (s, 1H), 9.22 (s, 2H), 8.03 (s, 1H), 7.71 (d, *J* = 2.0 Hz, 1H), 7.58 (d, *J* = 2.0 Hz, 1H), 7.32 (s, 1H), 6.28 (s, 1H), 4.89 (s, 1H), 4.07 (t, *J =* 6.0 Hz, 2H), 3.99 - 3.89 (m, 1H), 3.70 (t, *J* = 5.8 Hz, 2H), 2.30 (s, 3H), 1.96 (s, 3H), 1.32 (d, *J* = 6.8 Hz, 6H). MS (ESI) *m*/*z*: 419 [M+H]⁺.** |
| **I-182** | 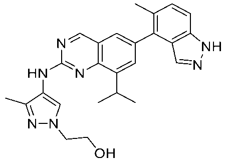 | **MS (ESI) *m*/*z*: 442 [M+H]⁺.** |
| **I-183** | 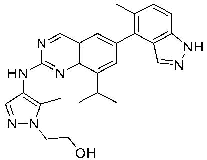 | **MS (ESI) *m*/*z*: 442 [M+H]⁺.** |
| **I-184** | 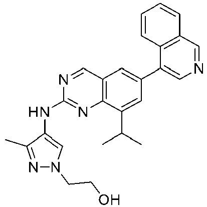 | **¹H NMR (600 MHz, DMSO-*d*₆) δ 9.38 (s, 1H), 9.37 - 9.27 (m, 2H), 8.56 (s, 1H), 8.40 - 8.30 (m, 1H), 8.27 (d, *J* = 8.2 Hz, 1H), 7.96 - 7.91 (m, 2H), 7.84 (t, *J* = 7.6 Hz, 1H), 7.80 - 7.73 (m, 2H), 4.95 (t, *J =* 5.3 Hz, 1H), 4.09 (t, *J =* 5.6 Hz, 2H), 4.06 - 3.99 (m, 1H), 3.76 (q, *J =* 5.5 Hz, 2H), 2.25 (s, 3H), 1.38 (d, *J* = 6.9 Hz, 6H). MS (ESI) *m*/*z*: 439 [M+H]⁺.** |
| **I-185** | 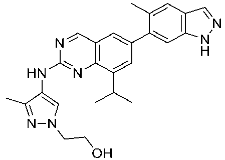 | **MS (ESI) *m*/*z*: 442 [M+H]⁺.** |
| **I-186** | 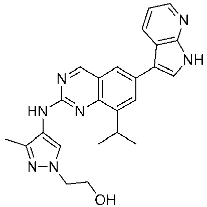 | **¹H NMR (600 MHz, DMSO-*d*₆) δ 9.31 (s, 1H), 9.21 (s, 1H), 8.36 - 8.24 (m, 2H), 8.11 (d, *J* = 2.1 Hz, 1H), 8.06 - 8.02 (m, 1H), 7.57 (s, 2H), 5.07 - 4.71 (m, 1H), 4.09 (t, *J* = 5.6 Hz, 2H), 4.06 - 3.98 (m, 1H), 3.75 (t, *J* = 5.7 Hz, 2H), 2.24 (s, 3H), 1.43 (d, *J* = 6.9 Hz, 6H). MS (ESI) *m*/*z*: 428 [M+H]⁺.** |
| **I-187** | 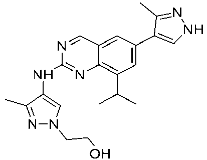 | **¹H NMR (600 MHz, DMSO-*d*₆) δ 12.67 (d, *J =* 50.2 Hz, 1H), 9.20 (s, 1H), 9.07 (s, 1H), 8.15 (d, *J* = 125.2 Hz, 1H), 7.78 - 7.70 (m, 3H), 4.89 (t, *J* = 5.3 Hz, 1H), 4.07 (t, *J* = 5.7 Hz, 2H), 4.00 - 3.93 (m, 1H), 3.74 (q, *J* = 5.6 Hz, 2H), 2.48 - 2.36 (m, 3H), 2.22 (s, 3H), 1.36 (d, *J* = 6.8 Hz, 6H). MS (ESI) *m*/*z:* 392 [M+H]⁺.** |
| **I-188** | 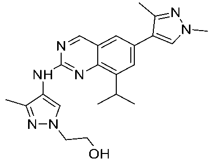 | **MS (ESI) *m*/*z*: 406 [M+H]⁺.** |
| **I-189** | 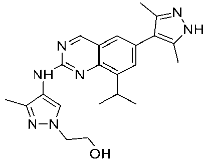 | **¹H NMR (600 MHz, DMSO-*d*₆) δ 12.44 - 12.23 (m, 1H), 9.20 (s, 1H), 9.14 - 9.01 (m, 1H), 8.36 - 8.16 (m, 1H), 7.59 (d, *J* = 2.0 Hz, 1H), 7.57 (d, *J* = 2.0 Hz, 1H), 4.89 (s, 1H), 4.07 (t, *J =* 5.7 Hz, 2H), 4.01 - 3.95 (m, 1H), 3.74 (t, *J* = 5.7 Hz, 2H), 2.26 (s, 6H), 2.22 (s, 3H), 1.35 (d, *J* = 6.9 Hz, 6H). MS (ESI) *m*/*z*: 406 [M+H]⁺.** |
| **I-190** | 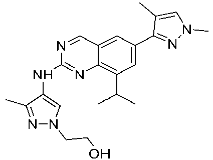 | **MS (ESI) *m*/*z*: 406 [M+H]⁺.** |
| **I-191** | 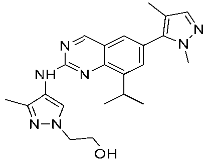 | **MS (ESI) *m*/*z*: 406 [M+H]⁺.** |
| **I-192** | 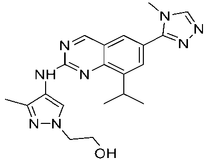 | **MS (ESI) *m*/*z*: 393 [M+H]⁺.** |
| **I-193** | 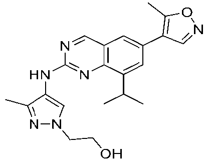 | **¹H NMR (600 MHz, DMSO-*d*₆) δ 9.30 - 9.14 (m, 2H), 8.97 (s, 1H), 8.33 - 8.21 (m, 1H), 7.85 (d, *J =* 2.0 Hz, 1H), 7.76 (d, *J =* 2.0 Hz, 1H), 4.08 (t, *J =* 5.6 Hz, 2H), 4.00 - 3.94 (m, 1H), 3.74 (t, *J =* 5.7 Hz, 2H), 2.65 (s, 3H), 2.23 (s, 3H), 1.37 (d, *J =* 6.9 Hz, 6H). MS (ESI) *m*/*z*: 393 [M+H]⁺.** |

### Synthesis of Compound II-1

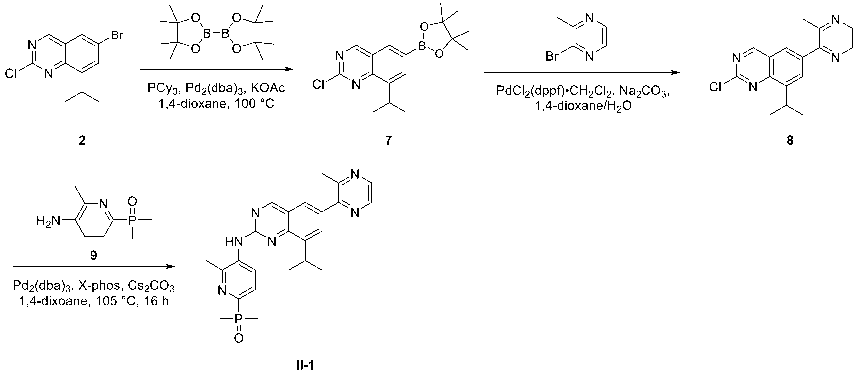

**1) Preparation of compound 7:**
   **Compound 2 (2.0 g, 7.0 mmol), bis(pinacolato)diboron (3.57 g, 14.0 mmol), Pd₂(dba)₃ (514 mg, 0.56 mmol), PCy₃ (393 mg, 1.4 mmol), and KOAc (2.06 g, 21.0 mmol) were dispersed in 1,4-dioxane (120 mL). After purging with nitrogen, the mixture was heated at 100°C for 1 h. LCMS analysis indicated the reaction was complete. The reaction mixture was filtered, and the filtrate was used directly in the next step.**
**2) Preparation of compound 8:**
   **2-bromo-3-methylpyrazine (1.34 g, 7.7 mmol), PdCl₂(dppf)•CH₂Cl₂ (575 mg, 0.7 mmol), Na₂CO₃ (2.23 g, 21.0 mmol), and H₂O (60 mL) were sequentially added to the filtrate from the previous step. After purging with nitrogen, the mixture was heated at 100°C for 1 h. LCMS analysis indicated the reaction was complete. The reaction mixture was filtered and concentrated. The residue was diluted with water and extracted with EtOAc. The organic layer was concentrated and purified by silica gel column chromatography to give compound 8 (1.9 g).·**
**3) Preparation of compound II-1:**
   **Compound 8 (15 mg, 0.05 mmol), compound 9 (10 mg, 0.05 mmol), Pd₂(dba)₃ (3 mg, 0.003 mmol), X-phos (3 mg, 0.006 mmol), and Cs₂CO₃ (33 mg, 0.1 mmol) were dispersed in 1,4-dioxane (2 mL). After purging with nitrogen, the mixture was heated at 105 °C for 16 h. LCMS analysis indicated the reaction was complete. The reaction mixture was filtered, concentrated, diluted with water, and extracted with EtOAc. The organic layer was concentrated and purified by silica gel column chromatography, and then further purified by preparative HPLC to give compound II-1 (4.2 mg).**

**The remaining compounds were synthesized by similar methods.**

**The specific compounds and structural identification data are listed in the table below.**

| **No.** | **Structure** | **NMR and/or MS data** |
|---|---|---|
| **II-1** | 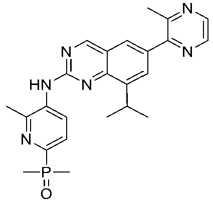 | **MS (ESI) *m*/*z*: 447 [M+H]⁺.** |
| **II-2** | 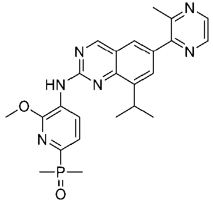 | **¹H NMR (400 MHz, DMSO-*d*₆) δ 9.47 (s, 1H), 9.07 - 9.04 (m, 1H), 8.65 (s, 1H), 8.63 (d, *J* = 2.4 Hz, 1H), 8.58 (d, *J* = 2.4 Hz, 1H), 8.17 (d, *J* =** 2.0 **Hz, 1H), 8.05 (d, *J* = 2.0 Hz, 1H), 7.67 - 7.64** (m, **1H), 4.07 - 4.00 (m, 4H), 2.67 (s, 3H), 1.69 (d,** *J* **= 13.6 Hz, 6H), 1.41 (d, *J =* 6.8 Hz, 6H). MS (ESI) *m*/*z*: 463 [M+H]⁺.** |
| **II-3** | 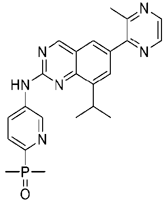 | **MS (ESI) *m*/*z:* 433 [M+H]⁺.** |
| **II-4** | 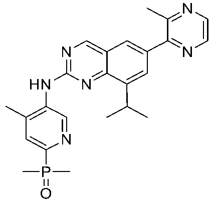 | **MS (ESI) *m*/*z*: 447 [M+H]⁺.** |
| **II-5** | 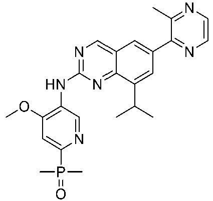 | **MS (ESI) *m*/*z*: 463 [M+H]⁺.** |
| **II-6** | 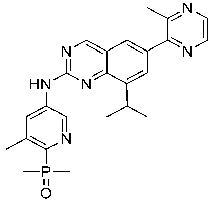 | **MS (ESI) *m*/*z*: 447 [M+H]⁺.** |
| **II-7** | 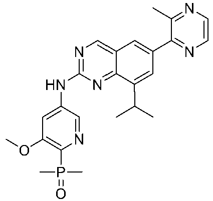 | **MS (ESI) *m*/*z*: 463 [M+H]⁺.** |
| **II-8** | 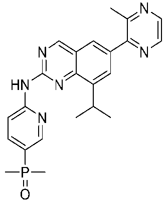 | **MS (ESI) *m*/*z:* 433 [M+H]⁺.** |
| **II-9** | 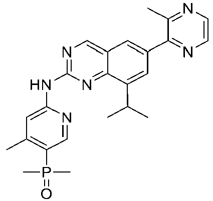 | **MS (ESI) *m*/*z*: 447 [M+H]⁺.** |
| **II-10** | 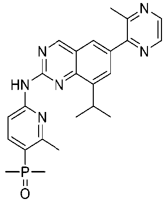 | **MS (ESI) *m*/*z*: 447 [M+H]⁺.** |
| **II-11** | 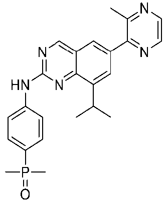 | **¹H NMR (400 MHz, DMSO-*d*₆) δ 10.30 (s, 1H), 9.43 (s, 1H), 8.63 (d, *J* = 2.4 Hz, 1H), 8.57 (d, *J* = 2.8 Hz, 1H), 8.22 - 8.19 (m, 2H), 8.13 (d, *J* = 2.0 Hz, 1H), 8.03 (d, *J* = 2.0 Hz, 1H), 7.77 - 7.72 (m, 2H), 4.11 - 4.04 (m, 1H), 2.68 (s, 3H), 1.66 (d, *J* = 13.2 Hz, 6H), 1.43 (d, *J* = 6.8 Hz, 6H). MS (ESI) *m*/*z*: 432 [M+H]⁺.** |
| **II-12** | 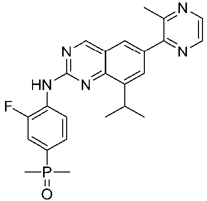 | **¹H NMR (400 MHz, DMSO-*d*₆) δ 9.61 (s, 1H), 9.44 (s, 1H), 8.65 - 8.54 (m, 3H), 8.14 (d, *J* = 2.0 Hz, 1H), 8.02 (d, *J* = 2.0 Hz, 1H), 7.70 - 7.61 (m, 2H), 4.02 - 3.95 (m, 1H), 2.67 (s, 3H), 1.69 (d, *J* = 13.2 Hz, 6H), 1.37 (d, *J =* 7.2 Hz, 6H). MS (ESI) *m*/*z*: 450 [M+H]⁺.** |
| **II-13** | 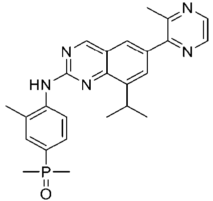 | **MS (ESI) *m*/*z*: 446 [M+H]⁺.** |
| **II-14** | 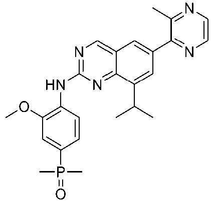 | **MS (ESI) *m*/*z*: 462 [M+H]⁺.** |
| **II-15** | 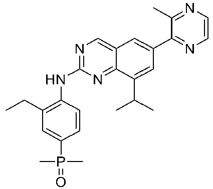 | **MS (ESI) *m*/*z*: 460 [M+H]⁺.** |
| **II-16** | 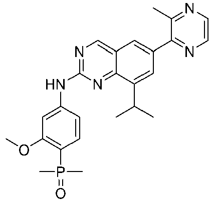 | **¹H NMR (400 MHz, DMSO-*d*₆) δ 10.31 (s, 1H), 9.44 (s, 1H), 8.63 (d, *J* = 2.4 Hz, 1H), 8.57 (d, *J* = 2.8 Hz, 1H), 8.21 - 8.19 (m, 1H), 8.13 (d, *J* = 2.0 Hz, 1H), 8.05 (d, *J* = 2.0 Hz, 1H), 7.68 - 7.56 (m, 2H), 4.21 - 4.14 (m, 1H), 3.96 (s, 3H), 2.67 (s, 3H), 1.64 (d, *J* = 13.6 Hz, 6H), 1.39 (d, *J =* 6.8 Hz, 6H). MS (ESI) *m*/*z*: 462 [M+H]⁺.** |
| **II-17** | 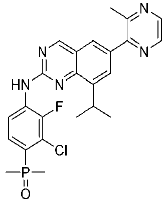 | **MS (ESI) *m*/*z*: 484 [M+H]⁺.** |
| **II-18** | 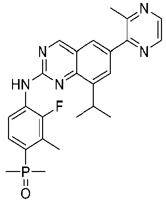 | **MS (ESI) *m*/*z*: 464 [M+H]⁺.** |
| **II-19** | 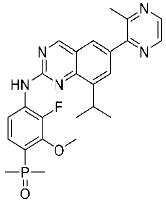 | **MS (ESI) *m*/*z*: 480 [M+H]⁺.** |
| **II-20** | 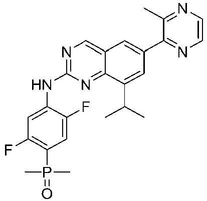 | **MS (ESI) *m*/*z*: 468 [M+H]⁺.** |
| **II-21** | 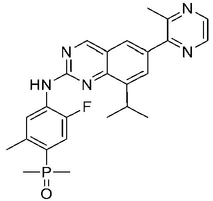 | **MS (ESI) *m*/*z*: 464 [M+H]⁺.** |
| **II-22** | 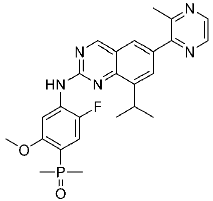 | **MS (ESI) *m*/*z*: 480 [M+H]⁺.** |
| **II-23** | 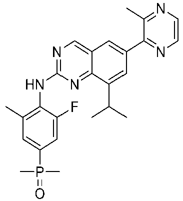 | **MS (ESI) *m*/*z*: 464 [M+H]⁺.** |
| **II-24** | 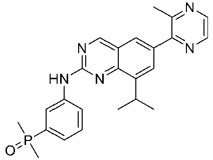 | **¹H NMR (600 MHz, Methanol-*d*₄) δ 9.27 (s, 1H), 8.58 (d, *J* = 2.5 Hz, 1H), 8.52 (d, *J* = 2.6 Hz, 1H), 8.49 (d, *J =* 13.6, 1.6 Hz, 1H), 8.19 (d, 1H), 7.99 - 7.96 (m, 2H), 7.55 (td, *J* = 7.9, 3.5 Hz, 1H), 7.48 - 7.42 (m, 1H), 4.23 (hept, *J* = 7.0 Hz, 1H), 2.70 (s, 3H), 1.86 (d, *J* = 13.3 Hz, 6H), 1.45 (d, *J* = 6.9 Hz, 6H). MS (ESI) m/z: 432 [M+H]⁺.** |
| **II-25** | 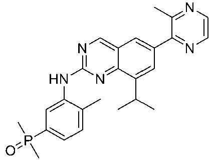 | **¹H NMR (600 MHz, DMSO-*d*₆) δ 9.36 (s, 1H), 9.14 (s, 1H), 8.61 (d, *J* = 2.2 Hz, 1H), 8.55 (d, *J* = 2.5 Hz, 1H), 8.32 (d, *J =* 12.4 Hz, 1H), 8.07 (d, *J* = 2.0 Hz, 1H), 7.95 (d, *J* = 2.0 Hz, 1H), 7.49 - 7.37 (m, 2H), 3.96 (p, *J* = 6.9 Hz, 1H), 2.66 (s, 3H), 2.38 (s, 3H), 1.66 (d, *J* = 13.2 Hz, 6H), 1.25 (d, *J* = 6.9 Hz, 6H). MS (ESI) *m*/*z*: 446 [M+H]⁺.** |
| **II-26** | 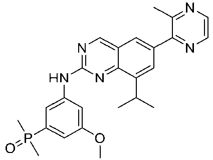 | **¹H NMR (600 MHz, Methanol-*d*₄) δ 9.24 (s, 1H), 8.56 (s, 1H), 8.50 (s, 1H), 8.10 (s, 1H), 7.97 (s, 1H), 7.95 (s, 1H), 7.81 (d, *J* = 13.3 Hz, 1H), 7.00 (d, *J* = 13.1 Hz, 1H), 4.23 (hept, *J* = 6.9 Hz, 1H), 3.93 (s, 3H), 2.68 (s, 3H), 1.84 (d, *J* = 13.1 Hz, 6H), 1.43 (d, *J* = 7.0 Hz, 6H). MS (ESI) *m*/*z*: 462 [M+H]⁺.** |
| **II-27** | 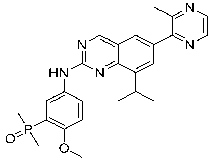 | **MS (ESI) *m*/*z*: 462 [M+H]⁺.** |
| **II-28** | 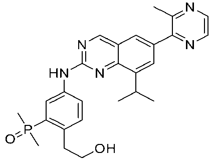 | **MS (ESI) *m*/*z*: 476 [M+H]⁺.** |
| **II-29** | 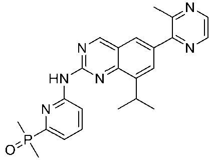 | **¹H NMR (600 MHz, DMSO-*d*₆) δ 10.35 (s, 1H), 9.50 (s, 1H), 8.69 (d, *J* = 8.4 Hz, 1H), 8.63 (dd, *J* = 2.5, 0.8 Hz, 1H), 8.58 (d, *J* = 2.5 Hz, 1H), 8.17 (d, *J* = 2.0 Hz, 1H), 8.07 - 8.01 (m, 2H), 7.59 (t, *J* = 7.1, 5.4, 0.9 Hz, 1H), 4.09 (h, *J =* 6.9 Hz, 1H), 2.68 (s, 3H), 1.70 (d, *J* = 13.5 Hz, 6H), 1.40 (d, *J* = 6.9 Hz, 6H). MS (ESI) *m*/*z:* 433 [M+H]⁺.** |
| **II-30** | 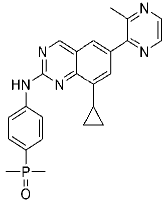 | **MS (ESI) *m*/*z*: 430 [M+H]⁺.** |
| **II-31** | 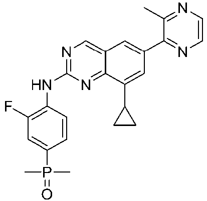 | **MS (ESI) *m*/*z*: 448 [M+H]⁺.** |
| **II-32** | 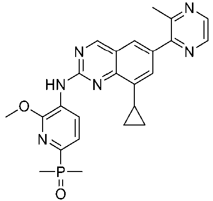 | **MS (ESI) *m*/*z*: 461 [M+H]⁺.** |
| **II-33** | 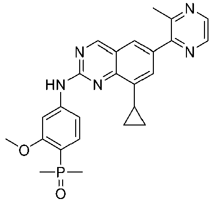 | **MS (ESI) *m*/*z*: 460 [M+H]⁺.** |
| **II-34** | 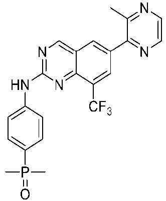 | **¹H NMR (600 MHz, Methanol-*d*₄) δ 9.41 (s, 1H), 8.61 (d, *J* = 2.5 Hz, 1H), 8.55 (d, *J* = 2.5 Hz, 1H), 8.47 (d, *J* = 1.9 Hz, 1H), 8.43 (d, *J* = 2.0 Hz, 1H), 8.27 (dd, *J* = 8.7, 2.3 Hz, 2H), 7.78 (dd, *J* = 11.6, 8.4 Hz, 2H), 2.73 (s, 3H), 1.81 (d, *J* = 13.3 Hz, 6H). MS (ESI) *m*/*z*: 458 [M+H]⁺.** |
| **II-35** | 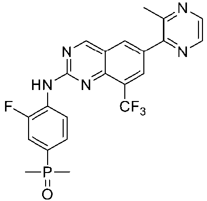 | **¹H NMR (600 MHz, DMSO-*d*₆) δ 10.06 (s, 1H), 9.59 (s, 1H), 8.65 (d, *J =* 2.4 Hz, 1H), 8.63 - 8.61 (m, 2H), 8.59 - 8.53 (m, 1H), 8.51 (d, *J* = 1.9 Hz, 1H), 7.67 (t, *J* = 11.2 Hz, 1H), 7.65 - 7.60 (m, 1H), 2.71 (s, 3H), 1.70 (d, *J* = 13.4 Hz, 6H). MS (ESI) *m*/*z*: 476 [M+H]⁺.** |
| **II-36** | 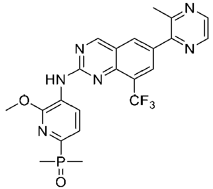 | **¹H NMR (600 MHz, Methanol-*d*₄) δ 9.47 (s, 1H), 9.38 (dd, *J* = 7.9, 3.5 Hz, 1H), 8.61 (d, *J* = 2.5 Hz, 1H), 7.75 - 7.59 (m, 2H), 7.45 (t, *J =* 4.1 Hz, 1H), 6.73 - 6.61 (m, 1H), 4.16 (s, 3H), 2.74 (s, 3H), 1.82 (d, *J* = 13.6 Hz, 6H). MS (ESI) m/z: 489 [M+H]⁺.** |
| **II-37** | 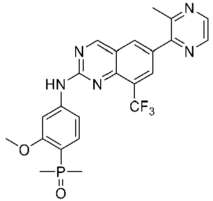 | **¹H NMR (600 MHz, DMSO-*d*₆) δ 10.68 (s, 1H), 9.64 (s, 1H), 8.71 (d, *J =* 2.4 Hz, 1H), 8.69 - 8.64 (m, 2H), 8.58 (s, 1H), 8.22 (s, 1H), 7.71 (dd, *J =* 12.3, 8.2 Hz, 1H), 7.59 (d, *J =* 8.4 Hz, 1H), 4.00 (s, 3H), 2.77 (s, 3H), 1.68 (d, *J =* 13.7 Hz, 6H). MS (ESI) *m*/*z*: 488 [M+H]⁺.** |
| **II-38** | 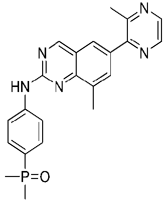 | **MS (ESI) *m*/*z*: 404 [M+H]⁺.** |
| **II-39** | 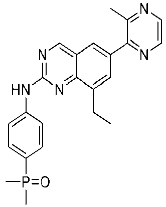 | **MS (ESI) *m*/*z*: 418 [M+H]⁺.** |
| **II-40** | 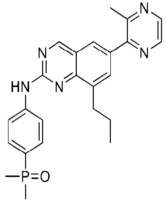 | **MS (ESI) *m*/*z*: 432 [M+H]⁺.** |
| **II-41** | 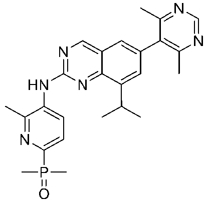 | **MS (ESI) m/z: 461[M+H]⁺.** |
| **II-42** | 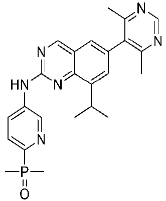 | **MS (ESI) m/z: 447 [M+H]⁺.** |
| **II-43** | 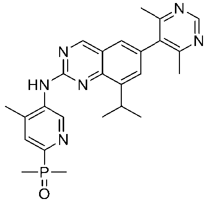 | **MS (ESI) m/z: 461 [M+H]⁺.** |
| **II-44** | 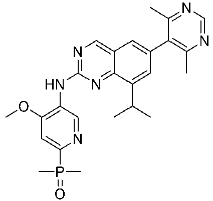 | **MS (ESI) m/z: 477 [M+H]⁺.** |
| **II-45** | 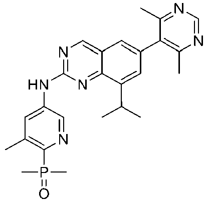 | **MS (ESI) m/z: 461 [M+H]⁺.** |
| **II-46** | 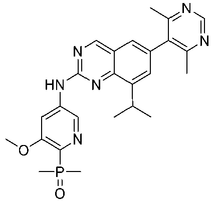 | **MS (ESI) *m*/*z*: 477 [M+H]⁺.** |
| **II-47** | 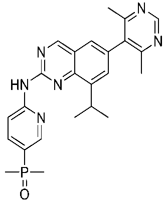 | **MS (ESI) *m*/*z*: 447 [M+H]⁺.** |
| **II-48** | 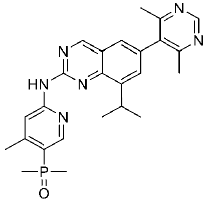 | **MS (ESI) *m*/*z*: 461 [M+H]⁺.** |
| **II-49** | 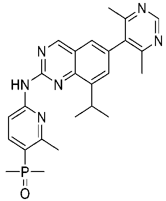 | **MS (ESI) m/z: 461 [M+H]⁺.** |
| **II-50** | 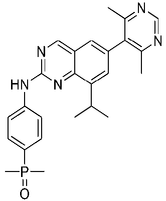 | **MS (ESI) *m*/*z*: 446 [M+H]⁺.** |
| **II-51** | 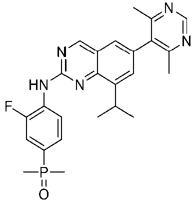 | **MS (ESI) *m*/*z*: 464 [M+H]⁺.** |
| **II-52** | 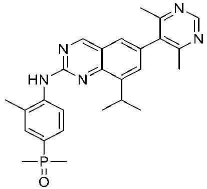 | **MS (ESI) *m*/*z*: 460 [M+H]⁺.** |
| **II-53** | 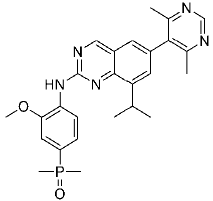 | **MS (ESI) *m*/*z*: 476 [M+H]⁺.** |
| **II-54** | 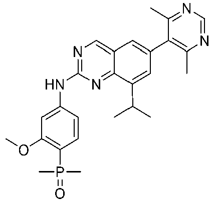 | **MS (ESI) *m*/*z*: 476 [M+H]⁺.** |
| **II-55** | 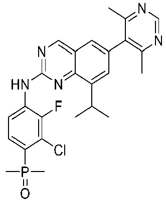 | **MS (ESI) *m*/*z*: 498 [M+H]⁺.** |
| **II-56** | 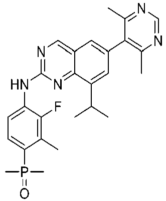 | **MS (ESI) *m*/*z*: 478 [M+H]⁺.** |
| **II-57** | 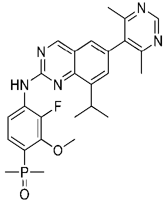 | **MS (ESI) *m*/*z*: 494 [M+H]⁺.** |
| **II-58** | 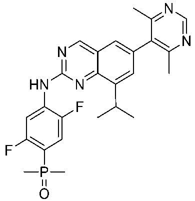 | **MS (ESI) *m*/*z*: 482 [M+H]⁺.** |
| **II-59** | 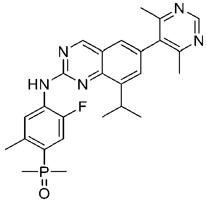 | **MS (ESI) *m*/*z*: 478 [M+H]⁺.** |
| **II-60** | 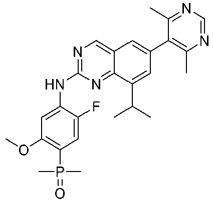 | **MS (ESI) *m*/*z*: 494 [M+H]⁺.** |
| **II-61** | 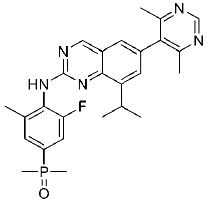 | **MS (ESI) *m*/*z*: 478 [M+H]⁺.** |
| **II-62** | 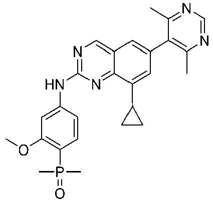 | **MS (ESI) *m*/*z*: 474 [M+H]⁺.** |
| **II-63** | 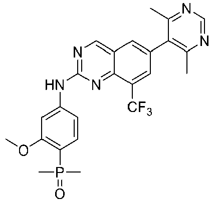 | **¹H NMR (600 MHz, DMSO-*d*₆) δ 10.59 (s, 1H), 9.48 (s, 1H), 8.96 (s, 1H), 8.28 - 8.22 (m, 2H), 8.16 (d, *J =* 4.4 Hz, 1H), 7.64 (dd, *J* = 12.2, 8.3 Hz, 1H), 7.54 (d, *J* = 8.3 Hz, 1H), 3.94 (s, 3H), 2.26 (s, 6H), 1.62 (d, *J* = 13.6 Hz, 6H). MS (ESI) *m*/*z*: 502 [M+H]⁺.** |
| **II-64** | 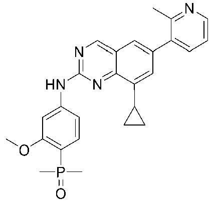 | **¹H NMR (400 MHz, DMSO-*d*₆) δ 10.28 (s, 1H), 9.38 (s, 1H), 8.49 (dd, *J =* 4.8, 1.6 Hz, 1H), 8.16 (d, *J* = 4.4 Hz, 1H), 7.77 (d, *J* = 2.0 Hz, 1H), 7.74 - 7.60 (m, 3H), 7.38 - 7.29 (m, 2H), 3.88 (s, 3H), 3.07 - 3.00 (m, 1H), 2.46 (s, 3H), 1.61 (d, *J* = 13.6 Hz, 6H), 1.17 - 1.09 (m, 2H), 0.96 - 0.89 (m, 2H). MS (ESI) *m*/*z*: 459 [M+H]⁺.** |
| **II-65** | 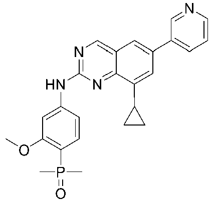 | **MS (ESI) *m*/*z*: 445 [M+H]⁺.** |
| **II-66** | 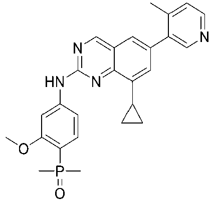 | **MS (ESI) *m*/*z*: 459 [M+H]⁺.** |
| **II-67** | 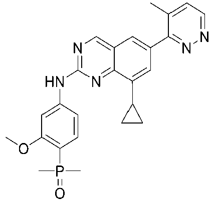 | **MS (ESI) *m*/*z*: 460 [M+H]⁺.** |
| **II-68** | 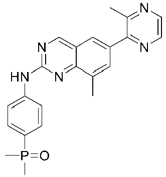 | **MS (ESI) *m*/*z*: 404 [M+H]⁺.** |
| **II-69** | 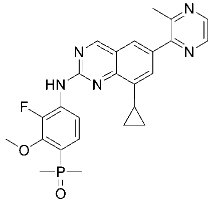 | **¹H NMR (400 MHz, DMSO-*d*₆) δ 9.75 (s, 1H), 9.44 (s, 1H), 8.60 (d, *J* = 2.4 Hz, 1H), 8.56 (d, *J* = 2.8 Hz, 1H), 8.31 - 8.27 (m, 1H), 8.07 (d, *J* = 2.0 Hz, 1H), 7.59 (d, *J* = 2.0 Hz, 1H), 7.55 - 7.50 (m, 1H), 4.01 (d, *J* = 2.4 Hz, 3H), 2.89 - 2.84 (m, 1H), 2.63 (s, 3H), 1.68 (d, J = 13.6 Hz, 6H), 1.13 - 1.09 (m, 2H), 0.87 - 0.86 (m, 2H). MS (ESI) *m*/*z*: 478 [M+H]⁺.** |
| **II-70** | 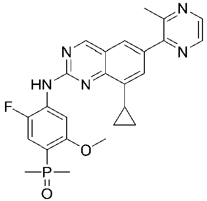 | **MS (ESI) *m*/*z*: 478 [M+H]⁺.** |
| **II-71** | 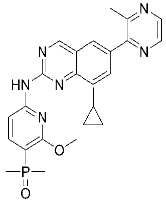 | **¹H NMR (600 MHz, DMSO-*d*₆) δ 10.33 (s, 1H), 9.49 (s, 1H), 8.61 (d, *J* = 2.5 Hz, 1H), 8.56 (d, *J* = 2.5 Hz, 1H), 8.46 (d, *J =* 8.2 Hz, 1H), 8.14 - 8.09 (m, 2H), 7.66 (d, *J* = 1.9 Hz, 1H), 3.99 (s, 3H), 2.97 - 2.89 (m, 1H), 2.64 (s, 3H), 1.64 (d, *J* = 13.7 Hz, 6H), 1.22 - 1.18 (m, 2H), 0.91 - 0.85 (m, 2H). MS (ESI) *m*/*z*: 461 [M+H]⁺.** |
| **II-72** | 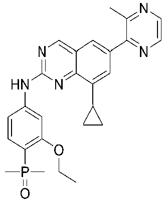 | **¹H NMR (600 MHz, DMSO-*d*₆) δ 10.31 (s, 1H), 9.43 (s, 1H), 8.60 (d, *J* = 2.4 Hz, 1H), 8.55 (d, *J* = 2.5 Hz, 1H), 8.15 (d, *J* = 4.6 Hz, 1H), 8.06 (d, *J* = 1.9 Hz, 1H), 7.69 - 7.60 (m, 2H), 7.58 (d, *J* = 1.9 Hz, 1H), 4.14 (q, *J* = 6.9 Hz, 2H), 3.05 - 2.98 (m, 1H), 2.63 (s, 3H), 1.63 (d, *J* = 13.6 Hz, 6H), 1.41 (t, *J* = 6.9 Hz, 3H), 1.17 - 1.12 (m, 2H), 0.93 - 0.89 (m, 2H). MS (ESI) *m*/*z*: 474 [M+H]⁺.** |
| **II-73** | 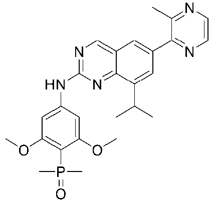 | **¹H NMR (600 MHz, DMSO-*d*₆) δ 10.23 (s, 1H), 9.44 (s, 1H), 8.63 (d, *J* = 2.4 Hz, 1H), 8.57 (d, *J* = 2.5 Hz, 1H), 8.14 (d, *J* = 2.0 Hz, 1H), 8.06 (d, *J* = 2.0 Hz, 1H), 7.55 (d, *J =* 3.7 Hz, 2H), 4.22 (hept, *J* = 6.7 Hz, 1H), 3.87 (s, 6H), 2.67 (s, 3H), 1.70 (d, *J* = 13.5 Hz, 6H), 1.37 (d, *J* = 7.0 Hz, 6H). MS (ESI) *m*/*z*: 492 [M+H]⁺.** |
| **II-74** | 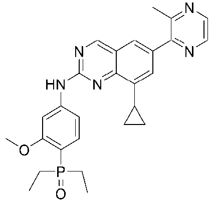 | **¹H NMR (600 MHz, DMSO-*d*₆) δ 10.33 (s, 1H), 9.44 (s, 1H), 8.60 (d, *J* = 2.4 Hz, 1H), 8.56 (d, *J* = 2.5 Hz, 1H), 8.11 (d, *J* = 4.3 Hz, 1H), 8.06 (d, *J* = 2.0 Hz, 1H), 7.71 (d, *J =* 8.3 Hz, 1H), 7.67 - 7.62 (m, 1H), 7.59 (d, *J* = 1.9 Hz, 1H), 3.86 (s, 3H), 3.09 - 2.95 (m, 1H), 2.64 (s, 3H), 1.96 - 1.82 (m, 4H), 1.18 - 1.13 (m, 2H), 0.96 - 0.88 (m, 8H). MS (ESI) *m*/*z*: 488 [M+H]⁺.** |
| **II-75** | 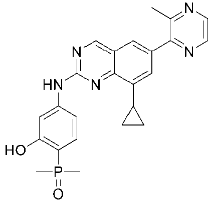 | **¹H NMR (400 MHz, DMSO- *d*₆) δ 10.85 (s, 1H), 10.20 (s, 1H), 9.41 (s, 1H), 8.60 (d, *J* = 2.4 Hz, 1H), 8.55 (d, *J* = 2.4 Hz, 1H), 8.05 (d, *J* = 2.0 Hz, 1H), 7.70 - 7.66 (m, 2H), 7.61 (d, *J* = 2.0 Hz, 1H), 7.53 - 7.47 (m, 1H), 2.97 - 2.91 (m, 1H), 2.63 (s, 3H), 1.69 (d, *J* = 13.6 Hz, 6H), 1.21 - 1.14 (m, 2H), 0.90 - 0.83 (m, 2H). MS (ESI) *m*/*z*: 446 [M+H]⁺.** |
| **II-76** | 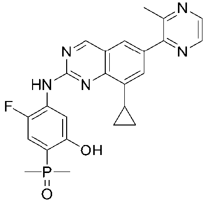 | **¹H NMR (600 MHz, DMSO-*d*₆) δ 10.76 (s, 1H), 9.45 - 9.42 (m, 2H), 8.60 (d, *J* = 2.4 Hz, 1H), 8.55 (d, *J =* 2.5 Hz, 1H), 8.11 - 8.06 (m, 2H), 7.60 (d, *J* = 1.9 Hz, 1H), 7.40 (dd, *J* = 12.7, 10.9 Hz, 1H), 2.94 (tt, *J* = 8.5, 5.3 Hz, 1H), 2.63 (s, 3H), 1.72 (d, *J = 13.8* Hz, 6H), 1.17 - 1.10 (m, 2H), 0.89 - 0.83 (m, 2H). MS (ESI) m/z: 464 [M+H]⁺.** |
| **II-77** | 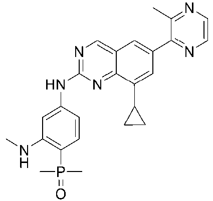 | **¹H NMR (600 MHz, DMSO-*d*₆) δ 10.08 (s, 1H), 9.40 (s, 1H), 8.60 (d, *J* = 2.4 Hz, 1H), 8.55 (d, *J* = 2.5 Hz, 1H), 8.04 (d, *J* = 1.8 Hz, 1H), 7.72 (d, *J* = 4.0 Hz, 1H), 7.55 (d, *J* = 1.9 Hz, 1H), 7.28 - 7.17 (m, 2H), 3.11 - 3.01 (m, 1H), 2.77 (s, 3H), 2.63 (s, 3H), 1.66 (d, *J* = 13.2 Hz, 6H), 1.15 - 1.09 (m, 2H), 0.92 - 0.88 (m, 2H). MS (ESI) *m*/*z*: 459 [M+H]⁺.** |

### Synthesis of Compounds III-20A and III-20B (Regioisomeric Mixture)

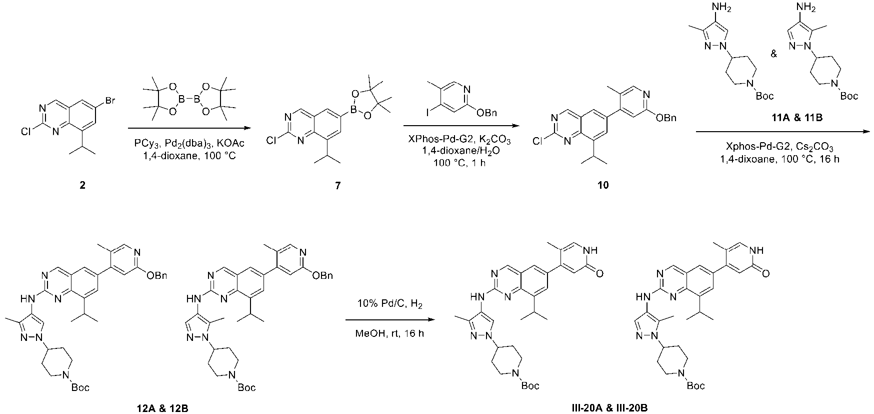

1) Preparation of compound 7:
   Compound 2 (1.0 g, 3.5 mmol), bis(pinacolato)diboron (1.33 g, 5.2 mmol), Pd₂(dba)₃ (257 mg, 0.28 mmol), PCy₃ (196 mg, 0.7 mmol), and KOAc (1.0 g, 10.2 mmol) were dispersed in 1,4-dioxane (20 mL). After purging with nitrogen, the mixture was heated at 100 °C for 1 h. LCMS analysis indicated the reaction was complete. The reaction mixture was filtered, and the filtrate was used directly in the next step.
2) Preparation of compound 10:
   2-(benzyloxy)-4-iodo-5-methylpyridine (1.25 g, 3.8 mmol), XPhos-Pd-G₂ (232 mg, 0.3 mmol), and K₂CO₃ (724 mg, 5.2 mmol) were sequentially added to the filtrate from the previous step. H₂O (10 mL) was then added. After purging with nitrogen, the mixture was heated at 100 °C for 1 h. LCMS analysis indicated the reaction was complete. The reaction mixture was filtered, concentrated, diluted with water, and extracted with EtOAc. The organic layer was concentrated and purified by silica gel column chromatography to give compound 10 (950 mg).
3) Preparation of mixture 12A & 12B:
   Compound 10 (18 mg, 0.046 mmol), compound 11A & 11B (15 mg, 0.054 mmol), Xphos Pd-G₂ (2.0 mg, 0.002 mmol), and Cs₂CO₃ (44 mg, 0.136 mmol) were dispersed in 1,4-dioxane (3.0 mL). After purging with nitrogen, the mixture was heated at 100 °C for 16 h. LCMS analysis indicated the reaction was complete. The reaction mixture was filtered, concentrated, diluted with water, and extracted with EtOAc. The organic phase was concentrated and purified by preparative TLC to afford mixture 12A & 12B (10 mg).
4) Preparation of mixture III-20A & III-20B:
   Mixture 12A & 12B (10 mg, 0.015 mmol) was dissolved in MeOH (10.0 mL). 10% Pd/C (2 mg) was added, and the mixture was stirred under a hydrogen atmosphere at room temperature for 16 h. LCMS analysis indicated the reaction was complete. The reaction mixture was filtered, concentrated, and purified by preparative HPLC to afford the regioisomeric mixture III-20A & III-20B (1.2 mg).

### Synthesis of compound III-74:

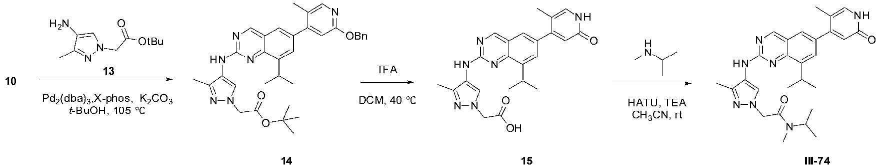

1) Preparation of compound 14:
   Compound 10 (200 mg, 0.5 mmol), compound 13 (158 mg, 0.75 mmol), Pd₂(dba)₃ (46 mg, 0.05 mmol), X-phos (36 mg, 0.075 mmol), and K₂CO₃ (207 mg, 1.5 mmol) were dispersed in t-BuOH (10 mL). After purging with nitrogen, the mixture was heated at 105 °C for 3.5 h. LCMS analysis indicated the reaction was complete. The reaction mixture was concentrated, diluted with water, and extracted with DCM. The organic layer was concentrated and purified by silica gel column chromatography to give compound 14 (85 mg).
2) Preparation of compound 15:
   Compound 14 (21.3 mg, 0.036 mmol) was dissolved in DCM (2 mL). TFA (1.5 mL) was added, and the mixture was stirred at 40 °C overnight. LCMS analysis indicated the reaction was complete. The reaction mixture was concentrated to give the trifluoroacetic acid salt of compound 15, which was used directly in the next step.
3) Preparation of compound III-74:
   Compound 15 (0.036 mmol), methylisopropylamine (4.2 mg, 0.057 mmol), HATU (20.6 mg, 0.057 mmol), and TEA (10.6 mg, 0.1 mmol) were dissolved in MeCN (2 mL). The mixture was stirred at 50 °C overnight. LCMS analysis indicated the reaction was complete. Water was added for dilution, and extraction was performed with DCM. The organic layer was concentrated and purified by silica gel column chromatography, followed by further purification by preparative HPLC to give compound III-74 (2.1 mg).

The remaining compounds were synthesized by similar methods.

The specific compounds and structural identification data are listed in the table below.

| **No.** | **Structure** | **NMR and/or MS data** |
|---|---|---|
| **III-1** | 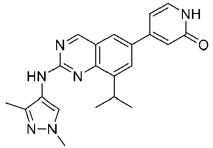 | **MS (ESI) *m*/*z*: 375 [M+H]⁺.** |
| **III-2** | 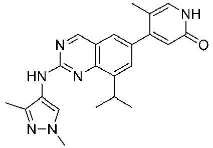 | **¹H NMR (400 MHz, DMSO-*d*₆) δ 11.47 (s, 1H), 9.23 (s, 2H), 8.11 (s, 1H), 7.73 (d, *J* = 2.0 Hz, 1H), 7.59 (d, *J* = 2.0 Hz, 1H), 7.31 (s, 1H), 6.28 (s, 1H), 4.00 - 3.89 (m, 1H), 3.79 (s, 3H), 2.20 (s, 3H), 1.96 (s, 3H), 1.34 (d, *J* = 6.8 Hz, 6H). MS (ESI) *m*/*z*: 389 [M+H]⁺.** |
| **III-3** | 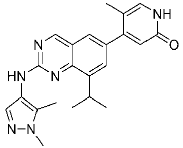 | **MS (ESI) *m*/*z*: 389 [M+H]⁺.** |
| **III-4** | 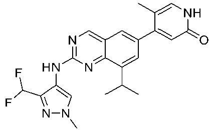 | **¹H NMR (400 MHz, DMSO-*d*₆) δ 11.53 (s, 1H), 9.46 (s, 1H), 9.30 (s, 1H), 8.32 (s, 1H), 7.78 (d, *J* = 2.0 Hz, 1H), 7.63 (d, *J =* 2.0 Hz, 1H), 7.41 - 7.15 (m, 2H), 6.29 (s, 1H), 4.02 - 3.95 (m, 1H), 3.94 (s, 3H), 1.96 (s, 3H), 1.35 (d, *J* = 6.8 Hz, 6H). MS (ESI) *m*/*z*: 425 [M+H]⁺.** |
| **III-5** | 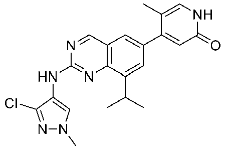 | **MS (ESI) *m*/*z*: 409 [M+H]⁺.** |
| **III-6** | 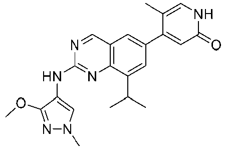 | **MS (ESI) *m*/*z*: 405 [M+H]⁺.** |
| **III-7** | 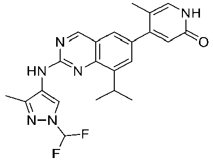 | **MS (ESI) *m*/*z*: 425 [M+H]⁺.** |
| **III-8** | 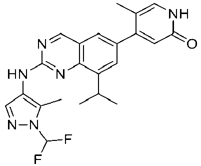 | **MS (ESI) *m*/*z*: 425 [M+H]⁺.** |
| **III-9** | 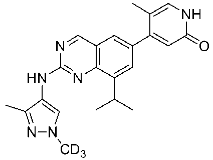 | **¹H NMR (400 MHz, DMSO-*d*₆) δ 11.48 (s, 1H), 9.23 (s, 1H), 9.20 (s, 1H), 8.10 (s, 1H), 7.72 (d, *J* = 2.0 Hz, 1H), 7.58 (d, *J =* 2.0 Hz, 1H), 7.31 (s, 1H), 6.28 (s, 1H), 3.99 - 3.89 (m, 1H), 2.20 (s, 3H), 1.96 (s, 3H), 1.34 (d, *J =* 6.8 Hz, 6H). MS (ESI) *m*/*z*: 392 [M+H]⁺.** |
| **III-10** | 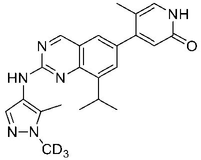 | **MS (ESI) *m*/*z*: 392 [M+H]⁺.** |
| **III-11** | 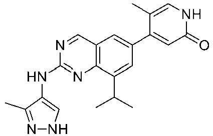 | **MS (ESI) *m*/*z*: 375 [M+H]⁺.** |
| **III-12** | 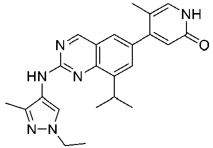 | **MS (ESI) *m*/*z*: 403 [M+H]⁺.** |
| **III-13** | 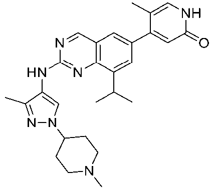 | **¹H NMR (400 MHz, DMSO-*d*₆) δ 11.54 (s, 1H), 9.32 (s, 1H), 9.26 (s, 1H), 8.28 (s, 1H), 7.74 (d, *J* = 2.0 Hz, 1H), 7.60 (d, *J* = 2.0 Hz, 1H), 7.32 (s, 1H), 6.29 (s, 1H), 4.39 - 4.33 (m, 1H), 3.97 - 3.88 (m, 1H), 2.81 - 2.79 (m, 2H), 2.45 - 2.05 (m, 10H), 2.03 - 1.99 (m, 1H), 1.96 (s, 3H), 1.50 - 1.42 (m, 1H), 1.41 - 1.33 (m, 6H). MS (ESI) m/z: 472 [M+H]⁺.** |
| **III-14** | 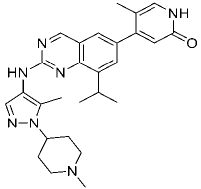 | **¹H NMR (400 MHz, DMSO-*d*₆) δ 11.55 (s, 1H), 9.21 (s, 1H), 9.15 (s, 1H), 8.03 (s, 1H), 7.70 (d, *J* = 2.0 Hz, 1H), 7.57 (d, *J* = 2.0 Hz, 1H), 7.31 (s, 1H), 6.28 (s, 1H), 4.11 - 4.02 (m, 1H), 3.98 - 3.88 (m, 1H), 2.93 - 2.84 (m, 2H), 2.30 (s, 3H), 2.21 (s, 3H), 2.07 - 2.03 (m, 4H), 1.96 (s, 3H), 1.81 - 1.77 (m, 2H), 1.30 (d, *J* = 6.8 Hz, 6H). MS (ESI) *m*/*z*: 472 [M+H]⁺.** |
| **III-15A&II I-15B** | 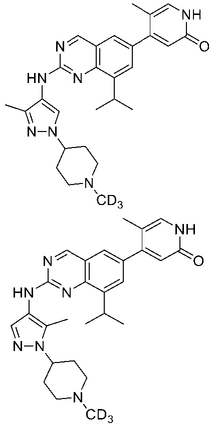 | **MS (ESI) m/z: 475 [M+H]⁺.** |
| **III-16** | 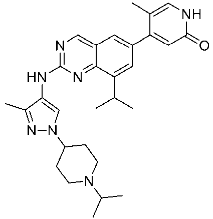 | **¹H NMR (600 MHz, DMSO-*d*₆) δ 9.40 (s, 1H), 9.27 (d, *J* = 3.4 Hz, 1H), 9.17 (s, 1H), 8.29 (s, 1H), 7.76 (d, *J* = 2.0 Hz, 1H), 7.61 (d, *J* = 2.0 Hz, 1H), 7.37 (s, 1H), 6.33 (s, 1H), 4.50 - 4.38 (m, 1H), 3.95 - 3.89 (m, 1H), 3.60 - 3.47 (m, 3H), 3.27 - 3.16 (m, 2H), 2.35 - 2.30 (m, 2H), 2.26 (s, 3H), 2.23 - 2.17 (m, 2H), 1.98 (s, 3H), 1.38 (d, *J* = 6.9 Hz, 6H), 1.29 (d, *J* = 6.7 Hz, 6H). MS (ESI) *m*/*z*: 500 [M+H]⁺.** |
| **III-17** | 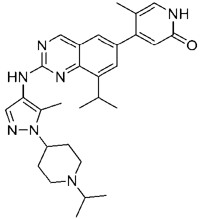 | **¹H NMR (600 MHz, DMSO-*d*₆) δ 9.30 - 9.19 (m, 2H), 9.13 - 9.08 (m, 1H), 8.09 (s, 1H), 7.73 (d, *J* = 2.0 Hz, 1H), 7.59 (d, *J* = 2.1 Hz, 1H), 7.35 (s, 1H), 6.31 (s, 1H), 4.60 - 4.49 (m, 1H), 3.97 - 3.86 (m, 1H), 3.50 - 3.47 (m, 3H), 3.28 - 3.16 (m, 2H), 2.41 - 2.26 (m, 5H), 2.13 - 2.06 (m, 2H), 1.97 (s, 3H), 1.31 (d, *J* = 6.9 Hz, 6H), 1.29 (d, *J* = 6.6 Hz, 6H). MS (ESI) *m*/*z*: 500 [M+H]⁺.** |
| **III-18** | 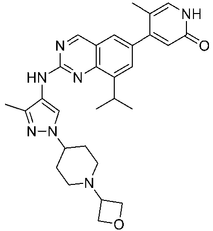 | **MS (ESI) *m*/*z*: 514 [M+H]⁺.** |
| **III-19** | 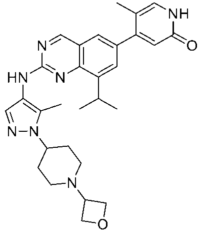 | **MS (ESI) *m*/*z*: 514 [M+H]⁺.** |
| **III-20A&II I-20B** | 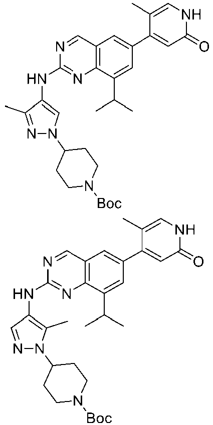 | **MS (ESI) *m*/*z*: 558 [M+H]⁺.** |
| **III-21** | 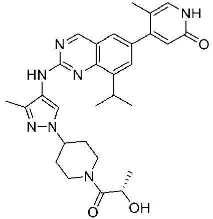 | **MS (ESI) *m*/*z:* 530 [M+H]⁺.** |
| **III-22** | 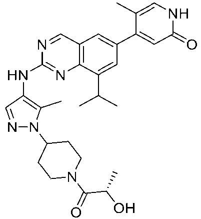 | **MS (ESI) *m*/*z:* 530 [M+H]⁺.** |
| **III-23** | 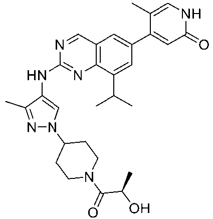 | **MS (ESI) *m*/*z:* 530 [M+H]⁺.** |
| **III-24** | 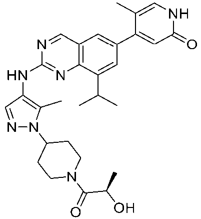 | **MS (ESI) *m*/*z:* 530 [M+H]⁺.** |
| **III-25** | 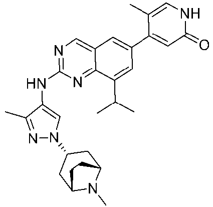 | **¹H NMR (600 MHz, Methanol-*d*₄) δ 8.25 - 8.17 (m, 1H), 7.91 (s, 1H), 7.46 (d, *J* = 1.9 Hz, 1H), 7.33 (d, *J* = 1.9 Hz, 1H), 6.54 (s, 1H), 5.73 (s, 1H), 4.02 - 3.96 (m, 1H), 2.85 (s, 3H), 2.65 - 2.61 (m, 1H), 2.60 - 2.53 (m, 2H), 2.50 - 2.43 (m, 4H), 2.39 - 2.34 (m, 1H), 2.32 - 2.30 (m, 1H), 2.27 - 2.24 (m, 1H), 2.24 (s, 3H), 2.23 - 2.20 (m, 1H), 2.05 (d, *J* = 1.0 Hz, 3H), 1.39 (d, *J* = 7.0 Hz, 6H). MS (ESI) *m*/*z*: 498 [M+H]⁺.** |
| **III-26** | 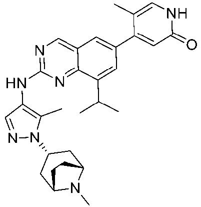 | **¹H NMR (600 MHz, Methanol-*d*₄) δ 8.18 - 7.98 (m, 1H), 7.62 (s, 1H), 7.48 (s, 1H), 7.46 (d, *J* = 1.9 Hz, 1H), 7.33 (d, *J* = 2.0 Hz, 1H), 6.55 (s, 1H), 4.75 (tt, *J* = 11.5, 5.8 Hz, 1H), 4.10 - 4.05 (m, 4H), 2.85 (s, 3H), 2.63 - 2.55 (m, 1H), 2.48 - 2.43 (m, 2H), 2.37 (s, 3H), 2.31 - 2.27 (m, 2H), 2.25 - 2.18 (m, 2H), 2.06 (d, *J* = 1.0 Hz, 3H), 1.39 - 1.34 (m, 6H). MS (ESI) *m*/*z*: 498 [M+H]⁺.** |
| **III-27** | 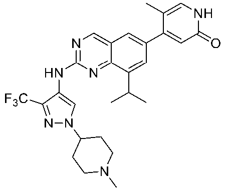 | **¹H NMR (600 MHz, Methanol-*d*₄) δ 9.23 (s, 1H), 8.61 (s, 1H), 7.76 (d, *J* = 2.0 Hz, 1H), 7.69 (d, *J* = 2.0 Hz, 1H), 7.51 - 7.47 (m, 1H), 6.61 (s, 1H), 4.70 - 4.62 (m, 1H), 4.07 - 4.00 (m, 1H), 3.78 - 3.71 (m, 2H), 3.58 - 3.32 (m, 1H), 3.29 - 3.27 (m, 1H), 2.97 (s, 3H), 2.54 - 2.34 (m, 4H), 2.10 (s, 3H), 1.43 (d, *J* = 6.9 Hz, 6H). MS (ESI) *m*/*z*: 526 [M+H]⁺.** |
| **III-28** | 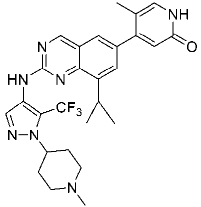 | **¹H NMR (600 MHz, Methanol-*d*₄) δ 9.22 (s, 1H), 8.45 (s, 1H), 7.75 (d, *J* = 2.0 Hz, 1H), 7.67 (d, *J* = 2.0 Hz, 1H), 7.52 - 7.49 (m, 1H), 6.62 (s, 1H), 4.72 - 4.65 (m, 1H), 4.08 - 4.00 (m, 1H), 3.73 - 3.67 (m, 2H), 3.36 - 3.33 (m, 1H), 3.30 - 3.28 (m, 1H), 2.95 (s, 3H), 2.62 - 2.52 (m, 2H), 2.35 - 2.28 (m, 2H), 2.10 (s, 3H), 1.37 (d, *J* = 6.9 Hz, 6H). MS (ESI) *m*/*z*: 526 [M+H]⁺.** |
| **III-29** | 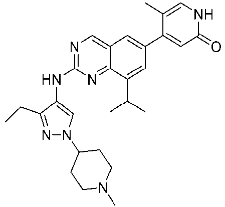 | **¹H NMR (600 MHz, Methanol-*d*₄) δ 7.92 (s, 1H), 7.49 (s, 1H), 7.47 (d, *J* = 1.9 Hz, 1H), 7.34 (d, *J* = 1.9 Hz, 1H), 6.55 (s, 1H), 5.73 (s, 1H), 4.54 - 4.45 (m, 1H), 4.04 - 3.96 (m, 1H), 3.77 - 3.65 (m, 2H), 3.29 - 3.21 (m, 2H), 2.94 (s, 3H), 2.68 - 2.61 (m, 2H), 2.45 - 2.37 (m, 4H), 2.08 - 2.03 (m, 3H), 1.41 (d, *J* = 7.0 Hz, 6H), 1.27 (t, *J =* 7.6 Hz, 3H). MS (ESI) *m*/*z*: 486 [M+H]⁺.** |
| **III-30** | 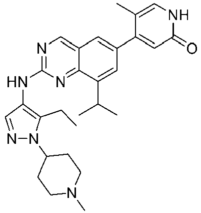 | **¹H NMR (600 MHz, Methanol-*d*₄) δ 7.66 (d, *J*= 14.7 Hz, 1H), 7.48 (s, 1H), 7.46 (d, *J* = 1.9 Hz, 1H), 7.33 (t, *J* = 1.6 Hz, 1H), 6.55 (s, 1H), 5.73 (s, 1H), 4.54 - 4.45 (m, 1H), 4.04 - 3.93 (m, 1H), 3.73 - 3.67 (m, 2H), 3.29 - 3.21 (m, 2H), 2.96 (s, 3H), 2.81 (q, *J* = 7.6 Hz, 2H), 2.44 - 2.32 (m, 2H), 2.25 - 2.18 (m, 2H), 2.06 (s, 3H), 1.41 (d, *J =* 7.0 Hz, 3H), 1.38 (d, *J* = 6.8 Hz, 3H), 1.28 - 1.25 (m, 3H). MS (ESI) *m*/*z*: 486 [M+H]⁺.** |
| **III-31** | 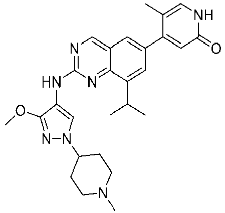 | **¹H NMR (600 MHz, Methanol-*d*₄) δ 9.13 (s, 1H), 8.27 (s, 1H), 7.67 (d, *J* = 2.0 Hz, 1H), 7.61 (d, *J* = 2.0 Hz, 1H), 7.39 - 7.36 (m, 1H), 6.51 (s, 1H), 4.16 - 4.08 (m, 1H), 4.05 - 3.99 (m, 1H), 3.97 (s, 3H), 3.26 - 3.18 (m, 2H), 2.64 - 2.57 (m, 2H), 2.54 (s, 3H), 2.28 - 2.21 (m, 2H), 2.19 - 2.12 (m, 2H), 2.06 (s, 3H), 1.43 (d, *J =* 7.0 Hz, 6H). MS (ESI) *m*/*z*: 488 [M+H]⁺.** |
| **III-32** | 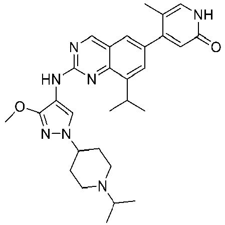 | **¹H NMR (600 MHz, Methanol-*d*₄) δ 7.80 (s, 1H), 7.44 (d, *J* = 2.0 Hz, 1H), 7.32 (d, *J* = 1.9 Hz, 1H), 6.59 (s, 1H), 6.49 (s, 1H), 5.77 (s, 1H), 4.42 - 4.32 (m, 2H), 3.95 (s, 3H), 3.66 - 3.57 (m, 4H), 3.28 - 3.22 (m, 1H), 2.46 - 2.33 (m, 4H), 2.09 (d, *J* = 1.0 Hz, 3H), 1.44 - 1.41 (m, 12H). MS (ESI) *m*/*z*: 516 [M+H]⁺.** |
| **III-33A&II I-33B** | 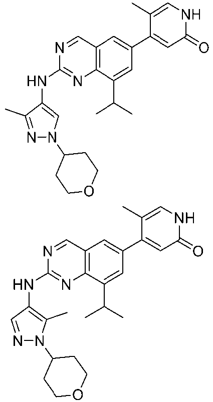 | **MS (ESI) *m*/*z*: 459 [M+H]⁺.** |
| **III-34** | 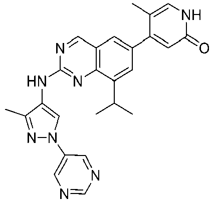 | **MS (ESI) *m*/*z*: 453 [M+H]⁺.** |
| **III-35** | 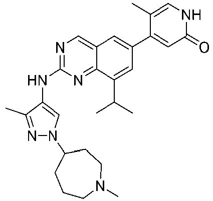 | **¹H NMR (600 MHz, Methanol-*d*₄) δ 7.89 (s, 1H), 7.48 (t, *J* = 0.9 Hz, 1H), 7.46 (d, *J* = 1.9 Hz, 1H), 7.34 (d, *J* = 1.9 Hz, 1H), 6.55 (s, 1H), 5.74 (s, 1H), 4.64 - 4.56 (m, 1H), 4.04 - 3.97 (m, 1H), 3.69 - 3.55 (m, 4H), 3.37 - 3.33 (m, 1H), 3.27 - 3.22 (m, 1H), 2.96 (s, 3H), 2.52 - 2.35 (m, 2H), 2.25 (s, 3H), 2.22 - 2.17 (m, 1H), 2.06 (s, 3H), 1.99 - 1.91 (m, 1H), 1.41 (d, *J* = 6.9 Hz, 6H). MS (ESI) *m*/*z*: 486 [M+H]⁺.** |
| **III-36** | 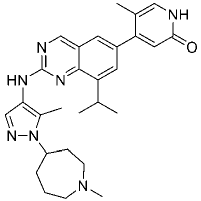 | **¹H NMR (600 MHz, Methanol-*d*₄) δ 8.16 - 8.00 (m, 1H), 7.48 (s, 1H), 7.46 (d, *J* = 1.9 Hz, 1H), 7.33 (d, *J* = 1.9 Hz, 1H), 6.54 (s, 1H), 5.73 (s, 1H), 4.78 - 4.70 (m, 1H), 4.02 - 3.94 (m, 1H), 3.66 - 3.55 (m, 4H), 3.41 - 3.33 (m, 1H), 3.30 - 3.21 (m, 1H), 2.97 (s, 3H), 2.62 - 2.43 (m, 2H), 2.33 (s, 3H), 2.29 - 2.23 (m, 2H), 2.06 (d, *J* = 1.0 Hz, 3H), 1.38 (d, *J* = 6.8 Hz, 6H) MS (ESI) *m*/*z*: 486 [M+H]⁺.** |
| **III-37A&II I-37B** | 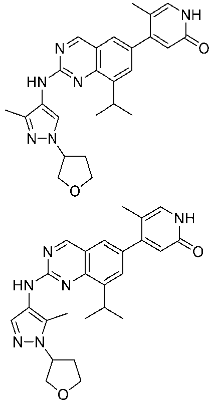 | **MS (ESI) *m*/*z*: 445 [M+H]⁺.** |
| **III-38** | 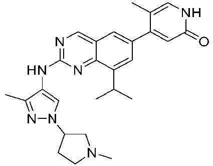 | **¹H NMR (600 MHz, Methanol-*d*₄) δ 7.53 (s, 1H), 7.46 (d, *J* = 1.9 Hz, 1H), 7.34 (d, *J* = 1.9 Hz, 1H), 6.65 (s, 1H), 6.55 (s, 1H), 5.75 (s, 1H), 4.09 - 3.97 (m, 2H), 3.65 - 3.52 (m, 2H), 3.18 - 3.11 (m, 2H), 3.06 (s, 3H), 2.56 - 2.38 (m, 2H), 2.33 (s, 3H), 2.11 (d, *J* = 1.0 Hz, 3H), 1.41 (d, *J* = 6.9 Hz, 6H). MS (ESI) *m*/*z*: 458 [M+H]⁺.** |
| **III-39** | 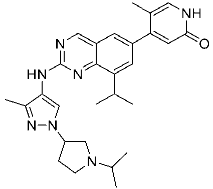 | **¹H NMR (600 MHz, Methanol-*d*₄) δ 7.73 (d, *J=* 2.0 Hz, 1H), 7.51 (s, 1H), 7.47 (d, *J* = 1.9 Hz, 1H), 7.34 (d, *J* = 1.9 Hz, 1H), 6.63 (s, 1H), 5.75 (s, 1H), 5.29 - 5.13 (m, 1H), 4.18 - 3.89 (m, 4H), 3.82 - 3.40 (m, 2H), 2.57 - 2.44 (m, 2H), 2.33 (s, 3H), 2.11 (s, 3H), 1.45 (d, *J* = 6.6 Hz, 6H), 1.41 (d, *J*= 6.9 Hz, 6H). MS (ESI) *m*/*z*: 486 [M+H]⁺.** |
| **III-40** | 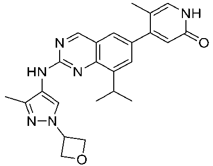 | **¹H NMR (400 MHz, DMSO-*d*₆) δ 11.54 (s, 1H), 9.42 (s, 1H), 9.27 (s, 1H), 8.47 (s, 1H), 7.75 (d, *J* = 2.0 Hz, 1H), 7.61 (d, *J* = 2.0 Hz, 1H), 7.33 (s, 1H), 6.30 (s, 1H), 5.53 - 5.49 (m, 1H), 4.97 (t, *J* = 7.0 Hz, 2H), 4.87 (t, *J =* 6.4 Hz, 2H), 4.01 - 3.85 (m, 1H), 2.29 (s, 3H), 1.97 (s, 3H), 1.37 (d, *J* = 7.2 Hz, 6H). MS (ESI) *m*/*z:* 431 [M+H]⁺.** |
| **III-41** | 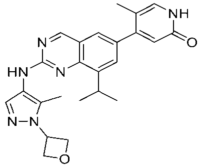 | **¹H NMR (400 MHz, DMSO-*d*₆) δ 11.56 (s, 1H), 9.26 - 9.23 (m, 2H), 8.22 (s, 1H), 7.72 (s, 1H), 7.59 (s, 1H), 7.32 (s, 1H), 6.30 (s, 1H), 5.60 - 5.57 (m, 1H), 4.97 (t, *J =* 6.0 Hz, 2H), 4.90 (t, *J =* 6.8 Hz, 2H), 4.03 - 3.89 (m, 1H), 2.25 (s, 3H), 1.96 (s, 3H), 1.32 (d, *J* = 6.8 Hz, 6H). MS (ESI) *m*/*z*: 431 [M+H]⁺.** |
| **III-42** | 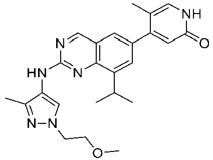 | **¹H NMR (400 MHz, DMSO-*d*₆) δ 11.35 (s, 1H), 9.28 (s, 1H), 9.25 (s, 1H), 8.25 (s, 1H), 7.74 (d, *J* = 2.0 Hz, 1H), 7.60 (d, *J* = 1.6 Hz, 1H), 7.32 (s, 1H), 6.29 (s, 1H), 4.19 (t, *J* = 5.2 Hz, 2H), 4.02 - 3.89 (m, 1H), 3.68 (t, *J* = 5.2 Hz, 2H), 3.27 (s, 3H), 2.23 (s, 3H), 1.96 (s, 3H), 1.35 (d, *J* = 7.2 Hz, 6H). MS (ESI) *m*/*z:* 433 [M+H]⁺.** |
| **III-43** | 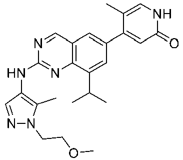 | **¹H NMR (400 MHz, DMSO-*d*₆) δ 11.43 (s, 1H), 9.22 (s, 1H), 9.18 (s, 1H), 7.99 (s, 1H), 7.71 (d, *J* = 2.0 Hz, 1H), 7.58 (d, *J* = 1.6 Hz, 1H), 7.32 (s, 1H), 6.28 (s, 1H), 4.19 (t, *J* = 5.4 Hz, 2H), 4.03 - 3.82 (m, 1H), 3.67 (t, *J* = 5.4 Hz, 2H), 3.24 (s, 3H), 2.28 (s, 3H), 1.96 (s, 3H), 1.30 (d, *J* = 6.8 Hz, 6H). MS (ESI) *m*/*z:* 433 [M+H]⁺.** |
| **III-44** | 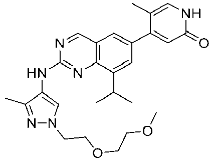 | **¹H NMR (400 MHz, MeOD) δ 9.15 (s, 1H), 8.29 (s, 1H), 7.67 (d, *J =* 1.6 Hz, 1H), 7.61 (d, *J =* 1.6 Hz, 1H), 7.38 (s, 1H), 6.52 (s, 1H), 4.27 (t, *J* = 5.4 Hz, 2H), 4.09 - 4.01 (m, 1H), 3.87 (t, *J* = 5.2 Hz, 2H), 3.60 (dd, *J =* 6.4, 2.8 Hz, 2H), 3.51 (dd, *J =* 6.4, 2.8 Hz, 2H), 3.29 (s, 3H), 2.31 (s, 3H), 2.07 (s, 3H), 1.41 (d, *J =* 6.8 Hz, 6H). MS (ESI) *m*/*z*: 477 [M+H]⁺.** |
| **III-45** | 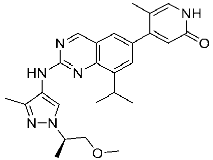 | **¹H NMR (400 MHz, MeOD) δ 9.15 (s, 1H), 8.36 (s, 1H), 7.68 (d, *J =* 2.0 Hz, 1H), 7.62 (d, *J* = 2.0 Hz, 1H), 7.37 (s, 1H), 6.52 (s, 1H), 4.50 - 4.45 (m, 1H), 4.09 - 4.02 (m, 1H), 3.73 - 3.62 (m, 2H), 3.33 (s, 3H), 2.32 (s, 3H), 2.06 (s, 3H), 1.54 (d, *J* = 6.8 Hz, 3H), 1.43 (dd, *J* = 6.8, 4.0 Hz, 6H). MS (ESI) *m*/*z*: 447 [M+H]⁺.** |
| **III-46** | 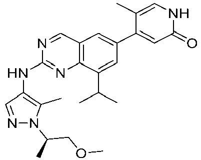 | **¹H NMR (400 MHz, MeOD) δ 9.13 (s, 1H), 8.04 (s, 1H), 7.66 (d, *J =* 2.0 Hz, 1H), 7.59 (d, *J =* 2.0 Hz, 1H), 7.37 (s, 1H), 6.51 (s, 1H), 4.62 - 4.57 (m, 1H), 4.05 - 3.98 (m, 1H), 3.76 - 3.71 (m, 1H), 3.66 - 3.63 (m, 1H), 3.28 (s, 3H), 2.33 (s, 3H), 2.06 (s, 3H), 1.47 (d, *J* = 6.8 Hz, 3H), 1.35 (d, *J* = 6.8 Hz, 6H). MS (ESI) *m*/*z*: 447 [M+H]⁺.** |
| **III-47A&II I-47B** | 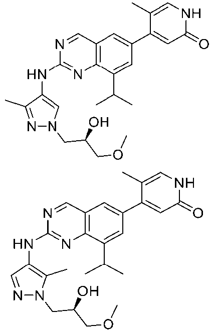 | **MS (ESI) m/z: 463 [M+H]⁺.** |
| **III-48** | 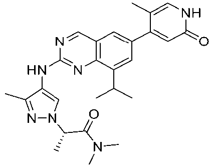 | **¹H NMR (400 MHz, DMSO-*d*₆) δ 11.60 (s, 1H), 9.40 (s, 1H), 9.26 (s, 1H), 8.37 (s, 1H), 7.75 (d, *J* = 2.0 Hz, 1H), 7.60 (d, *J =* 2.0 Hz, 1H), 7.33 (s, 1H), 6.30 (s, 1H), 5.46 (q, *J* = 6.8 Hz, 1H), 3.95 - 3.90 (m, 1H), 3.03 (s, 3H), 2.86 (s, 3H), 2.24 (s, 3H), 1.97 (s, 3H), 1.53 (d, *J* = 6.8 Hz, 3H), 1.37 - 1.33 (m, 6H). MS (ESI) *m*/*z*: 474 [M+H]⁺.** |
| **III-49** | 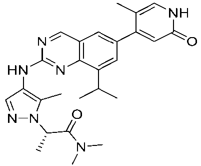 | **¹H NMR (400 MHz, DMSO-*d*₆) δ 11.62 (s, 1H), 9.40 (s, 1H), 9.26 (s, 1H), 8.37 (s, 1H), 7.75 (d, *J* = 2.0 Hz, 1H), 7.60 (d, *J =* 2.0 Hz, 1H), 7.34 (s, 1H), 6.31 (s, 1H), 5.46 (q, *J* = 6.8 Hz, 1H), 3.96 - 3.89 (m, 1H), 3.03 (s, 3H), 2.86 (s, 3H), 2.24 (s, 3H), 1.97 (s, 3H), 1.53 (d, *J* = 6.8 Hz, 3H), 1.37 - 1.33 (m, 6H). MS (ESI) *m*/*z*: 474 [M+H]⁺.** |
| **III-50A&II I-50B** | 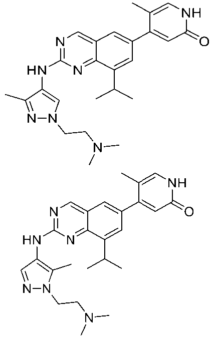 | **MS (ESI) m/z: 446 [M+H]⁺.** |
| **III-51** | 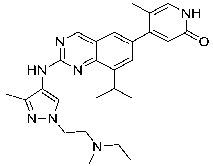 | **MS (ESI) m/z: 460 [M+H]⁺.** |
| **III-52** | 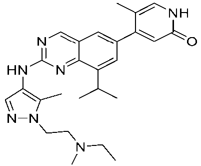 | **MS (ESI) m/z: 460 [M+H]⁺.** |
| **III-53A&II I-53B** | 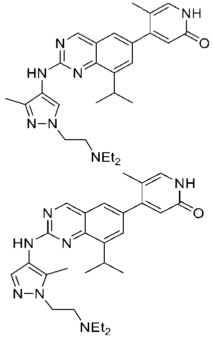 | **MS (ESI) *m*/*z*: 474 [M+H]⁺.** |
| **III-54** | 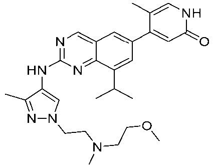 | **MS (ESI) *m*/*z*: 490 [M+H]⁺.** |
| **III-55** | 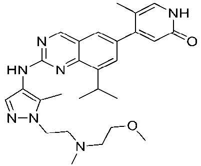 | **MS (ESI) *m*/*z*: 490 [M+H]⁺.** |
| **III-56** | 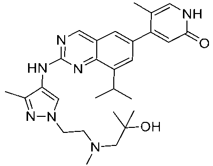 | **MS (ESI) *m*/*z*: 504 [M+H]⁺.** |
| **III-57** | 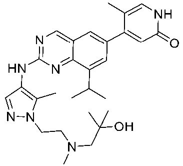 | **MS (ESI) *m*/*z*: 504 [M+H]⁺.** |
| **III-58A&II I-58B** | 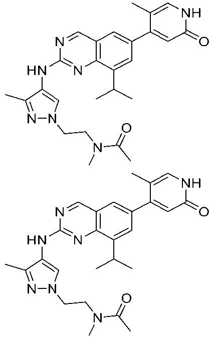 | **MS (ESI) *m*/*z*: 474 [M+H]⁺.** |
| **III-59** | 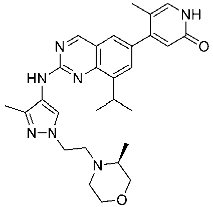 | **MS (ESI) *m*/*z*: 502 [M+H]⁺.** |
| **III-60** | 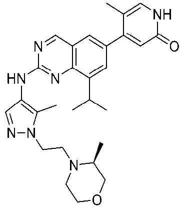 | **MS (ESI) *m*/*z*: 502 [M+H]⁺.** |
| **III-61** | 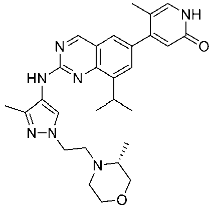 | **MS (ESI) *m*/*z*: 502 [M+H]⁺.** |
| **III-62** | 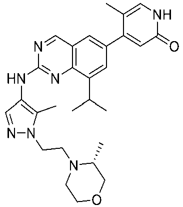 | **MS (ESI) *m*/*z*: 502 [M+H]⁺.** |
| **III-63A&II I-63B** | 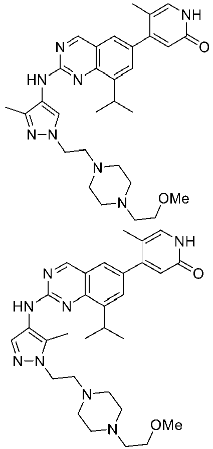 | **MS (ESI) *m*/*z*: 545 [M+H]⁺.** |
| **III-64** | 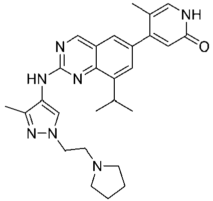 | **MS (ESI) *m*/*z*: 472 [M+H]⁺.** |
| **III-65** | 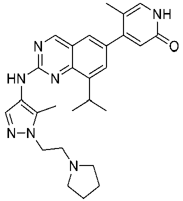 | **MS (ESI) *m*/*z*: 472 [M+H]⁺.** |
| **III-66A&II I-66B** | 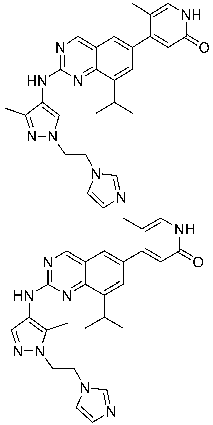 | **MS (ESI) *m*/*z*: 469 [M+H]⁺.** |
| **III-67** | 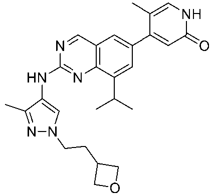 | **¹H NMR (600 MHz, Methanol-*d*₄) δ 9.28 (s, 1H), 8.89 (s, 1H), 7.77 (d, *J* = 2.0 Hz, 1H), 7.69 (d, *J* = 2.0 Hz, 1H), 7.48 - 7.41 (m, 1H), 6.55 (s, 1H), 4.78 - 4.72 (m, 1H), 4.69 - 4.63 (m, 1H), 4.40 (td, *J* = 12.2, 4.5 Hz, 1H), 4.14 - 3.97 (m, 2H), 3.76 (dd, *J* = 11.0, 5.3 Hz, 1H), 3.68 (dd, *J* = 11.1, 6.6 Hz, 1H), 2.57 (s, 3H), 2.53 - 2.47 (m, 1H), 2.35 - 2.30 (m, 1H), 2.07 (s, 3H), 2.05 - 2.01 (m, 1H), 1.41 (dd, *J* = 6.9, 2.3 Hz, 6H). MS (ESI) *m*/*z:* 459 [M+H]⁺.** |
| **III-68A&II I-68B** | 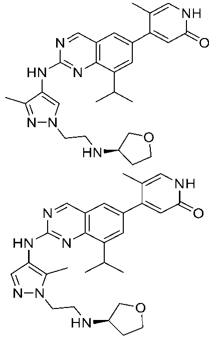 | **MS (ESI) *m*/*z*: 488 [M+H]⁺.** |
| **III-69** | 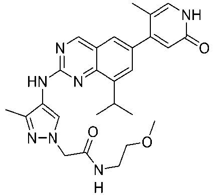 | **¹H NMR (600 MHz, DMSO-*d*₆) δ 9.38 - 9.30 (m, 1H), 9.26 (s, 1H), 8.29 - 8.21 (m, 1H), 8.18 (t, *J* = 5.6 Hz, 1H), 7.75 (d, *J* = 2.0 Hz, 1H), 7.59 (d, *J* = 2.0 Hz, 1H), 7.37 (s, 1H), 6.33 (s, 1H), 4.72 (s, 2H), 4.01 - 3.90 (m, 1H), 3.38 (t, *J* = 5.6 Hz, 2H), 3.28 (t, *J*= 5.6 Hz, 2H), 3.26 (s, 3H), 2.23 (s, 3H), 1.98 (s, 3H), 1.33 (d, *J =* 6.9 Hz, 6H). MS (ESI) *m*/*z*: 490 [M+H]⁺.** |
| **III-70** | 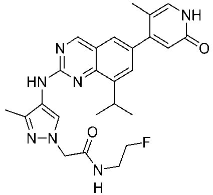 | **¹H NMR (600 MHz, DMSO-*d*₆) δ 11.56 (s, 1H), 9.34 (s, 1H), 9.26 (s, 1H), 8.39 (t, *J* = 5.6 Hz, 1H), 8.25 (s, 1H), 7.74 (d, *J* = 2.0 Hz, 1H), 7.59 (d, *J* = 2.0 Hz, 1H), 7.32 (s, 1H), 6.30 (s, 1H), 4.75 (s, 2H), 4.51 (t, *J =* 5.0 Hz, 1H), 4.43 (t, *J =* 5.0 Hz, 1H), 4.00 - 3.90 (m, 1H), 3.47 - 3.39 (m, 2H), 2.23 (s, 3H), 1.97 (s, 3H), 1.33 (d, *J* = 6.9 Hz, 6H). MS (ESI) *m*/*z*: 478 [M+H]⁺.** |
| **III-71** | 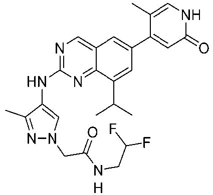 | **MS (ESI) *m*/*z*: 496 [M+H]⁺.** |
| **III-72** | 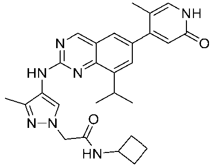 | **¹H NMR (600 MHz, DMSO-*d*₆) δ 11.64 - 11.51 (m, 1H), 9.41 - 9.28 (m, 1H), 9.26 (s, 1H), 8.42 (d, *J =* 7.8 Hz, 1H), 8.29 - 8.16 (m, 1H), 7.74 (d, *J* = 2.0 Hz, 1H), 7.59 (d, *J* = 2.0 Hz, 1H), 7.32 (s, 1H), 6.30 (s, 1H), 4.66 (s, 2H), 4.26 - 4.17 (m, 1H), 3.99 - 3.88 (m, 1H), 2.22 (s, 3H), 2.18 (qd, *J* = 7.7, 3.8 Hz, 2H), 1.97 (s, 3H), 1.96 - 1.87 (m, 2H), 1.69 - 1.62 (m, 2H), 1.33 (d, *J* = 6.9 Hz, 6H). MS (ESI) *m*/*z*: 486 [M+H]⁺.** |
| **III-73** | 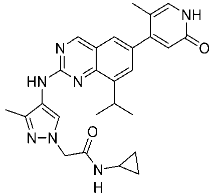 | **¹H NMR (600 MHz, DMSO-*d*₆) δ 11.69 - 11.45 (m, 1H), 9.40 - 9.29 (m, 1H), 9.26 (s, 1H), 8.27 (d, *J* = 4.3 Hz, 1H), 8.26 - 8.15 (m, 1H), 7.74 (d, *J = 2.0* Hz, 1H), 7.59 (d, *J* = 2.0 Hz, 1H), 7.33 (s, 1H), 6.30 (s, 1H), 4.64 (s, 2H), 4.01 - 3.89 (m, 1H), 2.72 - 2.64 (m, 1H), 2.23 (s, 3H), 1.97 (d, *J* = 1.0 Hz, 3H), 1.34 (d, *J* = 6.9 Hz, 6H), 0.69 - 0.62 (m, 2H), 0.48 - 0.41 (m, 2H). MS (ESI) *m*/*z*: 472 [M+H]⁺.** |
| **III-74** | 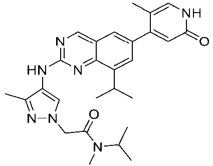 | **¹H NMR (600 MHz, DMSO-*d*₆) δ 9.36 - 9.30 (m, 1H), 9.26 (s, 1H), 8.24 - 8.12 (m, 1H), 7.75 (d, *J* = 2.0 Hz, 1H), 7.59 (s, 1H), 7.37 (s, 1H), 6.34 (s, 1H), 5.08 - 4.94 (m, 2H), 4.68 - 4.59 (m, 1H), 3.98 - 3.88 (m, 1H), 2.95 - 2.67 (m, 3H), 2.23 (s, 3H), 1.98 (s, 3H), 1.32 (d, *J =* 6.9 Hz, 6H), 1.17 - 1.03 (m, 6H). MS (ESI) *m*/*z*: 488 [M+H]⁺.** |
| **III-75** | 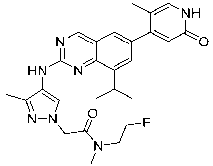 | **¹H NMR (600 MHz, DMSO-*d*₆) δ 9.40 - 9.30 (m, 1H), 9.26 (s, 1H), 8.25 - 8.13 (m, 1H), 7.75 (d, *J* = 1.9 Hz, 1H), 7.60 (d, *J* = 2.0 Hz, 1H), 7.41 (s, 1H), 6.37 (s, 1H), 5.12 - 4.96 (m, 2H), 4.71 - 4.49 (m, 2H), 3.96 - 3.90 (m, 1H), 3.81 - 3.73 (m, 2H), 3.14 - 2.83 (m, 3H), 2.23 (s, 3H), 1.99 (s, 3H), 1.32 (d, *J* = 6.9 Hz, 6H). MS (ESI) *m*/*z*: 492 [M+H]⁺.** |
| **III-76** | 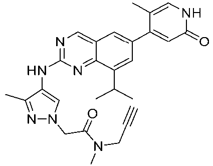 | **¹H NMR (600 MHz, DMSO-*d*₆) δ 9.45 - 9.29 (m, 1H), 9.26 (s, 1H), 7.75 (d, *J* = 2.1 Hz, 1H), 7.60 (s, 1H), 7.40 (s, 1H), 6.37 (s, 1H), 5.08 (s, 2H), 4.17 (d, *J* = 2.5 Hz, 2H), 3.98 - 3.90 (m, 1H), 3.25 - 3.21 (m, 1H), 3.13 - 2.85 (m, 3H), 2.23 (s, 3H), 1.99 (s, 3H), 1.32 (d, *J* = 6.9 Hz, 6H). MS (ESI) *m*/*z*: 484 [M+H]⁺.** |
| **III-77** | 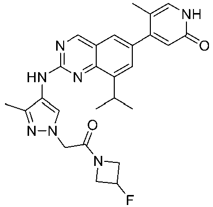 | **MS (ESI) *m*/*z*: 490 [M+H]⁺.** |
| **III-78** | 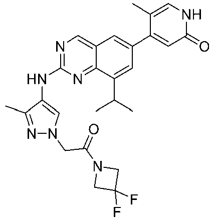 | **MS (ESI) *m*/*z*: 508 [M+H]⁺.** |
| **III-79** | 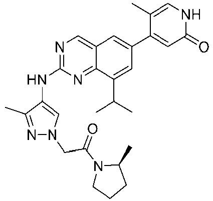 | **MS (ESI) *m*/*z*: 500 [M+H]⁺.** |
| **III-80** | 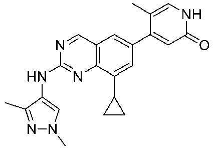 | **¹H NMR (400 MHz, DMSO-*d*₆) δ 11.54 (s, 1H), 9.28 (s, 1H), 9.23 (s, 1H), 8.20 (s, 1H), 7.67 (d, *J* = 2.0 Hz, 1H), 7.30 (s, 1H), 7.21 (s, 1H), 6.25 (s, 1H), 3.77 (s, 3H), 2.85 -2.70 (m, 1H), 2.21 (s, 3H), 1.92 (s, 3H), 1.12 - 1.07 (m, 2H), 0.86 - 0.82 (m, 2H). MS (ESI) *m*/*z*: 387 [M+H]⁺.** |
| **III-81** | 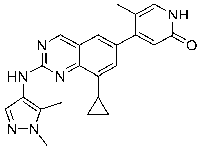 | **MS (ESI) *m*/*z*: 387[M+H]⁺.** |
| **III-82** | 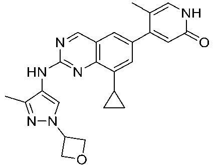 | **¹H NMR (400 MHz, DMSO-*d*₆) δ 11.49 (s, 1H), 9.36 (s, 1H), 9.26 (s, 1H), 8.48 (s, 1H), 7.69 (d, *J* = 2.0 Hz, 1H), 7.29 (s, 1H), 7.27 - 7.21 (m, 1H), 6.25 (s, 1H), 5.54 - 5.47 (m, 1H), 4.95 - 4.91 (m, 2H), 4.88 - 4.84 (m, 2H), 2.85 - 2.71 (m, 1H), 2.29 (s, 3H), 1.92 (s, 3H), 1.12 - 1.08 (m, 2H), 0.87 - 0.83 (m, 2H). MS (ESI) *m*/*z:* 429 [M+H]⁺.** |
| **III-83** | 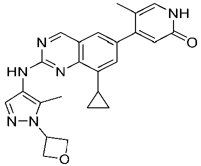 | **MS (ESI) *m*/*z*: 429 [M+H]⁺.** |
| **III-84** | 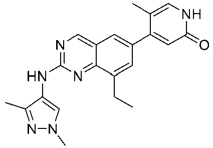 | **MS (ESI) *m*/*z:* 375 [M+H]⁺.** |
| **III-85** | 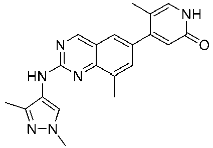 | **MS (ESI) *m*/*z*: 361 [M+H]⁺.** |
| **III-86** | 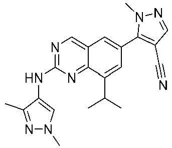 | **MS (ESI) *m*/*z*: 387 [M+H]⁺.** |
| **III-87** | 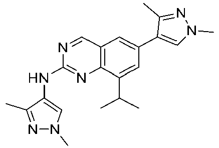 | **¹H NMR (600 MHz, DMSO-*d*₆) δ 9.20 (s, 1H), 9.16 - 9.03 (m, 1H), 8.17 - 8.06 (m, 1H), 8.01 (s, 1H), 7.71 (d, *J* = 2.1 Hz, 1H), 7.70 (d, *J* = 2.1 Hz, 1H), 3.97 - 3.89 (m, 1H), 3.81 (s, 3H), 3.79 (s, 3H), 2.37 (s, 3H), 2.20 (s, 3H), 1.36 (d, *J* = 7.0 Hz, 6H). MS (ESI) *m*/*z*: 376 [M+H]⁺.** |
| **III-88** | 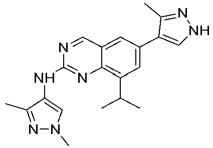 | **¹H NMR (600 MHz, DMSO-*d*₆) δ 12.76 - 12.58 (m, 1H), 9.19 (s, 1H), 9.11 - 8.95 (m, 1H), 8.18 - 7.97 (m, 1H), 7.79 - 7.69 (m, 3H), 3.99 - 3.89 (m, 1H), 3.78 (s, 3H), 2.47 - 2.38 (m, 3H), 2.20 (s, 3H), 1.36 (d, *J* = 6.9 Hz, 6H). MS (ESI) *m*/*z*: 362 [M+H]⁺.** |
| **III-89** | 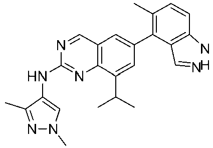 | **MS (ESI) *m*/*z*: 412 [M+H]⁺.** |
| **III-90** | 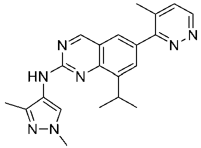 | **¹H NMR (600 MHz, DMSO-*d*₆) δ 9.39 - 9.32 (m, 1H), 9.30 (s, 1H), 9.14 (d, *J=* 5.2 Hz, 1H), 8.23 - 8.12 (m, 1H), 8.03 (d, *J* = 1.9 Hz, 1H), 7.92 (d, *J* = 2.0 Hz, 1H), 7.76 (d, *J=* 5.2 Hz, 1H), 4.03 - 3.95 (m, 1H), 3.81 (s, 3H), 2.45 (s, 3H), 2.22 (s, 3H), 1.37 (d, *J* = 6.9 Hz, 6H). MS (ESI) *m*/*z*: 374 [M+H]⁺.** |
| **III-91** | 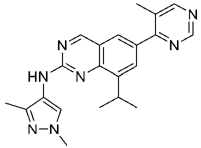 | **¹H NMR (600 MHz, DMSO-*d*₆) δ 9.41 - 9.34 (m, 1H), 9.31 (s, 1H), 9.13 (s, 1H), 8.77 (s, 1H), 8.19 - 8.13 (m, 1H), 8.12 (s, 1H), 7.99 (s, 1H), 4.02 - 3.93 (m, 1H), 3.80 (s, 3H), 2.47 (s, 3H), 2.22 (s, 3H), 1.36 (d, *J* = 6.9 Hz, 6H). MS (ESI) *m*/*z*: 374 [M+H]⁺.** |
| **III-92** | 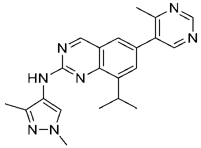 | **MS (ESI) *m*/*z*: 374 [M+H]⁺.** |
| **III-93** | 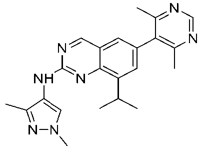 | **¹H NMR (600 MHz, DMSO-*d*₆) δ 9.29 - 9.15 (m, 2H), 8.91 (s, 1H), 8.22 - 8.04 (m, 1H), 7.66 (d, *J* = 2.0 Hz, 1H), 7.56 (d, *J* = 1.9 Hz, 1H), 4.03 - 3.90 (m, 1H), 3.79 (s, 3H), 2.25 (s, 6H), 2.21 (s, 3H), 1.34 (d, *J* = 6.9 Hz, 6H). MS (ESI) *m*/*z*: 388 [M+H]⁺.** |
| **III-94** | 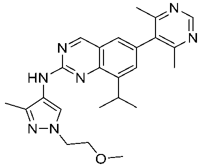 | **¹H NMR (600 MHz, Chloroform-d) δ 9.03 (s, 1H), 8.98 (s, 1H), 8.33 (s, 1H), 7.42 (d, *J* = 2.0 Hz, 1H), 7.39 (d, *J* = 1.9 Hz, 1H), 6.96 (s, 1H), 4.29 (t, *J* = 5.3 Hz, 2H), 4.08 (p, *J* = 7.0 Hz, 1H), 3.79 (t, *J=* 5.3 Hz, 2H), 3.39 (s, 3H), 2.35 (s, 3H), 2.32 (s, 6H), 1.41 (d, *J* = 6.9 Hz, 6H). MS (ESI) *m*/*z*: 432 [M+H]⁺.** |
| **III-95** | 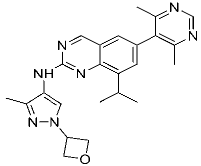 | **MS (ESI) *m*/*z*: 430 [M+H]⁺.** |
| **III-96** | 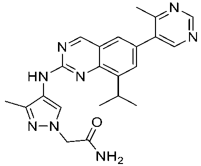 | **¹H NMR (600 MHz, DMSO-*d*₆) δ 9.45 - 9.34 (m, 1H), 9.32 (s, 1H), 9.13 (s, 1H), 8.78 (s, 1H), 8.38 - 8.24 (m, 1H), 7.89 (d, *J =* 2.0 Hz, 1H), 7.80 (d, *J* = 2.1 Hz, 1H), 7.46 (s, 1H), 7.31 (s, 1H), 4.74 (s, 2H), 4.09 - 3.98 (m, 1H), 2.58 (s, 3H), 2.29 (s, 3H), 1.41 (d, *J* = 6.9 Hz, 6H). MS (ESI) *m*/*z*: 417 [M+H]⁺.** |
| **III-97** | 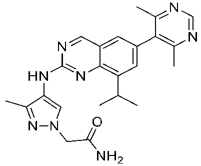 | **¹H NMR (600 MHz, DMSO-*d*₆) δ 9.32 - 9.25 (m, 1H), 9.21 (s, 1H), 8.91 (s, 1H), 8.28 - 8.19 (m, 1H), 7.66 (d, *J* = 2.0 Hz, 1H), 7.55 (d, *J* = 2.0 Hz, 1H), 7.39 (s, 1H), 7.25 (s, 1H), 4.68 (s, 2H), 4.04 - 3.91 (m, 1H), 2.25 (s, 6H), 2.23 (s, 3H), 1.32 (d, *J* = 6.9 Hz, 6H). MS (ESI) *m*/*z:* 431 [M+H]⁺.** |
| **III-98** | 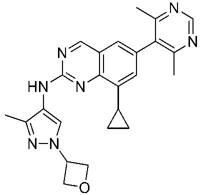 | **MS (ESI) *m*/*z*: 428 [M+H]⁺.** |
| **III-99** | 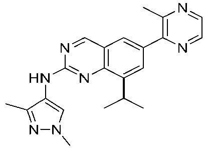 | **MS (ESI) *m*/*z*: 374 [M+H]⁺.** |
| **III-100** | 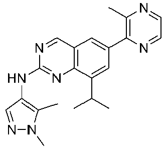 | **MS (ESI) *m*/*z*: 374 [M+H]⁺.** |
| **III-101** | 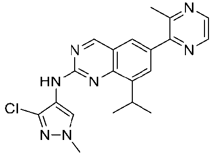 | **MS (ESI) m/z: 394 [M+H]⁺.** |
| **III-102** | 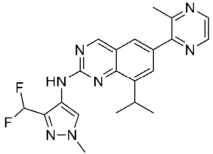 | **MS (ESI) *m*/*z*: 410 [M+H]⁺.** |
| **III-103** | 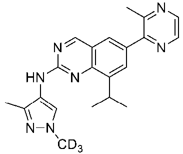 | **MS (ESI) *m*/*z*: 377 [M+H]⁺.** |
| **III-104** | 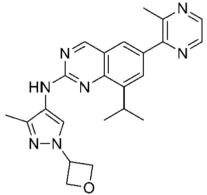 | **MS (ESI) *m*/*z*: 416 [M+H]⁺.** |
| **III-105** | 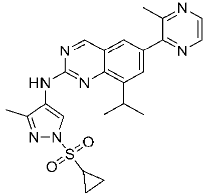 | **¹H NMR (600 MHz, Methanol-*d*₄) δ 9.20 (s, 1H), 8.87 (s, 1H), 8.49 (d, *J* = 2.6 Hz, 1H), 8.44 (d, *J* = 2.6 Hz, 1H), 7.91 (d, *J* = 2.0 Hz, 1H), 7.87 (d, *J* = 2.0 Hz, 1H), 4.01 - 3.94 (m, 1H), 2.80 - 2.72 (m, 1H), 2.60 (s, 3H), 2.38 (s, 3H), 1.55 - 1.46 (m, 2H), 1.37 (d, *J* = 7.0 Hz, 6H), 1.33 - 1.25 (m, 2H). MS (ESI) *m*/*z*: 464 [M+H]⁺.** |
| **III-106** | 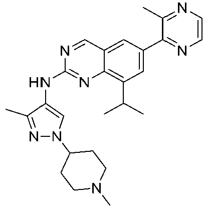 | **¹H NMR (600 MHz, DMSO-*d*₆) δ 9.59 - 9.48 (m, 1H), 9.31 (d, *J* = 4.7 Hz, 1H), 8.61 (d, *J* = 2.4 Hz, 1H), 8.55 (d, *J* = 2.5 Hz, 1H), 8.31 (s, 1H), 8.06 (d, *J* = 2.0 Hz, 1H), 7.95 (d, *J =* 2.1 Hz, 1H), 4.42 - 4.34 (m, 1H), 4.02 - 3.91 (m, 1H), 3.50 - 3.40 (m, 2H), 3.27 - 3.17 (m, 2H), 2.89 - 2.80 (m, 3H), 2.67 (s, 3H), 2.38 - 2.27 (m, 2H), 2.26 (s, 3H), 2.17 - 2.06 (m, 2H), 1.40 (d, *J* = 6.9 Hz, 6H). MS (ESI) *m*/*z*: 457 [M+H]⁺.** |
| **III-107** | 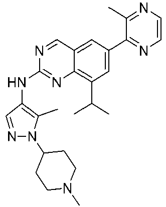 | **¹H NMR (600 MHz, DMSO-*d*₆) δ 9.58 - 9.45 (m, 1H), 9.28 (d, *J* = 4.2 Hz, 1H), 8.60 (d, *J* = 2.4 Hz, 1H), 8.55 (d, *J* = 2.5 Hz, 1H), 8.26 - 8.06 (m, 1H), 8.03 (d, *J* = 2.1 Hz, 1H), 7.93 (d, *J* = 2.1 Hz, 1H), 4.49 - 4.41 (m, 1H), 3.99 - 3.92 (m, 1H), 3.49 - 3.42 (m, 2H), 3.23 - 3.14 (m, 2H), 2.87 - 2.78 (m, 3H), 2.66 (s, 3H), 2.34 (s, 3H), 2.31 - 2.21 (m, 2H), 2.15 - 2.05 (m, 2H), 1.33 (d, *J* = 6.9 Hz, 6H). MS (ESI) *m*/*z*: 457 [M+H]⁺.** |
| **III-108** | 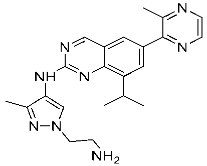 | **MS (ESI) *m*/*z*: 403 [M+H]⁺.** |
| **III-109** | 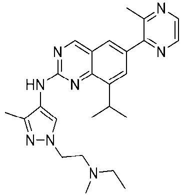 | **MS (ESI) *m*/*z*: 445 [M+H]⁺.** |
| **III-110** | 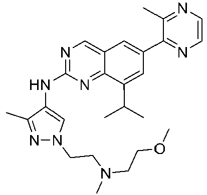 | **MS (ESI) *m*/*z*: 475 [M+H]⁺.** |
| **III-111** | 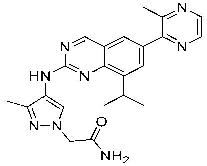 | **¹H NMR (600 MHz, DMSO-*d*₆) δ 9.41 - 9.25 (m, 2H), 8.61 (s, 1H), 8.55 (s, 1H), 8.32 - 8.17 (m, 1H), 8.05 (s, 1H), 7.93 (s, 1H), 7.41 (s, 1H), 7.29 - 7.24 (m, 1H), 4.69 (s, 2H), 4.05 - 3.92 (m, 1H), 2.67 (s, 3H), 2.24 (s, 3H), 1.35 (d, *J* = 6.9 Hz, 6H). MS (ESI) *m*/*z*: 417 [M+H]⁺.** |
| **III-112** | 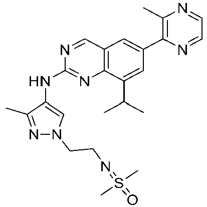 | **MS (ESI) *m*/*z*: 479 [M+H]⁺.** |
| **III-113** | 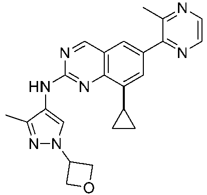 | **MS (ESI) *m*/*z*: 414 [M+H]⁺.** |
| **III-114** | 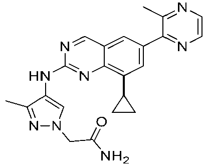 | **MS (ESI) *m*/*z*: 415 [M+H]⁺.** |
| **III-115** | 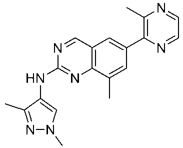 | **MS (ESI) *m*/*z*: 346 [M+H]⁺.** |
| **III-116** | 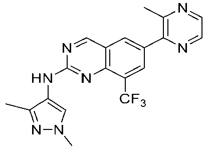 | **MS (ESI) *m*/*z*: 400 [M+H]⁺.** |
| **III-117** | 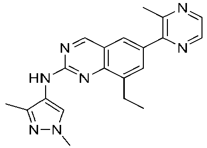 | **MS (ESI) *m*/*z*: 360 [M+H]⁺.** |
| **III-118** | 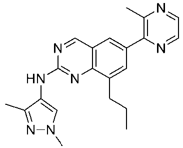 | **MS (ESI) *m*/*z*: 374 [M+H]⁺.** |
| **III-119** | 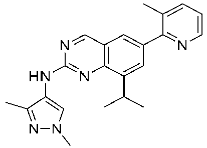 | **MS (ESI) *m*/*z*: 373 [M+H]⁺.** |
| **III-120** | 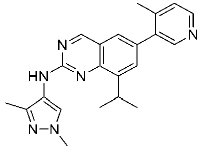 | **MS (ESI) *m*/*z*: 373 [M+H]⁺.** |
| **III-121** | 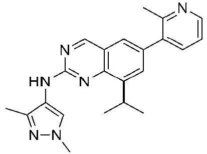 | **MS (ESI) *m*/*z*: 373 [M+H]⁺.** |
| **III-122** | 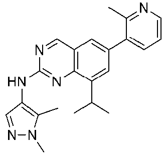 | **MS (ESI) *m*/*z*: 373 [M+H]⁺.** |
| **III-123** | 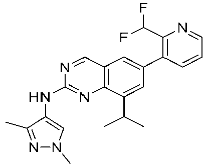 | **MS (ESI) *m*/*z*: 409 [M+H]⁺.** |
| **III-124** | 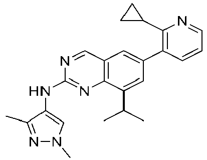 | **MS (ESI) *m*/*z*: 399 [M+H]⁺.** |
| **III-125** | 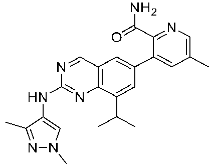 | **¹H NMR (600 MHz, DMSO-*d*₆) δ 9.20 (s, 1H), 9.14 (s, 1H), 8.43 (d, *J =* 1.9 Hz, 1H), 8.12 (s, 1H), 7.92 (d, *J* = 2.3 Hz, 1H), 7.78 - 7.74 (m, 1H), 7.72 (d, *J* = 2.0 Hz, 1H), 7.69 (d, *J* = 2.1 Hz, 1H), 7.45 (s, 1H), 3.95 - 3.89 (m, 1H), 3.79 (s, 3H), 2.41 (s, 3H), 2.21 (s, 3H), 1.33 (d, *J* = 6.9 Hz, 6H). MS (ESI) *m*/*z*: 416 [M+H]⁺.** |
| **III-126** | 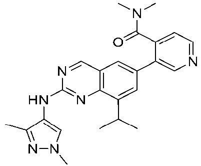 | **¹H NMR (600 MHz, DMSO-*d*₆) δ 9.40 - 9.31 (m, 1H), 9.27 (s, 1H), 8.89 (s, 1H), 8.73 (d, *J* = 5.2 Hz, 1H), 8.15 (s, 1H), 7.86 (d, *J* = 2.1 Hz, 1H), 7.65 (d, *J* = 2.1 Hz, 1H), 7.55 (d, *J =* 5.1 Hz, 1H), 4.09 - 4.01 (m, 1H), 2.83 (s, 3H), 2.56 (s, 3H), 2.54 (s, 3H), 2.22 (s, 3H), 1.31 (d, *J* = 6.9 Hz, 6H). MS (ESI) *m*/*z:* 430 [M+H]⁺.** |
| **III-127** | 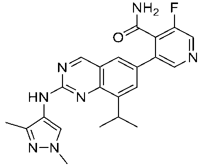 | **¹H NMR (600 MHz, DMSO-*d*₆) δ 9.32 (s, 1H), 9.25 (s, 1H), 8.70 (s, 1H), 8.65 (s, 1H), 8.28 (d, *J* = 2.1 Hz, 1H), 8.16 (s, 1H), 7.98 (d, *J* = 2.1 Hz, 1H), 7.89 (d, *J* = 2*.*1 Hz, 1H), 7.86 (d, *J* = 2.1 Hz, 1H), 4.13 - 4.07 (m, 1H), 3.80 (s, 3H), 2.22 (s, 3H), 1.34 (d, *J* = 6.9 Hz, 6H). MS (ESI) *m*/*z*: 420 [M+H]⁺.** |
| **III-128** | 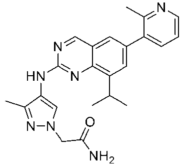 | **¹H NMR (600 MHz, DMSO-*d*₆) δ 9.46 - 9.32 (m, 1H), 9.29 (s, 1H), 8.81 (dd, *J =* 5.7, 1.6 Hz, 1H), 8.46 (dd, *J* = 7.9,1.5 Hz, 1H), 8.30 - 8.17 (m, 1H), 7.93 (dd, *J* = 7.9, 5.7 Hz, 1H), 7.86 (d, *J* = 2.1 Hz, 1H), 7.75 (d, *J* = 2.1 Hz, 1H), 7.42 (s, 1H), 7.25 (s, 1H), 4.69 (s, 2H), 4.03 - 3.96 (m, 1H), 2.70 (s, 3H), 2.24 (s, 3H), 1.35 (d, *J* = 6.9 Hz, 6H). MS (ESI) *m*/*z*: 416 [M+H]⁺.** |
| **III-129** | 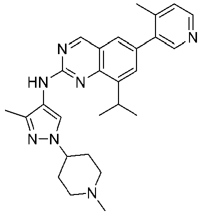 | **¹H NMR (600 MHz, DMSO-*d*₆) δ 9.32 (s, 1H), 9.25 (s, 1H), 8.49 (s, 1H), 8.46 (d, *J* = 5.0 Hz, 1H), 8.34 (s, 1H), 7.76 (d, *J* = 2.0 Hz, 1H), 7.67 (d, *J* = 2.0 Hz, 1H), 7.38 (d, *J =* 5.0 Hz, 1H), 4.04 - 3.99 (m, 1H), 3.98 - 3.94 (m, 1H), 2.89 - 2.83 (m, 2H), 2.33 (s, 3H), 2.24 (s, 3H), 2.22 (s, 3H), 2.12 - 2.01 (m, 4H), 1.95 - 1.85 (m, 2H), 1.37 (d, *J* = 6.9 Hz, 6H). MS (ESI) m/z: 456 [M+H]⁺.** |
| **III-130** | 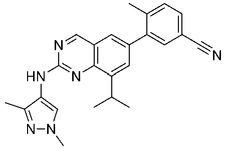 | **¹H NMR (600 MHz, Methanol-*d*₄) δ 9.16 (s, 1H), 8.25 - 8.15 (m, 1H), 7.82 (s, 1H), 7.63 (d, *J* = 1.8 Hz, 2H), 7.58 (d, *J* = 1.8 Hz, 1H), 7.43 (d, *J* = 1.9 Hz, 1H), 7.29 (d, *J* = 1.9 Hz, 1H), 4.05 - 3.99 (m, 1H), 3.88 (s, 3H), 2.33 (s, 3H), 2.24 (s, 3H), 1.40 (d, *J* = 7.0 Hz, 6H). MS (ESI) *m*/*z*: 397 [M+H]⁺.** |
| **III-131** | 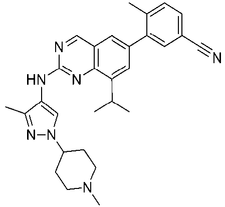 | **¹H NMR (600 MHz, DMSO-*d*₆) δ 9.33 (s, 1H), 9.24 (s, 1H), 8.34 (s, 1H), 7.81 (d, *J* = 1.8 Hz, 1H), 7.77 (dd, *J* = 7.9, 1.8 Hz, 1H), 7.75 (d, *J* = 2.0 Hz, 1H), 7.66 (d, *J* = 2.0 Hz, 1H), 7.57 (d, *J* = 8.0 Hz, 1H), 4.06 - 3.98 (m, 1H), 3.97 - 3.90 (m, 1H), 2.90 - 2.82 (m, 2H), 2.37 (s, 3H), 2.24 (s, 3H), 2.22 (s, 3H), 2.16 - 2.00 (m, 4H), 1.96 - 1.85 (m, 2H), 1.37 (d, *J* = 6.9 Hz, 6H). MS (ESI) m/z: 480[M+H]⁺.** |
| **III-132** | 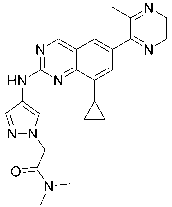 | **MS (ESI) *m*/*z*: 429 [M+H]⁺.** |
| **III-133** | 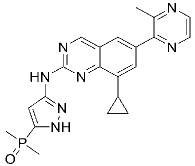 | **MS (ESI) *m*/*z*: 420 [M+H]⁺.** |

### Activity Test Example 1: Cell Antiproliferative Activity Assay

The activity of compounds against BRK kinase can be evaluated by their inhibition of the growth of the BRK kinase-stably transfected BaF3 cell line (BRK-BaF3) and wild-type BaF3 cells (WT-BaF3). The growth of the kinase-stably transfected cell line depends on its kinase activity, whereas the growth of wild-type BaF3 cells does not depend on this kinase activity; assessing the effect of compounds on the growth of WT-BaF3 cells can evaluate their broad-spectrum cytotoxicity. The larger the ratio of the half-maximal growth inhibitory concentration (IC₅₀) of a compound between WT-BaF3 and BRK-BaF3 cells, the better the selectivity of the compound.

### Cell Culture and Cell Viability Assay

BRK-BaF3, WT-BaF3, WEHI cell line, and breast cancer cell line T47D were cultured using RPMI 1640 Basal medium or DMEM (Dulbecco's Modified Eagle Medium) (Shanghai Peiyuan Biotechnology, L220KJ), supplemented with 10% FBS (Gibco, REF 10099-141C) and 1% P/S (Shanghai Peiyuan Biotechnology, S110JV), in an incubator (Thermo 3020) at 37°C with 5% CO₂. Cell viability was above 95%. Cells were passaged at a 3:1 ratio every 3-4 days. For WT-BaF3 cells, WEHI cells were first cultured, and their culture supernatant was filtered through a 0.22 µm filter. This supernatant was added at 10% to the regular culture medium to supplement IL-3 required for WT-BaF3 cell proliferation, which served as the culture medium for WT-BaF3. For testing, cells were first collected and counted, then prepared into a cell suspension at 100,000 cells/mL and plated at 100 µL per well. Drug concentrations started from 10 µM and were serially diluted 3:1. After incubation for 3 days, 20 µL of CCK-8 detection reagent (Targetmol C005) was added to each well. After incubation for 2-4 hours, OD450 was measured using a microplate reader (Varioskan LUX, Thermo Scientific). After subtracting the blank background, the curve was fitted and the IC₅₀ was calculated using XLfit software.

The following table exemplarily lists the IC₅₀ values of some compounds of the present invention against BRK-BaF3 and WT-BaF3 cells.

| **No.** | **BRK-BaF3 IC₅₀ (nM)** | **WT-BaF3 IC₅₀ (nM)** | **No.** | **BRK-BaF3 IC₅₀ (nM)** | **WT-BaF3 IC₅₀ (nM)** |
|---|---|---|---|---|---|
| **I-1** | +++++ | ++ | **II-7** | ++++ | ++ |
| **I-2** | +++++ | + | **II-8** | ++++ | + |
| **I-3** | +++++ | + | **II-9** | ++++ | + |
| **I-4** | +++++ | + | **II-10** | ++++ | + |
| **I-5** | +++++ | - | **II-11** | +++++ | ++ |
| **I-9** | +++++ | + | **II-12** | ++++ | + |
| **I-10** | ++++ | ++ | **II-13** | ++++ | + |
| **I-11** | +++++ | ++ | **II-14** | +++++ | + |
| **I-12** | +++++ | ++ | **II-15** | +++++ | + |
| **I-13** | +++++ | + | **II-16** | +++++ | ++ |
| **I-14** | +++++ | ++ | **II-17** | +++++ | ++ |
| **I-15** | ++++ | - | **II-18** | +++++ | + |
| **I-16** | +++++ | - | **II-21** | +++++ | ++ |
| **I-17** | +++++ | ++ | **II-22** | +++++ | ++ |
| **I-18** | +++++ | - | **II-30** | +++++ | ++ |
| **I-19** | ++++ | - | **II-31** | +++++ | + |
| **I-21** | +++++ | + | **II-32** | +++++ | ++ |
| **I-22** | +++++ | ++ | **II-33** | +++++ | +++ |
| **I-23** | ++++ | - | **II-34** | +++++ | + |
| **I-24** | +++++ | - | **II-35** | ++++ | + |
| **I-25** | +++++ | + | **II-42** | ++++ | + |
| **I-26** | +++++ | + | **II-43** | ++++ | + |
| **I-27** | +++++ | ++ | **II-44** | ++++ | + |
| **I-28** | ++++ | ++ | **II-45** | +++++ | + |
| **I-31** | +++++ | + | **II-50** | ++++ | + |
| **I-32** | +++++ | ++ | **II-53** | ++++ | + |
| **I-33** | +++++ | + | **II-54** | +++++ | + |
| **I-34** | +++++ | + | **II-56** | +++++ | + |
| **I-36** | +++++ | ++ | **II-59** | ++++ | + |
| **I-37** | +++++ | - | **II-62** | +++++ | +++ |
| **I-38** | +++++ | - | **II-64** | +++++ | ++ |
| **I-39** | ++++ | +++ | **II-67** | +++++ | +++ |
| **I-40** | +++++ | + | **II-68** | ++++ | + |
| **I-41** | +++++ | + | **II-69** | +++++ | + |
| **I-42** | +++++ | + | **II-70** | ++++ | ++ |
| **I-44** | +++++ | ++ | **II-71** | +++++ | ++ |
| **I-45** | +++++ | + | **II-72** | +++++ | ++ |
| **I-46** | +++++ | ++ | **II-73** | +++++ | ++ |
| **I-47** | +++++ | ++ | **II-74** | +++++ | ++ |
| **I-48** | +++++ | + | **II-75** | +++++ | + |
| **I-49** | +++++ | + | **II-76** | +++++ | + |
| **I-50** | +++++ | + | **II-77** | +++++ | +++ |
| **I-52** | +++++ | + | **III-1** | ++++ | - |
| **I-53** | +++++ | ++ | **III-2** | +++++ | + |
| **I-54** | +++++ | ++ | **III-3** | +++++ | - |
| **I-55** | +++++ | ++ | **III-4** | +++++ | ++ |
| **I-56** | +++++ | ++ | **III-5** | +++++ | - |
| **I-57** | +++++ | +++ | **III-6** | ++++ | - |
| **I-58** | ++++ | - | **III-7** | +++++ | - |
| **I-59** | +++++ | + | **III-8** | +++++ | - |
| **I-63** | ++++ | - | **III-9** | +++++ | ++ |
| **I-66** | ++++ | ++ | **III-10** | +++++ | - |
| **I-67** | +++++ | - | **III-11** | +++++ | - |
| **I-69** | ++++ | - | **III-12** | +++++ | - |
| **I-70** | +++++ | - | **III-13** | +++++ | - |
| **I-71** | +++++ | - | **III-14** | +++++ | ++ |
| **I-72** | +++++ | +++ | **III-15A&III-15B** | +++++ | - |
| **I-74** | +++++ | ++ | **III-16** | +++++ | - |
| **I-75** | +++++ | + | **III-17** | +++++ | - |
| **I-76** | +++++ | + | **III-18** | ++++ | - |
| **I-77** | +++++ | ++ | **III-25** | ++++ | - |
| **I-79** | +++++ | ++ | **III-26** | +++++ | - |
| **I-80** | +++++ | - | **III-27** | +++++ | - |
| **I-81** | +++++ | + | **III-28** | +++++ | - |
| **I-82** | ++++ | ++ | **III-29** | +++++ | - |
| **I-84** | +++++ | + | **III-30** | +++++ | - |
| **I-89** | ++++ | - | **III-31** | +++++ | - |
| **I-90** | +++++ | ++ | **III-32** | +++++ | - |
| **I-91** | ++++ | - | **III-33A&III-33B** | +++++ | - |
| **I-93** | ++++ | + | **III-34** | ++++ | - |
| **I-96** | ++++ | + | **III-35** | +++++ | - |
| **I-97** | +++++ | ++ | **III-36** | +++++ | - |
| **I-98** | +++++ | ++ | **III-37A&III-37B** | +++++ | - |
| **I-99** | +++++ | ++ | **III-38** | +++++ | - |
| **I-100** | +++++ | ++ | **III-39** | +++++ | - |
| **I-101** | +++++ | + | **III-40** | +++++ | - |
| **I-102** | +++++ | + | **III-41** | +++++ | + |
| **I-103** | +++++ | ++ | **III-42** | +++++ | - |
| **I-104** | +++++ | + | **III-43** | +++++ | - |
| **I-105** | +++++ | ++ | **III-44** | ++++ | - |
| **I-107** | +++++ | ++ | **III-45** | +++++ | - |
| **I-109** | +++++ | + | **III-46** | +++++ | - |
| **I-110** | +++++ | + | **III-47A&III-47B** | +++++ | - |
| **I-115** | ++++ | - | **III-48** | +++++ | - |
| **I-117** | ++++ | + | **III-49** | ++++ | - |
| **I-118** | ++++ | + | **III-50A&III-50B** | +++++ | - |
| **I-119** | +++++ | ++ | **III-51** | +++++ | - |
| **I-122** | +++++ | ++ | **III-52** | +++++ | - |
| **I-123** | ++++ | ++ | **III-53A&III-53B** | +++++ | - |
| **I-124** | ++++ | + | **III-54** | +++++ | - |
| **I-125** | +++++ | + | **III-55** | +++++ | - |
| **I-127** | +++++ | + | **III-56** | +++++ | - |
| **I-128** | +++++ | + | **III-57** | +++++ | - |
| **I-129** | ++++ | + | **III-59** | +++++ | - |
| **I-130** | ++++ | + | **III-60** | +++++ | - |
| **I-131** | +++++ | + | **III-61** | ++++ | - |
| **I-139** | +++++ | ++ | **III-62** | +++++ | - |
| **I-140** | +++++ | ++ | **III-64** | +++++ | - |
| **I-141** | ++++ | + | **III-65** | +++++ | - |
| **I-142** | +++++ | + | **III-68A&III-68B** | ++++ | - |
| **I-143** | ++++ | + | **III-70** | ++++ | - |
| **I-144** | +++++ | + | **III-71** | ++++ | - |
| **I-145** | ++++ | - | **III-72** | ++++ | - |
| **I-146** | +++++ | ++ | **III-73** | ++++ | - |
| **I-147** | +++++ | ++ | **III-74** | ++++ | - |
| **I-148** | +++++ | + | **III-76** | ++++ | - |
| **I-149** | ++++ | + | **III-80** | +++++ | - |
| **I-150** | +++++ | + | **III-81** | +++++ | - |
| **I-151** | ++++ | + | **III-82** | +++++ | |
| **I-152** | +++++ | + | **III-83** | +++++ | + |
| **I-153** | +++++ | + | **III-84** | ++++ | - |
| **I-155** | +++++ | ++ | **III-86** | +++++ | + |
| **I-156** | +++++ | - | **III-87** | +++++ | +++ |
| **I-158** | +++++ | - | **III-88** | +++++ | ++ |
| **I-15**9 | +++++ | - | **III-89** | +++++ | - |
| **I-160** | +++++ | +++ | **III-90** | +++++ | +++ |
| **I-161** | +++++ | - | **III-92** | +++++ | +++ |
| **I-162** | +++++ | ++ | **III-93** | +++++ | + |
| **I-163** | +++++ | - | **III-94** | +++++ | + |
| **I-166** | ++++ | - | **III-95** | ++++ | ++ |
| **I-169** | ++++ | - | **III-96** | ++++ | + |
| **I-170** | +++++ | + | **III-98** | ++++ | ++ |
| **I-171** | +++++ | - | **III-99** | +++++ | ++ |
| **I-172** | +++++ | - | **III-100** | +++++ | - |
| **I-173** | ++++ | - | **III-101** | +++++ | ++ |
| **I-174** | +++++ | - | **III-102** | +++++ | + |
| **I-175** | +++++ | - | **III-103** | +++++ | +++ |
| **I-180** | +++++ | - | **III-104** | +++++ | + |
| **I-1**8**1** | ++++ | + | **III-106** | +++++ | ++ |
| **I-182** | +++++ | - | **III-107** | +++++ | + |
| **I-183** | +++++ | - | **III-108** | +++++ | +++ |
| **I-184** | +++++ | - | **III-109** | **+++++** | + |
| **I-185** | ++++ | - | **III-110** | +++++ | + |
| **I-186** | ++++ | - | **III-111** | **+++++** | + |
| **I-187** | +++++ | ++ | **III-112** | ++++ | + |
| **I-188** | +++++ | ++ | **III-113** | +++++ | ++ |
| **I-189** | +++++ | ++ | **III-114** | +++++ | ++ |
| **I-190** | +++++ | ++ | **III-119** | +++++ | - |
| **I-191** | +++++ | ++ | **III-120** | +++++ | +++ |
| **I-193** | +++++ | +++ | **III-123** | +++++ | - |
| **II-1** | +++++ | + | **III-127** | ++++ | + |
| **II-2** | +++++ | + | **III-128** | +++++ | + |
| **II-3** | +++++ | + | **III-129** | +++++ | - |
| **II-4** | ++++ | + | **III-130** | +++++ | ++ |
| **II-5** | +++++ | + | **III-131** | +++++ | - |
| **II-6** | +++++ | + | **III-132** | +++++ | ++ |

| | | | | | |
|---|---|---|---|---|---|
| **Note: +++++ indicates IC₅₀ ≤ 20 nM; ++++ indicates 20 nM < IC₅₀ ≤ 100 nM; +++ indicates 100 nM < IC₅₀ ≤ 500 nM; ++ indicates 500 nM < IC₅₀ ≤ 2500 nM; + indicates IC₅₀ > 2500 nM; - indicates not tested.** | | | | | |

**As can be seen from the above table: (1) the compounds of the present invention have small half-maximal inhibitory concentrations (IC₅₀) against BRK-BaF3 cells, indicating excellent BRK kinase inhibitory activity; (2) the compounds of the present invention exhibit a large ratio of IC₅₀ values between WT-BaF3 and BRK-BaF3 cells, demonstrating excellent selectivity; (3) the compounds of the present invention have large IC₅₀ values against WT-BaF3 cells, indicating low broad-spectrum cytotoxicity.**

**In addition, the following table exemplarily lists the IC₅₀ values of some compounds of the present invention against T47D cells.**

| **No.** | **T47D IC₅₀ (nM)** | **No.** | **T47D IC₅₀ (nM)** |
|---|---|---|---|
| **I-14** | +++ | **II-64** | ++++ |
| **I-22** | +++ | **II-65** | ++++ |
| **I-27** | +++ | **II-66** | ++++ |
| **I-32** | ++++ | **II-67** | ++++ |
| **I-36** | ++++ | **II-69** | ++++ |
| **I-39** | ++++ | **II-70** | ++++ |
| **I-41** | +++ | **II-71** | ++++ |
| **I-46** | ++++ | **II-72** | ++++ |
| **I-48** | +++ | **II-74** | ++++ |
| **I-53** | ++++ | **II-77** | ++++ |
| **I-54** | +++ | **II-18** | +++ |
| **I-55** | ++++ | **II-22** | ++++ |
| **I-56** | ++++ | **II-33** | ++++ |
| **I-57** | +++ | **II-62** | +++ |
| **I-59** | ++++ | **III-2** | ++++ |
| **I-98** | +++ | **III-4** | +++ |
| **I-119** | +++ | **III-9** | ++++ |
| **I-125** | +++ | **III-14** | +++ |
| **I-140** | +++ | **III-87** | ++++ |
| **I-146** | +++ | **III-88** | +++ |
| **I-147** | +++ | **III-90** | ++++ |
| **I-160** | ++++ | **III-92** | ++++ |
| **I-162** | ++++ | **III-99** | ++++ |
| **I-187** | ++++ | **III-101** | +++ |
| **I-189** | +++ | **III-102** | +++ |
| **I-193** | ++++ | **III-103** | ++++ |
| **II-11** | ++++ | **III-120** | ++++ |
| **II-12** | +++ | **III-130** | ++++ |
| **II-16** | ++++ | **III-132** | ++++ |

| | | | |
|---|---|---|---|
| Note: ++++ represents IC₅₀ ≤ 500 nM; +++ represents 500 nM < IC₅₀ ≤ 1000 nM. | | | |

As can be seen from the above table, the compounds of the present invention have small IC₅₀ values against T47D cells, demonstrating excellent inhibitory activity against T47D cells.

Biological Activity Test Example 2: In Vitro Activity Test of Gastric Cancer PDX

Patient-derived xenograft (PDX) models are methods in which tumor tissue from cancer patients is transplanted into severely immunodeficient mice for culture and passaging, and used to test compound activity. The compounds of the present invention were tested using three PDX samples (GA0119, GA6208, and GA6871, all being gastric cancers with high BRK expression, all cultured in DMEM).

Preparation of PDX model tumor cell suspension: PDX model animals were housed in the animal facility, and tumor growth was observed weekly. When the PDX model tumor grew to an appropriate size, the tumor was removed and minced using sterile surgical instruments. Collagenase B digestion solution was added, and digestion was performed at 37°C for approximately 2 hours. Undigested tissue was removed by passing through a cell strainer. The strainer was washed with PBS 3-5 times, and the cell pellet was washed with PBS 4 times. Tumor cells were resuspended in culture medium. ACK lysis buffer was used to remove red blood cells when necessary.

Cell plating: The above cell suspension was taken and counted. The cell suspension concentration was adjusted to 5.75 × 10⁵ cells/mL by dilution with culture medium. 3.5 mL of the cell suspension and 6.5 mL of 1% methylcellulose solution were mixed thoroughly. 100 µL of the cell suspension was added to each well of a 96-well plate (including compound-treated cell plates and T0 control cell plates), followed by incubation overnight under conditions of 37°C, 5% CO₂, and 95% humidity.

Day 0 cell viability reading: The CellTiter-Glo^{®} reagent and T0 control cell culture plate were equilibrated at room temperature for 30 minutes. 100 µL of CellTiter-Glo^{®} reagent was added to each well. The plate was shaken on an orbital shaker for 20 minutes to ensure complete cell lysis. The cell culture plate was then equilibrated at room temperature for 10 minutes, and the chemiluminescence signal was read using an EnVision plate reader.

Day 0 compound treatment: The test compound (stock concentration 10 mM) was taken out and equilibrated at room temperature for use. The compound treatment cell plate was taken out. The test compound solution was added to the corresponding wells. The highest concentration for each compound was 30 µM, with 3-fold serial dilutions, 9 concentration points, each concentration point in triplicate (DMSO concentration normalized). The final concentration of DMSO in each cell well was 0.3%. The dosed 96-well plate was then placed in an incubator at 37°C, 5% CO₂, and 95% humidity for continued culture for 168 hours.

Day 7 cell viability reading: The CellTiter-Glo^{®} reagent and cell culture plate were equilibrated at room temperature for 30 minutes. 100 µL of CellTiter-Glo^{®} reagent was added to each well. The plate was shaken on an orbital shaker for 20 minutes to lyse the cells. The cell plate was then placed at room temperature for 10 minutes to stabilize the luminescence signal, and the luminescence value was read using an EnVision plate reader.

Data processing: Data were analyzed using GraphPad Prism 9.0 software. A nonlinear sigmoidal curve regression was used to fit the data and generate a dose-response curve, from which the IC₅₀ value was calculated. Cell survival rate (%) = (Lumₜₑₛₜ compound - Lum_{medium control}) / (Lum_{cell control -} Lum_{medium control}) × 100%. Lum represents the luminescence value.

Experimental results:

| **PDX** | **IC₅₀ (µM)** | | | | |
|---|---|---|---|---|---|
| | **I-21** | **I-81** | **I-109** | **II-69** | **cisplatin** |
| **GA0119** | **1.14** | **4.54** | **nd** | **8.01** | **1.38** |
| **GA6208** | **0.62** | **3.92** | **8.53** | **5.63** | **0.298** |
| **GA6871** | **2.13** | **7.13** | **16.96** | **9.28** | **4.28** |

| | | | | | |
|---|---|---|---|---|---|
| Note: nd indicates not tested. | | | | | |

As can be seen from the above table, the compounds of the present invention exhibit excellent in vitro inhibitory activity against gastric cancer PDX with high BRK expression.

### Biological Activity Test Example 3: In Vivo Efficacy Evaluation of Gastric Cancer PDX (GA6208)

Tumor tissue was collected from tumor-bearing mice of the gastric cancer xenograft model GA6208, cut into tumor pieces of 2-3 mm in diameter, and subcutaneously inoculated at the right front scapular region of Balb/c nude mice. When the mean tumor volume of the tumor-bearing mice reached approximately 150 mm³ (100-200 mm³), the mice were randomly divided into 4 groups of 8 mice each, and administered as follows: vehicle control (0.5% CMC-Na solution, oral gavage, twice daily), compound I-21 (100 mg/kg, oral gavage, twice daily), compound I-81 (100 mg/kg for the first 8 days, then 80 mg/kg for the subsequent 27 days, oral gavage, twice daily), and cisplatin (3 mg/kg, tail vein injection, once weekly), for a total of 35 days. The results are as follows:

| **Group** | **Compound** | **dose (mg/kg)** | **Day 35** | | |
|---|---|---|---|---|---|
| | | | **tumor volume (mean ± SEM)** | **TGI (%)** | **p value** |
| **1** | **vehicle** | **-** | **1412.91±173.07** | **-** | **-** |
| **2** | **I-21** | **100** | **782.88±71.02** | **44.59** | **0.0122** |
| **3** | **I-81** | **100/80** | **595.03±30.63** | **57.89** | **0.001** |
| **4** | **Cisplatin** | **3** | **1250.42±137.64** | **11.50** | **0.974** |

| | | | | | |
|---|---|---|---|---|---|
| Note: - indicates not applicable; TGI indicates tumor growth inhibition, calculated as: TGI (%) = [1 - (Ti - T0) / (Vi - V0)] × 100, where Ti is the mean tumor volume on day i after the start of treatment in the treated group, T0 is the mean tumor volume at the start of treatment in the treated group, V0 is the mean tumor volume at the start of treatment in the vehicle control group, and Vi is the mean tumor volume on day i after the start of treatment in the vehicle control group; comparison between treated groups and the vehicle control group was performed using one-way ANOVA followed by Dunnett's multiple comparison test. | | | | | |

As can be seen from the above table, the compounds of the present invention exhibit excellent in vivo efficacy against gastric cancer PDX with high BRK expression, superior to the chemotherapy drug cisplatin.

## Claims

1. A compound of formula O, or a stereoisomer thereof, a prodrug thereof, a pharmaceutically acceptable salt thereof, or a pharmaceutically acceptable solvate thereof, 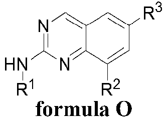 wherein:
R¹ is selected from the group consisting of 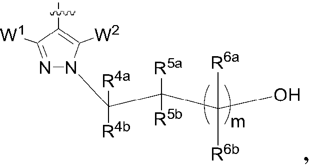 R¹ , 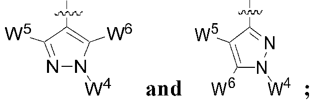
preferably, R¹ is selected from the group consisting of 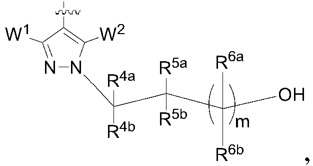 R^{1'} and 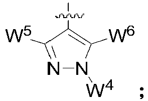
R^{1'} is selected from the group consisting of 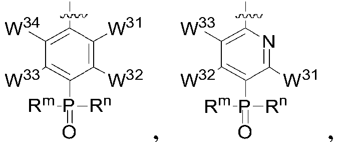 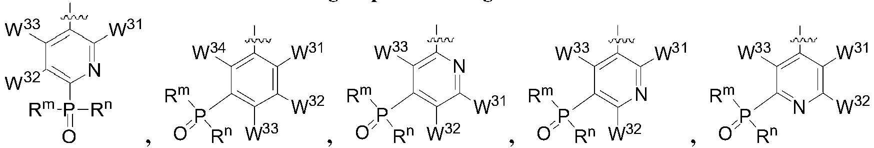 and 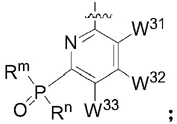
preferably, R^{1'} is selected from the group consisting of 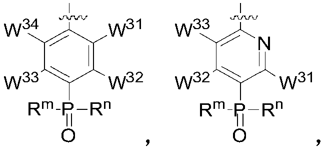 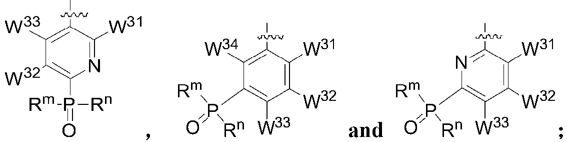
R^{m} and Rⁿ are each independently selected from the group consisting of C1-C6 alkyl;
preferably, R^{m} and Rⁿ are each independently selected from the group consisting of methyl and ethyl;
preferably, R^{1'} is selected from the group consisting of 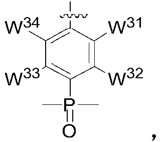 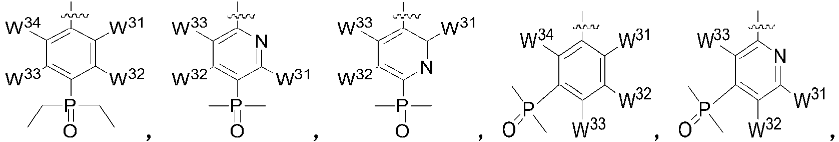 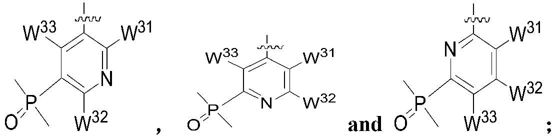
preferably, R^{1'} is selected from the group consisting of 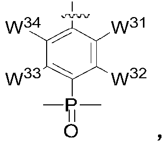 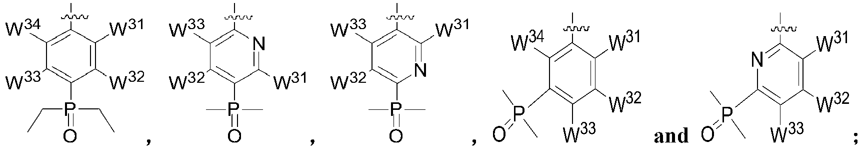
more preferably, R^{1'} is selected from the group consisting of 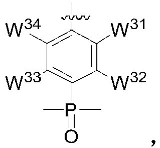 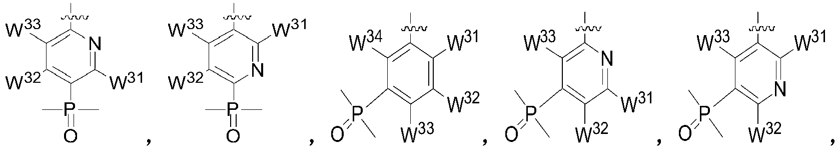 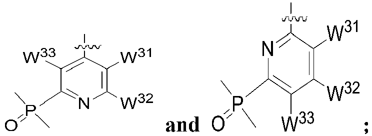
most preferably, R^{1'} is selected from the group consisting of 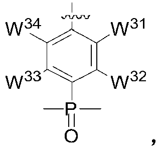 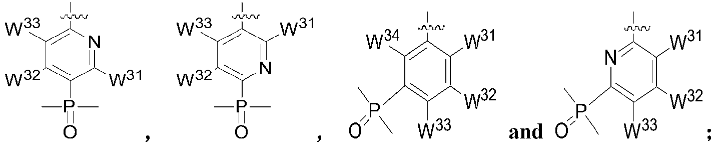
R^{4a} and R^{4b} are each independently selected from the group consisting of hydrogen and C1-C6 alkyl;
preferably, R^{4a} and R^{4b} are each independently selected from the group consisting of hydrogen and methyl;
R^{5a} and R^{5b} are each independently selected from the group consisting of hydrogen and C1-C6 alkyl;
preferably, R^{5a} and R^{5b} are each independently selected from the group consisting of hydrogen and methyl;
R^{6a} and R^{6b} are each independently selected from the group consisting of hydrogen and C1-C6 alkyl;
preferably, R^{6a} and R^{6b} are each independently selected from the group consisting of hydrogen and methyl;
m is selected from the group consisting of 0 and 1;
W¹ and W² are each independently selected from the group consisting of hydrogen, halogen, C1-C6 alkyl, and C1-C6 haloalkyl;
preferably, any one of W¹ and W² is selected from the group consisting of hydrogen, halogen, C1-C6 alkyl, and C1-C6 haloalkyl, and the other is hydrogen;
more preferably, any one of W¹ and W² is selected from the group consisting of hydrogen, chloro, methyl and difluoromethyl, and the other is hydrogen;
most preferably, 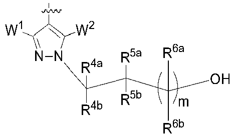 as a whole, is selected from the group consisting of: 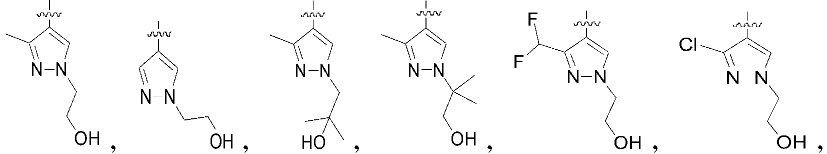 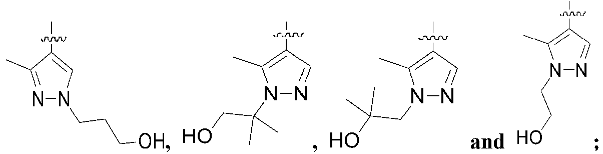
when W³¹, W³², W³³, and W³⁴ are simultaneously present, W³¹, W³², W³³, and W³⁴ are each independently selected from the group consisting of hydrogen, halogen, hydroxy, C1-C6 alkyl, hydroxy-substituted C1-C6 alkyl, C1-C6 alkoxy, and -NR^{x}R^{y};
preferably, when W³¹, W³², W³³, and W³⁴ are simultaneously present, W³¹, W³², W³³, and W³⁴ are each independently selected from the group consisting of hydrogen, halogen, C1-C6 alkyl, hydroxy-substituted C1-C6 alkyl, and C1-C6 alkoxy;
preferably, when W³¹, W³², W³³, and W³⁴ are simultaneously present, any one of W³¹, W³², W³³, and W³⁴ is selected from the group consisting of hydrogen, halogen, hydroxy, C1-C6 alkyl, C1-C6 alkoxy, hydroxy-substituted C1-C6 alkyl, and -NR^{x}R^{y}, and the remainder are hydrogen; or, any two of W³¹, W³², W³³, and W³⁴ are independently selected from the group consisting of hydrogen, halogen, hydroxy, C1-C6 alkyl, and C1-C6 alkoxy, and the remainder are hydrogen;
R^{X} and R^{Y} are each independently selected from the group consisting of hydrogen and C1-C6 alkyl; preferably, any one of R^{x} and R^{y} is selected from the group consisting of C1-C6 alkyl, and the other is hydrogen; more preferably, any one of R^{x} and R^{y} is methyl, and the other is hydrogen;
preferably, when W³¹, W³², W³³, and W³⁴ are simultaneously present, any one of W³¹, W³², W³³, and W³⁴ is selected from the group consisting of hydrogen, halogen, C1-C6 alkyl, C1-C6 alkoxy, and hydroxy-substituted C1-C6 alkyl, and the remainder are hydrogen; or, any two of W³¹, W³², W³³, and W³⁴ are independently selected from the group consisting of hydrogen, halogen, C1-C6 alkyl, and C1-C6 alkoxy, and the remainder are hydrogen;
more preferably, when W³¹, W³², W³³, and W³⁴ are simultaneously present, any one of W³¹, W³², W³³, and W³⁴ is selected from the group consisting of hydrogen, fluoro, hydroxy, methyl, ethyl, methoxy, ethoxy, 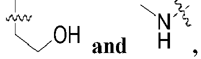 and the remainder are hydrogen; or, any two of W³¹, W³², W³³, and W³⁴ are independently selected from the group consisting of hydrogen, fluoro, chloro, hydroxy, methyl, and methoxy, and the remainder are hydrogen;
more preferably, when W³¹, W³², W³³, and W³⁴ are simultaneously present, any one of W³¹, W³², W³³, and W³⁴ is selected from the group consisting of hydrogen, fluoro, methyl, ethyl, methoxy and 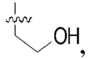 and the remainder are hydrogen; or, any two of W³¹, W³², W³³, and W³⁴ are independently selected from the group consisting of hydrogen, fluoro, chloro, methyl, and methoxy, and the remainder are hydrogen;
when W³¹, W³², and W³³ are simultaneously present but W³⁴ is absent, W³¹, W³², and W³³ are each independently selected from the group consisting of hydrogen, C1-C6 alkyl, and C1-C6 alkoxy;
preferably, when W³¹, W³², and W³³ are simultaneously present but W³⁴ is absent, any one of W³¹, W³², and W³³ is selected from the group consisting of hydrogen, C1-C6 alkyl, and C1-C6 alkoxy, and the remainder are hydrogen;
more preferably, when W³¹, W³², and W³³ are simultaneously present but W³⁴ is absent, any one of W³¹, W³², and W³³ is selected from the group consisting of hydrogen, methyl, and methoxy, and the remainder are hydrogen;
preferably, R^{1'} is selected from the group consisting of 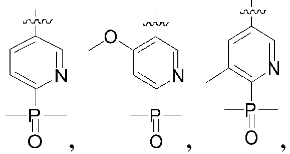 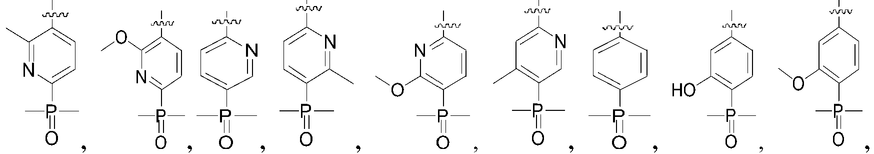 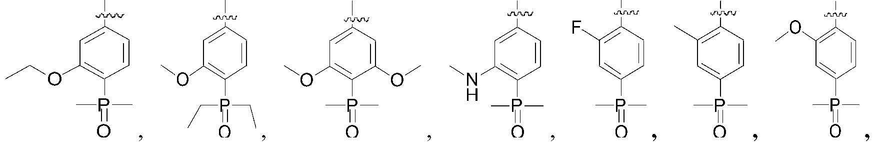 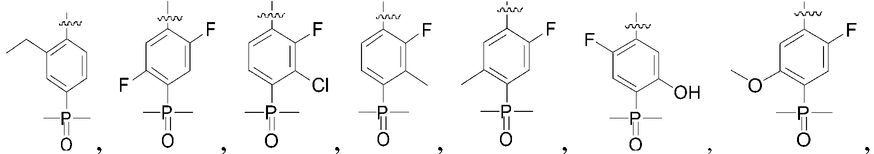 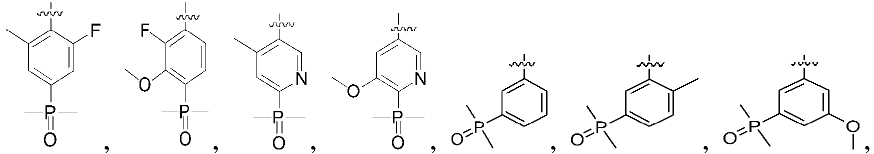 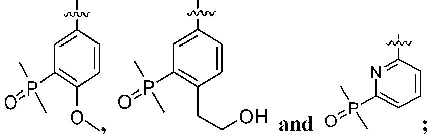
most preferably, R^{1'} is selected from the group consisting of 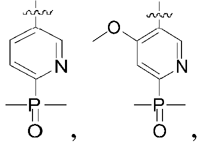 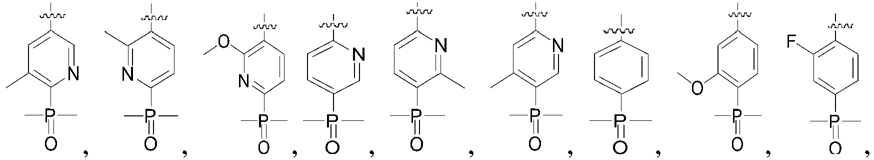 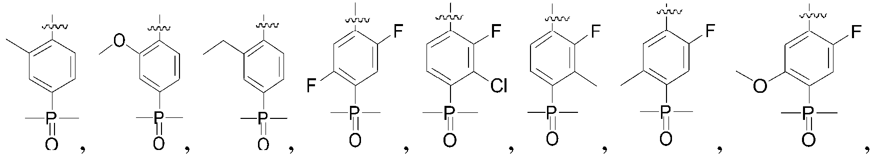 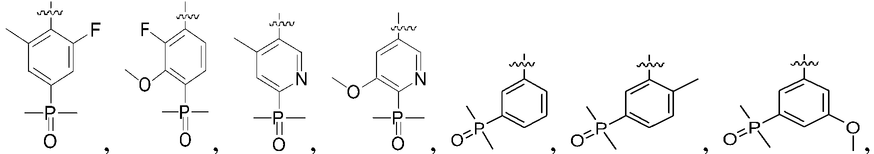 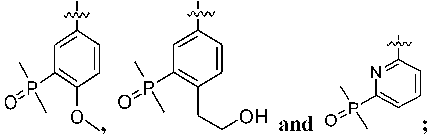
W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, O=S(R^{e}R^{f})=N-C1-C6 alkylene, R^{c}R^{d}N-C(=O)-C1-C6 alkylene, C3-C6 cycloalkyl-SO₂-, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene and 8- to 10-membered saturated bridged heterocyclyl, and the 3-to 7-membered saturated heterocyclyl, the 5- to 6-membered heteroaryl and the 8- to 10-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)- and 3-to 6-membered saturated heterocyclyl, and the C1-C6 alkylene is optionally substituted by hydroxy;
preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{c}R^{d}N-C(=O)-C1-C6 alkylene, C3-C6 cycloalkyl-SO₂-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, and the 3- to 7-membered saturated heterocyclyl and the 8-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)- and 4-membered saturated heterocyclyl, and the C1-C6 alkylene is optionally substituted by hydroxy;
more preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{c}R^{d}N-C(=O)-C1-C6 alkylene, C3-C6 cycloalkyl-SO₂-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, and the 3- to 7-membered saturated heterocyclyl and the 8-membered saturated bridged heterocyclyl are each independently optionally substituted by 1 or 2 (preferably 1) substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl (preferably 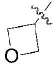 ), and the C1-C6 alkylene is optionally substituted by hydroxy, the 3- to 7-membered saturated heterocyclyl is selected from the group consisting of 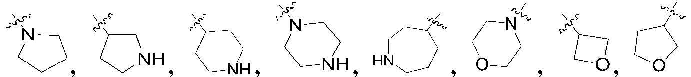 and 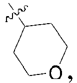 the 5- to 6-membered heteroaryl is selected from the group consisting of 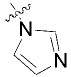 and 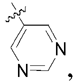 the 8-membered bridged heterocyclyl is 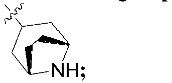
R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 3- to 6-membered saturated heterocyclyl; or, R^{c}, R^{d}, and the N atom to which they are both attached together form a 3- to 6-membered saturated heterocyclic ring, and the 3- to 6-membered saturated heterocyclic ring is optionally substituted by 1 or 2 substituent(s) selected from the group consisting of C1-C6 alkyl and halogen;
preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 5-membered saturated heterocyclyl; or, R^{c}, R^{d}, and the N atom to which they are both attached together form a 4- to 5-membered saturated heterocyclic ring, and the 4- to 5-membered saturated heterocyclic ring is optionally substituted by 1 or 2 substituent(s) selected from the group consisting of C1-C6 alkyl and halogen;
more preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 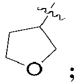 or, R^{c}, R^{d}, and the N atom to which they are both attached together form 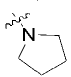 or 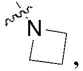 and the 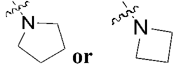 is independently optionally substituted by 1 or 2 substituent(s) selected from the group consisting of C1-C6 alkyl and halogen;
most preferably, R^{c} and R^{d} are each independently selected from the group consisting of hydrogen, methyl, ethyl, isopropyl, 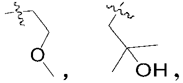 acetyl, 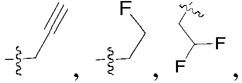 cyclopropyl, cyclobutyl, and 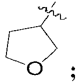 or, R^{c} and R^{d}, and the N atom to which they are both attached together form 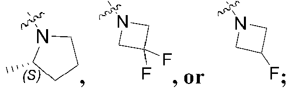
R^{e} and R^{f} are each independently selected from the group consisting of hydrogen and C1-C6 alkyl;
preferably, R^{e} and R^{f} are each independently selected from the group consisting of C1-C6 alkyl;
more preferably, R^{e} and R^{f} are methyl;
further preferably, W⁴ is selected from the group consisting of hydrogen, methyl, ethyl, difluoromethyl, trideuteromethyl, 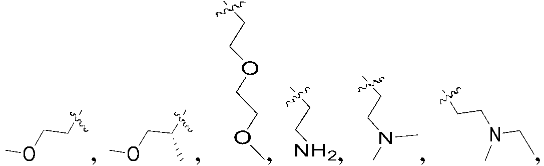 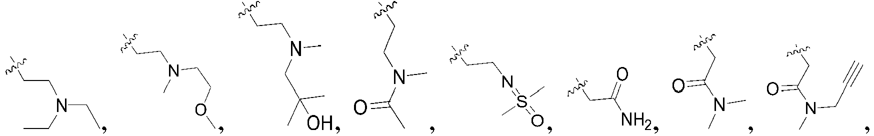 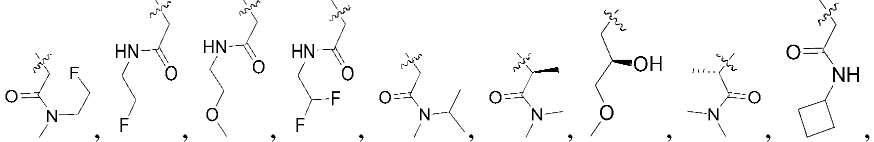 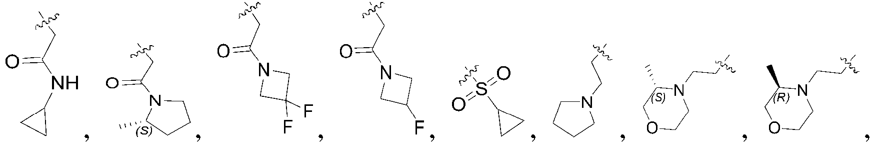 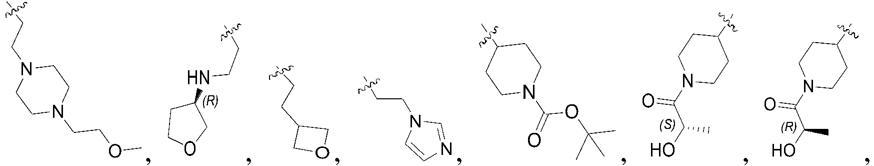 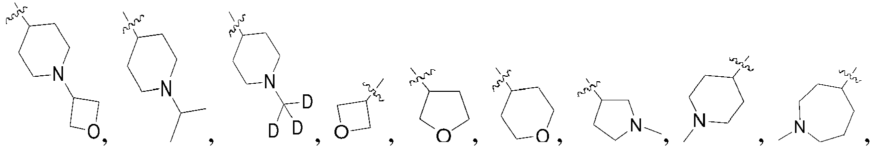 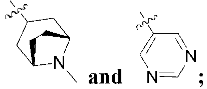
most preferably, W⁴ is selected from the group consisting of hydrogen, methyl, ethyl, difluoromethyl, trideuteromethyl, 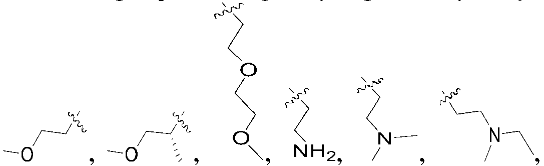 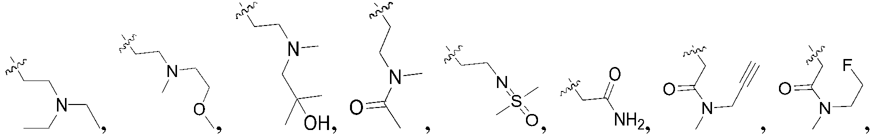 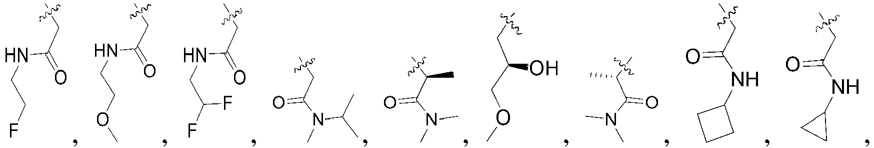 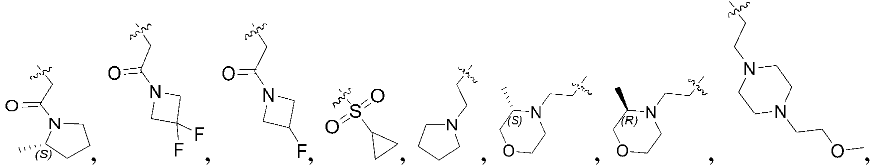 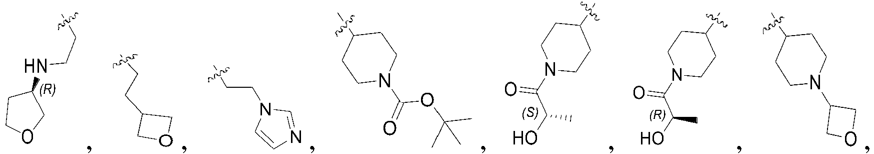 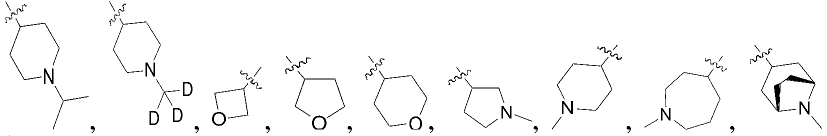
and 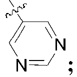
W⁵ and W⁶ are each independently selected from the group consisting of hydrogen, halogen, C1-C6 alkyl, C1-C6 alkoxy, C1-C6 haloalkyl and 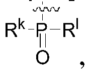 wherein W⁵ and W⁶ are not hydrogen at the same time;
preferably, W⁵ and W⁶ are each independently selected from the group consisting of hydrogen, halogen, C1-C6 alkyl, C1-C6 alkoxy and C1-C6 haloalkyl, wherein W⁵ and W⁶ are not hydrogen at the same time;
preferably, any one of W⁵ and W⁶ is selected from the group consisting of hydrogen, halogen, C1-C6 alkyl, C1-C6 alkoxy, C1-C6 haloalkyl and 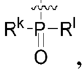 and the other is hydrogen;
R^{k} and R^{l} are each independently selected from the group consisting of C1-C6 alkyl;
preferably, R^{k} and R^{l} are methyl;
preferably, any one of W⁵ and W⁶ is selected from the group consisting of halogen, C1-C6 alkyl, C1-C6 alkoxy, and C1-C6 haloalkyl, and the other is hydrogen;
more preferably, any one of W⁵ and W⁶ is selected from the group consisting of hydrogen, chloro, methyl, ethyl, methoxy, difluoromethyl, trifluoromethyl and 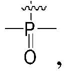 and the other is hydrogen;
more preferably, any one of W⁵ and W⁶ is selected from the group consisting of chloro, methyl, ethyl, methoxy, difluoromethyl and trifluoromethyl, and the other is hydrogen;
further preferably, 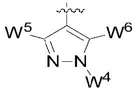 as a whole, is selected from the group consisting of 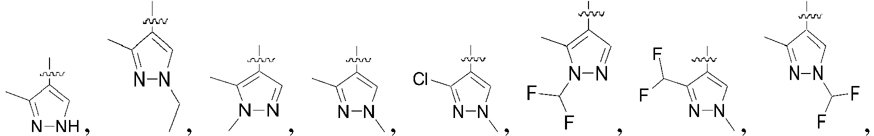 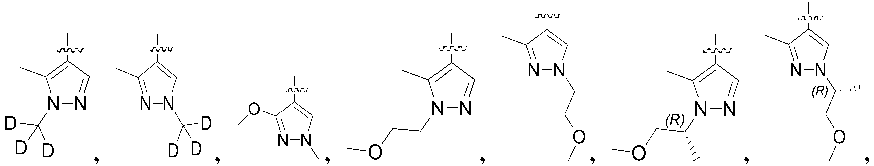 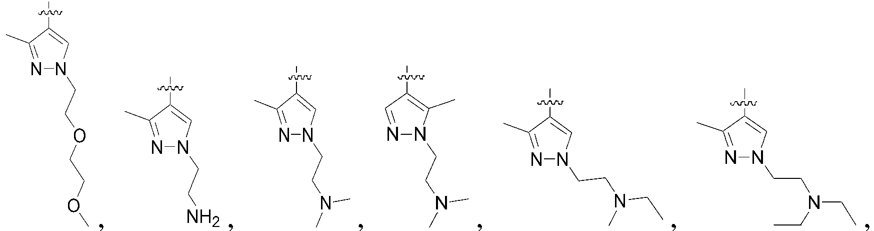 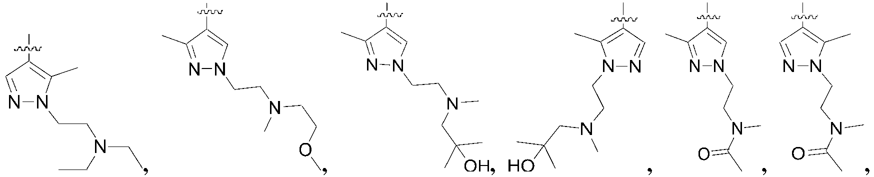 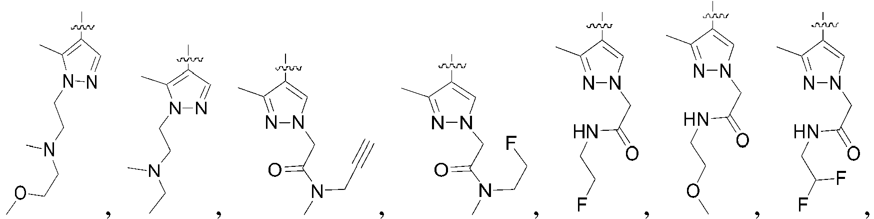 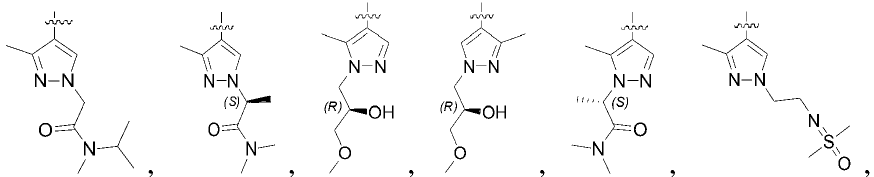 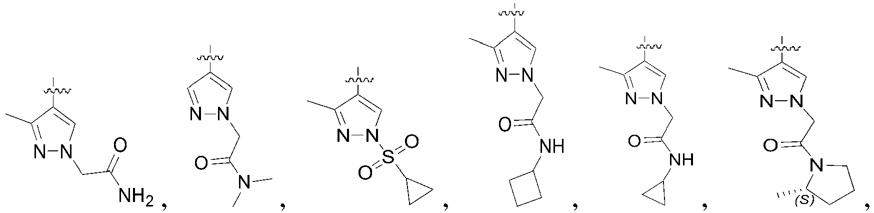 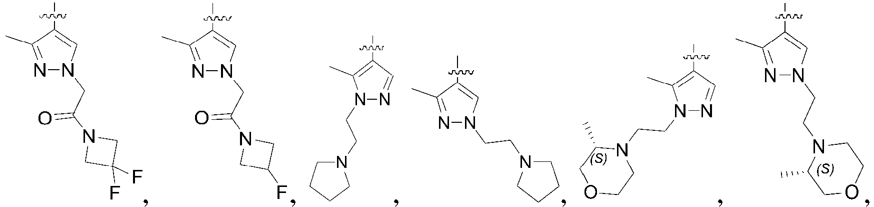 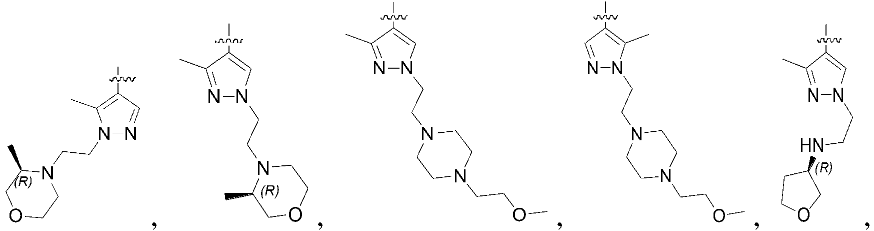 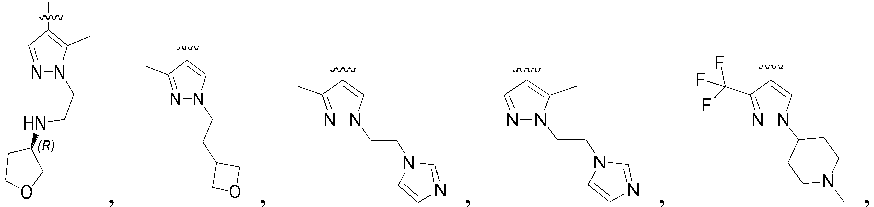 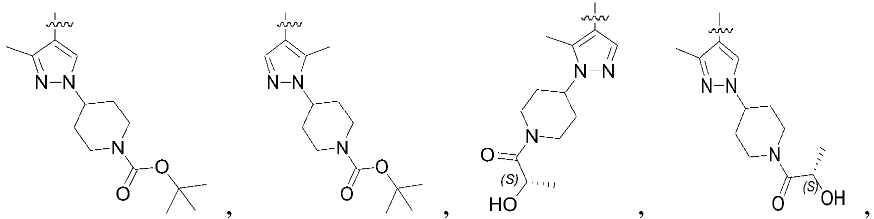 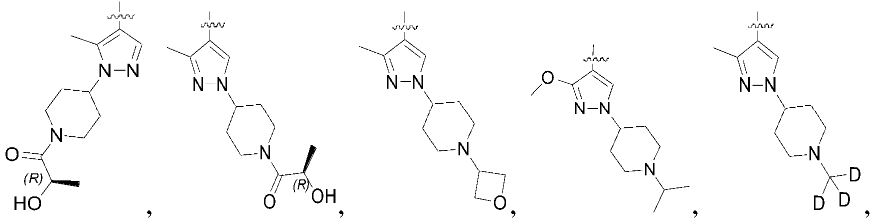 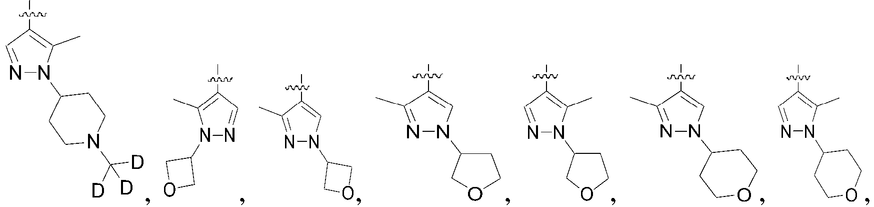 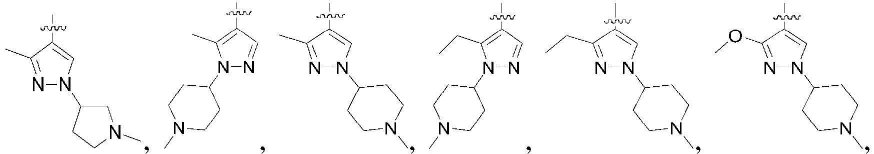 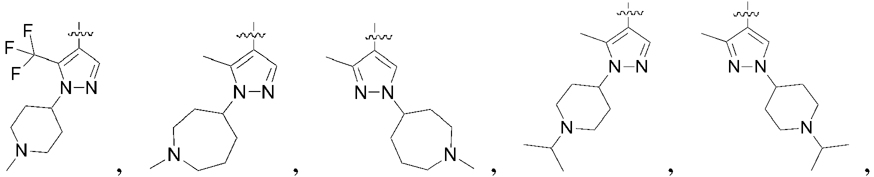 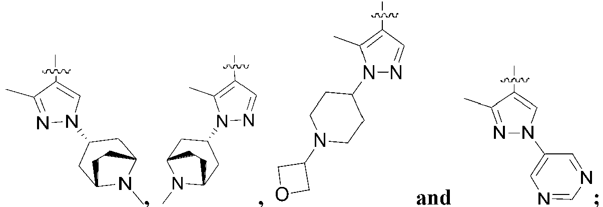
most preferably, 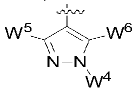 as a whole, is selected from the group consisting of 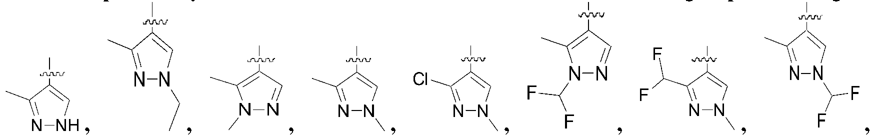 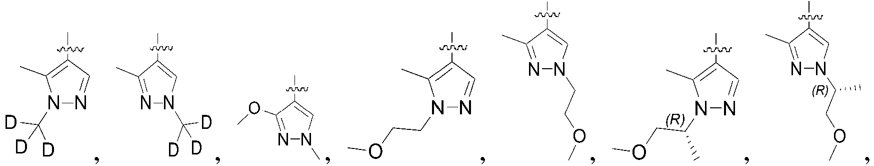 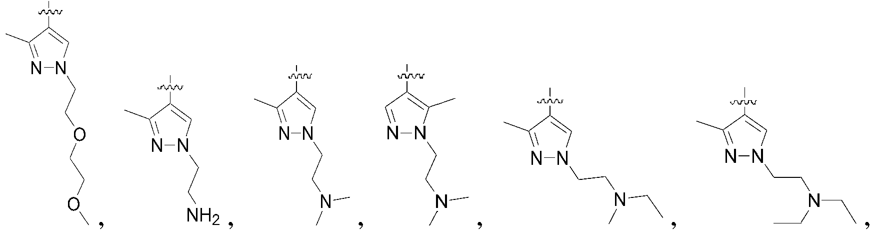 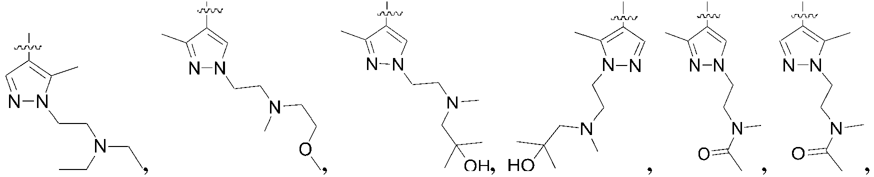 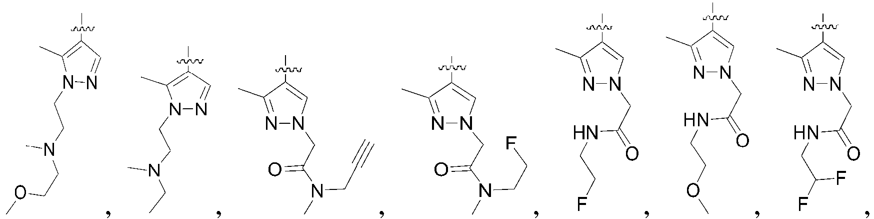 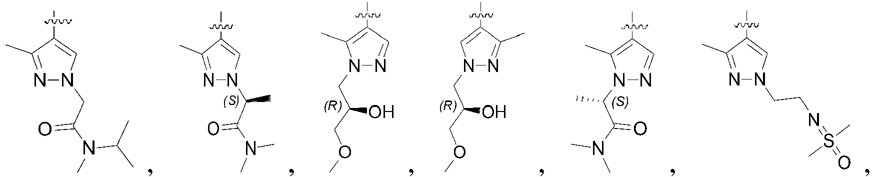 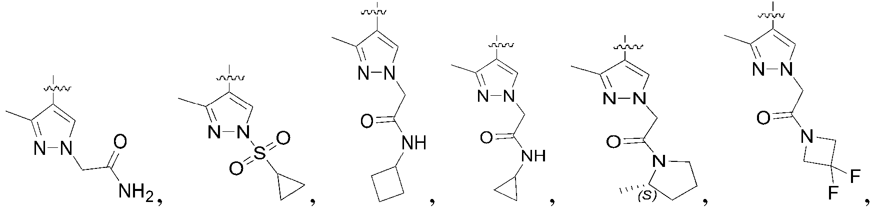 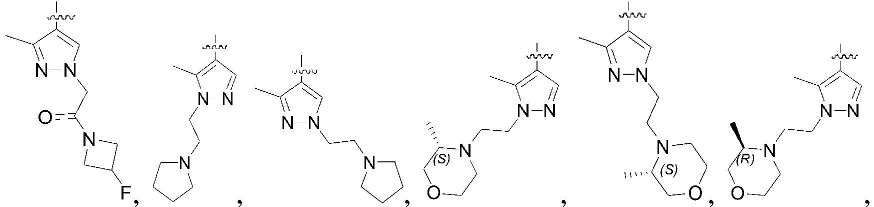 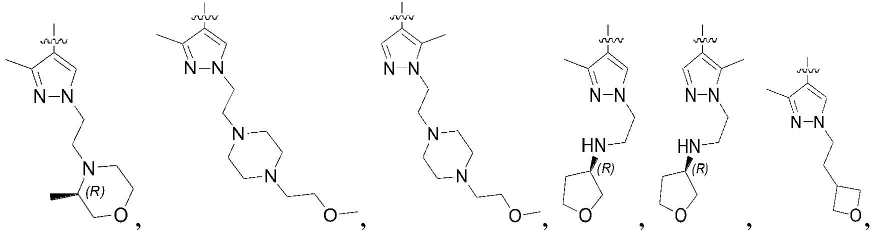 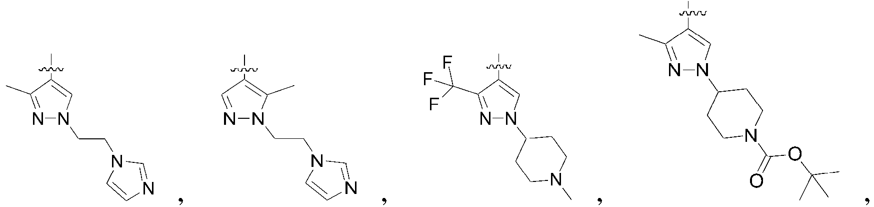 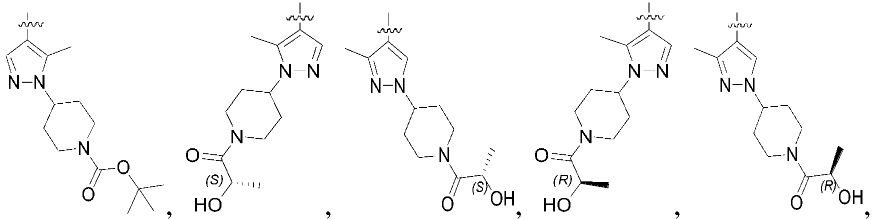 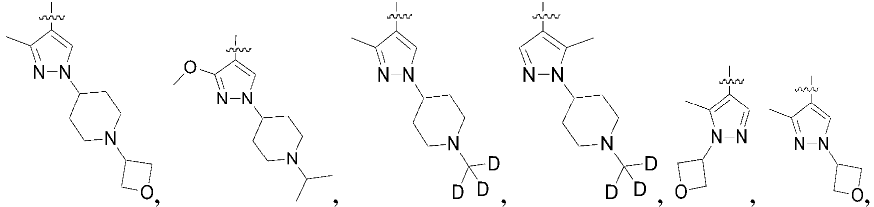 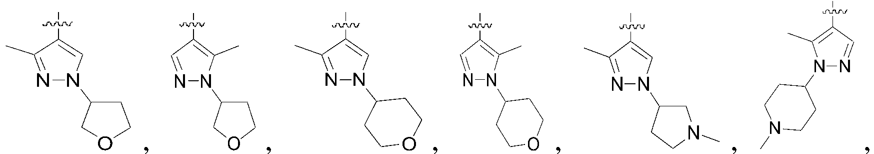 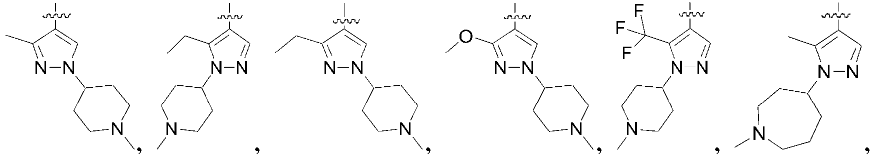 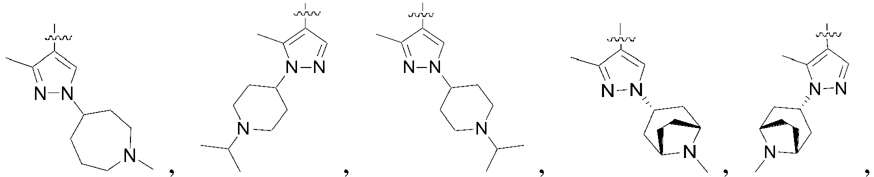 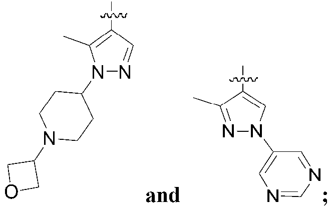
most preferably, 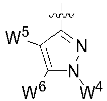 as a **whole,** is 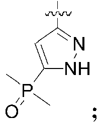
R² is selected from the group consisting of hydrogen, C1-C6 alkyl, C3-C6 cycloalkyl and C1-C6 haloalkyl;
preferably, R² is selected from the group consisting of C1-C6 alkyl, C3-C6 cycloalkyl and C1-C6 haloalkyl;
more preferably, R² is selected from the group consisting of methyl, ethyl, n-propyl, 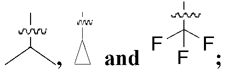
R³ is selected from the group consisting of:
1) 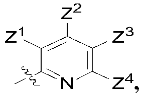 wherein:
Z¹, Z², Z³, and Z⁴ are each independently selected from the group consisting of hydrogen, halogen, nitro, cyano, C1-C6 alkyl, C1-C6 alkoxy, C1-C6 haloalkyl, C3-C6 cycloalkyl, and - NR^{a}R^{b}, wherein R^{a} and R^{b} are each independently selected from the group consisting of hydrogen and C1-C6 alkyl;
preferably, Z¹, Z², Z³, and Z⁴ are each independently selected from the group consisting of hydrogen, halogen, cyano, C1-C6 alkyl, C1-C6 haloalkyl, C1-C6 alkoxy, and -NR^{a}R^{b}, wherein R^{a} and R^{b} are each independently selected from the group consisting of hydrogen and C1-C6 alkyl;
more preferably, among Z¹, Z², Z³, and Z⁴, Z¹ is selected from the group consisting of C1-C6 alkyl, halogen, and C1-C6 alkoxy, and the remainder are hydrogen; or, among Z¹, Z², Z³, and Z⁴, Z¹ and Z⁴ are each independently selected from the group consisting of halogen, cyano, C1-C6 alkyl, C1-C6 haloalkyl, and amino, and the remainder are hydrogen; or, Z¹, Z², Z³, and Z⁴ are all hydrogen;
more preferably, Z¹, Z², Z³, and Z⁴ are each independently selected from the group consisting of hydrogen, fluoro, cyano, methyl, trifluoromethyl, amino, and methoxy;
further preferably, among Z¹, Z², Z³, and Z⁴, Z¹ is selected from the group consisting of methyl, fluoro, and methoxy, and the remainder are hydrogen; or, among Z¹, Z², Z³, and Z⁴, Z¹ and Z⁴ are each independently selected from the group consisting of fluoro, cyano, methyl, trifluoromethyl, and amino, and the remainder are hydrogen; or, Z¹, Z², Z³, and Z⁴ are all hydrogen;
most preferably, 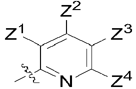 as a whole, is selected from the group consisting of 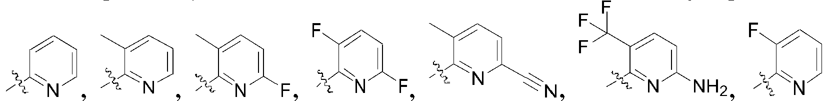 and 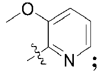
2) 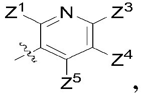 wherein:
Z¹, Z³, Z⁴, and Z⁵ are each independently selected from the group consisting of hydrogen, nitro, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 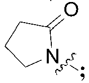 wherein R^{a} and R^{b} are each independently selected from the group consisting of hydrogen, C1-C6 alkyl, and C3-C6 cycloalkyl;
preferably, Z¹, Z³, Z⁴, and Z⁵ are each independently selected from the group consisting of hydrogen, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 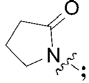 wherein R^{a} and R^{b} are each independently selected from the group consisting of hydrogen, C1-C6 alkyl, and C3-C6 cycloalkyl;
more preferably, among Z¹, Z³, Z⁴, and Z⁵, any one is selected from the group consisting of cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 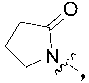 wherein R^{a} and R^{b} are each independently selected from the group consisting of hydrogen, C1-C6 alkyl, and C3-C6 cycloalkyl, and the remainder are hydrogen; or, among Z¹, Z³, Z⁴, and Z⁵, any two are selected from the group consisting of cyano, halogen, C1-C6 alkyl, amino and 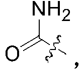 and the remainder are hydrogen; or, Z¹, Z³, Z⁴, and Z⁵ are all hydrogen;
more preferably, Z¹, Z³, Z⁴, and Z⁵ are each independently selected from the group consisting of hydrogen, fluoro, chloro, bromo, cyano, methyl, ethyl, methoxy, isopropoxy, difluoromethyl, acetyl, cyclopropyl, 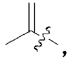 amino, 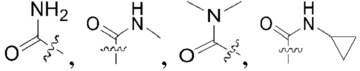 and 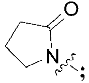
further preferably, among Z¹, Z³, Z⁴, and Z⁵, any one is selected from the group consisting of cyano, fluoro, chloro, bromo, methyl, ethyl, methoxy, isopropoxy, difluoromethyl, acetyl, cyclopropyl, 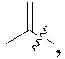 amino, 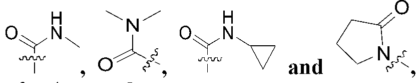 and the remainder are hydrogen; or, among Z¹, Z³, Z⁴, and Z⁵, any two are selected from the group consisting of fluoro, chloro, bromo, methyl, cyano, amino and 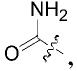 and the remainder are hydrogen; or, Z¹, Z³, Z⁴, and Z⁵ are all hydrogen;
most preferably, 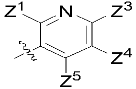 as a whole, is selected from the group consisting of 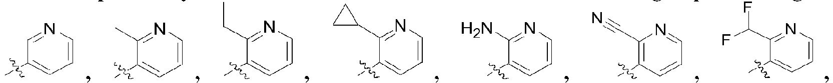 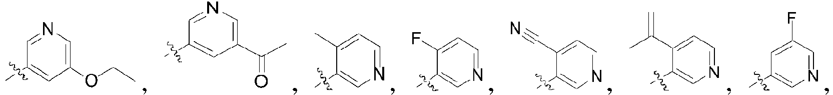 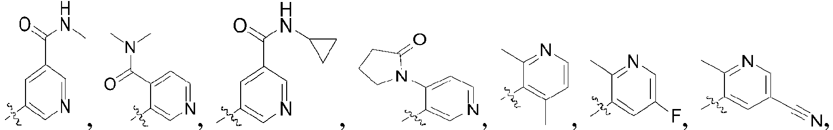 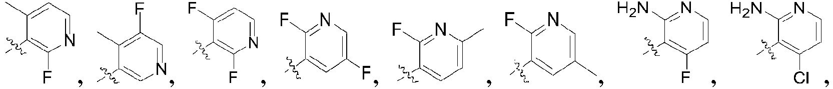 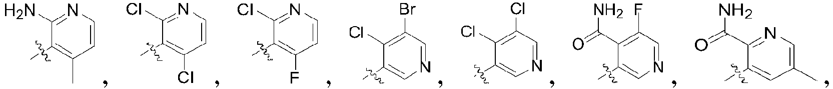 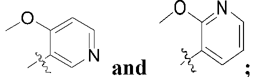
3) 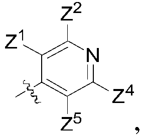 wherein:
Z¹, Z², Z⁴, and Z⁵ are each independently selected from the group consisting of hydrogen, halogen, cyano, nitro, C1-C6 alkyl, C1-C6 alkoxy, C1-C6 alkyl-C(=O)-, and -NR^{a}R^{b}, wherein R^{a} and R^{b} are each independently selected from the group consisting of hydrogen and C1-C6 alkyl;
preferably, Z¹, Z², Z⁴, and Z⁵ are each independently selected from the group consisting of hydrogen, halogen, C1-C6 alkyl, C1-C6 alkoxy, C1-C6 alkyl-C(=O)-, and -NR^{a}R^{b}, wherein R^{a} and R^{b} are each independently selected from the group consisting of hydrogen and C1-C6 alkyl;
more preferably, among Z¹, Z², Z⁴, and Z⁵, any one is selected from the group consisting of C1-C6 alkyl, halogen, C1-C6 alkoxy, amino, and C1-C6 alkyl-C(=O)-, and the remainder are hydrogen; or, among Z¹, Z², Z⁴, and Z⁵, any two are selected from the group consisting of halogen and C1-C6 alkyl, and the remainder are hydrogen; or, Z¹, Z², Z⁴, and Z⁵ are all hydrogen;
more preferably, Z¹, Z², Z⁴, and Z⁵ are each independently selected from the group consisting of hydrogen, fluoro, methyl, methoxy, acetyl, and amino;
further preferably, among Z¹, Z², Z⁴, and Z⁵, any one is selected from the group consisting of methyl, fluoro, methoxy, amino, and acetyl, and the remainder are hydrogen; or, among Z¹, Z², Z⁴, and Z⁵, any two are selected from the group consisting of fluoro and methyl, and the remainder are hydrogen; or, Z¹, Z², Z⁴, and Z⁵ are all hydrogen;
most preferably, 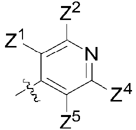 as a whole, is selected from the group consisting of 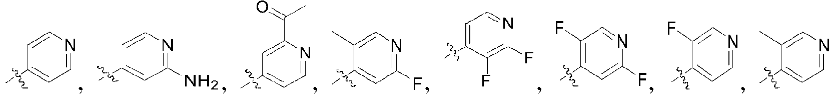 and 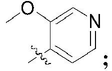
4) 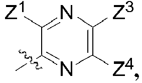 wherein:
Z¹, Z³, and Z⁴ are each independently selected from the group consisting of hydrogen, halogen, cyano, nitro, C1-C6 alkyl, C1-C6 alkoxy, hydroxy-substituted C1-C6 alkyl, -NR^{a}R^{b}, and R^{a}R^{b}N-C(=O)-; wherein R^{a} and R^{b} are each independently selected from the group consisting of hydrogen and C1-C6 alkyl;
preferably, Z¹, Z³, and Z⁴ are each independently selected from the group consisting of hydrogen, halogen, cyano, C1-C6 alkyl, C1-C6 alkoxy, hydroxy-substituted C1-C6 alkyl, - NR^{a}R^{b}, and R^{a}R^{b}N-C(=O)-; wherein R^{a} and R^{b} are each independently selected from the group consisting of hydrogen and C1-C6 alkyl;
more preferably, among Z¹, Z³, and Z⁴, any one is selected from the group consisting of halogen, cyano, C1-C6 alkyl, C1-C6 alkoxy, hydroxy-substituted C1-C6 alkyl, -NR^{a}R^{b}, and R^{a}R^{b}N-C(=O)-, wherein R^{a} and R^{b} are each independently selected from the group consisting of hydrogen and C1-C6 alkyl, and the remainder are hydrogen; or, among Z¹, Z³, and Z⁴, any two are selected from the group consisting of C1-C6 alkyl, and the remainder is hydrogen; or, Z¹, Z³, and Z⁴ are all hydrogen;
more preferably, Z¹, Z³, and Z⁴ are each independently selected from the group consisting of hydrogen, fluoro, chloro, cyano, methyl, methoxy, hydroxymethyl, amino and 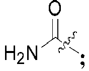
further preferably, among Z¹, Z³, and Z⁴, any one is selected from the group consisting of fluoro, chloro, cyano, methyl, methoxy, hydroxymethyl, amino and 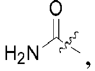 and the remainder are hydrogen; or, among Z¹, Z³, and Z⁴, any two are methyl, and the remainder is hydrogen; or, Z¹, Z³, and Z⁴ are all hydrogen;
most preferably, 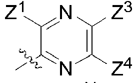 as a whole, is selected from the group consisting of 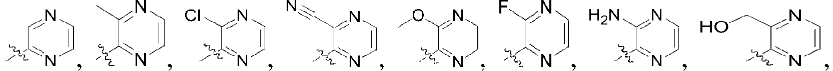 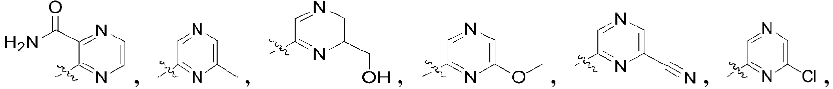 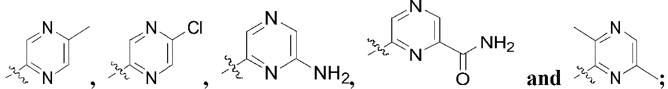
5) 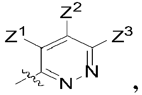 wherein:
Z¹, Z², and Z³ are each independently selected from the group consisting of hydrogen, cyano, halogen, nitro, and C1-C6 alkyl;
preferably, Z¹, Z², and Z³ are each independently selected from the group consisting of hydrogen, cyano, and C1-C6 alkyl;
more preferably, among Z¹, Z², and Z³, any one is selected from the group consisting of hydrogen, cyano, and C1-C6 alkyl, and the remainder are hydrogen;
more preferably, Z¹, Z², and Z³ are each independently selected from the group consisting of hydrogen, cyano, and methyl;
further preferably, among Z¹, Z², and Z³, any one is selected from the group consisting of hydrogen, cyano, and methyl, and the remainder are hydrogen;
most preferably, 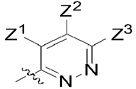 as a whole, is selected from the group consisting of 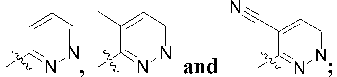
6) 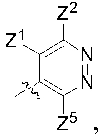 wherein:
Z¹, Z², and Z⁵ are each independently selected from the group consisting of hydrogen, cyano, halogen, nitro, and C1-C6 alkyl;
preferably, Z¹, Z², and Z⁵ are all hydrogen;
7) 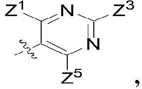 wherein:
Z¹, Z³, and Z⁵ are each independently selected from the group consisting of hydrogen, hydroxy, halogen, cyano, nitro, C1-C6 alkyl, C1-C6 alkoxy, and -NR^{a}R^{b}, wherein R^{a} and R^{b} are each independently selected from the group consisting of hydrogen and C1-C6 alkyl;
preferably, Z¹, Z³, and Z⁵ are each independently selected from the group consisting of hydrogen, hydroxy, halogen, C1-C6 alkyl, C1-C6 alkoxy, and -NR^{a}R^{b}, wherein R^{a} and R^{b} are each independently selected from the group consisting of hydrogen and C1-C6 alkyl;
more preferably, among Z¹, Z³, and Z⁵, any one is selected from the group consisting of C1-C6 alkyl, C1-C6 alkoxy, and amino, and the remainder are hydrogen; or, among Z¹, Z³, and Z⁵, any two are selected from the group consisting of hydroxy, halogen, C1-C6 alkyl, C1-C6 alkoxy, and amino, and the remainder is hydrogen; or, Z¹, Z³, and Z⁵ are all hydrogen;
more preferably, Z¹, Z³, and Z⁵ are each independently selected from the group consisting of hydrogen, hydroxy, fluoro, chloro, methyl, methoxy, and amino;
further preferably, among Z¹, Z³, and Z⁵, any one is selected from the group consisting of methyl, methoxy, and amino, and the remainder are hydrogen; or, among Z¹, Z³, and Z⁵, any two are selected from the group consisting of hydroxy, fluoro, chloro, methyl, methoxy, and amino, and the remainder is hydrogen; or, Z¹, Z³, and Z⁵ are all hydrogen;
most preferably, 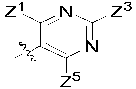 as a whole, is selected from the group consisting of 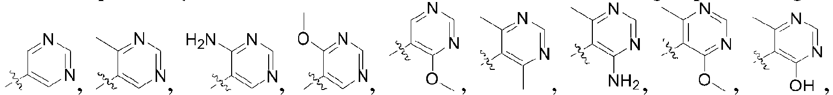 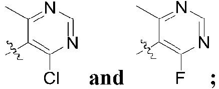
8) 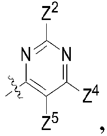 wherein:
Z², Z⁴, and Z⁵ are each independently selected from the group consisting of hydrogen, halogen, cyano, nitro, and C1-C6 alkyl;
preferably, Z², Z⁴, and Z⁵ are each independently selected from the group consisting of hydrogen, halogen, and C1-C6 alkyl;
more preferably, among Z², Z⁴, and Z⁵, any one is selected from the group consisting of halogen and C1-C6 alkyl, and the remainder are hydrogen; or, among Z², Z⁴, and Z⁵, any two are selected from the group consisting of halogen and C1-C6 alkyl, and the remainder is hydrogen; or, Z², Z⁴, and Z⁵ are all hydrogen;
more preferably, Z², Z⁴, Z⁵ are each independently selected from the group consisting of hydrogen, fluoro, chloro, bromo and methyl;
further preferably, among Z², Z⁴, and Z⁵, any one is selected from the group consisting of fluoro, chloro, bromo, and methyl, and the remainder are hydrogen; or, among Z², Z⁴, and Z⁵, any two are selected from the group consisting of fluoro, bromo, and methyl, and the remainder is hydrogen; or, Z², Z⁴, and Z⁵ are all hydrogen;
most preferably, 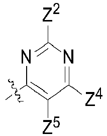 as a whole, is selected from the group consisting of 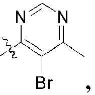 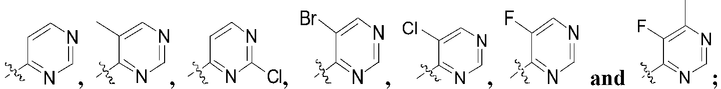
9) 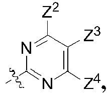 wherein:
Z², Z³, and Z⁴ are each independently selected from the group consisting of hydrogen, cyano, halogen, nitro, and C1-C6 alkyl;
preferably, Z², Z³, and Z⁴ are all hydrogen;
10) 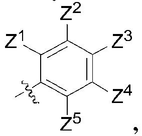 wherein:
Z¹, Z², Z³, Z⁴, and Z⁵ are each independently selected from the group consisting of hydrogen, hydroxy, cyano, nitro, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, and the 5-membered heterocyclic ring or the 5-membered heteroaromatic ring is independently optionally substituted by 1 to 2 halogen atoms, and the remainder are each independently selected from the group consisting of hydrogen and C1-C6 alkyl;
preferably, Z¹, Z², Z³, Z⁴, and Z⁵ are each independently selected from the group consisting of hydrogen, hydroxy, cyano, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, and the 5-membered heterocyclic ring or the 5-membered heteroaromatic ring is independently optionally substituted by 1 to 2 halogen atoms, and the remainder are each independently selected from the group consisting of hydrogen and C1-C6 alkyl;
more preferably, among Z¹, Z², Z³, Z⁴, and Z⁵, any two are selected from the group consisting of hydroxy, cyano, halogen, and C1-C6 alkyl, and the remainder are hydrogen; or, among Z¹, Z², Z³, Z⁴, and Z⁵, Z¹ and Z² or Z² and Z³, and the carbon atoms to which they are respectively attached together form 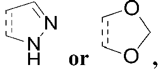 and the 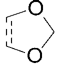 is optionally substituted by 1 or 2 halogen atoms, and the remainder are each independently selected from the group consisting of hydrogen and C1-C6 alkyl;
further preferably, among Z¹, Z², Z³, Z⁴, and Z⁵, any two are selected from the group consisting of hydroxy, cyano, fluoro, and methyl, and the remainder are hydrogen; or, among Z¹, Z², Z³, Z⁴, and Z⁵, Z¹ and Z² or Z² and Z³, and the carbon atoms to which they are respectively attached together form 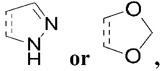 and the 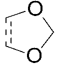 is optionally substituted by 1 or 2 fluoro atoms, and the remainder are each independently selected from the group consisting of hydrogen and methyl;
most preferably, 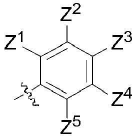
as a whole, is selected from the group consisting of 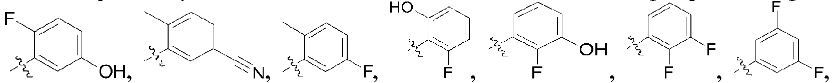 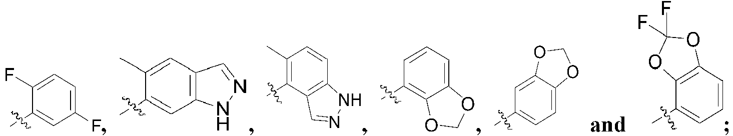
11) 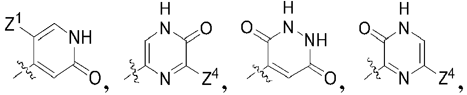 wherein Z¹ is selected from the group consisting of hydrogen and C1-C6 alkyl, preferably selected from the group consisting of hydrogen and methyl; each Z⁴ is independently selected from the group consisting of halogen and C1-C6 alkyl, preferably selected from the group consisting of chloro and methyl; and
12) 5- to 9-membered heteroaryl, and the 5- to 9-membered heteroaryl is optionally substituted by 1 or 2 substituent(s) selected from the group consisting of C1-C6 alkyl and cyano; preferably, the 5- to 9-membered heteroaryl is selected from the group consisting of 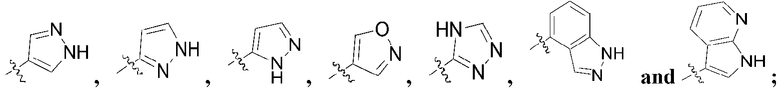 and the 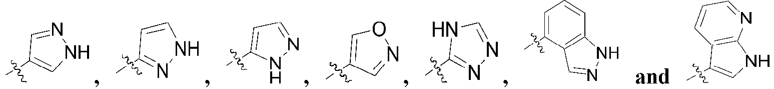 are each independently optionally substituted by 1 or 2 substituents selected from the group consisting of C1-C6 alkyl and cyano; more preferably, the 5- to 9-membered heteroaryl is selected from the group consisting of 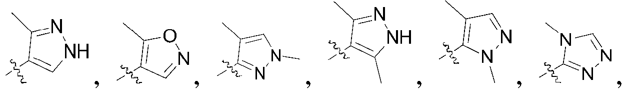 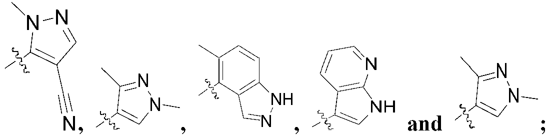 preferably, R³ is selected from the group consisting of

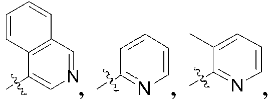

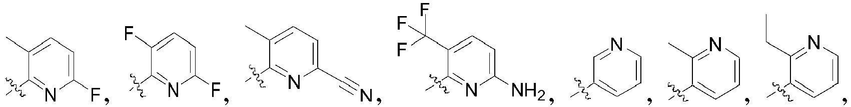

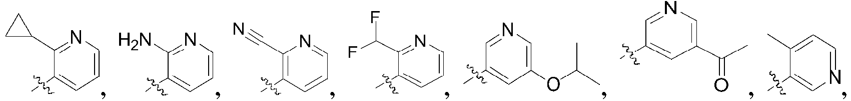

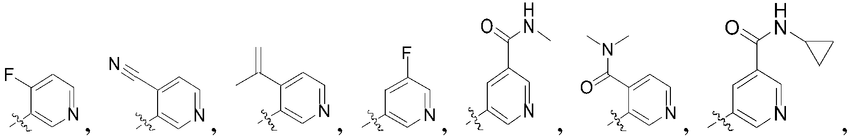

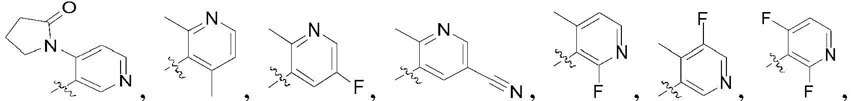

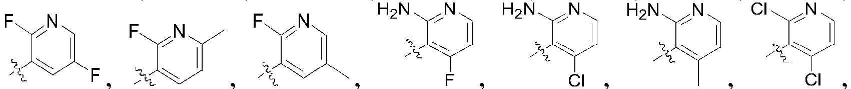

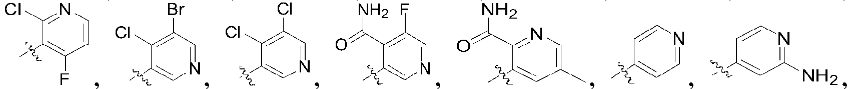

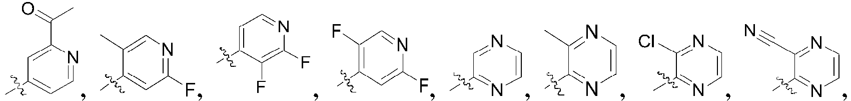

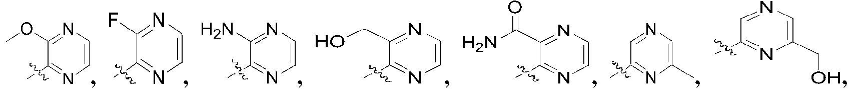

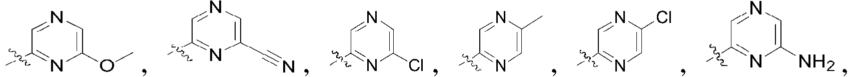

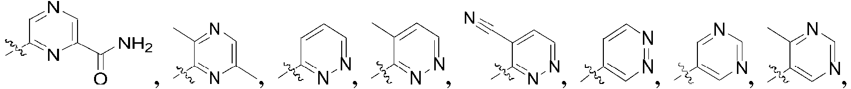

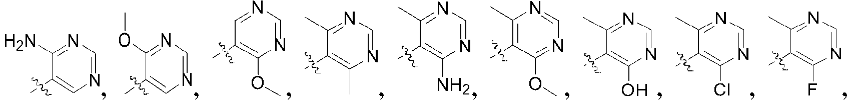

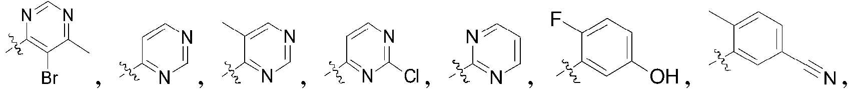

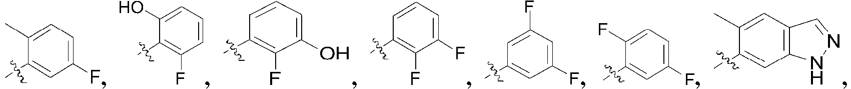

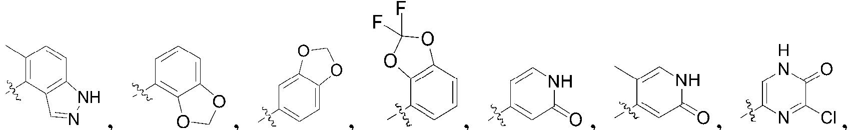

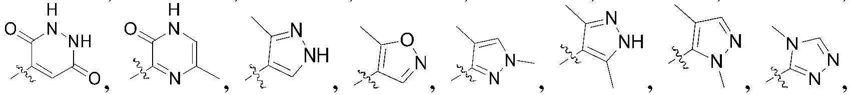

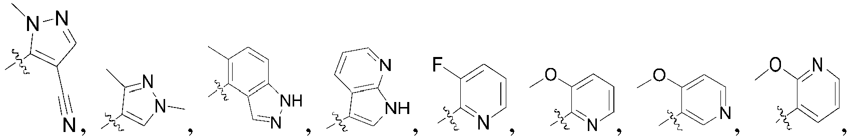

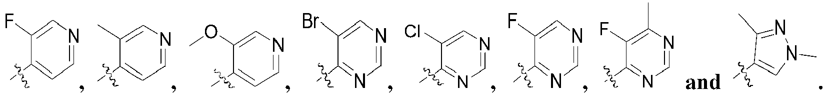

2. The compound or a stereoisomer thereof, a prodrug thereof, a pharmaceutically acceptable salt thereof, or a pharmaceutically acceptable solvate thereof according to claim 1, wherein the compound has the structure of formula I, 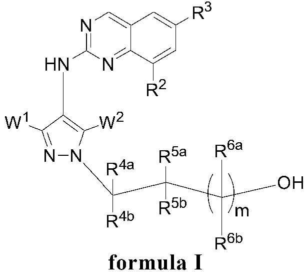 wherein:
R² is selected from the group consisting of hydrogen, C1-C6 alkyl, C3-C6 cycloalkyl and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 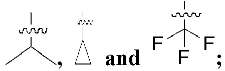
R³ is selected from the group consisting of:
1) 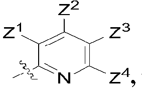 wherein:
Z¹, Z², Z³, and Z⁴ are each independently selected from the group consisting of hydrogen, halogen, nitro, cyano, C1-C6 alkyl, C1-C6 alkoxy, C1-C6 haloalkyl, C3-C6 cycloalkyl, and - NR^{a}R^{b}, wherein R^{a} and R^{b} are each independently selected from the group consisting of hydrogen and C1-C6 alkyl;
preferably, Z¹, Z², Z³, and Z⁴ are each independently selected from the group consisting of hydrogen, halogen, cyano, C1-C6 alkyl, C1-C6 haloalkyl, C1-C6 alkoxy, and -NR^{a}R^{b}, wherein R^{a} and R^{b} are each independently selected from the group consisting of hydrogen and C1-C6 alkyl;
more preferably, among Z¹, Z², Z³, and Z⁴, Z¹ is selected from the group consisting of C1-C6 alkyl, halogen, and C1-C6 alkoxy, and the remainder are hydrogen; or, among Z¹, Z², Z³, and Z⁴, Z¹ and Z⁴ are each independently selected from the group consisting of halogen, cyano, C1-C6 alkyl, C1-C6 haloalkyl, and amino, and the remainder are hydrogen; or, Z¹, Z², Z³, and Z⁴ are all hydrogen;
more preferably, Z¹, Z², Z³, and Z⁴ are each independently selected from the group consisting of hydrogen, fluoro, cyano, methyl, trifluoromethyl, amino, and methoxy;
further preferably, among Z¹, Z², Z³, and Z⁴, Z¹ is selected from the group consisting of methyl, fluoro, and methoxy, and the remainder are hydrogen; or, among Z¹, Z², Z³, and Z⁴, Z¹ and Z⁴ are each independently selected from the group consisting of fluoro, cyano, methyl, trifluoromethyl, and amino, and the remainder are hydrogen; or, Z¹, Z², Z³, and Z⁴ are all hydrogen;
most preferably, 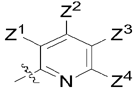
as a whole, is selected from the group consisting of 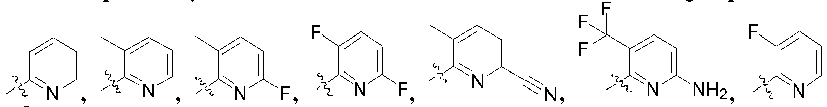 and 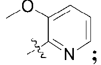
2) 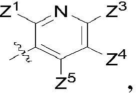 wherein:
Z¹, Z³, Z⁴, and Z⁵ are each independently selected from the group consisting of hydrogen, nitro, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 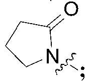 wherein R^{a} and R^{b} are each independently selected from the group consisting of hydrogen, C1-C6 alkyl, and C3-C6 cycloalkyl;
preferably, Z¹, Z³, Z⁴, and Z⁵ are each independently selected from the group consisting of hydrogen, cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 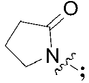 wherein R^{a} and R^{b} are each independently selected from the group consisting of hydrogen, C1-C6 alkyl, and C3-C6 cycloalkyl;
more preferably, among Z¹, Z³, Z⁴, and Z⁵, any one is selected from the group consisting of cyano, halogen, C1-C6 alkyl, C1-C6 alkoxy, C1-C6 haloalkyl, C1-C6 alkyl-C(=O)-, C3-C6 cycloalkyl, C2-C6 alkenyl, -NR^{a}R^{b}, R^{a}R^{b}N-C(=O)- and 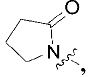 wherein R^{a} and R^{b} are each independently selected from the group consisting of hydrogen, C1-C6 alkyl, and C3-C6 cycloalkyl, and the remainder are hydrogen; or, among Z¹, Z³, Z⁴, and Z⁵, any two are selected from the group consisting of cyano, halogen, C1-C6 alkyl, and amino, and the remainder are hydrogen; or, Z¹, Z³, Z⁴, and Z⁵ are all hydrogen;
more preferably, Z¹, Z³, Z⁴, and Z⁵ are each independently selected from the group consisting of hydrogen, fluoro, chloro, bromo, cyano, methyl, ethyl, methoxy, isopropoxy, difluoromethyl, acetyl, cyclopropyl, 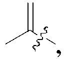 amino, 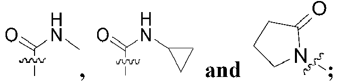
further preferably, among Z¹, Z³, Z⁴, and Z⁵, any one is selected from the group consisting of cyano, fluoro, chloro, bromo, methyl, ethyl, methoxy, isopropoxy, difluoromethyl, acetyl, cyclopropyl, 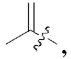 amino, 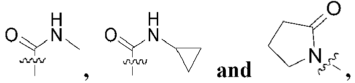 and the remainder are hydrogen; or, among Z¹, Z³, Z⁴, and Z⁵, any two are selected from the group consisting of fluoro, chloro, bromo, methyl, cyano, and amino, and the remainder are hydrogen; or, Z¹, Z³, Z⁴, and Z⁵ are all hydrogen;
most preferably, 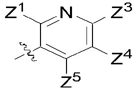 as a whole, is selected from the group consisting of 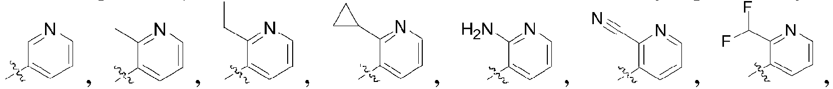 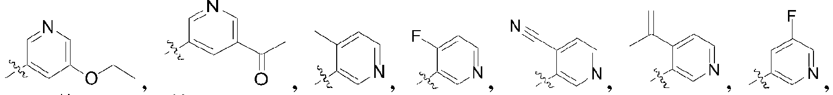 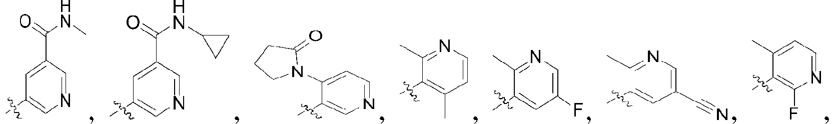 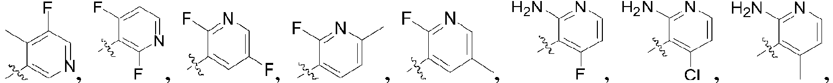 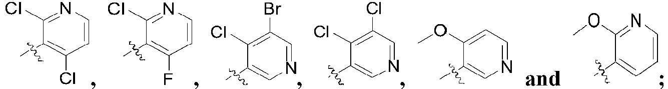
3) 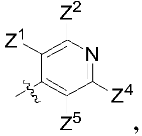 wherein:
Z¹, Z², Z⁴, and Z⁵ are each independently selected from the group consisting of hydrogen, halogen, cyano, nitro, C1-C6 alkyl, C1-C6 alkoxy, C1-C6 alkyl-C(=O)- and -NR^{a}R^{b}, wherein R^{a} and R^{b} are each independently selected from the group consisting of hydrogen and C1-C6 alkyl;
preferably, Z¹, Z², Z⁴, and Z⁵ are each independently selected from the group consisting of hydrogen, halogen, C1-C6 alkyl, C1-C6 alkoxy, C1-C6 alkyl-C(=O)- and -NR^{a}R^{b}, wherein R^{a} and R^{b} are each independently selected from the group consisting of hydrogen and C1-C6 alkyl;
more preferably, among Z¹, Z², Z⁴, and Z⁵, any one is selected from the group consisting of C1-C6 alkyl, halogen, C1-C6 alkoxy, amino and C1-C6 alkyl-C(=O)-, and the remainder are hydrogen; or, among Z¹, Z², Z⁴, and Z⁵, any two are selected from the group consisting of halogen and C1-C6 alkyl, and the remainder are hydrogen; or, Z¹, Z², Z⁴, and Z⁵ are all hydrogen;
more preferably, Z¹, Z², Z⁴, Z⁵ are each independently selected from the group consisting of hydrogen, fluoro, methyl, methoxy, acetyl and amino;
further preferably, among Z¹, Z², Z⁴, and Z⁵, any one is selected from the group consisting of methyl, fluoro, methoxy, amino and acetyl, and the remainder are hydrogen; or, among Z¹, Z², Z⁴, and Z⁵, any two are selected from the group consisting of fluoro and methyl, and the remainder are hydrogen; or, Z¹, Z², Z⁴, and Z⁵ are all hydrogen;
most preferably, 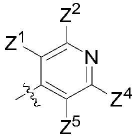 as a whole, is selected from the group consisting of 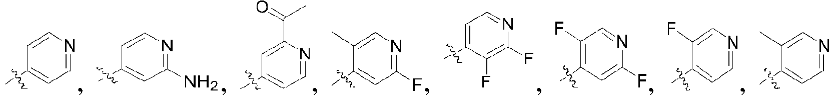 and 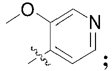
4) 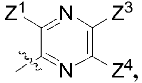 wherein:
Z¹, Z³, and Z⁴ are each independently selected from the group consisting of hydrogen, halogen, cyano, nitro, C1-C6 alkyl, C1-C6 alkoxy, hydroxy-substituted C1-C6 alkyl, -NR^{a}R^{b}, and R^{a}R^{b}N-C(=O)-; wherein R^{a} and R^{b} are each independently selected from the group consisting of hydrogen and C1-C6 alkyl;
preferably, Z¹, Z³, and Z⁴ are each independently selected from the group consisting of hydrogen, halogen, cyano, C1-C6 alkyl, C1-C6 alkoxy, hydroxy-substituted C1-C6 alkyl, - NR^{a}R^{b}, and R^{a}R^{b}N-C(=O)-; wherein R^{a} and R^{b} are each independently selected from the group consisting of hydrogen and C1-C6 alkyl;
more preferably, among Z¹, Z³, and Z⁴, any one is selected from the group consisting of halogen, cyano, C1-C6 alkyl, C1-C6 alkoxy, hydroxy-substituted C1-C6 alkyl, -NR^{a}R^{b}, and R^{a}R^{b}N-C(=O)-, wherein R^{a} and R^{b} are each independently selected from the group consisting of hydrogen and C1-C6 alkyl, and the remainder are hydrogen; or among Z¹, Z³, and Z⁴, any two are independently selected from the group consisting of C1-C6 alkyl, and the remainder is hydrogen; or Z¹, Z³, and Z⁴ are all hydrogen;
more preferably, Z¹, Z³, and Z⁴ are each independently selected from the group consisting of hydrogen, fluoro, chloro, cyano, methyl, methoxy, hydroxymethyl, amino and 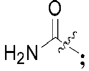
further preferably, among Z¹, Z³, and Z⁴, any one is selected from the group consisting of fluoro, chloro, cyano, methyl, methoxy, hydroxymethyl, amino and 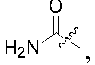 and the remainder are hydrogen; or, among Z¹, Z³, and Z⁴, any two are methyl, and the remainder is hydrogen; or, Z¹, Z³, and Z⁴ are all hydrogen;
most preferably, 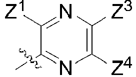 as a whole, is selected from the group consisting of 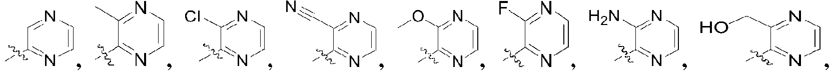 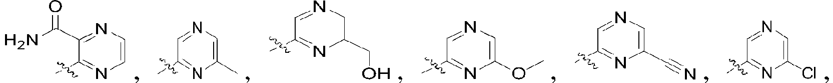 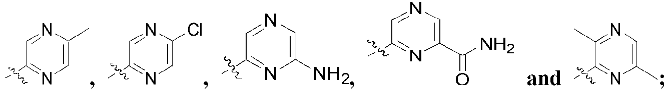
5) 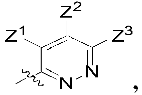 wherein:
Z¹, Z², and Z³ are each independently selected from the group consisting of hydrogen, cyano, halogen, nitro, and C1-C6 alkyl;
preferably, Z¹, Z², and Z³ are each independently selected from the group consisting of hydrogen, cyano, and C1-C6 alkyl;
more preferably, among Z¹, Z², and Z³, any one is selected from the group consisting of hydrogen, cyano, and C1-C6 alkyl, and the remainder are hydrogen;
more preferably, Z¹, Z², and Z³ are each independently selected from the group consisting of hydrogen, cyano, and methyl;
further preferably, among Z¹, Z², and Z³, any one is selected from the group consisting of hydrogen, cyano, and methyl, and the remainder are hydrogen;
most preferably, 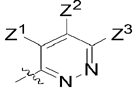 as a whole, is selected from the group consisting of 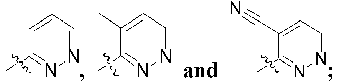
6) 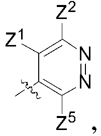 wherein:
Z¹, Z², and Z⁵ are each independently selected from the group consisting of hydrogen, cyano, halogen, nitro, and C1-C6 alkyl;
preferably, Z¹, Z², and Z⁵ are all hydrogen;
7) 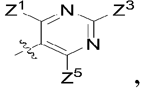 wherein:
Z¹, Z³, and Z⁵ are each independently selected from the group consisting of hydrogen, hydroxy, halogen, cyano, nitro, C1-C6 alkyl, C1-C6 alkoxy, and -NR^{a}R^{b}, wherein R^{a} and R^{b} are each independently selected from the group consisting of hydrogen and C1-C6 alkyl;
preferably, Z¹, Z³, and Z⁵ are each independently selected from the group consisting of hydrogen, hydroxy, halogen, C1-C6 alkyl, C1-C6 alkoxy, and -NR^{a}R^{b}, wherein R^{a} and R^{b} are each independently selected from the group consisting of hydrogen and C1-C6 alkyl;
more preferably, among Z¹, Z³, and Z⁵, any one is selected from the group consisting of C1-C6 alkyl, C1-C6 alkoxy, and amino, and the remainder are hydrogen; or, among Z¹, Z³, and Z⁵, any two are selected from the group consisting of hydroxy, halogen, C1-C6 alkyl, C1-C6 alkoxy, and amino, and the remainder is hydrogen; or, Z¹, Z³, and Z⁵ are all hydrogen;
more preferably, Z¹, Z³, and Z⁵ are each independently selected from the group consisting of hydrogen, hydroxy, fluoro, chloro, methyl, methoxy, and amino;
further preferably, among Z¹, Z³, and Z⁵, any one is selected from the group consisting of methyl, methoxy, and amino, and the remainder are hydrogen; or, among Z¹, Z³, and Z⁵, any two are selected from the group consisting of hydroxy, fluoro, chloro, methyl, methoxy, and amino, and the remainder is hydrogen; or, Z¹, Z³, and Z⁵ are all hydrogen;
most preferably, 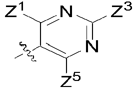 as a whole, is selected from the group consisting of 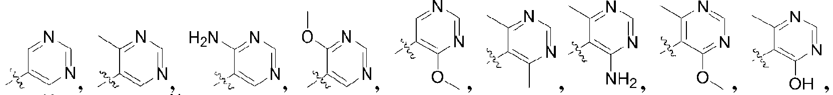 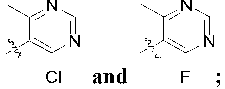
8) 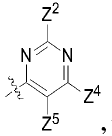 wherein:
Z², Z⁴, and Z⁵ are each independently selected from the group consisting of hydrogen, halogen, cyano, nitro, and C1-C6 alkyl;
preferably, Z², Z⁴, and Z⁵ are each independently selected from the group consisting of hydrogen, halogen, and C1-C6 alkyl;
more preferably, among Z², Z⁴, and Z⁵, any one is selected from the group consisting of halogen and C1-C6 alkyl, and the remainder are hydrogen; or, among Z², Z⁴, and Z⁵, any two are selected from the group consisting of halogen and C1-C6 alkyl, and the remainder is hydrogen; or, Z², Z⁴, and Z⁵ are all hydrogen;
more preferably, Z², Z⁴, and Z⁵ are each independently selected from the group consisting of hydrogen, fluoro, chloro, bromo, and methyl;
further preferably, among Z², Z⁴, and Z⁵, any one is selected from the group consisting of fluoro, chloro, bromo, and methyl, and the remainder are hydrogen; or, among Z², Z⁴, and Z⁵, any two are selected from the group consisting of fluoro, bromo, and methyl, and the remainder is hydrogen; or, Z², Z⁴, and Z⁵ are all hydrogen;
most preferably, 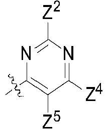 as a whole, is selected from the group consisting of 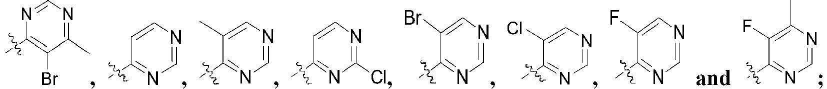
9) 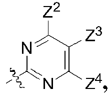 wherein:
Z², Z³, and Z⁴ are each independently selected from the group consisting of hydrogen, cyano, halogen, nitro, and C1-C6 alkyl;
preferably, Z², Z³, and Z⁴ are all hydrogen;
10) 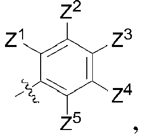 wherein:
Z¹, Z², Z³, Z⁴, and Z⁵ are each independently selected from the group consisting of hydrogen, hydroxy, cyano, nitro, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, wherein the 5-membered heterocyclic ring or the 5-membered heteroaromatic ring is independently optionally substituted by 1 or 2 halogen atoms, and the remainder are each independently selected from the group consisting of hydrogen and C1-C6 alkyl;
preferably, Z¹, Z², Z³, Z⁴, and Z⁵ are each independently selected from the group consisting of hydrogen, hydroxy, cyano, halogen, and C1-C6 alkyl; or, among Z¹, Z², Z³, Z⁴, and Z⁵, Z¹ and Z² or Z² and Z³, and the carbon atoms to which they are respectively attached together form a 5-membered heterocyclic ring or a 5-membered heteroaromatic ring, wherein the 5-membered heterocyclic ring or the 5-membered heteroaromatic ring is independently optionally substituted by 1 or 2 halogen atoms, and the remainder are each independently selected from the group consisting of hydrogen and C1-C6 alkyl;
more preferably, among Z¹, Z², Z³, Z⁴, and Z⁵, any two are independently selected from the group consisting of hydroxy, cyano, halogen, and C1-C6 alkyl, and the remainder are hydrogen; or, among Z¹, Z², Z³, Z⁴, and Z⁵, Z¹ and Z² or Z² and Z³, and the carbon atoms to which they are respectively attached together form 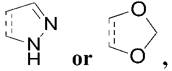 wherein 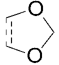 is optionally substituted by 1 or 2 halogen atoms, and the remainder are each independently selected from the group consisting of hydrogen and C1-C6 alkyl;
further preferably, among Z¹, Z², Z³, Z⁴, and Z⁵, any two are independently selected from the group consisting of hydroxy, cyano, fluoro, and methyl, and the remainder are hydrogen; or, among Z¹, Z², Z³, Z⁴, and Z⁵, Z¹ and Z² or Z² and Z³, and the carbon atoms to which they are respectively attached together form 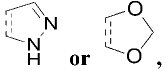 wherein the 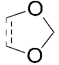 is optionally substituted by 1 or 2 fluoro atoms, and the remainder are each independently selected from the group consisting of hydrogen and methyl;
most preferably, 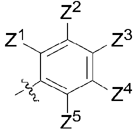 as a whole, is selected from the group consisting of 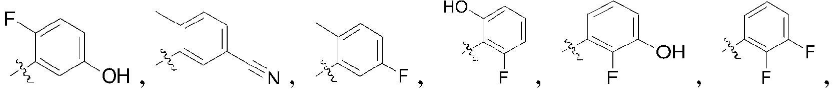 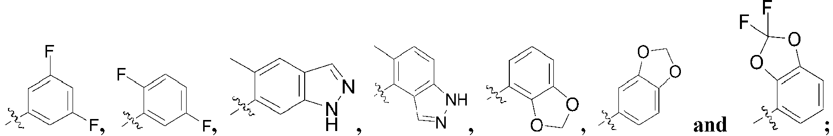
11) 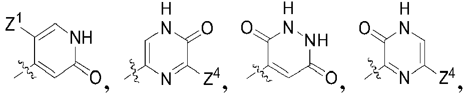 wherein, Z¹ is selected from the group consisting of C1-C6 alkyl, preferably is methyl; each Z⁴ is independently selected from the group consisting of halogen and C1-C6 alkyl, preferably is independently selected from the group consisting of chloro and methyl; and
12) 5- to 9-membered heteroaryl, and the 5- to 9-membered heteroaryl is optionally substituted by 1 or 2 substituents selected from the group consisting of C1-C6 alkyl; preferably, the 5- to 9-membered heteroaryl is selected from the group consisting of 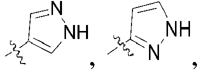 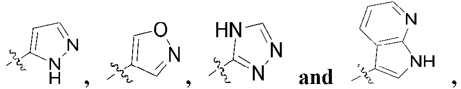 and the 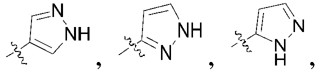 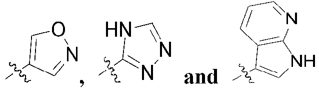 are each independently optionally substituted by 1 or 2 substituents selected from C1-C6 alkyl; more preferably, the 5- to 9-membered heteroaryl is selected from the group consisting of 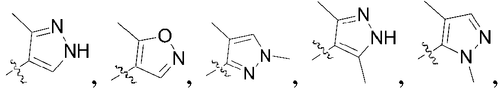 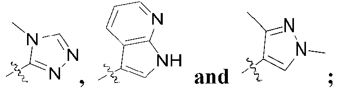
preferably, R³ is selected from the group consisting of 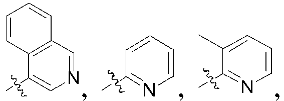 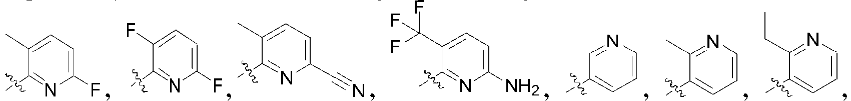 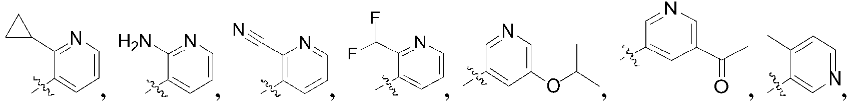 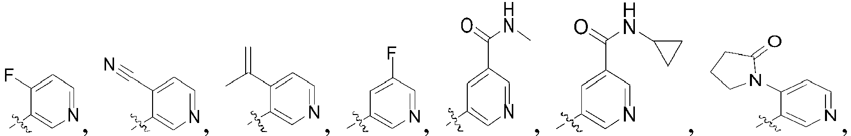 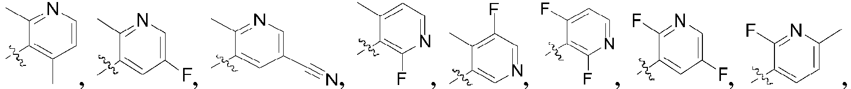 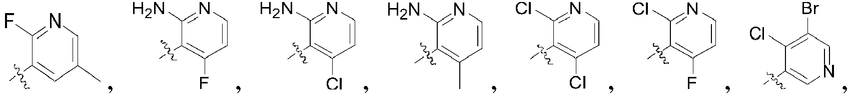 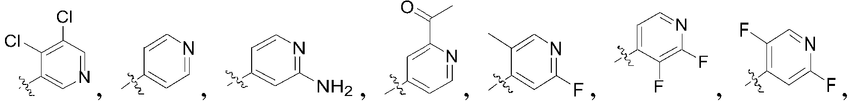 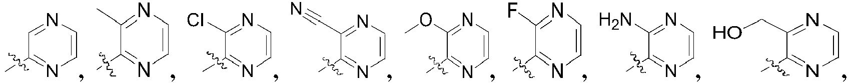 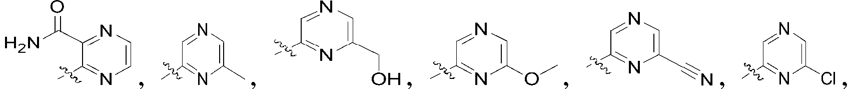 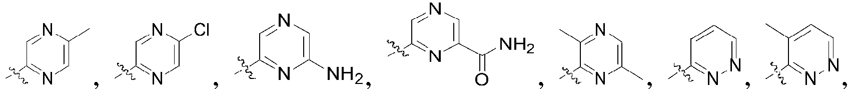 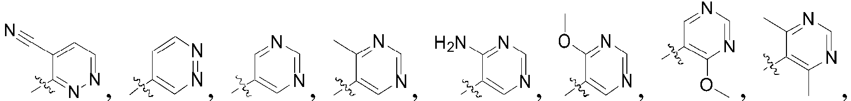 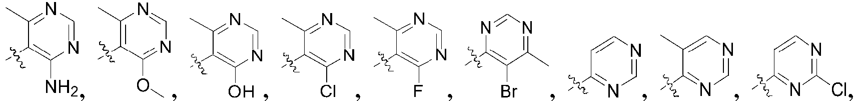 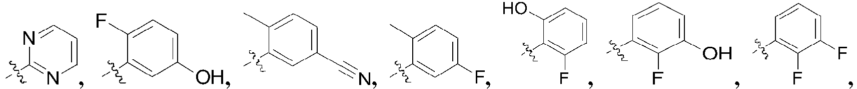 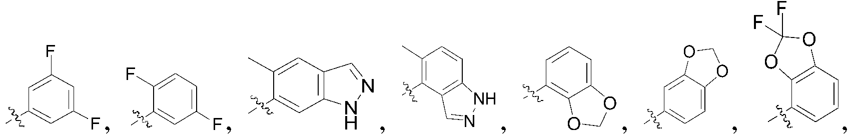 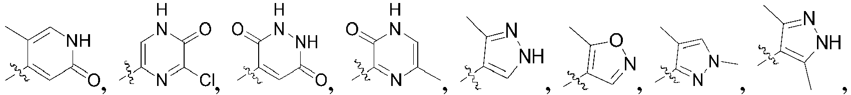 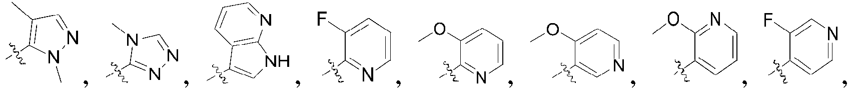 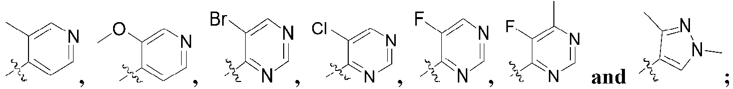 R^{4a} and R^{4b} are each independently selected from the group consisting of hydrogen and C1-C6 alkyl;
preferably, R^{4a} and R^{4b} are each independently selected from the group consisting of hydrogen and methyl;
R^{5a} and R^{5b} are each independently selected from the group consisting of hydrogen and C1-C6 alkyl;
preferably, R^{5a} and R^{5b} are each independently selected from the group consisting of hydrogen and methyl;
R^{6a} and R^{6b} are each independently selected from the group consisting of hydrogen and C1-C6 alkyl;
preferably, R^{6a} and R^{6b} are each independently selected from the group consisting of hydrogen and methyl;
m is selected from the group consisting of 0 and 1;
W¹ and W² are each independently selected from the group consisting of hydrogen, halogen, C1-C6 alkyl, and C1-C6 haloalkyl;
preferably, any one of W¹ and W² is selected from the group consisting of hydrogen, halogen, C1-C6 alkyl, and C1-C6 haloalkyl, and the other is hydrogen;
more preferably, any one of W¹ and W² is selected from the group consisting of hydrogen, chloro, methyl, and difluoromethyl, and the other is hydrogen;
most preferably, 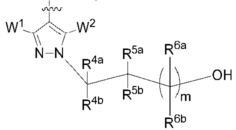 as a whole, is selected from the group consisting of:

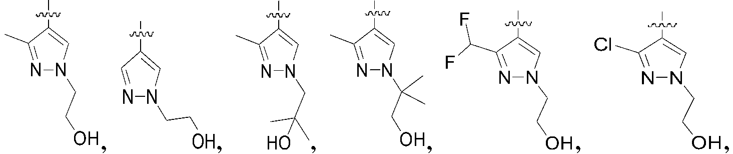

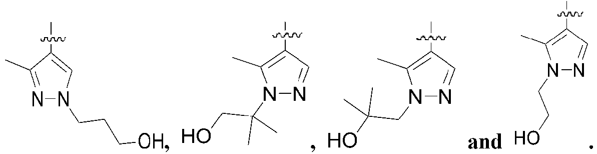

3. The compound or a stereoisomer thereof, a prodrug thereof, a pharmaceutically acceptable salt thereof, or a pharmaceutically acceptable solvate thereof according to claim 1, wherein the compound has the structure of formula II, 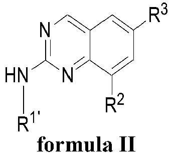 wherein:
R^{1'} is selected from the group consisting of 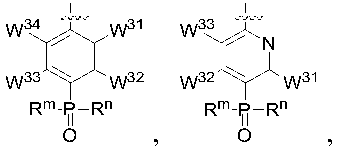 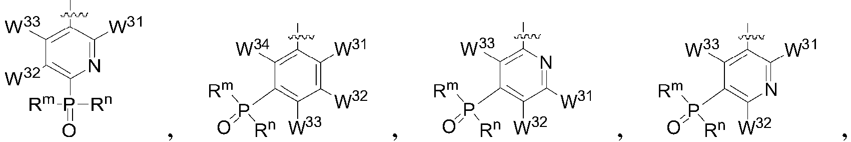 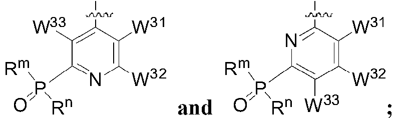
preferably, R^{1'} is selected from the group consisting of 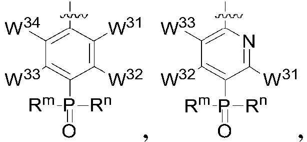 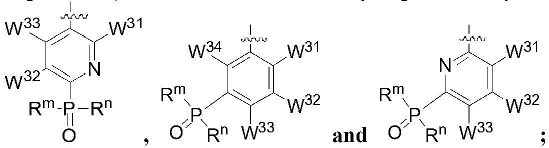
R^{m} and Rⁿ are each independently selected from the group consisting of C1-C6 alkyl;
preferably, R^{m} and Rⁿ are each independently selected from the group consisting of methyl and ethyl;
preferably, R^{1'} is selected from the group consisting of 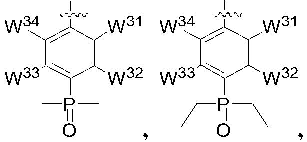 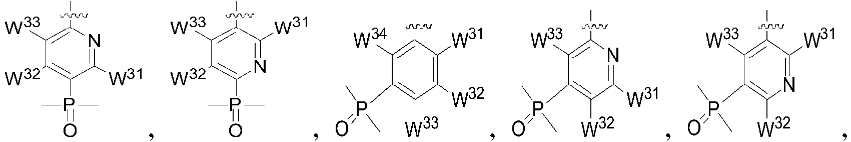 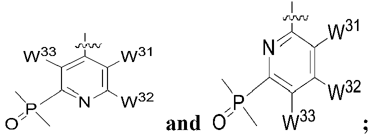
preferably, R^{1'} is selected from the group consisting of 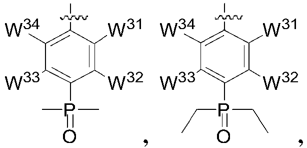 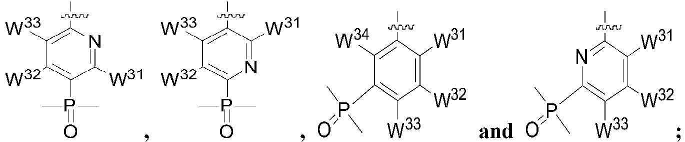
more preferably, R^{1'} is selected from the group consisting of 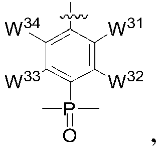 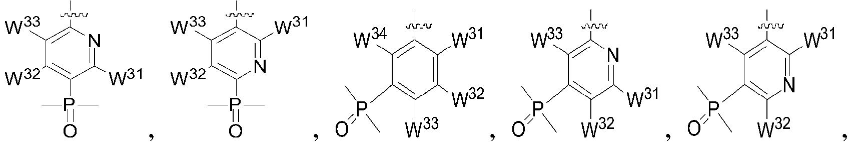 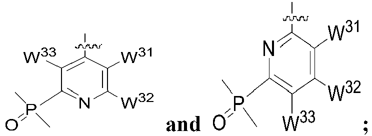
most preferably, R^{1'} is selected from the group consisting of 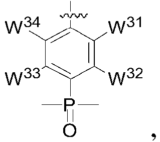 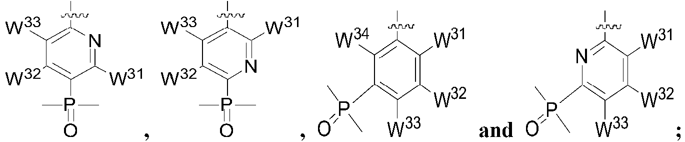
when W³¹, W³², W³³, and W³⁴ are simultaneously present, W³¹, W³², W³³, and W³⁴ are each independently selected from the group consisting of hydrogen, halogen, hydroxy, C1-C6 alkyl, hydroxy-substituted C1-C6 alkyl, C1-C6 alkoxy, and -NR^{x}R^{y};
preferably, when W³¹, W³², W³³, and W³⁴ are simultaneously present, W³¹, W³², W³³, and W³⁴ are each independently selected from the group consisting of hydrogen, halogen, C1-C6 alkyl, hydroxy-substituted C1-C6 alkyl, and C1-C6 alkoxy;
preferably, when W³¹, W³², W³³, and W³⁴ are simultaneously present, among W³¹, W³², W³³, and W³⁴, any one is selected from the group consisting of hydrogen, halogen, hydroxy, C1-C6 alkyl, C1-C6 alkoxy, hydroxy-substituted C1-C6 alkyl, and -NR^{x}R^{y}, and the remainder are hydrogen; or, among W³¹, W³², W³³, and W³⁴, any two are independently selected from the group consisting of hydrogen, halogen, hydroxy, C1-C6 alkyl, and C1-C6 alkoxy, and the remainder are hydrogen;
R^{x} and R^{y} are each independently selected from the group consisting of hydrogen and C1-C6 alkyl; preferably, any one of R^{x} and R^{y} is selected from the group consisting of C1-C6 alkyl, and the other is hydrogen; more preferably, any one of R^{x} and R^{y} is methyl, and the other is hydrogen;
preferably, when W³¹, W³², W³³, and W³⁴ are simultaneously present, among W³¹, W³², W³³, and W³⁴, any one is selected from the group consisting of hydrogen, halogen, C1-C6 alkyl, C1-C6 alkoxy, and hydroxy-substituted C1-C6 alkyl, and the remainder are hydrogen; or, among W³¹, W³², W³³, and W³⁴, any two are selected from the group consisting of hydrogen, halogen, C1-C6 alkyl, and C1-C6 alkoxy, and the remainder are hydrogen;
more preferably, when W³¹, W³², W³³, and W³⁴ are simultaneously present, among W³¹, W³², W³³, and W³⁴, any one is selected from the group consisting of hydrogen, fluoro, hydroxy, methyl, ethyl, methoxy, ethoxy, 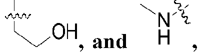 and the remainder are hydrogen; or, among W³¹, W³², W³³, and W³⁴, any two are selected from the group consisting of hydrogen, fluoro, chloro, hydroxy, methyl, and methoxy, and the remainder are hydrogen;
more preferably, when W³¹, W³², W³³, and W³⁴ are simultaneously present, among W³¹, W³², W³³, and W³⁴, any one is selected from the group consisting of hydrogen, fluoro, methyl, ethyl, methoxy, and 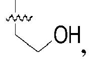 and the remainder are hydrogen; or, among W³¹, W³², W³³, and W³⁴, any two are selected from the group consisting of hydrogen, fluoro, chloro, methyl, and methoxy, and the remainder are hydrogen;
when W³¹, W³², and W³³ are simultaneously present but W³⁴ is absent, W³¹, W³², and W³³ are each independently selected from the group consisting of hydrogen, C1-C6 alkyl, and C1-C6 alkoxy;
preferably, when W³¹, W³², and W³³ are simultaneously present but W³⁴ is absent, among W³¹, W³², and W³³, any one is selected from the group consisting of hydrogen, C1-C6 alkyl, and C1-C6 alkoxy, and the remainder are hydrogen;
more preferably, when W³¹, W³², and W³³ are simultaneously present but W³⁴ is absent, among W³¹, W³², and W³³, any one is selected from the group consisting of hydrogen, methyl, and methoxy, and the remainder are hydrogen;
preferably, R^{1'} is selected from the group consisting of 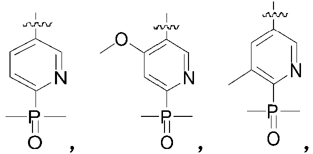 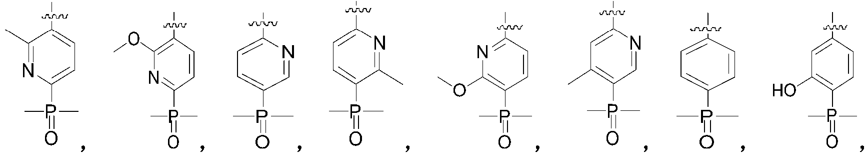 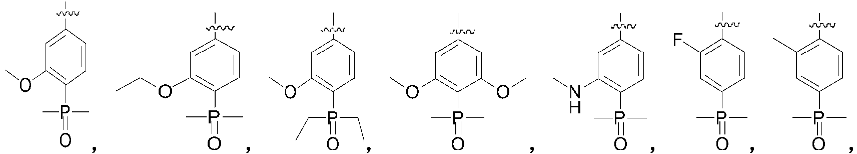 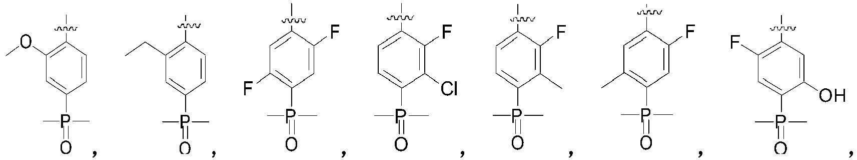 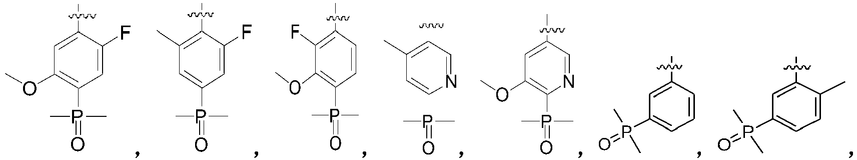 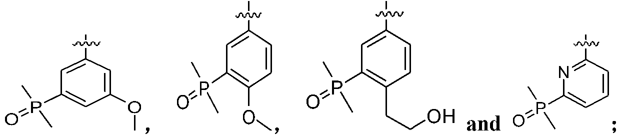
most preferably, R^{1'} is selected from the group consisting of 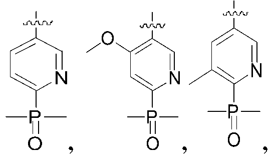 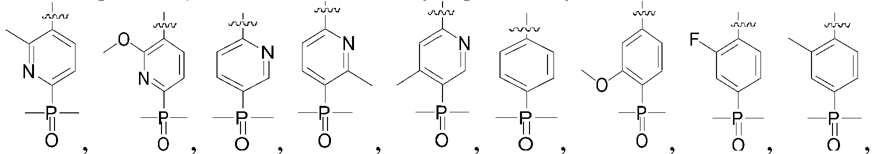 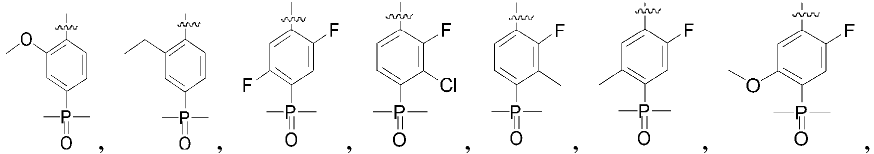 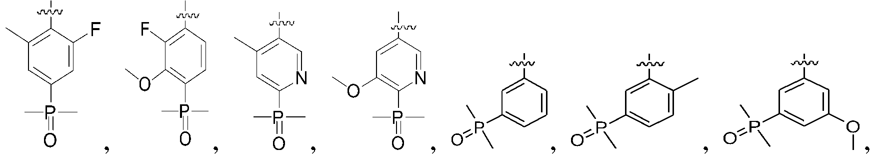 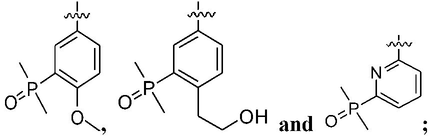
R² is selected from the group consisting of hydrogen, C1-C6 alkyl, C3-C6 cycloalkyl, and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 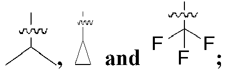
R³ is selected from the group consisting of:
1) 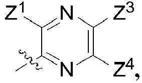 wherein:
Z¹, Z³, and Z⁴ are each independently selected from the group consisting of hydrogen, cyano, halogen, nitro, and C1-C6 alkyl;
preferably, Z¹, Z³, and Z⁴ are each independently selected from the group consisting of hydrogen and C1-C6 alkyl;
more preferably, among Z¹, Z³, and Z⁴, any one is selected from the group consisting of C1-C6 alkyl, and the remainder are hydrogen;
further preferably, among Z¹, Z³, and Z⁴, any one is methyl, and the remainder are hydrogen;
most preferably, 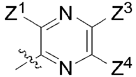 as a **whole,** is 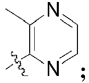
2) 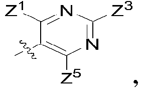 wherein:
Z¹, Z³, and Z⁵ are each independently selected from the group consisting of hydrogen, cyano, halogen, nitro, and C1-C6 alkyl;
preferably, Z¹, Z³, and Z⁵ are each independently selected from the group consisting of hydrogen and C1-C6 alkyl;
more preferably, among Z¹, Z³, and Z⁵, any two are independently selected from the group consisting of C1-C6 alkyl, and the remainder is hydrogen;
more preferably, Z¹, Z³, and Z⁵ are each independently selected from the group consisting of hydrogen and methyl;
further preferably, among Z¹, Z³, and Z⁵, any two are methyl, and the remainder is hydrogen;
most preferably, 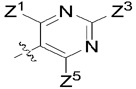 as a **whole,** is 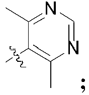
3) 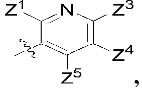 wherein:
Z¹, Z³, Z⁴, and Z⁵ are each independently selected from the group consisting of hydrogen, nitro, cyano, halogen, and C1-C6 alkyl;
preferably, Z¹, Z³, Z⁴, and Z⁵ are each independently selected from the group consisting of hydrogen and C1-C6 alkyl;
more preferably, among Z¹, Z³, Z⁴, and Z⁵, any one is selected from the group consisting of hydrogen and C1-C6 alkyl, and the remainder are hydrogen;
more preferably, Z¹, Z³, Z⁴, and Z⁵ are each independently selected from the group consisting of hydrogen and methyl;
further preferably, among Z¹, Z³, Z⁴, and Z⁵, any one is selected from the group consisting of hydrogen and methyl, and the remainder are hydrogen;
most preferably, 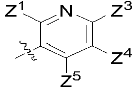 as a whole, is selected from the group consisting of 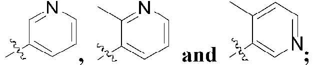
and 4) 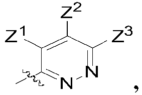 wherein:
Z¹, Z², and Z³ are each independently selected from the group consisting of hydrogen, cyano, halogen, nitro, and C1-C6 alkyl;
preferably, Z¹, Z², and Z³ are each independently selected from the group consisting of hydrogen and C1-C6 alkyl;
more preferably, among Z¹, Z², and Z³, any one is selected from the group consisting of C1-C6 alkyl, and the remainder are hydrogen;
further preferably, among Z¹, Z², and Z³, any one is methyl, and the remainder are hydrogen;
most preferably, 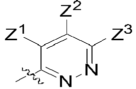
as a **whole,** is 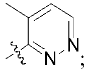
preferably, R³ is selected from the group consisting of:
1) 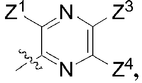 wherein:
Z¹, Z³, and Z⁴ are each independently selected from the group consisting of hydrogen, cyano, halogen, nitro, and C1-C6 alkyl;
preferably, Z¹, Z³, and Z⁴ are each independently selected from the group consisting of hydrogen and C1-C6 alkyl;
more preferably, among Z¹, Z³, and Z⁴, any one is selected from the group consisting of C1-C6 alkyl, and the remainder are hydrogen;
further preferably, among Z¹, Z³, and Z⁴, any one is methyl, and the remainder are hydrogen;
most preferably, 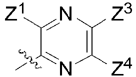 as a **whole,** is 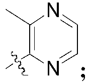
and 2) 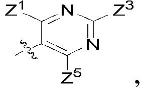 wherein:
Z¹, Z³, and Z⁵ are each independently selected from the group consisting of hydrogen, cyano, halogen, nitro, and C1-C6 alkyl;
preferably, Z¹, Z³, and Z⁵ are each independently selected from the group consisting of hydrogen and C1-C6 alkyl;
more preferably, among Z¹, Z³, and Z⁵, any two are selected from the group consisting of C1-C6 alkyl, and the remainder is hydrogen;
more preferably, Z¹, Z³, and Z⁵ are each independently selected from the group consisting of hydrogen and methyl;
further preferably, among Z¹, Z³, and Z⁵, any two are methyl, and the remainder is hydrogen;
most preferably, 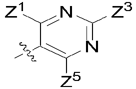 as a **whole,** is 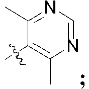
preferably, R³ is selected from the group consisting of 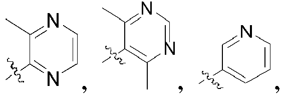 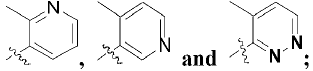
most preferably, R³ is selected from the group consisting of

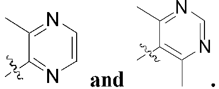

4. The compound or a stereoisomer thereof, a prodrug thereof, a pharmaceutically acceptable salt thereof, or a pharmaceutically acceptable solvate thereof according to claim 1, wherein the compound has the structure of formula III, 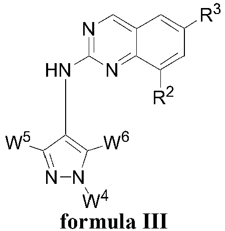 wherein:
W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, O=S(R^{e}R^{f})=N-C1-C6 alkylene, R^{c}R^{d}N-C(=O)-C1-C6 alkylene, C3-C6 cycloalkyl-SO₂-, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene and 8- to 10-membered saturated bridged heterocyclyl, and the 3-to 7-membered saturated heterocyclyl, the 5- to 6-membered heteroaryl, and the 8- to 10-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 3- to 6-membered saturated heterocyclyl, and the C1-C6 alkylene is optionally substituted by hydroxy;
preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{c}R^{d}N-C(=O)-C1-C6 alkylene, C3-C6 cycloalkyl-SO₂-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, wherein the 3- to 7-membered saturated heterocyclyl and the 8-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl, and the C1-C6 alkylene is optionally substituted by hydroxy;
more preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, O=S(R^{e}R^{f})=N-C1-C6 alkylene, R^{c}R^{d}N-C1-C6 alkylene, R^{c}R^{d}N-C(=O)-C1-C6 alkylene, C3-C6 cycloalkyl-SO₂-, 3- to 7-membered saturated heterocyclyl, 3-to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, wherein the 3- to 7-membered saturated heterocyclyl and the 8-membered saturated bridged heterocyclyl are each independently optionally substituted by 1 or 2 (preferably 1) substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl (preferably 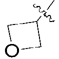 ), the C1-C6 alkylene is optionally substituted by hydroxy, the 3- to 7-membered saturated heterocyclyl is selected from the group consisting of 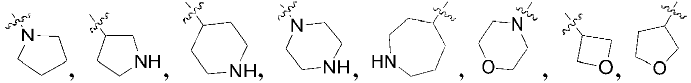 and 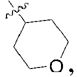 the 5- to 6-membered heteroaryl is selected from the group consisting of 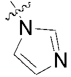 and 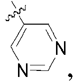 and the 8-membered bridged heterocyclyl is 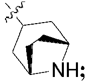
R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 3- to 6-membered saturated heterocyclyl; or, R^{c}, R^{d}, and the N atom to which they are both attached together form a 3- to 6-membered saturated heterocyclic ring, wherein the 3- to 6-membered saturated heterocyclic ring is optionally substituted by 1 or 2 substituent(s) selected from the group consisting of C1-C6 alkyl and halogen;
preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 5-membered saturated heterocyclyl; or, R^{c}, R^{d}, and the N atom to which they are both attached together form a 4- to 5-membered saturated heterocyclic ring, wherein the 4- to 5-membered saturated heterocyclic ring is optionally substituted by 1 or 2 substituent(s) selected from the group consisting of C1-C6 alkyl and halogen;
more preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl, and 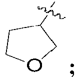 or, R^{c}, R^{d}, and the N atom to which they are both attached together form 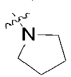 or 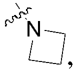 wherein the 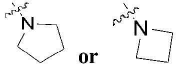 is independently optionally substituted by 1 or 2 substituents selected from the group consisting of C1-C6 alkyl and halogen;
most preferably, R^{c} and R^{d} are each independently selected from the group consisting of hydrogen, methyl, ethyl, isopropyl, 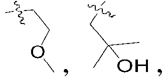 acetyl, 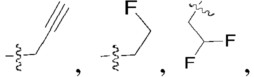 cyclopropyl, cyclobutyl and 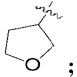 or, R^{c}, R^{d}, and the N atom to which they are both attached together form 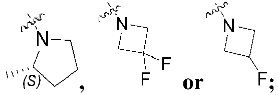
R^{e} and R^{f} are each independently selected from the group consisting of hydrogen and C1-C6 alkyl;
preferably, R^{e} and R^{f} are each independently selected from the group consisting of C1-C6 alkyl;
more preferably, R^{e} and R^{f} are methyl;
further preferably, W⁴ is selected from the group consisting of hydrogen, methyl, ethyl, difluoromethyl, trideuteromethyl, 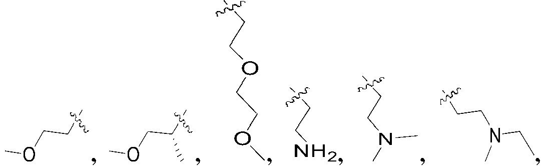 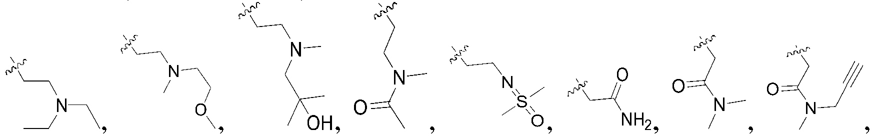 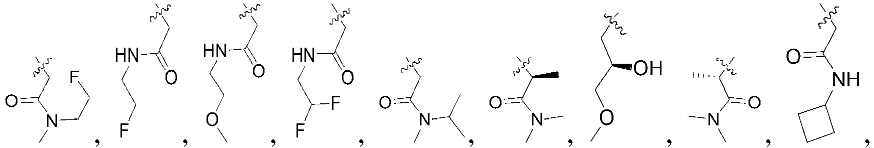 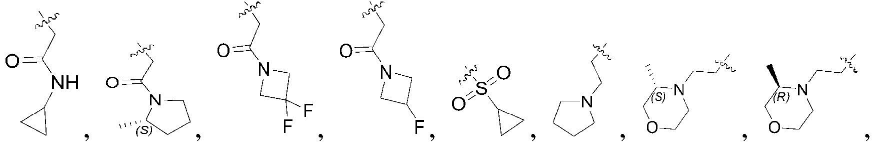 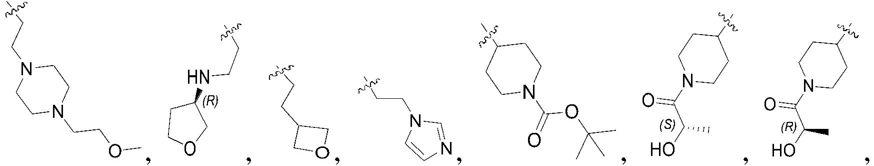 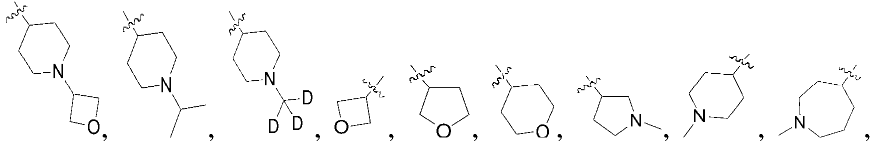 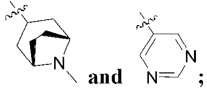
most preferably, W⁴ is selected from the group consisting of hydrogen, methyl, ethyl, difluoromethyl, trideuteromethyl, 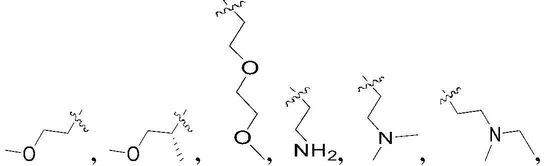 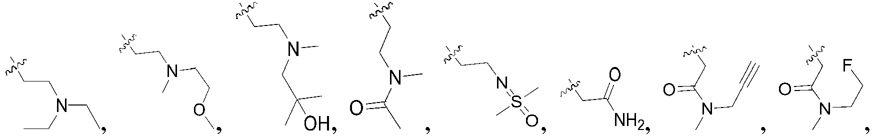 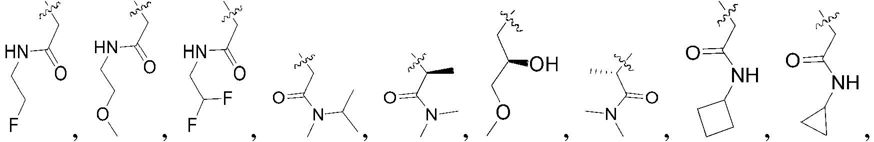 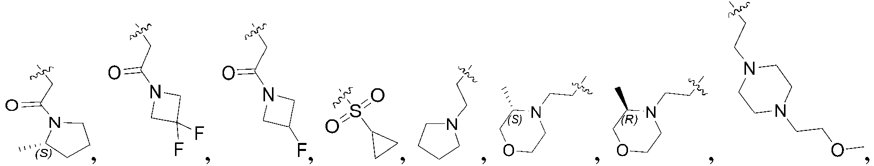 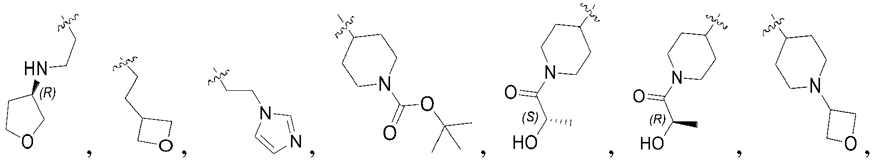 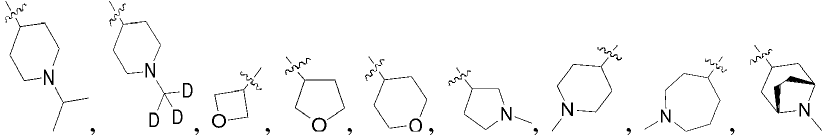 and 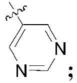
W⁵ and W⁶ are each independently selected from the group consisting of hydrogen, halogen, C1-C6 alkyl, C1-C6 alkoxy and C1-C6 haloalkyl, wherein W⁵ and W⁶ are not hydrogen at the same time;
preferably, any one of W⁵ and W⁶ is selected from the group consisting of hydrogen, halogen, C1-C6 alkyl, C1-C6 alkoxy, and C1-C6 haloalkyl, and the other is hydrogen;
preferably, any one of W⁵ and W⁶ is selected from the group consisting of halogen, C1-C6 alkyl, C1-C6 alkoxy, and C1-C6 haloalkyl, and the other is hydrogen;
more preferably, any one of W⁵ and W⁶ is selected from the group consisting of hydrogen, chloro, methyl, ethyl, methoxy, difluoromethyl, and trifluoromethyl, and the other is hydrogen;
more preferably, any one of W⁵ and W⁶ is selected from the group consisting of chloro, methyl, ethyl, methoxy, difluoromethyl, and trifluoromethyl, and the other is hydrogen;
further preferably, 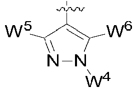 as a whole, is selected from the group consisting of 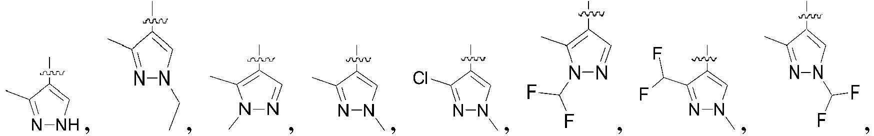 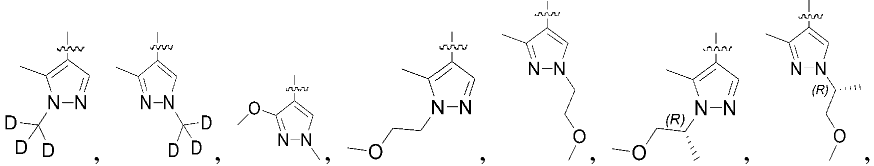 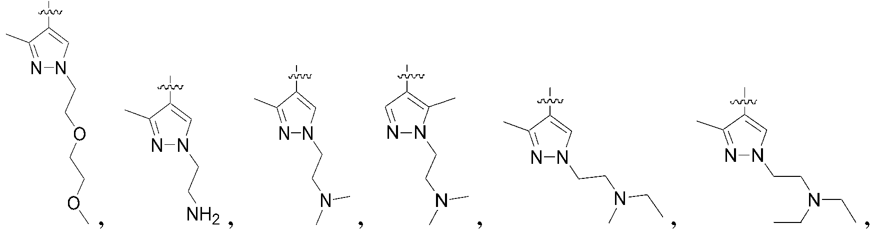 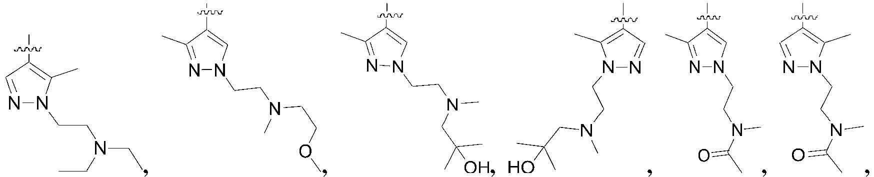 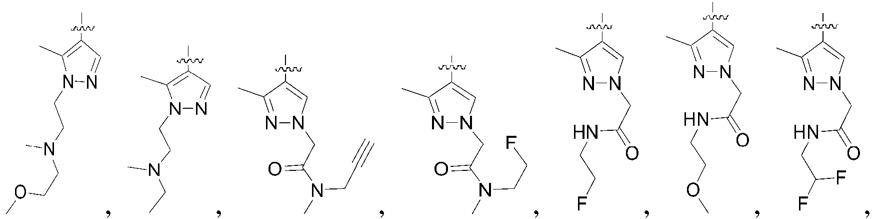 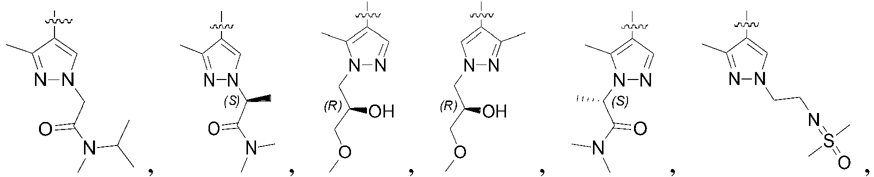 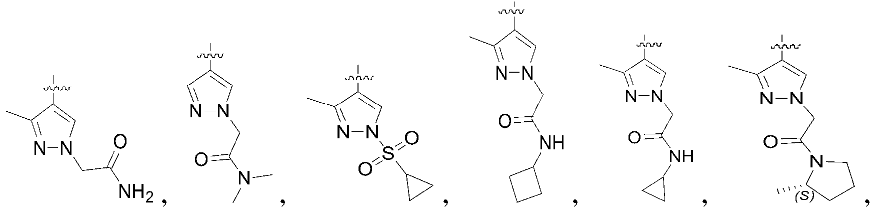 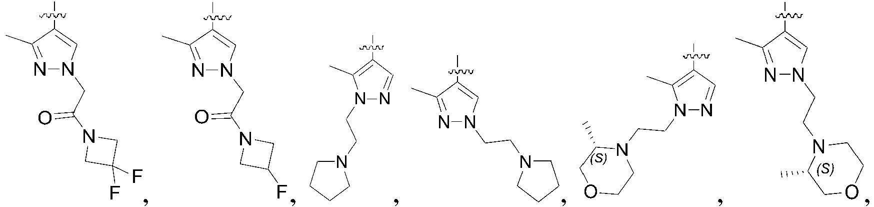 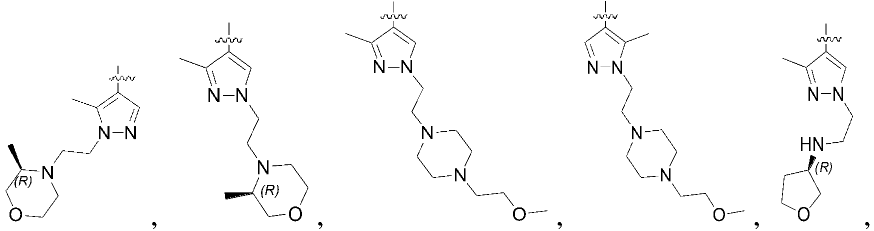 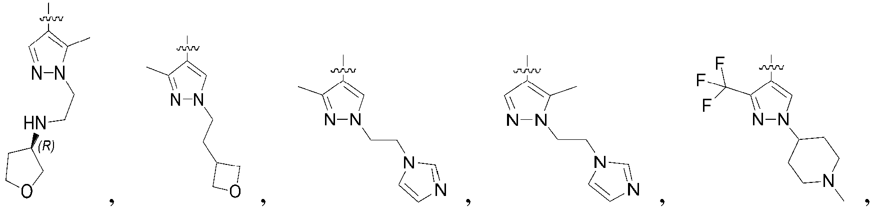 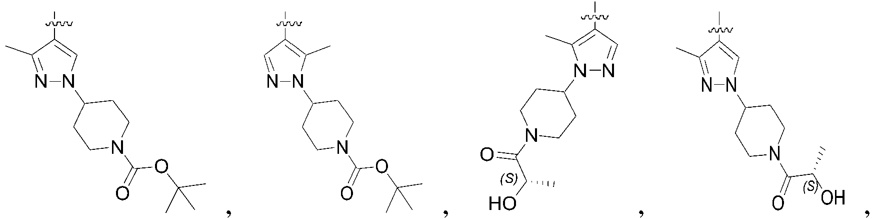 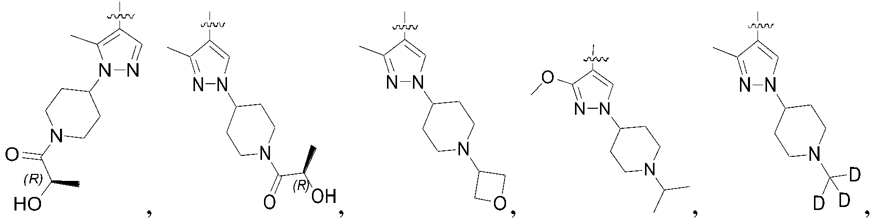 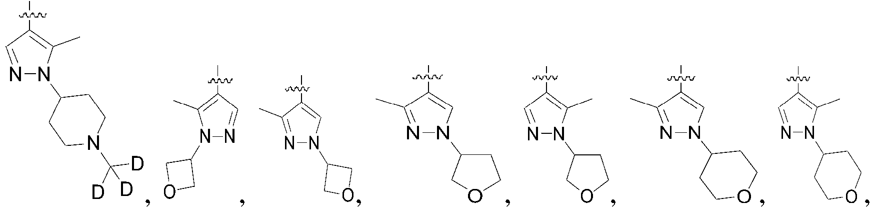 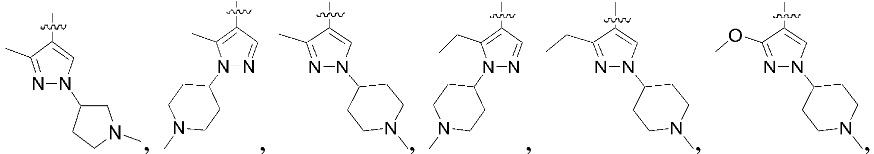 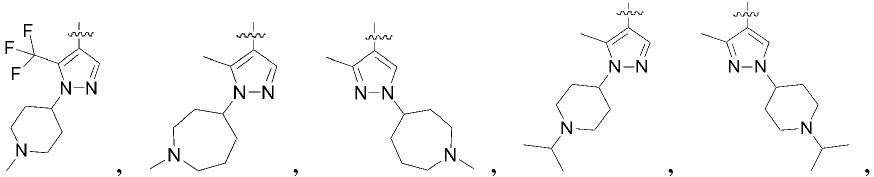 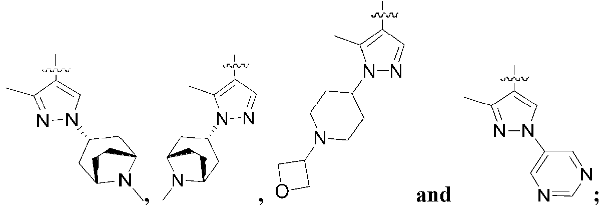
most preferably, 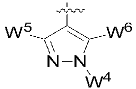 as a whole, is selected from the group consisting of 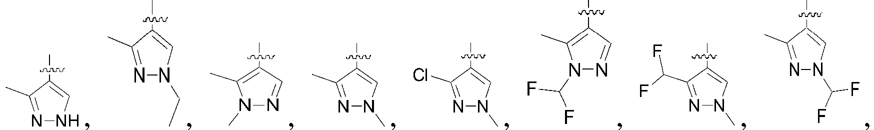 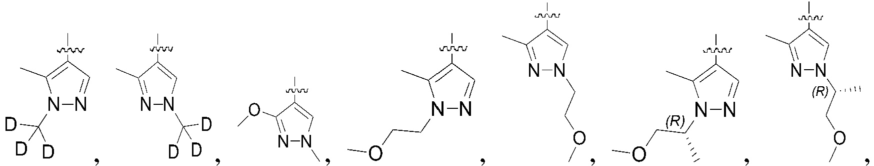 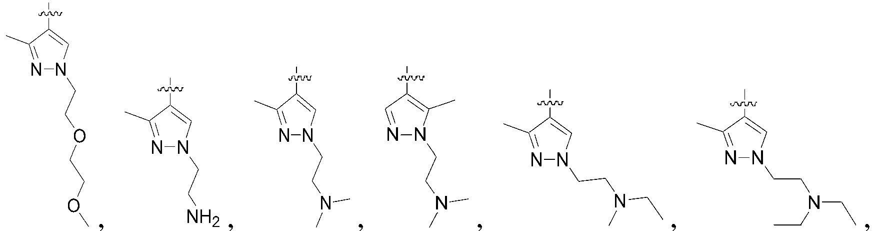 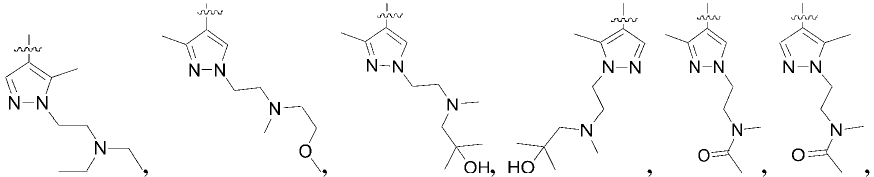 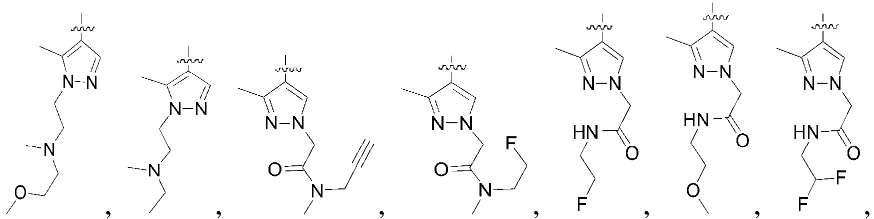 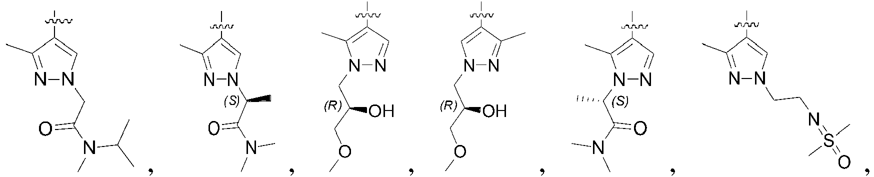 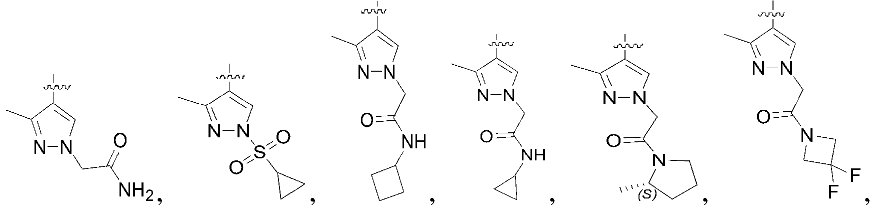 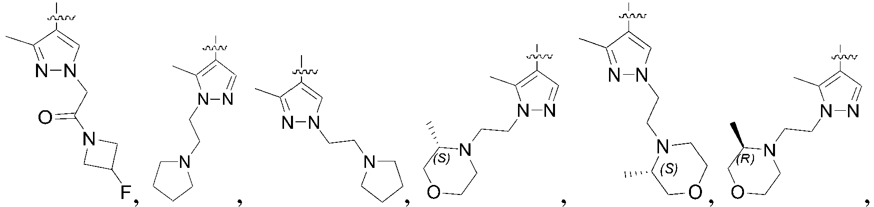 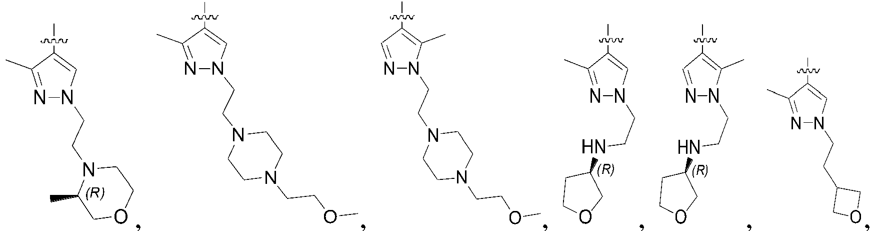 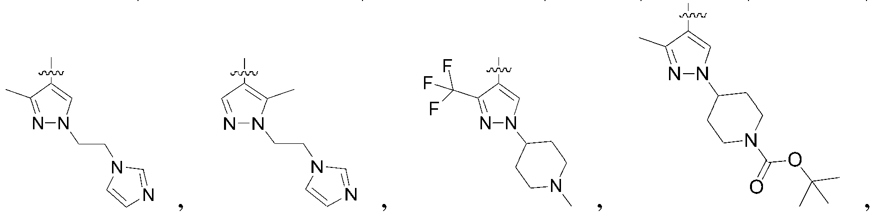 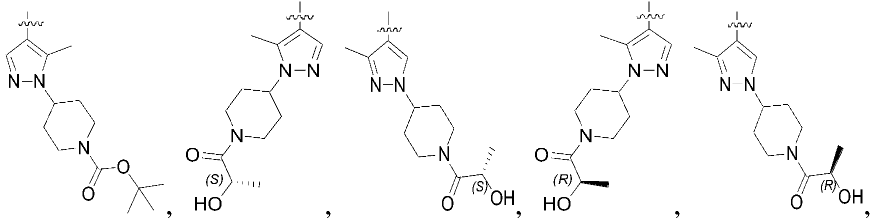 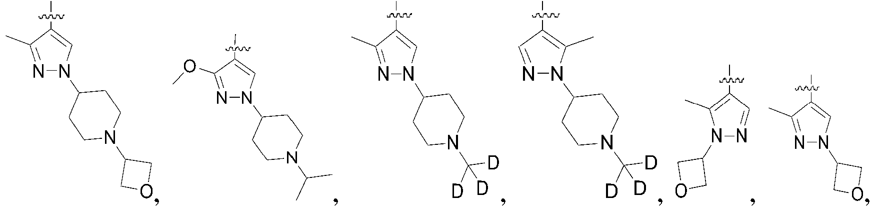 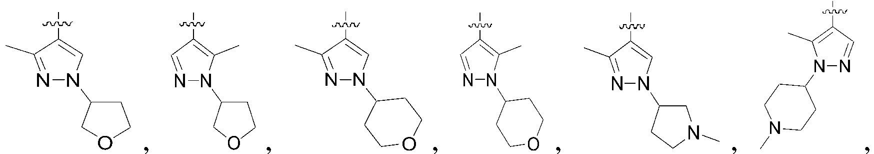 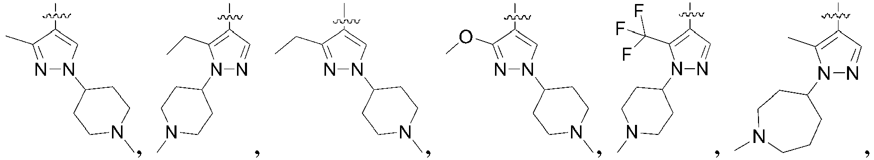 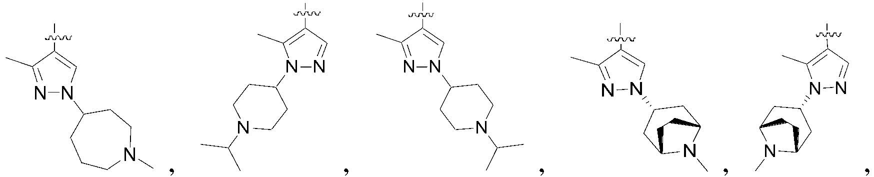 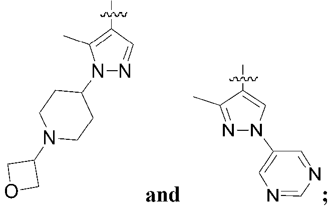 R² is selected from the group consisting of hydrogen, C1-C6 alkyl, C3-C6 cycloalkyl, and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 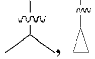 and trifluoromethyl;
R³ is selected from the group consisting of:
1) 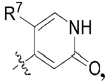 wherein:
R⁷ is selected from the group consisting of hydrogen and C1-C6 alkyl;
preferably, R⁷ is selected from the group consisting of hydrogen and methyl;
2) 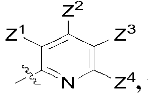 wherein:
Z¹, Z², Z³, and Z⁴ are each independently selected from the group consisting of hydrogen, cyano, halogen, nitro, and C1-C6 alkyl;
preferably, Z¹, Z², Z³, and Z⁴ are each independently selected from the group consisting of hydrogen and C1-C6 alkyl;
more preferably, among Z¹, Z², Z³, and Z⁴, any one is selected from the group consisting of C1-C6 alkyl, and the remainder are hydrogen;
further preferably, among Z¹, Z², Z³, and Z⁴, any one is methyl, and the remainder are hydrogen;
most preferably, 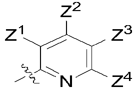 as a **whole,** is 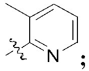
3) 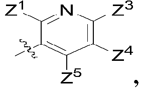 wherein:
Z¹, Z³, Z⁴, and Z⁵ are each independently selected from the group consisting of hydrogen, halogen, nitro, cyano, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
preferably, Z¹, Z³, Z⁴, and Z⁵ are each independently selected from the group consisting of hydrogen, halogen, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
more preferably, among Z¹, Z³, Z⁴, and Z⁵, any one is selected from the group consisting of C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl, and the remainder are hydrogen; or, among Z¹, Z³, Z⁴, and Z⁵, any two are selected from the group consisting of halogen, C1-C6 alkyl, and **R^{a}R^{b}N-C(=O)-**, wherein R^{a} and R^{b} are each independently selected from the group consisting of hydrogen and C1-C6 alkyl, and the remainder are hydrogen;
more preferably, Z¹, Z³, Z⁴, and Z⁵ are each independently selected from the group consisting of hydrogen, fluoro, methyl, difluoromethyl, cyclopropyl, 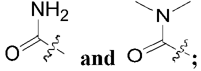
further preferably, among Z¹, Z³, Z⁴, and Z⁵, any one is selected from the group consisting of methyl, 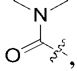 cyclopropyl, and difluoromethyl, and the remainder are hydrogen; or, among Z¹, Z³, Z⁴, and Z⁵, any two are independently selected from the group consisting of fluoro, methyl and 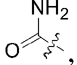 and the remainder are hydrogen;
most preferably, 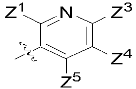 as a whole, is selected from the group consisting of 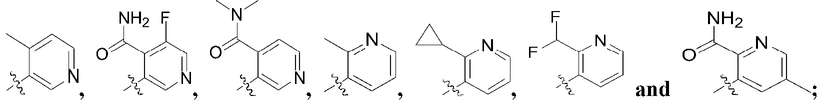
4) 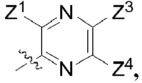 wherein:
Z¹, Z³, and Z⁴ are each independently selected from the group consisting of hydrogen, cyano, halogen, nitro, and C1-C6 alkyl;
preferably, **Z¹,** Z³, and Z⁴ are each independently selected from the group consisting of hydrogen and C1-C6 alkyl;
more preferably, among Z¹, Z³, and Z⁴, any one is selected from the group consisting of C1-C6 alkyl, and the remainder are hydrogen;
further preferably, among Z¹, Z³, and Z⁴, any one is methyl, and the remainder are hydrogen;
most preferably, 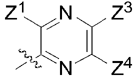 as a **whole,** is 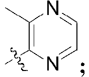
5) 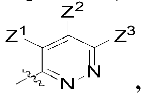 **wherein:**
Z¹, Z², and Z³ are each independently selected from the group consisting of hydrogen, cyano, halogen, nitro, and C1-C6 alkyl;
preferably, Z¹, Z², and Z³ are each independently selected from the group consisting of hydrogen and C1-C6 alkyl;
more preferably, among Z¹, Z², and Z³, any one is selected from the group consisting of C1-C6 alkyl, and the remainder are hydrogen;
further preferably, among Z¹, Z², and Z³, any one is methyl, and the remainder are hydrogen;
most preferably, 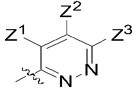 as a **whole,** is 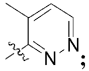
6) 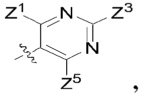 **wherein:**
Z¹, Z³, and Z⁵ are each independently selected from the group consisting of hydrogen, cyano, halogen, nitro, and C1-C6 alkyl;
preferably, Z¹, Z³, and Z⁵ are each independently selected from the group consisting of hydrogen and C1-C6 alkyl;
more preferably, among Z¹, Z³, and Z⁵, any one is selected from the group consisting of C1-C6 alkyl, and the remainder are hydrogen; or, among Z¹, Z³, and Z⁵, any two are selected from the group consisting of C1-C6 alkyl, and the remainder is hydrogen;
more preferably, Z¹, Z³, and Z⁵ are each independently selected from the group consisting of hydrogen and methyl;
further preferably, among Z¹, Z³, and Z⁵, any one is methyl and the remainder are hydrogen; or, among Z¹, Z³, and Z⁵, any two are methyl and the remainder is hydrogen;
most preferably, 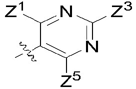 as a whole, is selected from the group consisting of 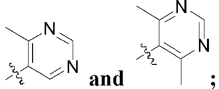
7) 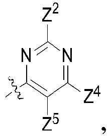 wherein:
Z², Z⁴, and Z⁵ are each independently selected from the group consisting of hydrogen, cyano, halogen, nitro, and C1-C6 alkyl;
preferably, Z², Z⁴, and Z⁵ are each independently selected from the group consisting of hydrogen and C1-C6 alkyl;
more preferably, among Z², Z⁴, and Z⁵, any one is selected from the group consisting of C1-C6 alkyl, and the remainder are hydrogen;
further preferably, among Z², Z⁴, and Z⁵, any one is methyl, and the remainder are hydrogen;
most preferably, 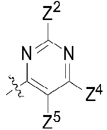 as a whole, is 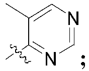
8) 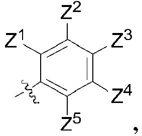 wherein:
Z¹, Z², Z³, Z⁴, and Z⁵ are each independently selected from the group consisting of hydrogen, cyano, halogen, nitro, and C1-C6 alkyl;
preferably, Z¹, Z², Z³, Z⁴, and Z⁵ are each independently selected from the group consisting of hydrogen, cyano, and C1-C6 alkyl;
more preferably, among Z¹, Z², Z³, Z⁴, and Z⁵, any two are independently selected from the group consisting of cyano and C1-C6 alkyl, and the remainder are hydrogen;
further preferably, among Z¹, Z², Z³, Z⁴, and Z⁵, any two are independently selected from the group consisting of cyano and methyl, and the remainder are hydrogen;
most preferably, 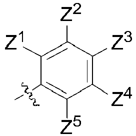
as a whole, is 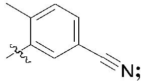 and
9) 5- to 9-membered heteroaryl, wherein the 5- to 9-membered heteroaryl is optionally substituted by 1 or 2 substituent(s) selected from the group consisting of C1-C6 alkyl and cyano; preferably, the 5- to 9-membered heteroaryl is selected from the group consisting of 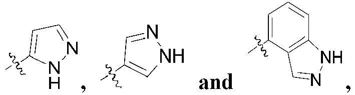 wherein the 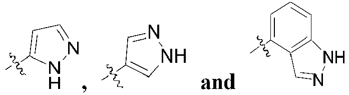 are each independently optionally substituted by 1 or 2 substituent(s) selected from the group consisting of C1-C6 alkyl and cyano; more preferably, the 5- to 9-membered heteroaryl selected from the group consisting of 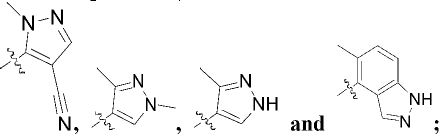
preferably, R³ is selected from the group consisting of 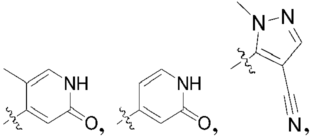 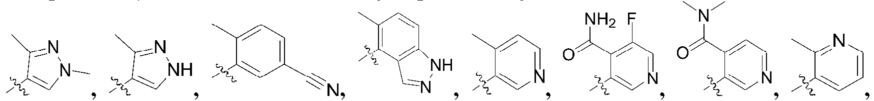 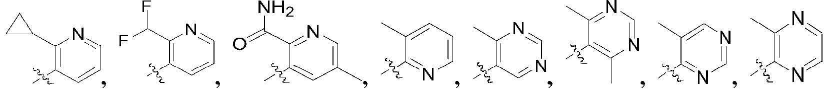
and

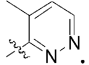

5. The compound or a stereoisomer thereof, a prodrug thereof, a pharmaceutically acceptable salt thereof, or a pharmaceutically acceptable solvate thereof according to claim 4, wherein the compound has the structure of formula III-1, 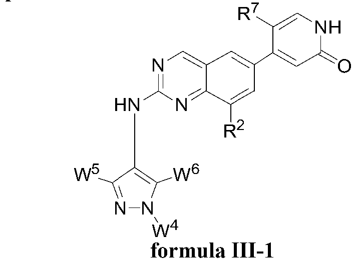 wherein:
W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, R^{e}R^{d}N-C(=O)-C1-C6 alkylene, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene, and 8- to 10-membered saturated bridged heterocyclyl, wherein the 3- to 7-membered saturated heterocyclyl, the 5-to 6-membered heteroaryl, and the 8- to 10-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 3- to 6-membered saturated heterocyclyl, and the C1-C6 alkylene is optionally substituted by hydroxy;
preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, R^{e}R^{d}N-C(=O)-C1-C6 alkylene, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene, and 8-membered saturated bridged heterocyclyl, wherein the 3- to 7-membered saturated heterocyclyl and the 8-membered saturated bridged heterocyclyl are each independently optionally substituted by 1 or 2 substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl, and the C1-C6 alkylene is optionally substituted by hydroxy;
more preferably, W⁴ is selected from the group consisting of hydrogen, C1-C6 alkyl, C1-C6 haloalkyl, deuterated C1-C6 alkyl, C1-C6 alkoxy-C1-C6 alkylene, C1-C6 alkoxy-C1-C6 alkylene-O-C1-C6 alkylene, R^{c}R^{d}N-C1-C6 alkylene, R^{e}R^{d}N-C(=O)-C1-C6 alkylene, 3- to 7-membered saturated heterocyclyl, 3- to 7-membered saturated heterocyclyl-C1-C6 alkylene, 5- to 6-membered heteroaryl, 5- to 6-membered heteroaryl-C1-C6 alkylene and 8-membered saturated bridged heterocyclyl, and the 3- to 7-membered saturated heterocyclyl, the 8-membered saturated bridged heterocyclyl are each independently optionally substituted by 1 or 2 (preferably 1) substituent(s) selected from the group consisting of C1-C6 alkyl, deuterated C1-C6 alkyl, C1-C6 alkoxy-C1-C6 alkylene, C1-C6 alkoxy-C(=O)-, hydroxy-C1-C6 alkylene-C(=O)-, and 4-membered saturated heterocyclyl (preferably 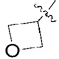 ), the C1-C6 alkylene is optionally substituted by hydroxy, the 3- to 7-membered saturated heterocyclyl is selected from the group consisting 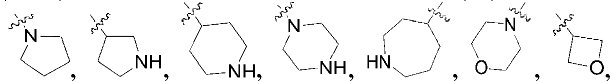 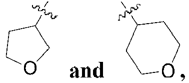 the 5- to 6-membered heteroaryl is selected from the group consisting of 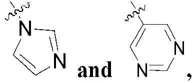 and the 8-membered bridged heterocyclyl is 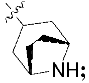
R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 3- to 6-membered saturated heterocyclyl; or, R^{c}, R^{d}, and the N atom to which they are both attached together form a 3- to 6-membered saturated heterocyclic ring, wherein the 3- to 6-membered saturated heterocyclic ring is optionally substituted by 1 or 2 substituent(s) selected from the group consisting of C1-C6 alkyl and halogen;
preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O)-, C2-C6 alkynyl-C1-C6 alkylene, C1-C6 haloalkyl and 5-membered saturated heterocyclyl; or, R^{c}**,** R^{d}**,** and the N atom to which they are both attached together form a 4- to 5-membered saturated heterocyclic ring, wherein the 4- to 5-membered saturated heterocyclic ring is optionally substituted by 1 or 2 substituent(s) selected from the group consisting of C1-C6 alkyl and halogen;
more preferably, R^{c} and R^{d} are each independently selected from the group consisting of hydrogen, C1-C6 alkyl, C3-C6 cycloalkyl, C1-C6 alkoxy-C1-C6 alkylene, hydroxy-substituted C1-C6 alkyl, C1-C6 alkyl-C(=O) -, C2-C6 alkynyl-C1-C6 alkylene, C1-C6 haloalkyl and 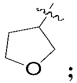 or, R^{c}**,** R^{d}**,** and the N atom to which they are both attached together form 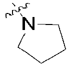 or 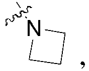 and the 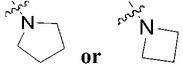 is independently optionally substituted by 1 or 2 substituent(s) selected from the group consisting of C1-C6 alkyl and halogen;
most preferably, R^{c}**,** R^{d} are each independently selected from the group consisting of hydrogen, methyl, ethyl, isopropyl, 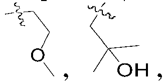 acetyl, 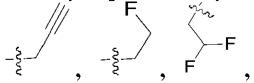 cyclopropyl, cyclobutyl and 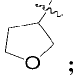 or**,** R^{c}**,** R^{d}**,** and the N atom to which they are both attached together form 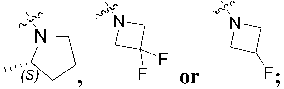
most preferably, W⁴ is selected from the group consisting of hydrogen, methyl, ethyl, difluoromethyl, trideuteromethyl, 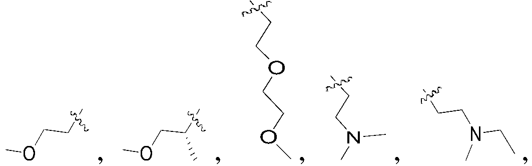 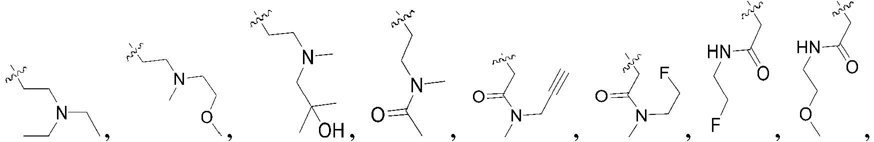 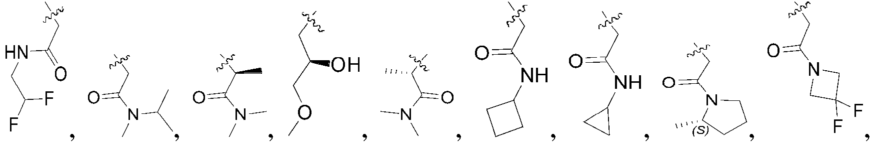 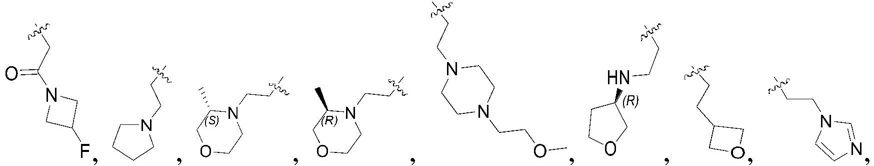 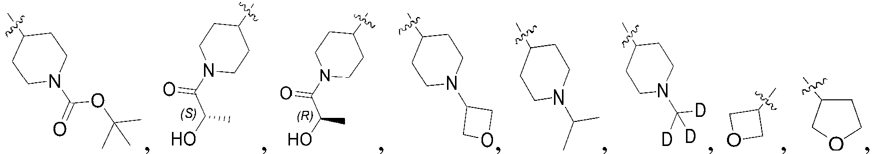 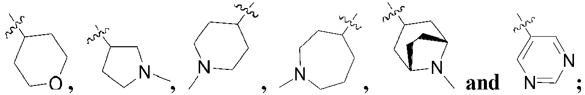
W⁵ and W⁶ are each independently selected from the group consisting of hydrogen, halogen, C1-C6 alkyl, C1-C6 alkoxy, and C1-C6 haloalkyl, wherein W⁵ and W⁶ are not hydrogen at the same time;
preferably, any one of W⁵ and W⁶ is selected from the group consisting of halogen, C1-C6 alkyl, C1-C6 alkoxy, and C1-C6 haloalkyl, and the other is hydrogen;
more preferably, any one of W⁵ and W⁶ is selected from the group consisting of chloro, methyl, ethyl, methoxy, difluoromethyl, and trifluoromethyl, and the other is hydrogen;
most preferably, 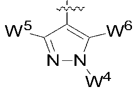 as a whole, is selected from the group consisting of 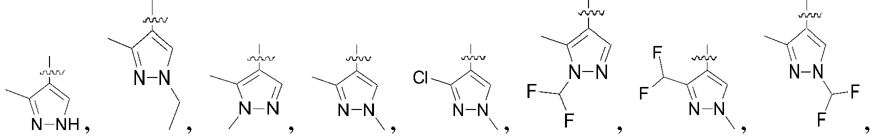 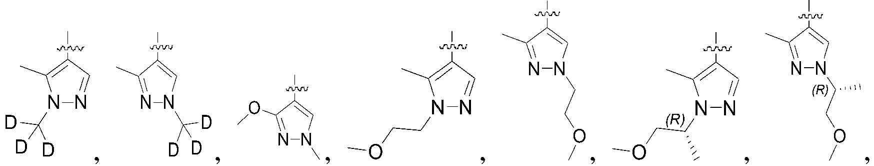 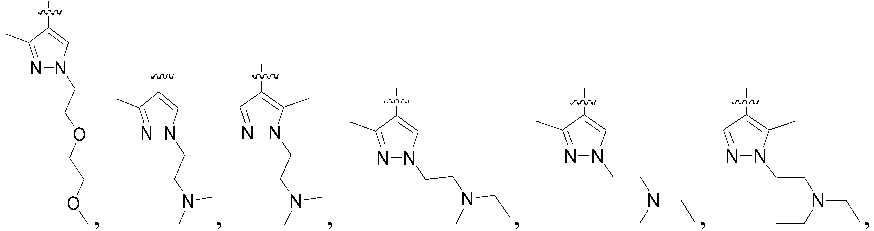 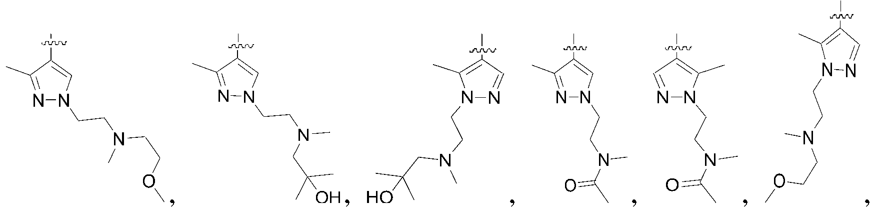 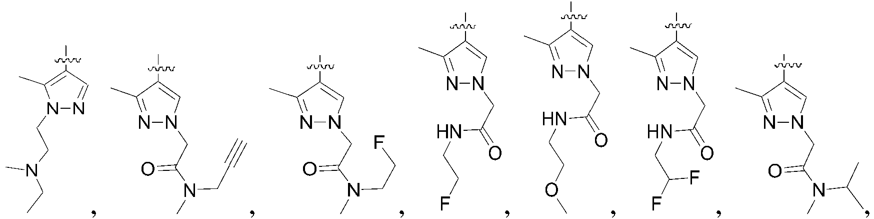 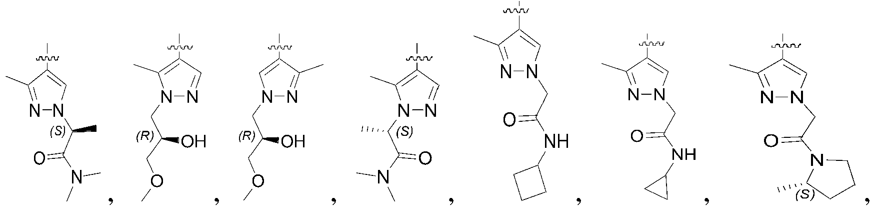 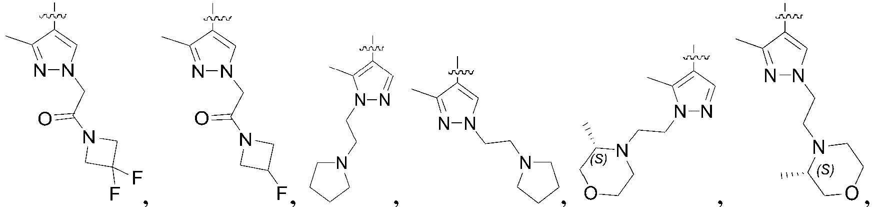 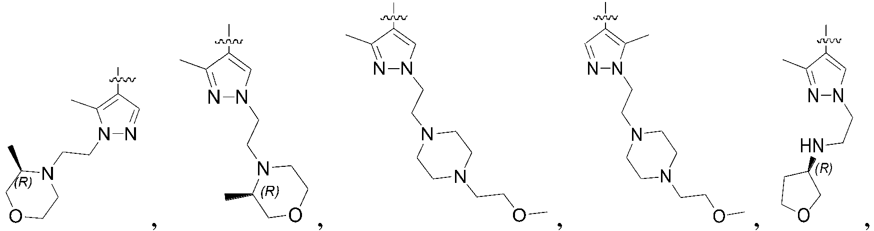 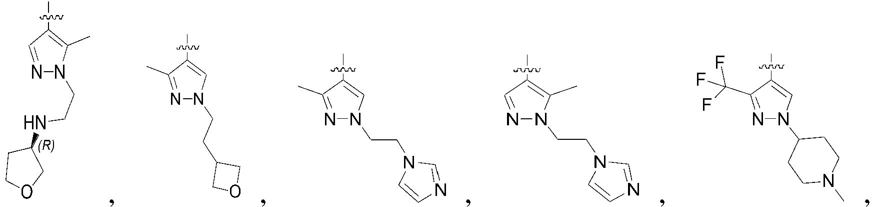 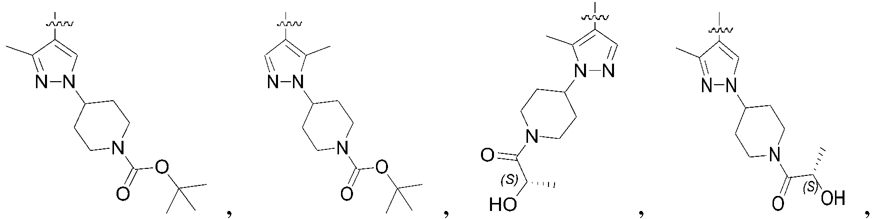 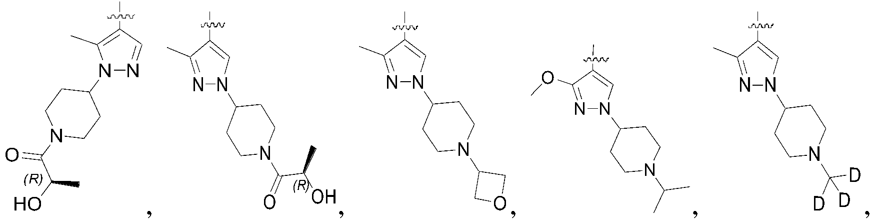 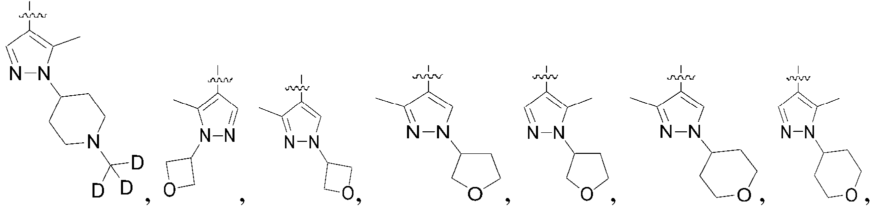 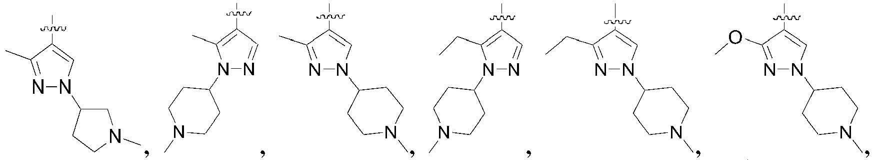 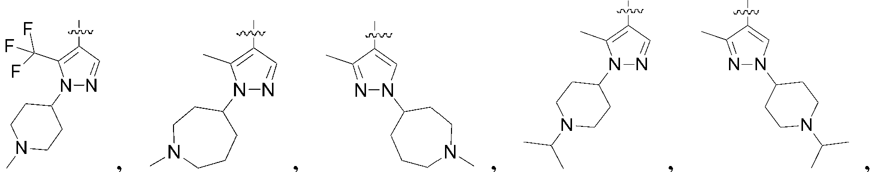 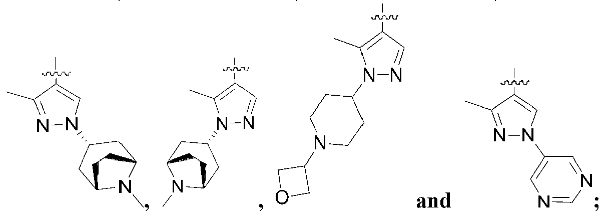 R² is selected from the group consisting of hydrogen, C1-C6 alkyl, C3-C6 cycloalkyl, and C1-C6 haloalkyl;
preferably, R² is selected from the group consisting of C1-C6 alkyl and C3-C6 cycloalkyl;
more preferably, R² is selected from the group consisting of methyl, ethyl, 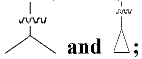
R⁷ is selected from the group consisting of hydrogen and C1-C6 alkyl;
preferably, R⁷ is selected from the group consisting of hydrogen and methyl.

6. The compound or a stereoisomer thereof, a prodrug thereof, a pharmaceutically acceptable salt thereof, or a pharmaceutically acceptable solvate thereof according to claim 4, wherein the compound has the structure of formula III-2, 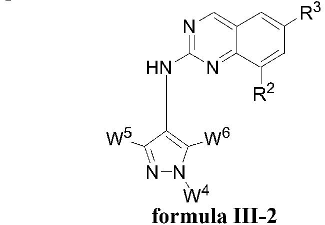 wherein:
W⁴ is selected from the group consisting of C1-C6 alkyl, deuterated C1-C6 alkyl, C1-C6 alkoxy-C1-C6 alkylene, 3- to 6-membered saturated heterocyclyl, O=S(R^{e}R^{f})=N-C1-C6 alkylene, R^{c}R^{d}N-C1-C6 alkylene, R^{e}R^{d}N-C(=O)-C1-C6 alkylene, and C3-C6 cycloalkyl-SO₂-, wherein the 3- to 6-membered saturated heterocyclyl is optionally substituted by C1-C6 alkyl;
preferably, W⁴ is selected from the group consisting of C1-C6 alkyl, deuterated C1-C6 alkyl, C1-C6 alkoxy-C1-C6 alkylene, 4- to 6-membered saturated heterocyclyl, O=S(R^{e}R^{f})=N-C1-C6 alkylene, R^{c}R^{d}N-C1-C6 alkylene, R^{e}R^{d}N-C(=O)-C1-C6 alkylene, and C3-C6 cycloalkyl-SO₂-, wherein the 4- to 6-membered saturated heterocyclyl is optionally substituted by C1-C6 alkyl;
more preferably, W⁴ is selected from the group consisting of C1-C6 alkyl, deuterated C1-C6 alkyl, C1-C6 alkoxy-C1-C6 alkylene, 4- to 6-membered saturated heterocyclyl, O=S(R^{e}R^{f})=N-C1-C6 alkylene, R^{c}R^{d}N-C1-C6 alkylene, R^{e}R^{d}N-C(=O)-C1-C6 alkylene, and C3-C6 cycloalkyl-SO₂-, wherein the 4- to 6-membered saturated heterocyclyl is optionally substituted by C1-C6 alkyl, and the 4- to 6-membered saturated heterocyclyl is selected from the group consisting of 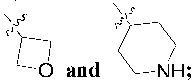
R^{e} and R^{d} are each independently selected from the group consisting of hydrogen, C1-C6 alkyl, and C1-C6 alkoxy-C1-C6 alkylene;
preferably, R^{c} and R^{d} are each independently selected from the group consisting of hydrogen, methyl, ethyl and 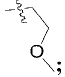
R^{e} and R^{f} are each independently selected from the group consisting of hydrogen and C1-C6 alkyl;
preferably, R^{e} and R^{f} are each independently selected from the group consisting of C1-C6 alkyl;
more preferably, R^{e} and R^{f} are methyl;
further preferably, W⁴ is selected from the group consisting of methyl, trideuteromethyl, 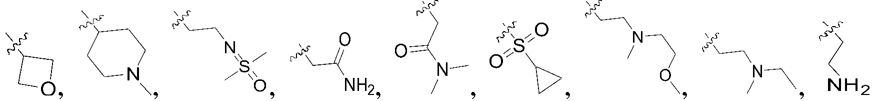 and 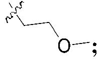
most preferably, W⁴ is selected from the group consisting of methyl, trideuteromethyl, 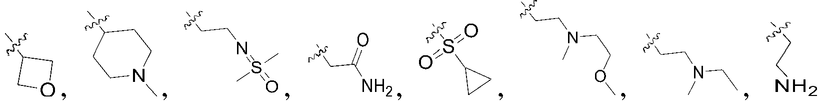
and 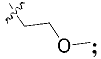
W⁵, W⁶ are each independently selected from the group consisting of hydrogen, halogen, C1-C6 alkyl and C1-C6 haloalkyl, and W⁵ and W⁶ are not hydrogen at the same time;
preferably, any one of W⁵ and W⁶ is selected from the group consisting of hydrogen, halogen, C1-C6 alkyl, and C1-C6 haloalkyl, and the other is hydrogen;
preferably, any one **of W⁵** and W⁶ is selected from the group consisting of halogen, C1-C6 alkyl, and C1-C6 haloalkyl, and the other is hydrogen;
more preferably, any one of W⁵ and W⁶ is selected from the group consisting of hydrogen, chloro, methyl, and difluoromethyl, and the other is hydrogen;
more preferably, any one of W⁵ and W⁶ is selected from the group consisting of chloro, methyl, and difluoromethyl, and the other is hydrogen;
further preferably, 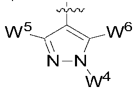 as a whole, is selected from the group consisting of 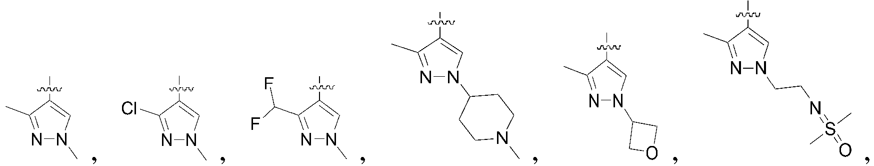 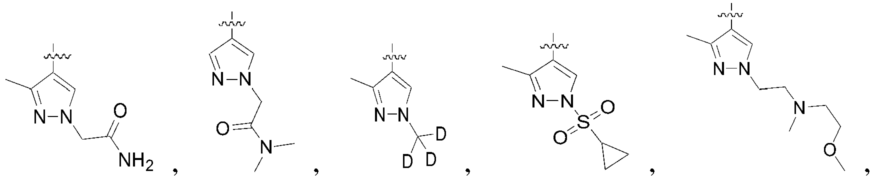 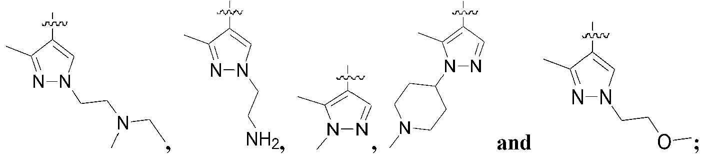
most preferably, 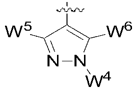 as a whole, is selected from the group consisting of 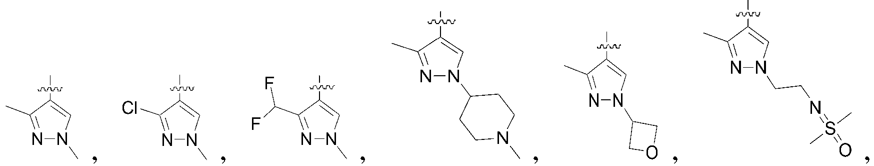 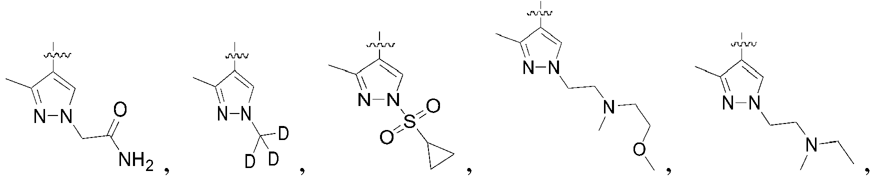 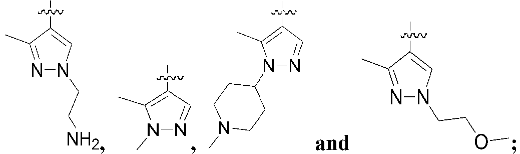
R² is selected from the group consisting of hydrogen, C1-C6 alkyl, C3-C6 cycloalkyl, and C1-C6 haloalkyl;
preferably, R² is selected from the group consisting of C1-C6 alkyl, C3-C6 cycloalkyl, and C1-C6 haloalkyl;
more preferably, R² is selected from the group consisting of methyl, ethyl, n-propyl, 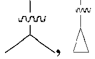
and trifluoromethyl;
R³ is selected from the group consisting of:
1) 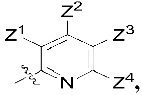 wherein:
Z¹, Z², Z³**,** and Z⁴ are each independently selected from the group consisting of hydrogen, cyano, halogen, nitro, and C1-C6 alkyl;
preferably, Z¹, Z², Z³, and Z⁴ are each independently selected from the group consisting of hydrogen and C1-C6 alkyl;
more preferably, among Z¹, Z², Z³, and Z⁴, any one is selected from the group consisting of C1-C6 alkyl, and the remainder are hydrogen;
further preferably, among Z¹, Z², Z³, and Z⁴, any one is methyl, and the remainder are hydrogen;
most preferably, 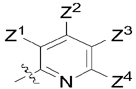 as a whole, is 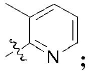
2) 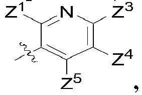 wherein:
Z¹, Z³, Z⁴, and Z⁵ are each independently selected from the group consisting of hydrogen, halogen, nitro, cyano, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
preferably, Z¹, Z³, Z⁴, and Z⁵ are each independently selected from the group consisting of hydrogen, halogen, C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl;
more preferably, among Z¹, Z³, Z⁴, and Z⁵, any one is selected from the group consisting of C1-C6 alkyl, C1-C6 haloalkyl, C3-C6 cycloalkyl, and R^{a}R^{b}N-C(=O)-, wherein R^{a} and R^{b} are each independently selected from the group consisting of hydrogen and C1-C6 alkyl, and the remainder are hydrogen; or, among Z¹, Z³, Z⁴, and Z⁵, any two are selected from the group consisting of halogen, C1-C6 alkyl, and R^{a}R^{b}N-C(=O)-, wherein R^{a} and R^{b} are each independently selected from the group consisting of hydrogen and C1-C6 alkyl, and the remainder are hydrogen;
more preferably, Z¹, Z³, Z⁴, and Z⁵ are each independently selected from the group consisting of hydrogen, fluoro, methyl, difluoromethyl, cyclopropyl, 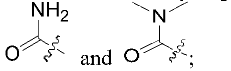
further preferably, among Z¹, Z³, Z⁴, and Z⁵, any one is selected from the group consisting of methyl, 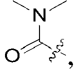 cyclopropyl, and difluoromethyl, and the remainder are hydrogen; or, among Z¹, Z³, Z⁴, and Z⁵, any two are selected from the group consisting of fluoro, methyl and 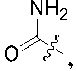 and the remainder are hydrogen;
most preferably, 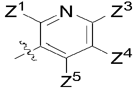 as a whole, is selected from the group consisting of 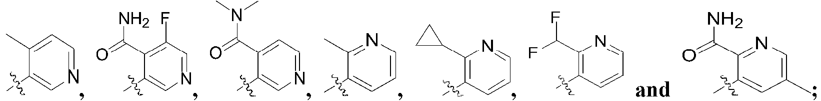
3) 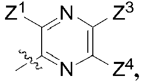 wherein:
Z¹, Z³, and Z⁴ are each independently selected from the group consisting of hydrogen, cyano, halogen, nitro, and C1-C6 alkyl;
preferably, Z¹, Z³, and Z⁴ are each independently selected from the group consisting of hydrogen and C1-C6 alkyl;
more preferably, among Z¹, Z³, and Z⁴, any one is selected from the group consisting of C1-C6 alkyl, and the remainder are hydrogen;
further preferably, among Z¹, Z³, and Z⁴, any one is methyl, and the remainder are hydrogen;
most preferably, 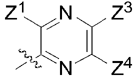 as a whole, is 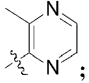
4) 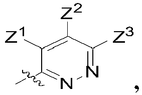 wherein:
Z¹, Z², and Z³ are each independently selected from the group consisting of hydrogen, cyano, halogen, nitro, and C1-C6 alkyl;
preferably, Z¹, Z², and Z³ are each independently selected from the group consisting of hydrogen and C1-C6 alkyl;
more preferably, among Z¹, Z², and Z³, any one is selected from the group consisting of C1-C6 alkyl, and the remainder are hydrogen;
further preferably, among Z¹, Z², and Z³, any one is methyl, and the remainder are hydrogen;
most preferably, 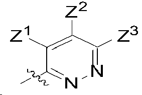 as a whole, is 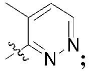
5) 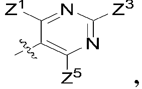 wherein:
Z¹, Z³, and Z⁵ are each independently selected from the group consisting of hydrogen, cyano, halogen, nitro, and C1-C6 alkyl;
preferably, Z¹, Z³, and Z⁵ are each independently selected from the group consisting of hydrogen and C1-C6 alkyl;
more preferably, among Z¹, Z³, and Z⁵, any one is selected from the group consisting of C1-C6 alkyl, and the remainder are hydrogen; or, among Z¹, Z³, and Z⁵, any two are selected from the group consisting of C1-C6 alkyl, and the remainder is hydrogen;
more preferably, Z¹, Z³, and Z⁵ are each independently selected from the group consisting of hydrogen and methyl.
further preferably, among Z¹, Z³, and Z⁵, any one is methyl, and the remainder are hydrogen; or, among Z¹, Z³, and Z⁵, any two are methyl, and the remainder is hydrogen;
most preferably, 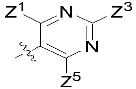 as a whole, is selected from the group consisting of 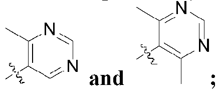
6) 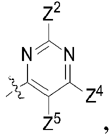 wherein:
Z², Z⁴, and Z⁵ are each independently selected from the group consisting of hydrogen, cyano, halogen, nitro, and C1-C6 alkyl;
preferably, Z², Z⁴, and Z⁵ are each independently selected from the group consisting of hydrogen and C1-C6 alkyl;
more preferably, among Z², Z⁴, and Z⁵, any one is selected from the group consisting of C1-C6 alkyl, and the remainder are hydrogen;
further preferably, among Z², Z⁴, and Z⁵, any one is methyl, and the remainder are hydrogen;
most preferably, 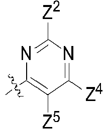 as a whole, is 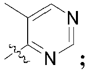
7) 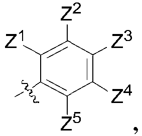 wherein:
Z¹, Z², Z³, Z⁴, and Z⁵ are each independently selected from the group consisting of hydrogen, cyano, halogen, nitro, and C1-C6 alkyl;
preferably, Z¹, Z², Z³, Z⁴, and Z⁵ are each independently selected from the group consisting of hydrogen, cyano, and C1-C6 alkyl;
more preferably, among Z¹, Z², Z³, Z⁴, and Z⁵, any two are independently selected from the group consisting of cyano and C1-C6 alkyl, and the remainder are hydrogen;
further preferably, among Z¹, Z², Z³, Z⁴, and Z⁵, any two are selected from the group consisting of cyano and methyl, and the remainder are hydrogen;
most preferably, 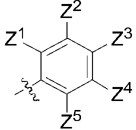 as a whole, is 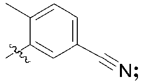 and
8) 5- to 9-membered heteroaryl, and the 5- to 9-membered heteroaryl is optionally substituted by 1 or 2 substituent(s) selected from the group consisting of C1-C6 alkyl and cyano; preferably, the 5- to 9-membered heteroaryl is selected from the group consisting of 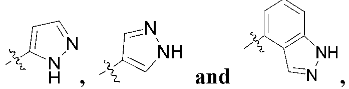 and the 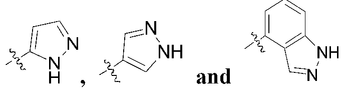 are each independently optionally substituted by 1 or 2 substituent(s) selected from the group consisting of C1-C6 alkyl and cyano; more preferably, the 5- to 9-membered heteroaryl is selected from the group consisting of 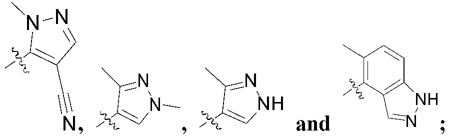
preferably, R³ is selected from the group consisting of

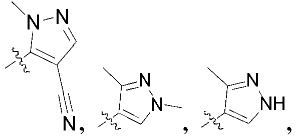

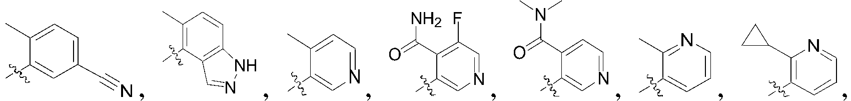

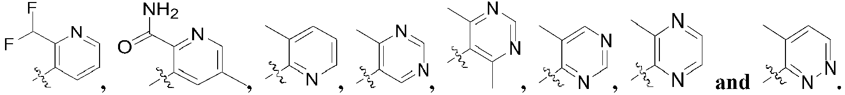

7. The compound or a stereoisomer thereof, a prodrug thereof, a pharmaceutically acceptable salt thereof, or a pharmaceutically acceptable solvate thereof according to any one of claims 1-6, wherein the compound is selected from the group consisting of:
| **No.** | **Structure** | **No.** | **Structure** |
|---|---|---|---|
| **I-1** | 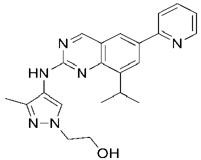 | **I-98** | 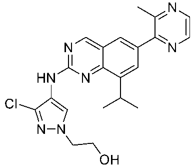 |
| **I-2** | 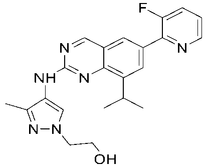 | **I-99** | 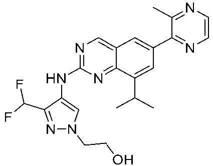 |
| **I-3** | 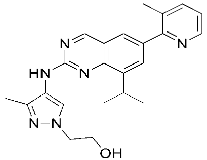 | **I-100** | 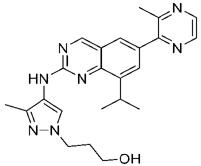 |
| **I-4** | 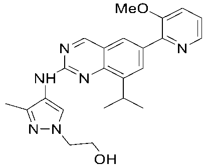 | **I-101** | 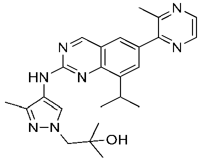 |
| **I-5** | 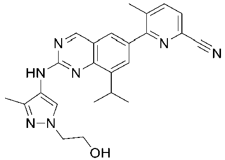 | **I-102** | 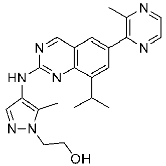 |
| **I-6** | 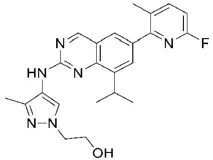 | **I-103** | 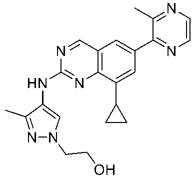 |
| **I-7** | 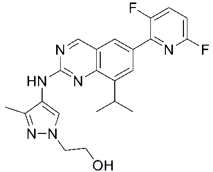 | **I-104** | 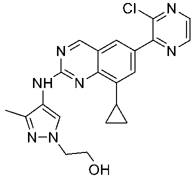 |
| **I-8** | 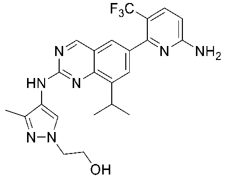 | **I-105** | 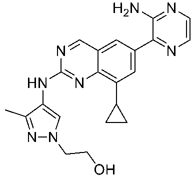 |
| **I-9** | 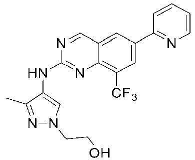 | **I-106** | 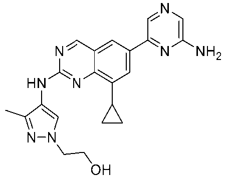 |
| **I-10** | 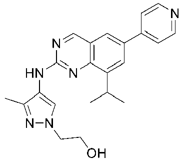 | **I-107** | 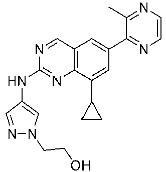 |
| **I-11** | 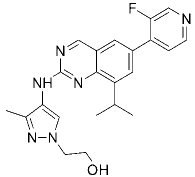 | **I-108** | 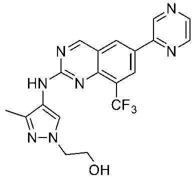 |
| **I-12** | 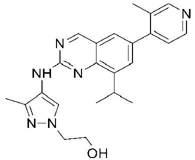 | **I-109** | 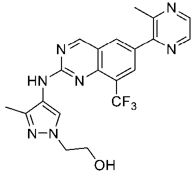 |
| **I-13** | 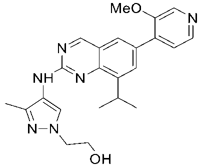 | **I-110** | 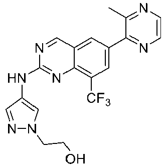 |
| **I-14** | 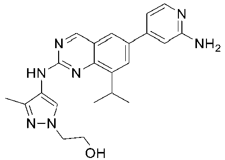 | **I-111** | 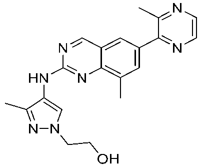 |
| **I-15** | 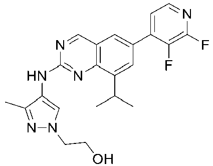 | **I-112** | 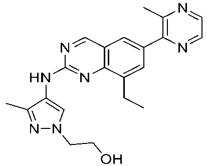 |
| **I-16** | 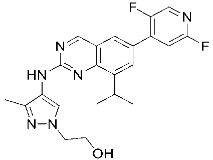 | **I-113** | 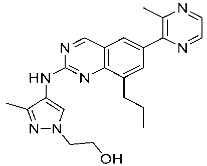 |
| **I-17** | 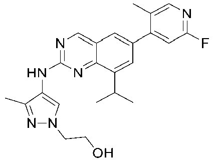 | **I-114** | 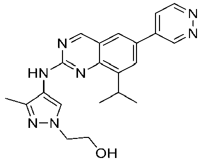 |
| **I-18** | 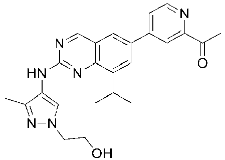 | **I-115** | 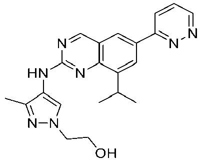 |
| **I-19** | 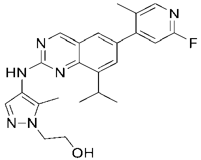 | **I-116** | 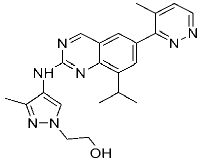 |
| **I-20** | 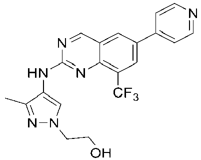 | **I-117** | 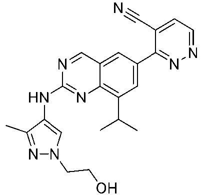 |
| **I-21** | 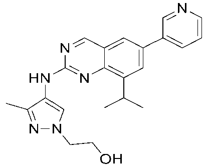 | **I-118** | 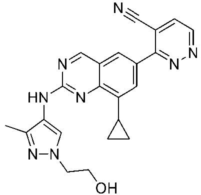 |
| **I-22** | 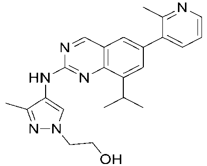 | **I-119** | 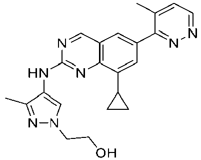 |
| **I-23** | 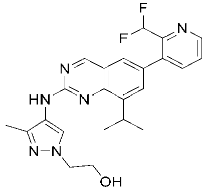 | **I-120** | 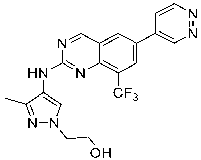 |
| **I-24** | 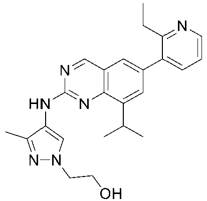 | **I-121** | 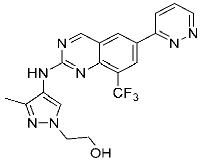 |
| **I-25** | 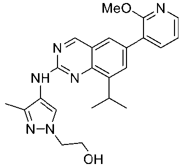 | **I-122** | 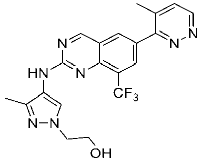 |
| **I-26** | 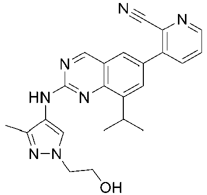 | **I-123** | 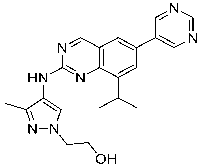 |
| **I-27** | 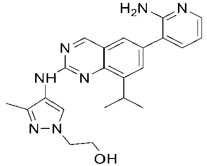 | **I-124** | 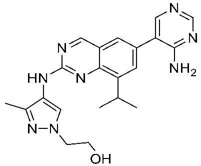 |
| **I-28** | 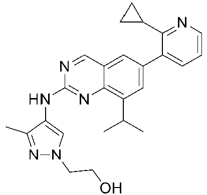 | **I-125** | 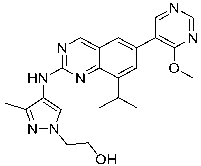 |
| **I-29** | 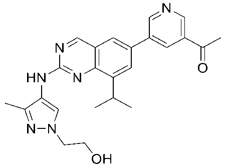 | **I-126** | 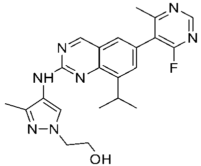 |
| **I-30** | 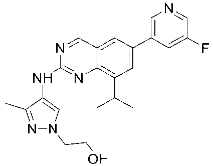 | **I-127** | 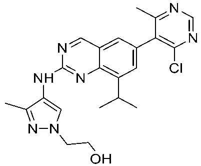 |
| **I-31** | 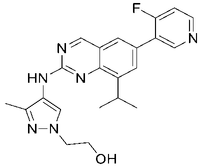 | **I-128** | 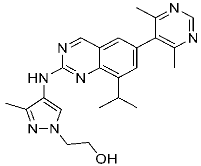 |
| **I-32** | 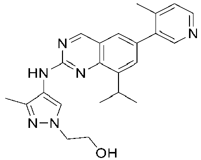 | **I-129** | 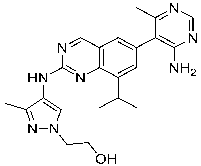 |
| **I-33** | 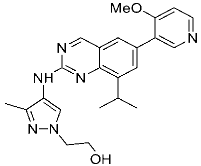 | **I-130** | 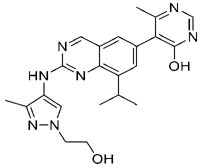 |
| **I-34** | 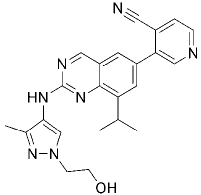 | **I-131** | 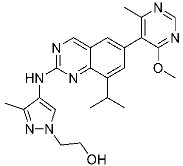 |
| **I-35** | 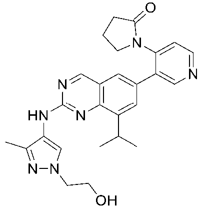 | **I-132** | 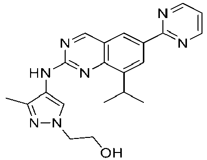 |
| **I-36** | 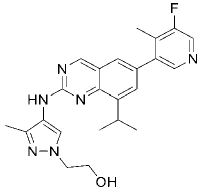 | **I-133** | 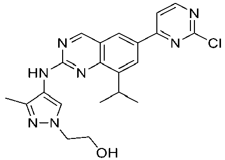 |
| **I-37** | 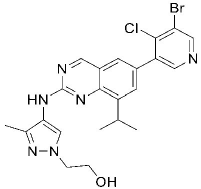 | **I-134** | 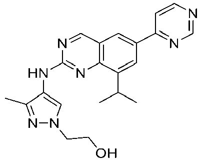 |
| **I-38** | 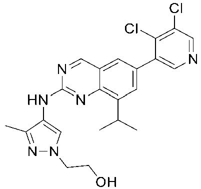 | **I-135** | 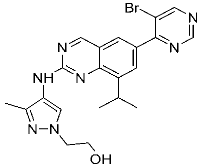 |
| **I-39** | 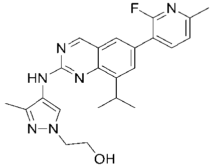 | **I-136** | 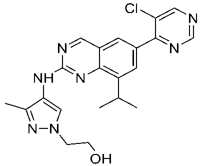 |
| **I-40** | 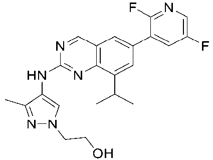 | **I-137** | 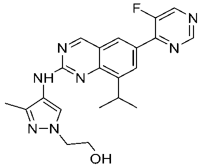 |
| **I-41** | 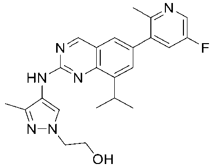 | **I-138** | 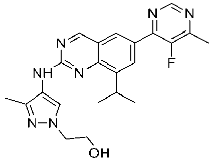 |
| **I-42** | 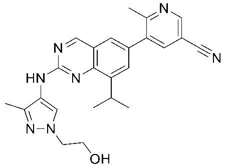 | **I-139** | 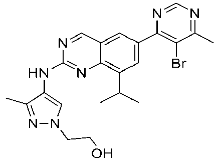 |
| **I-43** | 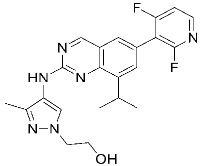 | **I-140** | 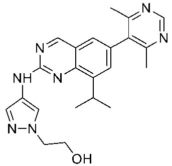 |
| **I-44** | 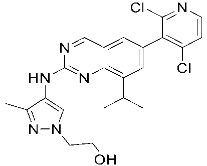 | **I-141** | 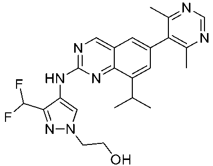 |
| **I-45** | 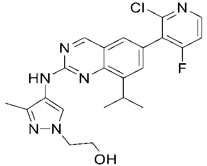 | **I-142** | 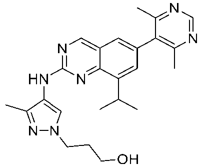 |
| **I-46** | 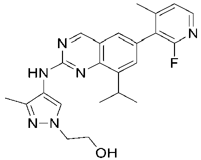 | **I-143** | 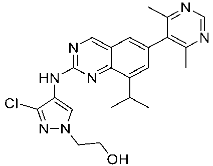 |
| **I-47** | 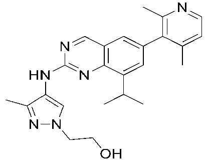 | **I-144** | 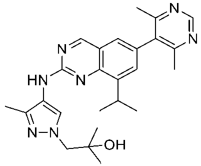 |
| **I-48** | 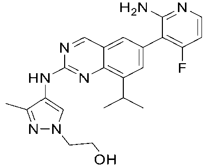 | **I-145** | 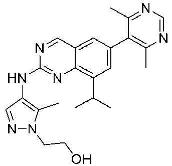 |
| **I-49** | 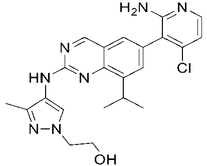 | **I-146** | 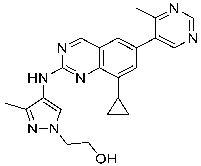 |
| **I-50** | 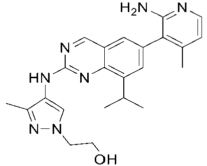 | **I-147** | 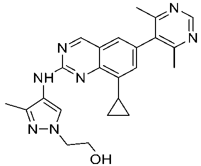 |
| **I-51** | 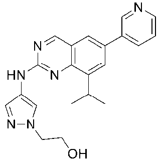 | **I-148** | 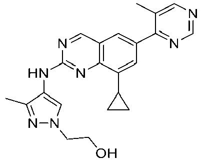 |
| **I-52** | 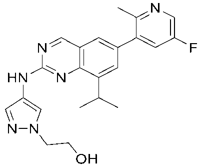 | **I-149** | 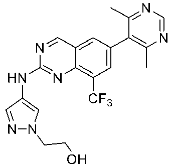 |
| **I-53** | 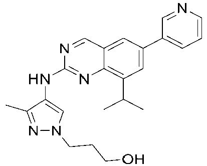 | **I-150** | 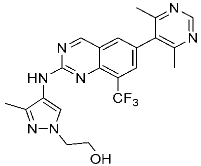 |
| **I-54** | 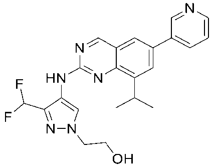 | **I-151** | 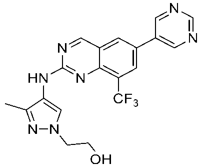 |
| **I-55** | 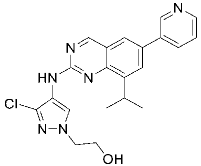 | **I-152** | 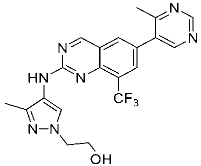 |
| **I-56** | 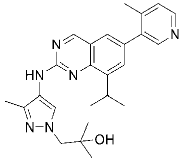 | **I-153** | 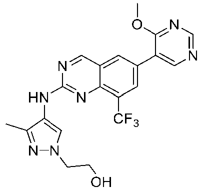 |
| **I-57** | 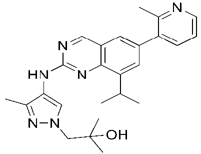 | **I-154** | 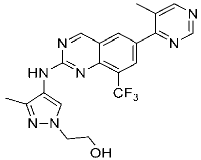 |
| **I-58** | 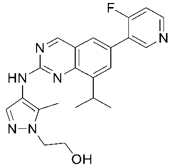 | **I-155** | 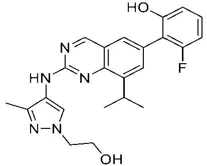 |
| **I-59** | 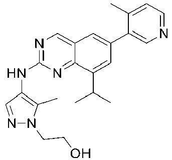 | **I-156** | 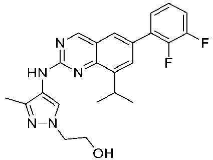 |
| **I-60** | 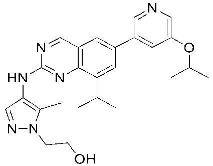 | **I-157** | 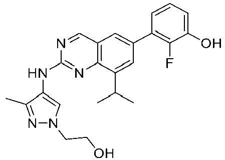 |
| **I-61** | 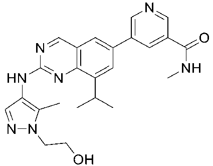 | **I-158** | 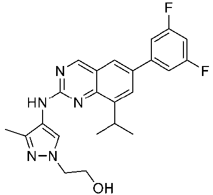 |
| **I-62** | 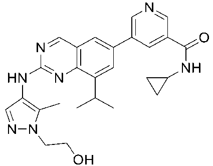 | **I-159** | 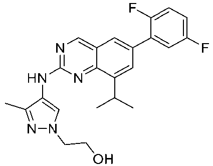 |
| **I-63** | 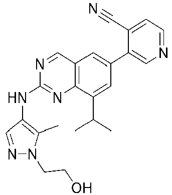 | **I-160** | 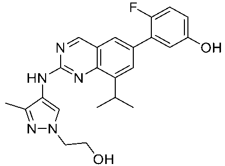 |
| **I-64** | 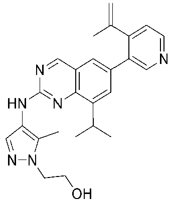 | **I-161** | 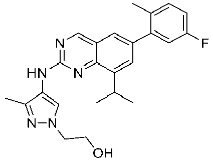 |
| **I-65** | 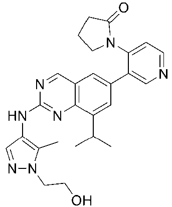 | **I-162** | 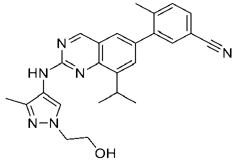 |
| **I-66** | 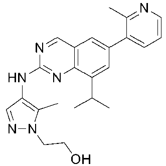 | **I-163** | 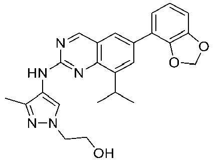 |
| **I-67** | 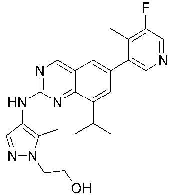 | **I-164** | 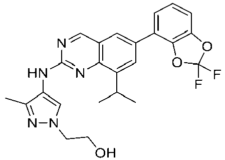 |
| **I-68** | 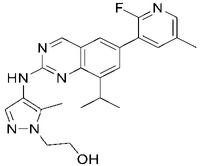 | **I-165** | 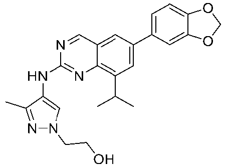 |
| **I-69** | 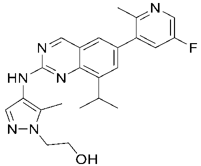 | **I-166** | 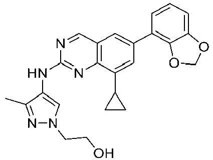 |
| **I-70** | 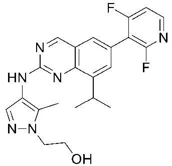 | **I-167** | 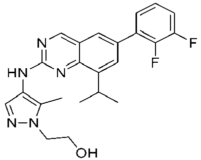 |
| **I-71** | 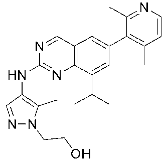 | **I-168** | 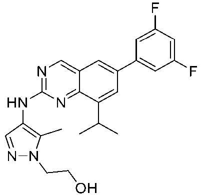 |
| **I-72** | 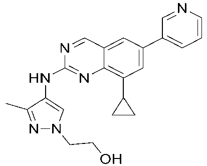 | **I-169** | 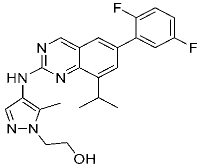 |
| **I-73** | 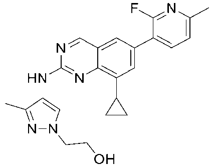 | **I-170** | 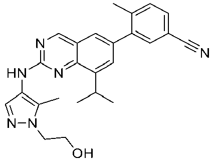 |
| **I-74** | 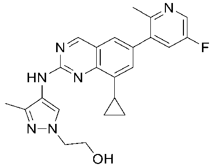 | **I-171** | 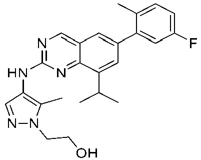 |
| **I-75** | 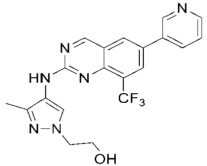 | **I-172** | 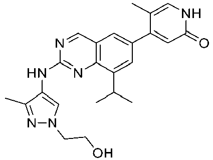 |
| **I-76** | 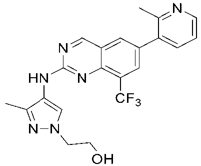 | **I-173** | 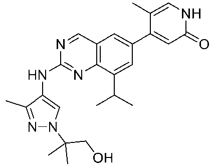 |
| **I-77** | 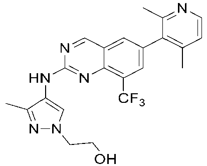 | **I-174** | 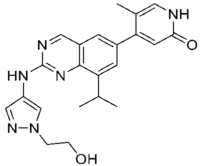 |
| **I-78** | 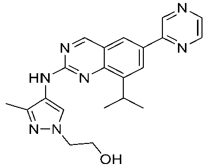 | **I-175** | 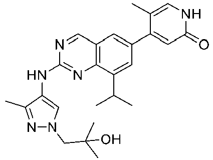 |
| **I-79** | 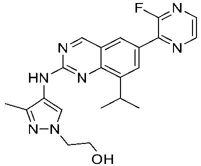 | **I-176** | 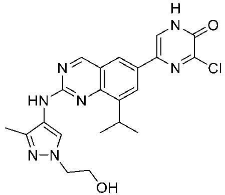 |
| **I-80** | 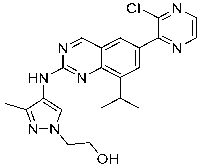 | **I-177** | 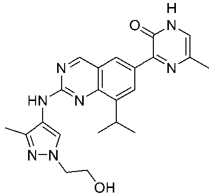 |
| **I-81** | 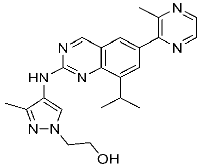 | **I-178** | 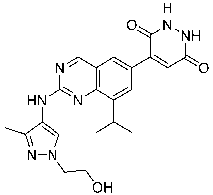 |
| **I-82** | 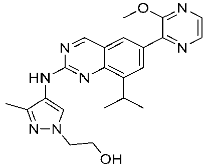 | **I-179** | 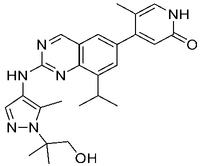 |
| **I-83** | 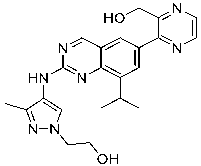 | **I-180** | 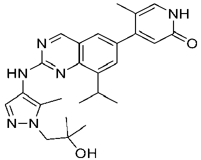 |
| **I-84** | 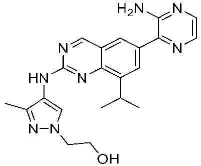 | **I-181** | 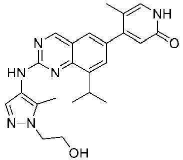 |
| **I-85** | 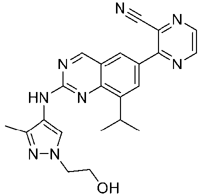 | **I-182** | 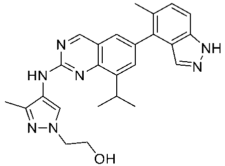 |
| **I-86** | 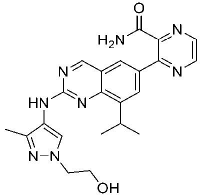 | **I-183** | 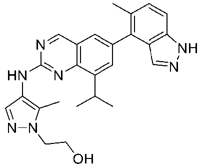 |
| **I-87** | 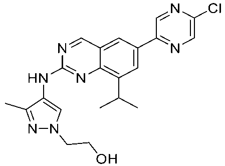 | **I-184** | 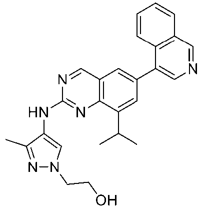 |
| **I-88** | 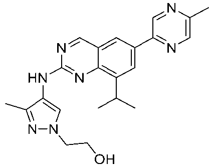 | **I-185** | 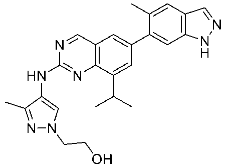 |
| **I-89** | 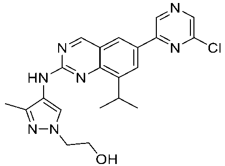 | **I-186** | 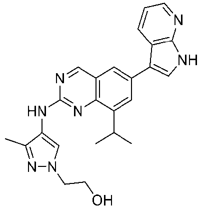 |
| **I-90** | 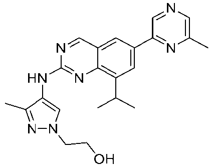 | **I-187** | 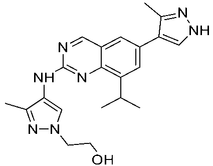 |
| **I-91** | 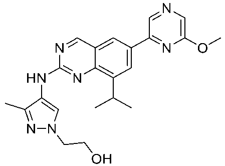 | **I-188** | 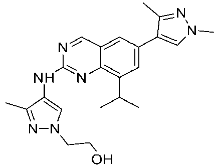 |
| **I-92** | 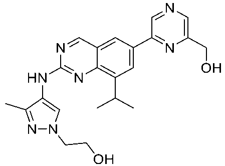 | **I-189** | 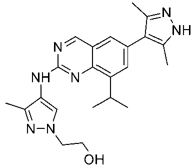 |
| **I-93** | 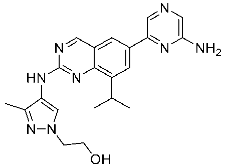 | **I-190** | 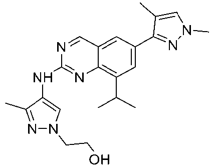 |
| **I-94** | 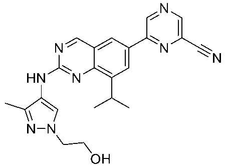 | **I-191** | 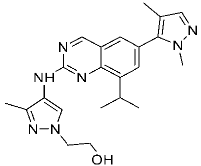 |
| **I-95** | 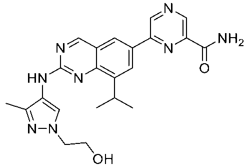 | **I-192** | 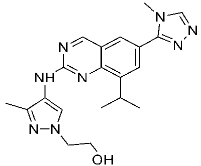 |
| **I-96** | 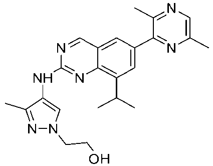 | **I-193** | 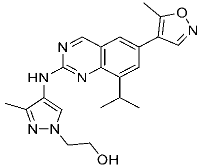 |
| **I-97** | 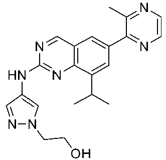 | | |
| **II-1** | 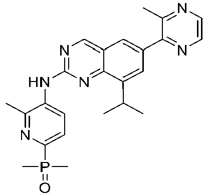 | **II-33** | 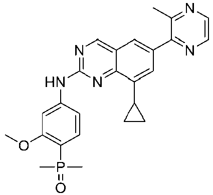 |
| **II-2** | 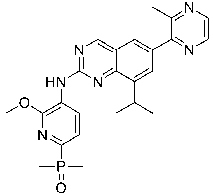 | **II-34** | 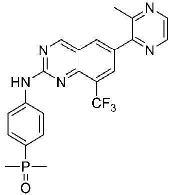 |
| **II-3** | 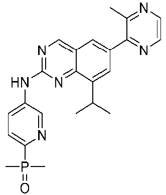 | **II-35** | 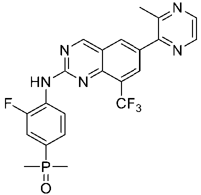 |
| **II-4** | 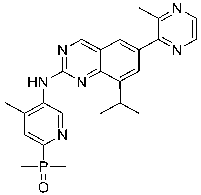 | **II-36** | 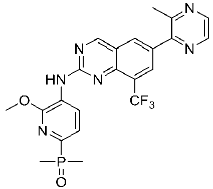 |
| **II-5** | 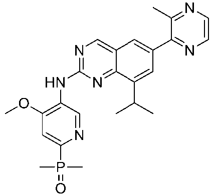 | **II-37** | 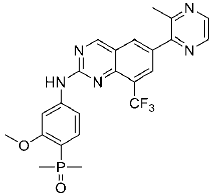 |
| **II-6** | 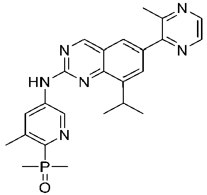 | **II-38** | 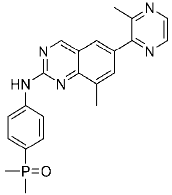 |
| **II-7** | 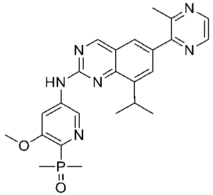 | **II-39** | 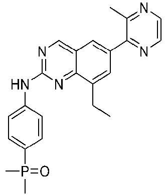 |
| **II-8** | 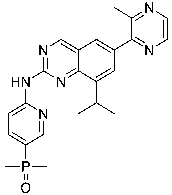 | **II-40** | 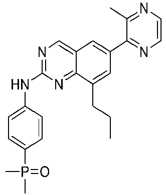 |
| **II-9** | 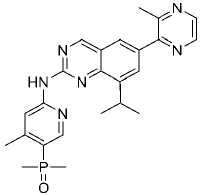 | **II-41** | 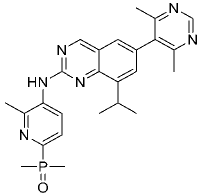 |
| **II-10** | 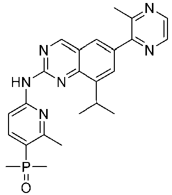 | **II-42** | 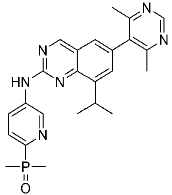 |
| **II-11** | 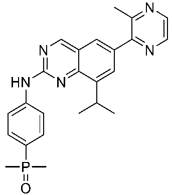 | **II-43** | 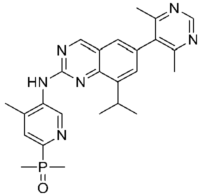 |
| **II-12** | 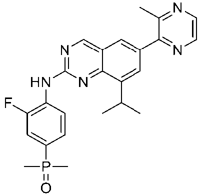 | **II-44** | 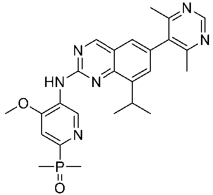 |
| **II-13** | 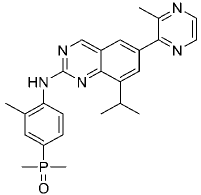 | **II-45** | 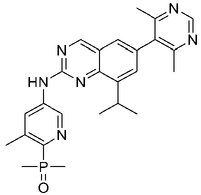 |
| **II-14** | 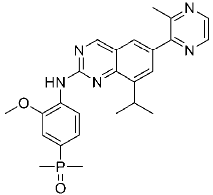 | **II-46** | 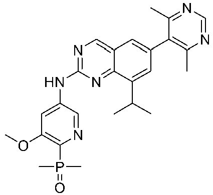 |
| **II-15** | 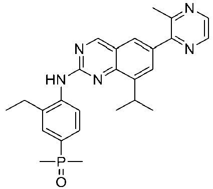 | **II-47** | 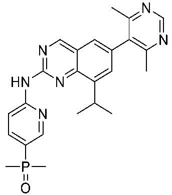 |
| **II-16** | 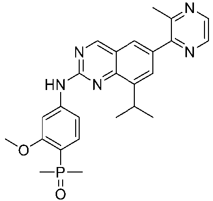 | **II-48** | 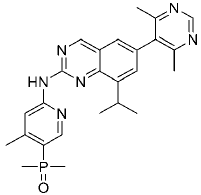 |
| **II-17** | 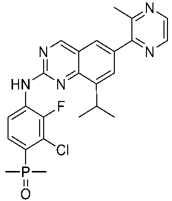 | **II-49** | 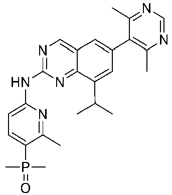 |
| **II-18** | 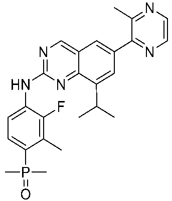 | **II-50** | 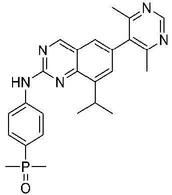 |
| **II-19** | 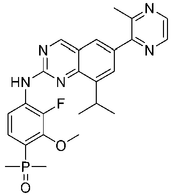 | **II-51** | 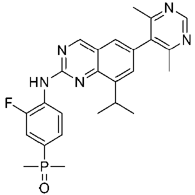 |
| **II-20** | 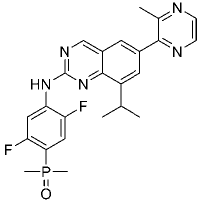 | **II-52** | 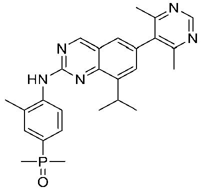 |
| **II-21** | 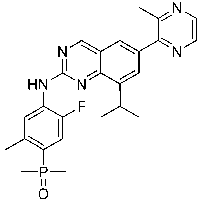 | **II-53** | 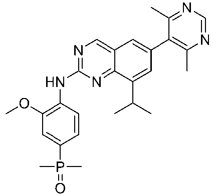 |
| **II-22** | 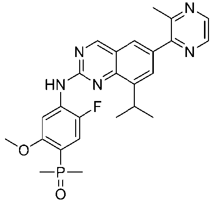 | **II-54** | 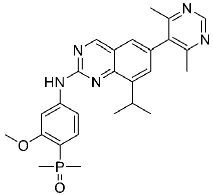 |
| **II-23** | 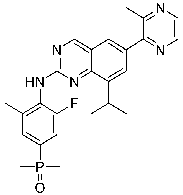 | **II-55** | 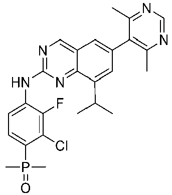 |
| **II-24** | 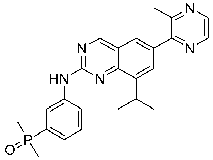 | **II-56** | 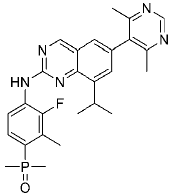 |
| **II-25** | 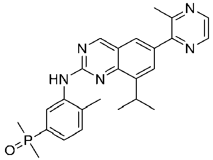 | **II-57** | 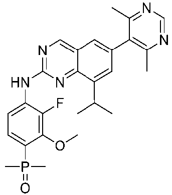 |
| **II-26** | 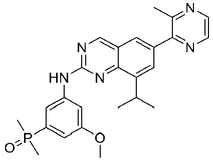 | **II-58** | 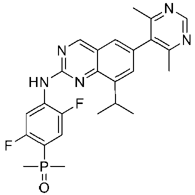 |
| **II-27** | 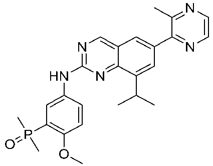 | **II-59** | 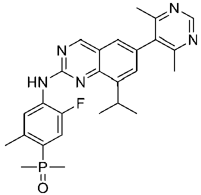 |
| **II-28** | 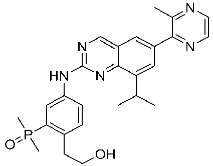 | **II-60** | 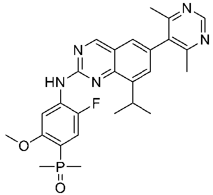 |
| **II-29** | 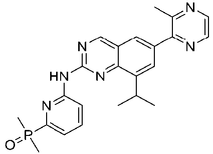 | **II-61** | 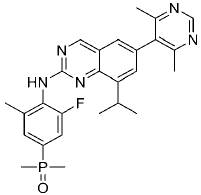 |
| **II-30** | 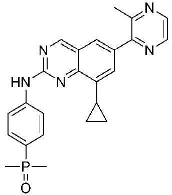 | **II-62** | 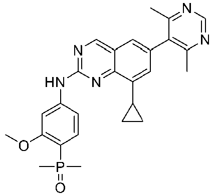 |
| **II-31** | 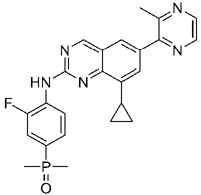 | **II-63** | 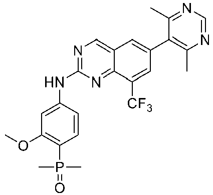 |
| **II-32** | 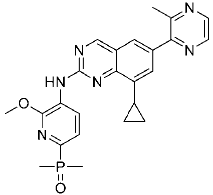 | | |
| **III-1** | 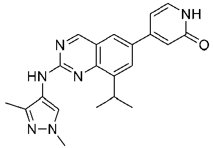 | **III-67** | 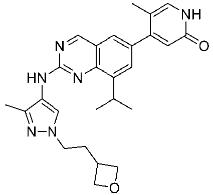 |
| **III-2** | 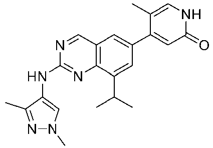 | **III-68A&III-68B** | 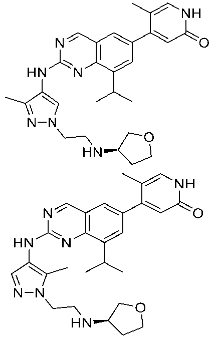 |
| **III-3** | 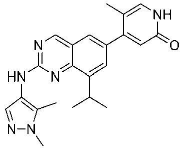 | **III-69** | 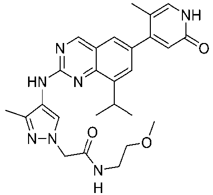 |
| **III-4** | 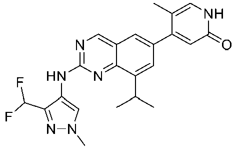 | **III-70** | 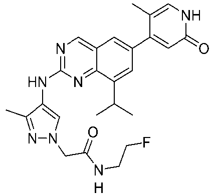 |
| **III-5** | 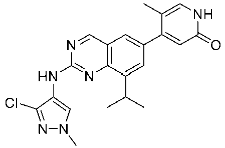 | **III-71** | 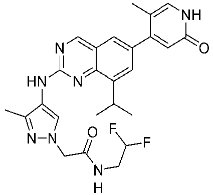 |
| **III-6** | 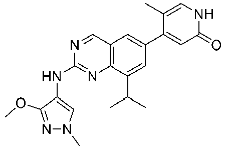 | **III-72** | 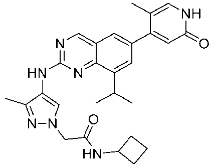 |
| **III-7** | 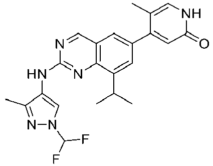 | **III-73** | 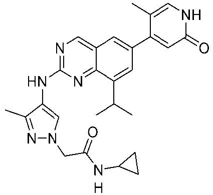 |
| **III-8** | 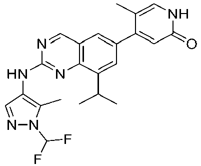 | **III-74** | 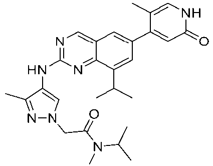 |
| **III-9** | 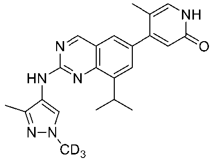 | **III-75** | 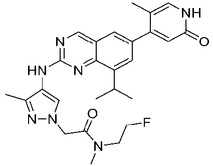 |
| **III-10** | 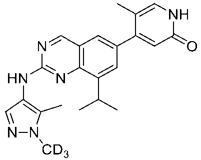 | **III-76** | 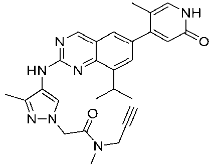 |
| **III-11** | 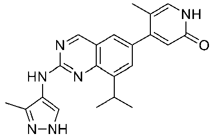 | **III-77** | 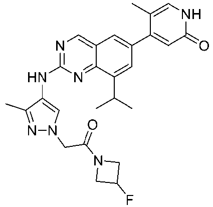 |
| **III-12** | 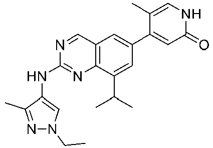 | **III-78** | 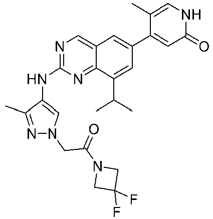 |
| **III-13** | 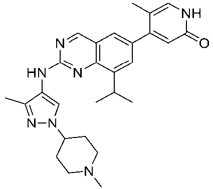 | **III-79** | 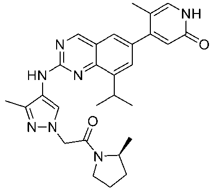 |
| **III-14** | 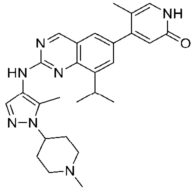 | **III-80** | 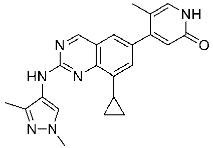 |
| **III-15A&III-15B** | 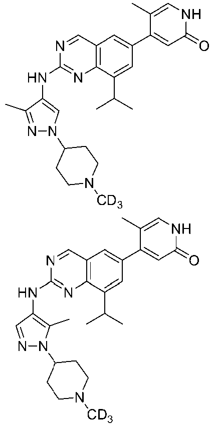 | **III-81** | 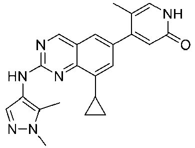 |
| **III-16** | 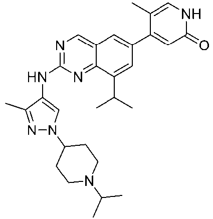 | **III-82** | 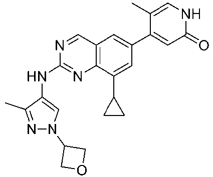 |
| **III-17** | 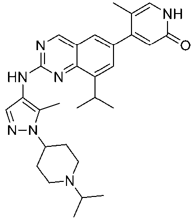 | **III-83** | 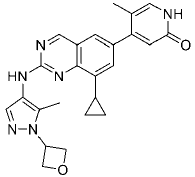 |
| **III-18** | 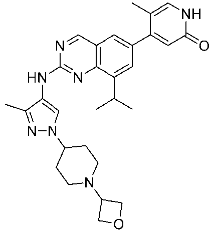 | **III-84** | 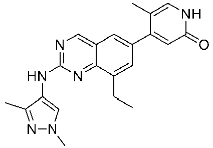 |
| **III-19** | 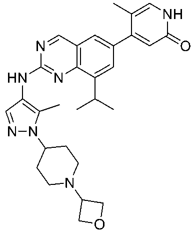 | **III-85** | 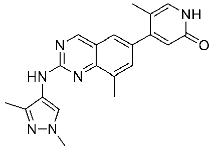 |
| **III-20A&III-20B** | 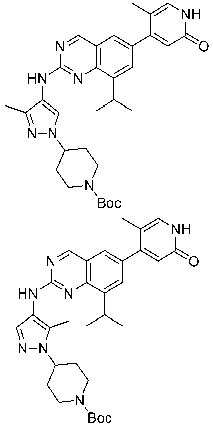 | **III-86** | 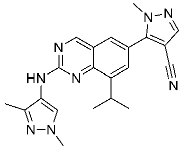 |
| **III-21** | 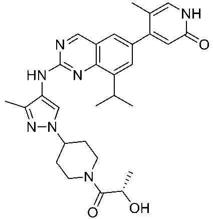 | **III-87** | 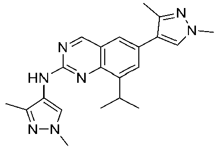 |
| **III-22** | 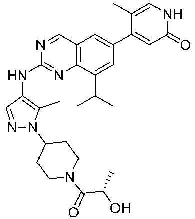 | **III-88** | 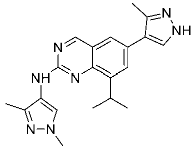 |
| **III-23** | 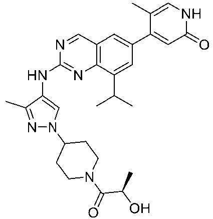 | **III-89** | 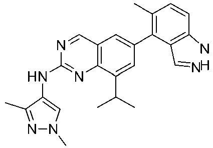 |
| **III-24** | 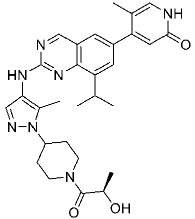 | **III-90** | 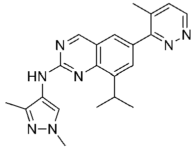 |
| **III-25** | 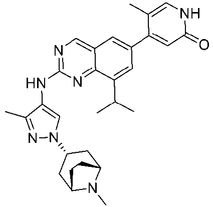 | **III-91** | 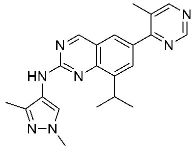 |
| **III-26** | 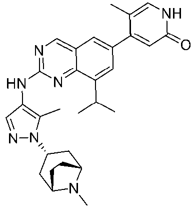 | **III-92** | 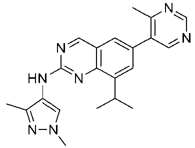 |
| **III-27** | 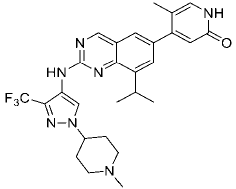 | **III-93** | 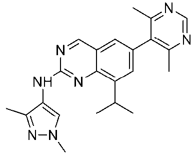 |
| **III-28** | 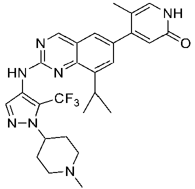 | **III-94** | 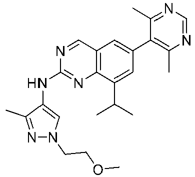 |
| **III-29** | 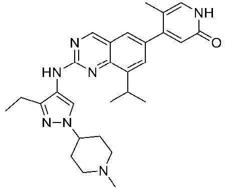 | **III-95** | 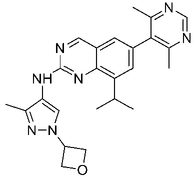 |
| **III-30** | 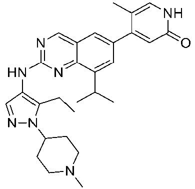 | **III-96** | 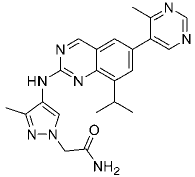 |
| **III-31** | 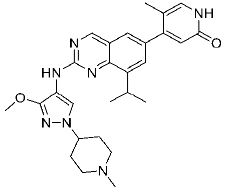 | **III-97** | 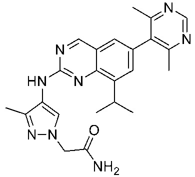 |
| **III-32** | 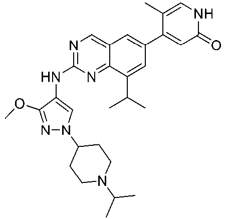 | **III-98** | 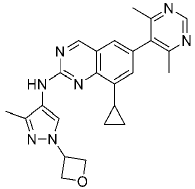 |
| **III-33A&III-33B** | 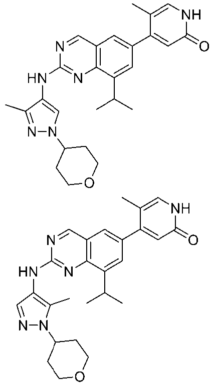 | **III-99** | 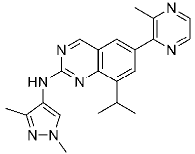 |
| **III-34** | 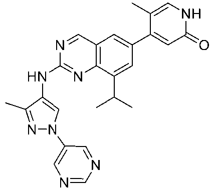 | **III-100** | 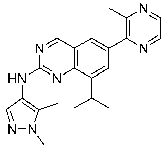 |
| **III-35** | 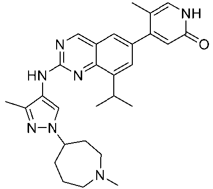 | **III-101** | 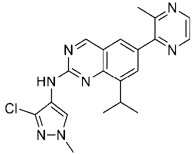 |
| **III-36** | 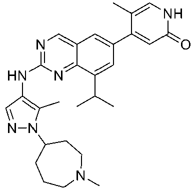 | **III-102** | 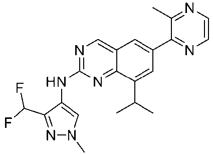 |
| **III-37A&III-37B** | 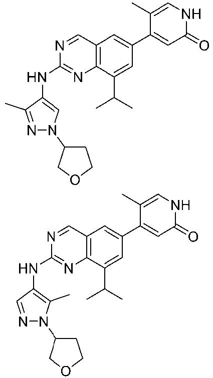 | **III-103** | 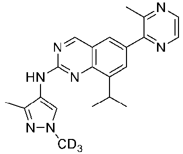 |
| **III-38** | 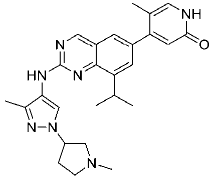 | **III-104** | 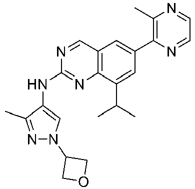 |
| **III-39** | 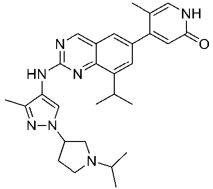 | **III-105** | 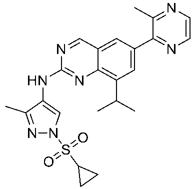 |
| **III-40** | 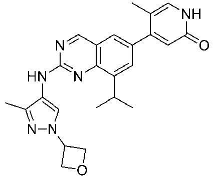 | **III-106** | 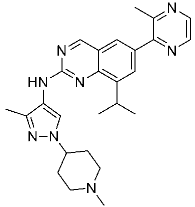 |
| **III-41** | 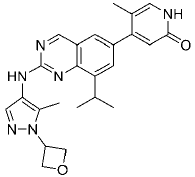 | **III-107** | 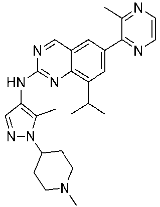 |
| **III-42** | 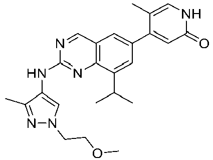 | **III-108** | 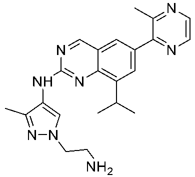 |
| **III-43** | 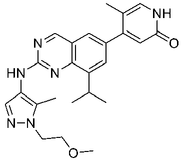 | **III-109** | 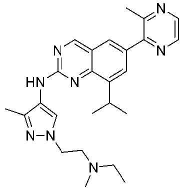 |
| **III-44** | 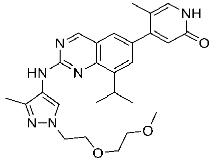 | **III-110** | 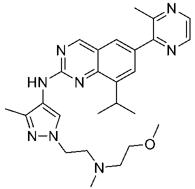 |
| **III-45** | 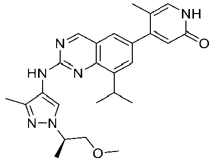 | **III-111** | 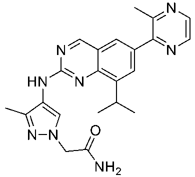 |
| **III-46** | 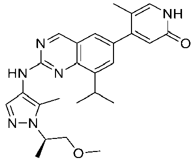 | **III-112** | 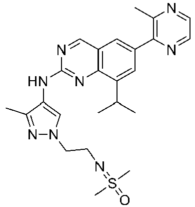 |
| **III-47A&III-47B** | 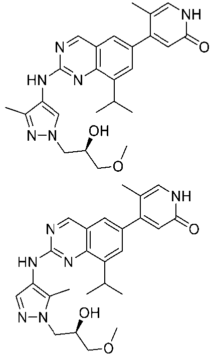 | **III-113** | 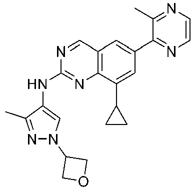 |
| **III-48** | 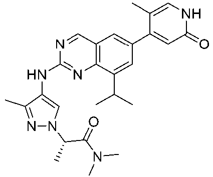 | **III-114** | 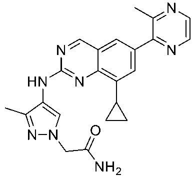 |
| **III-49** | 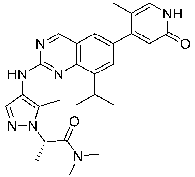 | **III-115** | 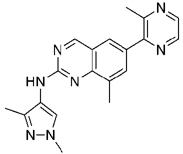 |
| **III-50A&III-50B** | 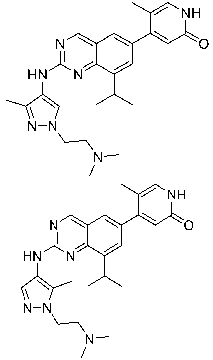 | **III-116** | 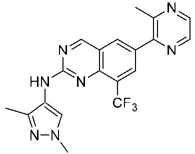 |
| **III-51** | 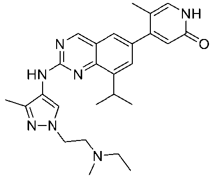 | **III-117** | 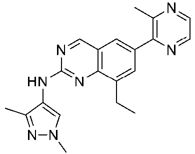 |
| **III-52** | 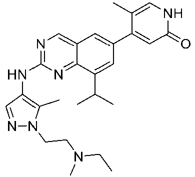 | **III-118** | 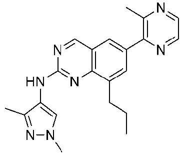 |
| **III-53A&III-53B** | 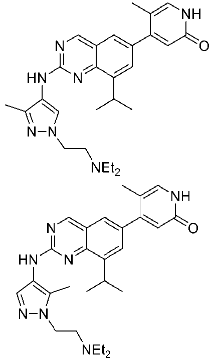 | **III-119** | 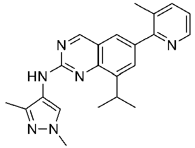 |
| **III-54** | 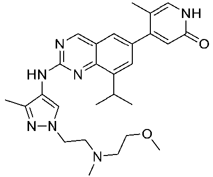 | **III-120** | 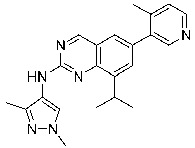 |
| **III-55** | 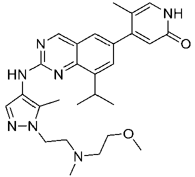 | **III-121** | 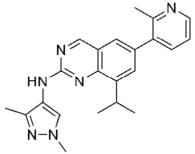 |
| **III-56** | 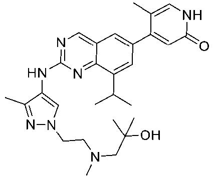 | **III-122** | 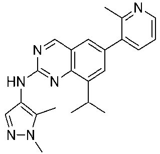 |
| **III-57** | 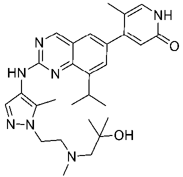 | **III-123** | 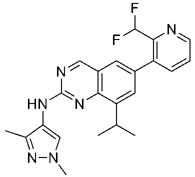 |
| **III-58A&III-58B** | 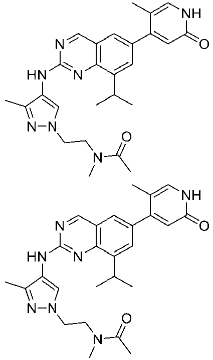 | **III-124** | 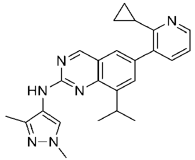 |
| **III-59** | 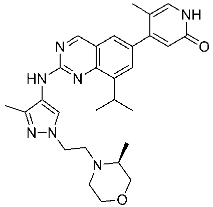 | **III-125** | 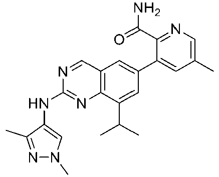 |
| **III-60** | 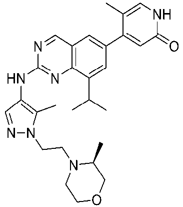 | **III-126** | 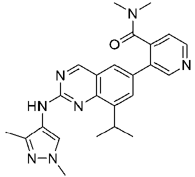 |
| **III-61** | 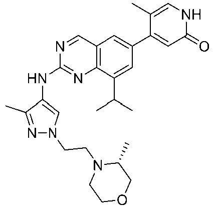 | **III-127** | 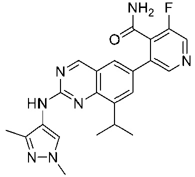 |
| **III-62** | 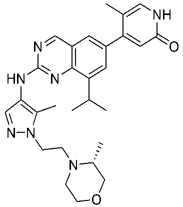 | **III-128** | 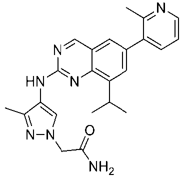 |
| **III-63A&III-63B** | 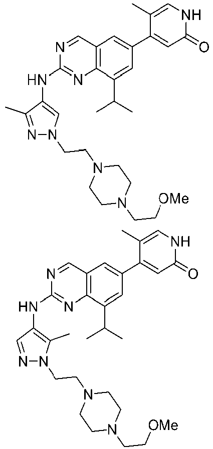 | **III-129** | 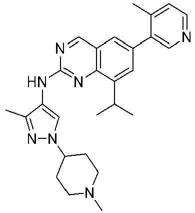 |
| **III-64** | 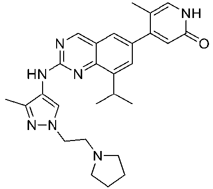 | **III-130** | 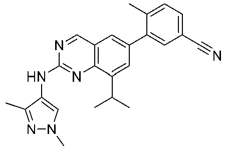 |
| **III-65** | 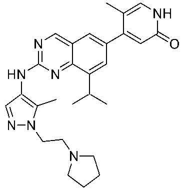 | **III-131** | 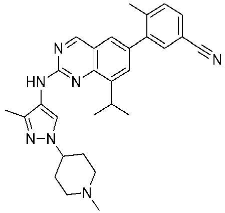 |
| **III-66A&III-66B** | 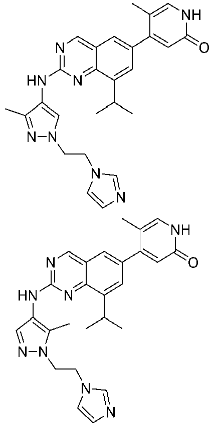 | | |
| **II-64** | 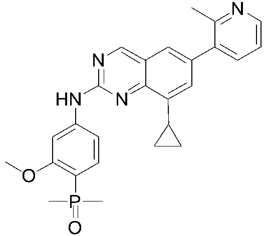 | **II-71** | 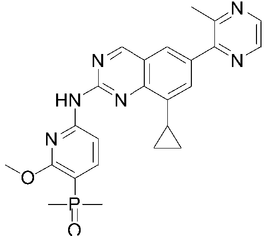 |
| **II-65** | 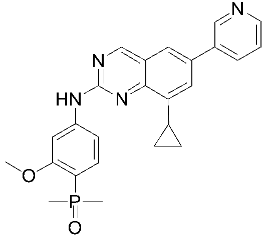 | **II-72** | 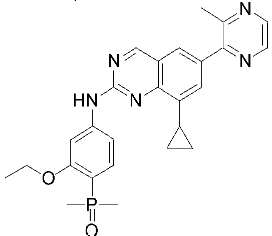 |
| **II-66** | 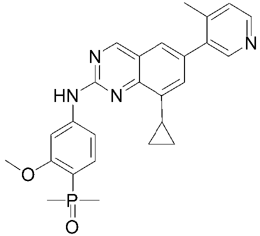 | **II-73** | 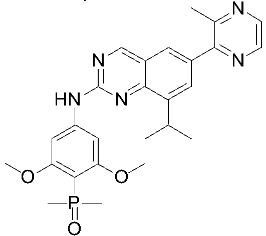 |
| **II-67** | 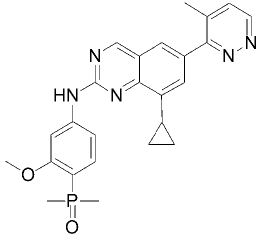 | **II-74** | 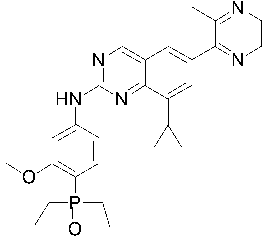 |
| **II-68** | 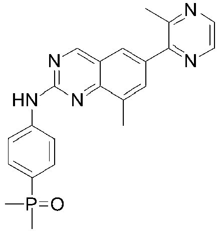 | **II-75** | 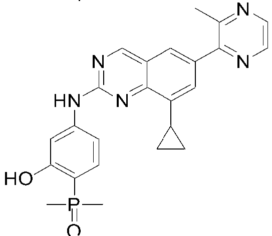 |
| **II-69** | 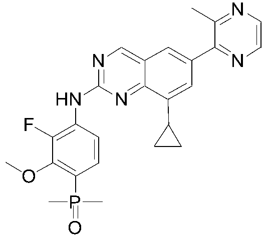 | **II-76** | 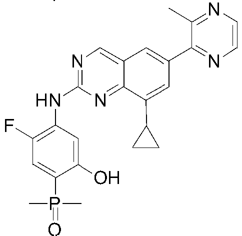 |
| **II-70** | 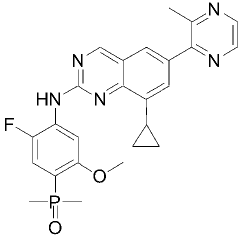 | **II-77** | 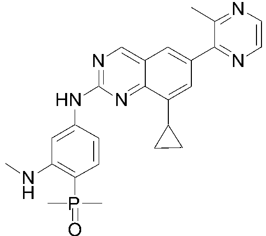 |
| **III-132** | 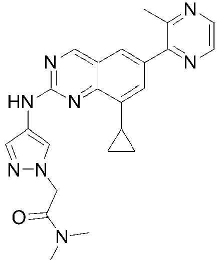 | **III-133** | 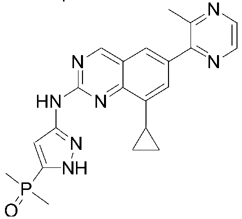 |

8. A method for preparing the compound according to any one of claims 1-7, comprising: 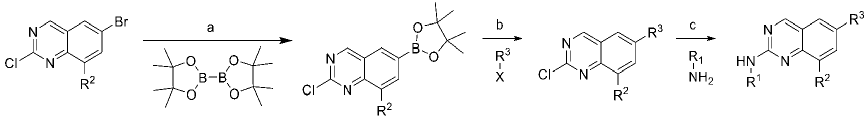 or 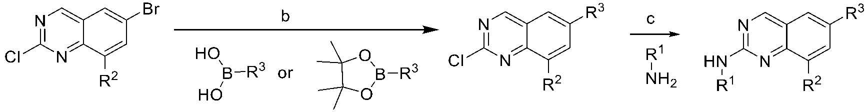 or 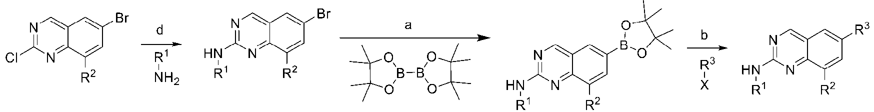 or 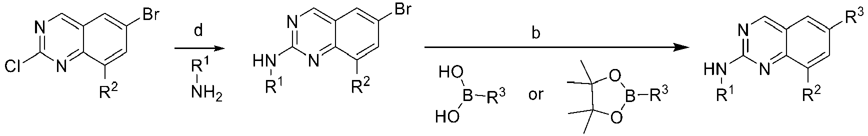
wherein X is halogen, and R¹, R², and R³ are each independently as defined in any one of claims 1-7,
reaction conditions are as follows:
(a) a palladium-catalyzed coupling reaction;
(b) a palladium-catalyzed coupling reaction;
(c) a palladium-catalyzed coupling reaction, a nucleophilic substitution reaction under acidic conditions, or a nucleophilic substitution reaction under basic conditions;
(d) a nucleophilic substitution reaction under acidic conditions or a nucleophilic substitution reaction under basic conditions;
the palladium catalyst is selected from the group consisting of palladium acetate, tetrakis(triphenylphosphine)palladium, bis(triphenylphosphine)palladium(II) dichloride, [1,1'-bis(diphenylphosphino)ferrocene]palladium(II) dichloride, and tris(dibenzylideneacetone)dipalladium;
the basic conditions refer to conditions in the presence of any of the following: triethylamine, diisopropylethylamine, pyridine, sodium bicarbonate, sodium carbonate, potassium carbonate, cesium carbonate, lithium hydroxide, sodium hydroxide, potassium hydroxide, sodium hydride, or potassium hydride; and
the acidic conditions refer to conditions in the presence of any of the following: acetic acid, trifluoroacetic acid, hydrochloric acid, methanesulfonic acid, p-toluenesulfonic acid, or camphorsulfonic acid.

9. A pharmaceutical composition comprising the compound or a stereoisomer thereof, a prodrug thereof, a pharmaceutically acceptable salt thereof, or a pharmaceutically acceptable solvate thereof according to any one of claims 1-7, and optionally a pharmaceutically acceptable excipient.

10. Use of the compound or a stereoisomer thereof, a prodrug thereof, a pharmaceutically acceptable salt thereof, or a pharmaceutically acceptable solvate thereof according to any one of claims 1-7, or the pharmaceutical composition according to claim 9, in the manufacture of a medicament for preventing and/or treating a BRK kinase-mediated disease;
preferably, the BRK kinase-mediated disease is selected from the group consisting of breast cancer, gastric cancer, leukemia, skin cancer, head and neck cancer, thyroid cancer, pancreatic cancer, cervical cancer, bladder cancer, ovarian cancer, nasopharyngeal carcinoma, non-small cell lung cancer, prostate cancer, colorectal cancer, and esophageal cancer.
